# EUROPÄISCHE PATENTSCHRIFT

(11) **EP 1 366 043 B1**
(45) Veröffentlichungstag und Bekanntmachung des Hinweises auf die Patenterteilung: **20.06.2007**
(21) Anmeldenummer: 02702331.6
(22) Anmeldetag: 31.01.2002
(51) Int. Cl.: C07D 409/04, A01N 43/36, C07D 495/04, C07D 333/28, C07D 333/22, C07D 333/58, C07D 401/04, C07D 213/61, C07D 213/50

(54) **2-HETEROARYL-3,4-DIHYDRO-2H-PYRROL-DERIVATE UND IHRE VERWENDUNG ALS SCHÄDLINGSBEKÄMPFUNGSMITTEL**
2-HETEROARYL-3,4-DIHYDRO-2H-PYRROLE DERIVATIVES AND THE USE THEREOF AS PESTICIDES
DERIVES DE 2-HETEROARYL-3,4-DIHYDRO-2H-PYRROL ET LEUR UTILISATION EN TANT QU'AGENTS DE LUTTE CONTRE LES PARASITES

(30) Priorität: 13.02.2001 DE 10106457
(43) Veröffentlichungstag der Anmeldung: 03.12.2003
(73) Patentinhaber: Bayer CropScience AG, 40789 Monheim (DE)
(72) Erfinder: PLANT, Andrew, Wokingham, Berkshire RG41 5PD (GB); FISCHER, Rüdiger, 50259 Pulheim (DE); SEITZ, Thomas, 40764 Langenfeld (DE); ERDELEN, Christoph, 42799 Leichlingen (DE); TURBERG, Andreas, 42781 Haan (DE); HANSEN, Olaf, 42799 Leichlingen (DE)
(86) Internationale Anmeldenummer: PCT/EP2002/000992
(87) Internationale Veröffentlichungsnummer: WO 2002/064588

(56) Entgegenhaltungen:
- WO-A-00/21958
- WO-A-98/22438
- WILLE, GREGOR ET AL: "A short synthesis of the bacterial pigments violacein and deoxyviolacein" SYNTHESIS (2001), (5), 759-762 , XP002201463

## Beschreibung

Die vorliegende Erfindung betrifft neue Δ¹-Pyrroline, mehrere Verfahren zu ihrer Herstellung und ihre Verwendung als Schädlingsbekämpfungsmittel.

Es ist bereits bekannt, dass zahlreiche Δ¹-Pyrroline insektizide Eigenschaften besitzen (vgl. WO 00/21958, WO 99/59968, WO 99/59967 und WO 98/22438). Die Wirksamkeit dieser Stoffe ist gut, lässt aber in manchen Fällen zu wünschen übrig.

Es wurden nun Δ¹-Pyrroline der Formel (I) 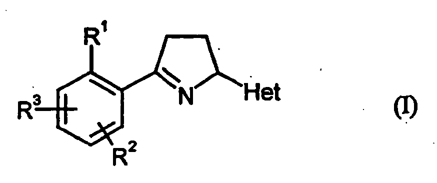 gefunden, in welcher
- R¹: für Halogen, jeweils gegebenenfalls substituiertes Alkyl, Alkoxy oder -S(O)_{w}R⁴ steht,
- R² und R³: unabhängig voneinander für Wasserstoff Halogen oder für jeweils gegebenenfalls substituiertes Alkyl, Alkoxy oder Alkoxyalkyl stehen,
- R⁴: für gegebenenfalls substituiertes Alkyl steht,
- Het: für gegebenenfalls einfach oder mehrfach, gleich oder verschieden durch R⁵ substituiertes Heteroaryl steht,
- R⁵: für die Gruppierung -X-Y-Z-E steht, mit der Maßgabe, dass Y nicht für eine direkte Bindung steht, wenn X nicht für eine direkte Bindung steht,
- X: für eine direkte Bindung, Sauerstoff, -S(O)_{w}-, -NR⁶-, Carbonyl, Carbonyloxy, Oxycarbonyl, Oxysulfonyl (OSO₂), Alkylen, Halogenalkylen, Alkenylen, Halogenalkenylen, Alkinylen, Alkylenoxy, Oxyalkylen, Oxyalkylenoxy, -S(O)_{w}-alkylen, Cyclopropylen oder Oxiranylen steht,
- Y: für eine direkte Bindung oder für jeweils gegebenenfalls substituiertes Phenylen, Naphthylen, Tetrahydronaphthylen oder Heterocyclylen steht,
- Z: für eine direkte Bindung oder -(CH₂)ₙ- steht,
- E: für Wasserstoff, Halogen, Hydroxy, Cyano, Formyl, Nitro, Trialkylsilyl, Pentafluorthio, -S(O)_{w}R⁷, -OSO₂R⁷, -NR⁸R⁹, -COR⁷, -CO₂R⁷, -OC(O)R⁷, -CONR¹⁰R¹¹, -N(R¹²)COR¹³, -C(R¹⁴)=N-OR¹⁵, -SO₂NR¹⁶R¹⁷; für jeweils gegebenenfalls substituiertes Alkyl, Alkenyl, Alkinyl, Alkoxy, Alkenyloxy, Cycloalkyl, Cycloalkylalkyl, Cycloalkyloxy, Aryl, Arylalkyl, Aryloxy, Aryloxyalkyl, gesättigtes oder ungesättigtes Heterocyclyl oder Heterocyclylalkyl steht,
- R⁶: für jeweils gegebenenfalls substituiertes Alkyl, Cycloalkyl, Cycloalkylalkyl, Aryl oder Arylalkyl steht,
- R⁷: für jeweils gegebenenfalls substituiertes Alkyl, Cycloalkyl, Aryl oder Arylalkyl steht,
- R⁸ und R⁹: unabhängig voneinander für Wasserstoff, -SO₂R⁷, -COR⁷, -CO₂R⁷, für jeweils gegebenenfalls substituiertes Alkyl, Alkenyl, Cycloalkyl, Cycloalkylalkyl, Aryl, Arylalkyl oder gesättigtes oder ungesättigtes Heterocyclyl oder Heterocyclylalkyl stehen,
- R⁸ und R⁹: außerdem gemeinsam für jeweils gegebenenfalls substituiertes Alkenylen oder Alkylen stehen, wobei, die Alkylenkette jeweils durch -O-, -S- oder -NR¹⁸- unterbrochen sein kann,
- R¹⁰ und R¹¹: unabhängig voneinander für Wasserstoff -SO₂R⁷, für jeweils gegebenenfalls substituiertes Alkyl, Alkenyl, Cycloalkyl, Cycloalkylalkyl, Aryl, Arylalkyl oder gesättigtes oder ungesättigtes Heterocyclyl oder Heterocyclylalkyl stehen,
- R¹⁰ und R¹¹: außerdem gemeinsam für gegebenenfalls substituiertes Alkylen stehen,
wobei die Alkylenkette jeweils durch -O-, -S- oder -NR¹⁸- unterbrochen sein kann,
- R¹² und R¹³: unabhängig voneinander für Wasserstoff, für jeweils gegebenenfalls substituiertes Alkyl, Cycloalkyl, Cycloalkylalkyl, Aryl oder Arylalkyl stehen,
- R¹² und R¹³: außerdem gemeinsam für jeweils gegebenenfalls substituiertes Alkylen oder Alkenylen stehen,
- R¹⁴ und R¹⁵: unabhängig voneinander für Wasserstoff, für jeweils gegebenenfalls substituiertes Alkyl oder Alkenyl stehen,
- R¹⁶ und R¹⁷: unabhängig voneinander für Wasserstoff, für gegebenenfalls substituiertes Alkyl oder Cycloalkyl stehen,
- R¹⁶ und R¹⁷: außerdem gemeinsam für gegebenenfalls substituiertes Alkylen, Alkoxyalkylen oder Alkylthioalkylen stehen,
- R¹⁸: für Wasserstoff, -SO₂R⁷, -COR⁷ oder -CO₂R⁷; für jeweils gegebenenfalls substituiertes Alkyl, Alkenyl, Cycloalkyl, Cycloalkylalkyl, Aryl, Arylalkyl oder gesättigtes oder ungesättigtes Heterocyclyl oder Heterocyclylalkyl steht,
- w: für 0, 1 oder 2 steht,
- n: für 1, 2, 3 oder 4 steht.

Die Verbindungen der Formel (I) können gegebenenfalls in Abhängigkeit von der Art und Anzahl der Substituenten als geometrische und/oder optische Isomere, Regioisomere bzw. Konfigurationsisomere oder deren Isomerengemische in unterschiedlicher Zusammensetzung vorliegen. Sowohl die reinen Isomere als auch die Isomerengemische werden erfindungsgemäß beansprucht.

Weiterhin wurde gefunden, dass man die neuen Verbindungen der Formel (I) nach einem der im Folgenden beschriebenen Verfahren erhalten kann.

Δ¹-Pyrroline der Formel (I) 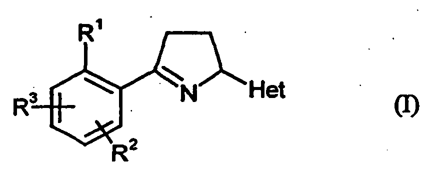 in welcher
R¹, R², R³ und Het die oben angegebenen Bedeutungen haben,
lassen sich herstellen, indem man
A) Aminoketone der Formel (II) 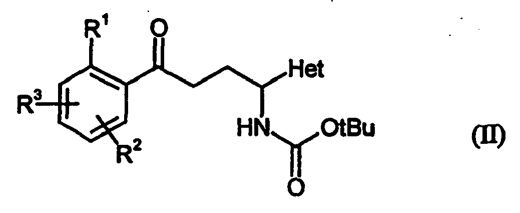 in welcher
   R¹, R², R³ und Het die oben angegebenen Bedeutungen haben,
   mit einer Lewissäure oder einer Protonsäure gegebenenfalls in Gegenwart eines Verdünnungsmittels behandelt.
   oder
B) Azide der Formel (III) 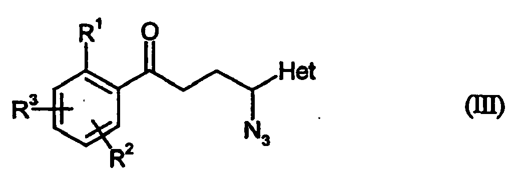 in welcher
   R¹, R², R³ und Het die oben angegebenen Bedeutungen haben,
   mit einem Trialkylphosphin oder einem Triarylphosphin oder einem Trialkylphosphit oder einem Reduktionsmittel in Gegenwart eines Verdünnungsmittels und gegebenenfalls in Gegenwart eines Katalysators umsetzt,
   oder
C) Amide der Formel (IV) 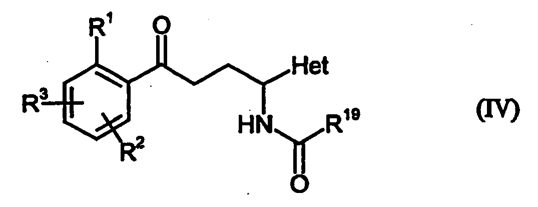 in welcher
   - R¹⁹: für Alkyl, Halogenalkyl, Aryl oder Arylalkyl steht und
   - R¹, R², R³ und Het: die oben angegebenen Bedeutungen haben,
   mit einem N-Entacylierungsmittel gegebenenfalls in Gegenwart eines Verdünnungsmittelsumsetzt.

Δ¹-Pyrroline der Formel (I-a) 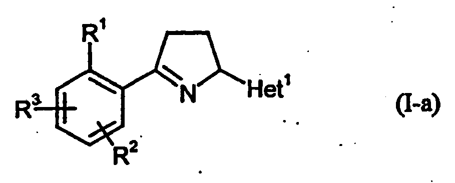 in welcher
- Het¹: für einfach durch R⁵⁻¹ substituiertes Heteroaryl steht,
- R⁵⁻¹: für die Gruppierung -Y¹-E steht,
- Y¹: für jeweils gegebenenfalls substituiertes Phenylen oder Heterocyclylen steht,
- R¹, R², R³ und E: die oben angegebenen Bedeutungen haben,
lassën sich herstellen, indem man
D) Δ¹-Pyrroline der Formel (I-b) 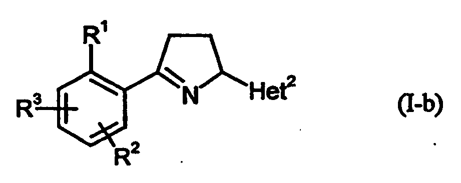 in welcher
   - Het²: für einfach durch R⁵⁻² substituiertes Heteroaryl steht,
   - R⁵⁻²: für Chlor, Brom, Iod, -OSO₂CF₃ oder -OSO₂(CF₂)₃CF₃ steht und
   - R¹, R² und R³: die oben angegebenen Bedeutungen haben,
   mit (Hetero)cyclen der Formel (V) 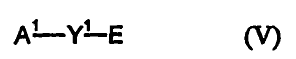 in welcher
   - Y¹ und E: die oben angegebenen Bedeutungen haben und
   - A¹: für Chlor, Brom, Iod, -OSO₂CF₃ oder -OSO₂(CF₂)₃CF₃ steht,
   in Gegenwart eines Katalysators, in Gegenwart eines Diboronsäureesters und gegebenenfalls in Gegenwart eines Säurebindemittels und gegebenenfalls in Gegenwart eines Verdünnungsmittels in einer Tandem-Reaktion umsetzt,
   oder
E) Δ¹-Pyrroline der Formel (VI) 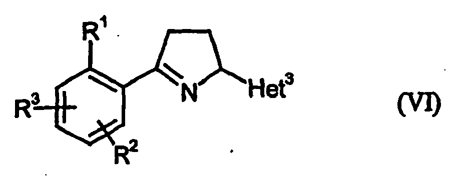 in welcher
   - Het³: für einfach durch A²-substituiertes Heteroaryl steht,
   - A²: für -B(OH)₂, (4,4,5,5-Tetramethyl-1,3,2-dioxaborolan)-2-yl, (5,5-Dimethyl-1,3,2-dioxaborinan)-2-yl, (4,4,6-Trimethyl-1,3,2-dioxaborinan)-2-yl oder 1,3,2-Benwdioxaborol-2-yl steht und
   - R¹, R² und R³: die oben angegebenen Bedeutungen haben,
   mit Heterocyclen der Formel (V) 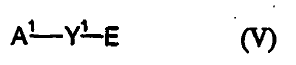 in welcher
   Y¹, E und A¹ die oben angegebenen Bedeutungen haben,
   in Gegenwart eines Katalysators, gegebenenfalls in Gegenwart eines Säurebindemittels und gegebenenfalls in Gegenwart eines Verdünnungsmittels umsetzt,
   oder
F) Δ¹-Pyrroline der Formel (I-b) 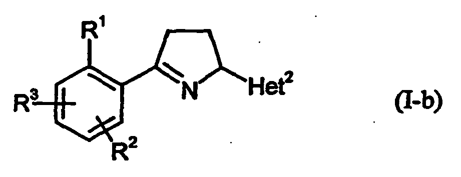 in welcher
   R¹, R², R³ und Het² die oben angegebenen Bedeutungen haben,
   mit Boronsäure-Derivaten der Formel (VII) 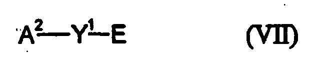 in welcher
   Y¹, E und A² die oben angegebenen Bedeutungen haben,
   in Gegenwart eines Katalysators, gegebenenfalls in Gegenwart eines Säurebindemittels und gegebenenfalls in Gegenwart eines Verdünnungsmittels umsetzt,
   oder
G) Δ¹-Pyrroline der Formel (I-c) 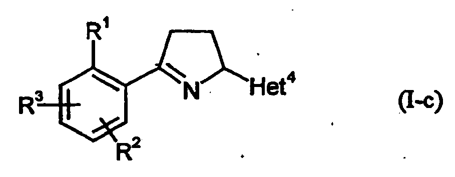 in welcher
   - Het⁴: für einfach durch R⁵⁻³ substituiertes Heteroaryl steht,
   - R⁵⁻³: für Brom oder Iod steht und
   - R¹, R² und R³: die oben angegebenen Bedeutungen haben,
   mit metallorganischen Verbindungen der Formel (VIII) 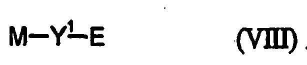 in welcher
   - Y¹ und E: die oben angegebenen Bedeutungen haben und
   - M: für ZnCl, Sn(Me)₃ oder Sn(n-Bu)₃ steht,
   in Gegenwart eines Katalysators, gegebenenfalls in Gegenwart eines Säurebindemittels und gegebenenfalls in Gegenwart eines Verdünnungsmittels umsetzt.

Schließlich wurde gefunden, dass die erfindungsgemäßen Verbindungen der Formel (I) sehr gute insektizide Eigenschaften besitzen und sich sowohl im Pflanzenschutz als auch im Materialschutz zur Bekämpfung unerwünschter Schädlinge, wie Insekten, verwenden lassen.

Die erfindungsgemäßen Δ¹-Pyrroline sind durch die Formel (I) allgemein definiert.
- R¹: steht bevorzugt für Halogen, Alkyl, Halogenalkyl, Alkoxy, Halogenalkoxy oder -S(O)_{w}R⁴.
- R² und R³: stehen unabhängig voneinander bevorzugt für Wasserstoff, Halogen, Alkyl, Halogenalkyl, Alkoxy, Halogenalkoxy oder Alkoxyalkyl.
- R⁴: steht bevorzugt für Alkyl oder Halogenalkyl.
- Het: steht bevorzugt für gegebenenfalls einfach bis vierfach, gleich oder verschieden durch R⁵ substituiertes 5- bis 14-gliedriges, 1 bis 3 aromatische Ringe enthaltendes Heteroaryl mit einem oder mehreren Heteroatomen aus der Reihe Stickstoff, Sauerstoff und Schwefel.
- R⁵: steht bevorzugt für die Gruppierung -X-Y-Z-E mit der Maßgabe, dass Y nicht für eine direkte Bindung steht, wenn X nicht für eine direkte Bindung steht
- X: steht bevorzugt für eine direkte Bindung, Sauerstoff, -S(O)_{w}-, -NR⁶-, Carbonyl, Carbonyloxy, Oxycarbonyl, Oxysulfonyl (OSO₂), Alkylen, Halogenalkylen, Alkenylen, Halogenalkenylen, Alkinylen, Alkylenoxy, Oxyalkylen, Oxyalkylenoxy, -S(O)_{w}-alkylen, Cyclopropylen oder Oxiranylen.
- Y: steht bevorzugt für eine direkte Bindung oder für jeweils gegebenenfalls einfach bis vierfach, gleich oder verschieden durch Reste aus der Liste W¹ substituiertes Phenylen, Naphthylen, Tetrahydronaphthylen oder 5- bis 10-gliedriges, gesättigtes oder ungesättigtes Heterocyclylen mit einem oder mehreren Heteroatomen aus der Reihe Stickstoff, Sauerstoff und Schwefel.
- Z: steht bevorzugt für eine direkte Bindung oder -(CH₂)ₙ-.
- E: steht bevorzugt für Wasserstoff, Halogen, Hydroxy, Cyano, Formyl, Nitro, Trialkylsilyl, Pentafluorthio, -S(O)_{w}R⁷, -OSO₂R⁷, -NR⁸R⁹, -COR⁷, -CO₂R⁷, -OC(O)R⁷, -CONR¹⁰R¹¹, -N(R¹²)COR¹³, -C(R¹⁴)=N-OR¹⁵, -SO₂NR¹⁶R¹⁷; für jeweils gegebenenfalls einfach- oder mehrfach, gleich oder verschieden durch Halogen, Cyano, Alkoxy und/oder -NR⁸R⁹ substituiertes Alkyl, Alkenyl, Alkinyl, Alkoxy, Alkenyloxy; oder für jeweils gegebenenfalls einfach oder mehrfach, gleich oder verschieden durch Halogen, Cyano, Nitro, Alkyl, Halogenalkyl, Alkenyl, Halogenalkenyl, Alkoxy, Halogenalkoxy, Alkylthio und/oder Halogenalkylthio substituiertes Cycloalkyl, Cycloalkylalkyl, Cycloalkyloxy, Aryl, Arylalkyl, Aryloxy, Aryloxyalkyl, gesättigtes oder ungesättigtes 5- bis 10-gliedriges Heterocyclyl oder Heterocyclylalkyl mit einem oder mehreren Heteroatomen aus der Reihe Stickstoff, Sauerstoff und Schwefel.
- W¹: steht bevorzugt für Halogen, Cyano, Formyl, Nitro, Trialkylsilyl, Alkyl, Halogenalkyl, Alkoxy, Halogenalkoxy, Alkenyl, Halogenalkenyl, Alkenyloxy, Halogenalkenyloxy, Alkylcarbonyl, Alkoxycarbonyl, -S(O)_{w}R⁷, -C(R¹⁴)=N-OR¹⁵, -SO₂NR¹⁶R¹⁷, -(CH₂)ₚNR¹⁶R¹⁷, -(CH₂)ₚN(R¹⁶)COR¹⁷, -(CH₂)ₚN(R¹⁶)SO₂R¹⁷, -OSO₂R¹⁶, -OSO₂NR¹⁶R¹⁷.
- R⁶: steht bevorzugt für Alkyl, Halogenalkyl oder für jeweils gegebenenfalls einfach oder mehrfach, gleich oder verschieden durch Halogen, Alkyl, Halogenalkyl, Alkoxy, Halogenalkoxy, Alkylthio und/oder Halogenalkylthio substituiertes Cycloalkyl, Cycloalkylalkyl, Aryl oder Arylalkyl.
- R⁷: steht bevorzugt für gegebenenfalls einfach oder mehrfach, gleich oder verschieden durch Halogen und/oder -NR⁸R⁹ substituiertes Alkyl, für jeweils gegebenenfalls einfach oder mehrfach, gleich oder verschieden durch Halogen, Cyano, Alkyl, Halogenalkyl, Alkoxy, Halogenalkoxy, Alkylthio, Halogenalkylthio substituiertes Cycloalkyl, Aryl oder Arylalkyl.
- R⁸ und R⁹: stehen unabhängig voneinander bevorzugt für Wasserstoff -SO₂R⁷, -COR⁷, -CO₂R⁷, für jeweils gegebenenfalls einfach oder mehrfach, gleich oder verschieden durch Halogen, Alkylcarbonyl, Alkylcarbonyloxy, Alkylamino, Dialkylamino, Alkoxy, Halogenalkoxy, Alkylthio und/oder Halogenalkylthio substituiertes Alkyl oder Alkenyl; für jeweils gegebenenfalls einfach oder mehrfach, gleich oder verschieden durch Halogen, Cyano, Alkyl, Halogenalkyl, Alkoxy, Halogenalkoxy, Alkylthio und/oder Halogenalkylthio substituiertes Cycloalkyl, Cycloalkylalkyl, Aryl, Arylalkyl oder gesättigtes oder ungesättigtes, 5- bis 10-gliedriges Heterocyclyl oder Heterocyclylalkyl mit einem oder mehreren Heteroatomen aus der Reihe Stickstoff, Sauerstoff und Schwefel.
- R⁸ und R⁹: stehen außerdem gemeinsam bevorzugt für gegebenenfalls einfach oder mehrfach, gleich oder verschieden durch Halogen, Cyano, Alkoxy, Halogenalkoxy, Alkylthio und/oder Halogenalkylthio substituiertes Alkenylen oder für gegebenenfalls einfach oder mehrfach, gleich oder verschieden durch Halogen, Cyano, Alkyl, Halogenalkyl, Alkoxy, Halogenalkoxy, Alkylthio und/oder Halogenalkylthio substituiertes Alkylen, wobei die Alkylenkette jeweils durch -O-, -S- oder NR¹⁸- unterbrochen sein kann.
- R¹⁰ und R¹¹: stehen unabhängig voneinander bevorzugt für Wasserstoff, -SO₂R⁷, für jeweils gegebenenfalls einfach oder mehrfach, gleich oder verschieden durch Halogen, Alkylamino, Dialkylamino, Alkoxy und/oder Alkylthio substituiertes Alkyl oder Alkenyl; für jeweils gegebenenfalls einfach oder mehrfach, gleich oder verschieden durch Halogen, Cyano, Alkyl, Halogenalkyl, Alkoxy, Halogenalkoxy, Alkylthio und/oder Halogenalkylthio substituiertes Cycloalkyl, Cycloalkylalkyl, Aryl, Arylalkyl oder gesättigtes oder ungesättigtes, 5-bis 10-gliedriges Heterocyclyl oder Heterocyclylalkyl mit einem oder mehreren Heteroatomen aus der Reihe Stickstoff, Sauerstoff und Schwefel.
- R¹⁰ und R¹¹: stehen außerdem gemeinsam bevorzugt für gegebenenfalls einfach oder mehrfach, gleich oder verschieden durch Halogen, Cyano, Alkyl, Halogenalkyl, Alkoxy, Halogenalkoxy, Alkylthio und/oder Halogenalkylthio substituiertes Alkylen, wobei die Alkylenkette jeweils durch -O-, -S- oder -NR¹⁸-unterbrochen sein kann.
- R¹² und R¹³: stehen unabhängig voneinander bevorzugt für Wasserstoff, für gegebenenfalls einfach oder mehrfach, gleich oder verschieden durch Halogen, Cyano, Alkoxy und/oder Alkylthio substituiertes Alkyl, für jeweils gegebenenfalls einfach oder mehrfach, gleich oder verschieden durch Halogen, Cyano, Alkyl, Halogenalkyl, Alkoxy, Halogenalkoxy, Alkylthio und/oder Halogenalkylthio substituiertes Cycloalkyl, Cycloalkylalkyl, Aryl oder Arylalkyl.
- R¹² und R¹³: stehen außerdem gemeinsam bevorzugt für jeweils gegebenenfalls einfach oder mehrfach, gleich oder verschieden durch Halogen, Alkyl, Halogenalkyl, Alkoxy, Halogenalkoxy, Alkylthio und/oder Halogenalkylthio substituiertes Alkylen oder Alkenylen.
- R¹⁴ und R¹⁵: stehen unabhängig voneinander bevorzugt für Wasserstoff, für Alkyl, Halogenalkyl, Alkenyl oder Halogenalkenyl.
- R¹⁶ und R¹⁷: stehen unabhängig voneinander bevorzugt für Wasserstoff, Alkyl, Halogenalkyl oder für gegebenenfalls einfach oder mehrfach, gleich oder verschieden durch Halogen oder Alkyl substituiertes Cycloalkyl.
- R¹⁶ und R¹⁷: stehen außerdem gemeinsam bevorzugt für jeweils gegebenenfalls einfach oder mehrfach, gleich oder verschieden durch Halogen oder Alkyl substituiertes Alkylen, Alkoxyalkylen oder Alkylthioalkylen.
- R¹⁸: steht bevorzugt für Wasserstoff, -SO₂R⁷, -COR⁷ oder -CO₂R⁷; für jeweils gegebenenfalls einfach oder mehrfach, gleich oder verschieden durch Halogen, Cyano, Alkylamino, Dialkylamino, Alkoxy, Halogenalkoxy, Alkylthio und/oder Halogenalkylthio substituiertes Alkyl oder Alkenyl; für jeweils gegebenenfalls einfach oder mehrfach, gleich oder verschieden durch Halogen, Cyano, Alkyl, Halogenalkyl, Alkoxy, Halogenalkoxy, Alkylthio und/oder Halogenalkylthio substituiertes Cycloalkyl, Cycloalkylalkyl, Aryl, Arylalkyl, gesättigtes oder ungesättigtes, 5- bis 10-gliedriges Heterocyclyl oder Heterocyclylalkyl mit einem oder mehreren Heteroatomen aus der Reihe Stickstoff, Sauerstoff und Schwefel.
- w: steht bevorzugt für 0, 1 oder 2.
- n: steht bevorzugt für 1, 2, 3 oder 4.
- p: steht bevorzugt für 0, 1 oder 2 steht.
- R¹: steht besonders bevorzugt für Halogen, C₁-C₆-Alkyl, C₁-C₆-Halogenalkyl, C₁-C₆-Alkoxy, C₁-C₆-Halogenalkoxy oder -S(O)_{w}R⁴.
- R² und R³: stehen unabhängig voneinander besonders bevorzugt für Wasserstoff, Halogen, C₁-C₆-Alkyl, C₁-C₆-Halogenalkyl, C₁-C₆-Alkoxy, C₁-C₆-Halogenalkoxy oder C₁-C₆-Alkoxy-C₁-C₆-alkyl.
- R⁴: steht besonders bevorzugt für C₁-C₆-Alkyl oder C₁-C₆-Halogenalkyl.
- Het: steht besonders bevorzugt für gegebenenfalls einfach bis vierfach, gleich oder verschieden durch R⁵ substituiertes 5- bis 14-gliedriges, 1 bis 3 aromatische Ringe enthaltendes Heteroaryl mit 1 bis 4 Heteroatomen, welches 0 bis 4 Stickstoffatome, 0 bis 2 nicht benachbarte Sauerstoffatome und/oder 0 bis 2 nicht benachbarte Schwefelatome enthält (insbesondere Thienyl, Benzothienyl, Furyl, Benzofuryl, Indolyl, Thienothienyl, Thienofuryl Thienobenzothienyl, Thienobenzofuryl, Pyridinyl, Pyrimidinyl, Pyridazinyl, Pyrazinyl, Triazolyl oder Tetrazolyl).
- R⁵: steht besonders bevorzugt für die Gruppierung X-Y-Z-E mit der Maßgabe, dass Y nicht für eine direkte Bindung steht, wenn X nicht für eine direkte Bindung steht.
- X: steht besonders bevorzugt für eine direkte Bindung, Sauerstoff, -S(O)_{w}-, -NR⁶-, Carbonyl, Carbonyloxy, Oxycarbonyl, Oxysulfonyl (OSO₂), C₁-C₆-Alkylen, C₁-C₆-Halogenalkylen, C₂-C₆-Alkenylen, C₂-C₆-Halogenalkenylen, C₂-C₆-Alkinylen, C₁-C₆-Alkylenoxy, C₁-C₆-Oxyalkylen, Oxy-C₁-C₆-alkylenoxy, -S(O)_{w}-C₁-C₆-alkylen, Cyclopropylen oder Oxiranylen.
- Y: steht besonders bevorzugt für eine direkte Bindung oder für jeweils gegebenenfalls einfach bis vierfach, gleich oder verschieden durch Reste aus der Liste W¹ substituiertes 1,4-Phenylen, 1,3-Phenylen, 1,2-Phenylen, 2,6-Naphthylen, 2,7-Naphthylen, 1,4-Naphthylen, 2,6-(1,2,3,4-Tetrahydro)-naphthylen, 2,7-(1,2,3,4-Tetrahydro)naphthylen, 1,4-(1,2,3,4-Tetrahydro)-naphthylen, 5,8-(1,2,3,4-Tetrahydro)naphthylen; oder für jeweils gegebenenfalls einfach bis vierfach, gleich oder verschieden durch Reste aus der Liste W¹ substituiertes 5- oder 6-gliedriges, gesättigtes oder ungesättigtes Heterocyclylen mit 1 bis 3 Heteroatomen, welches 0 bis 3 Stickstoffatome, 0 bis 2 nicht benachbarte Sauerstoffatome und/oder 0 bis 2 nicht benachbarte Schwefelatome enthält (insbesondere Furylen, Thienylen, Pyrrolylen, Oxazolylen, Thiazolylen, Pyridinylen, Pyrimidinylen, Pyridazinylen oder Pyrazinylen).
- Z: steht besonders bevorzugt für eine direkte Bindung oder -(CH₂)ₙ-.
- E: steht besonders bevorzugt für Wasserstoff, Fluor, Chlor, Brom, Hydroxy, Cyano, Formyl, Nitro, Tri-(C₁-C₆-alkyl)silyl, Pentafluorthio, -S(O)_{w}R⁷, -OSO₂R⁷, -NR⁸R⁹, -COR⁷, -CO₂R⁷, -OC(O)R⁷, -CONR¹⁰R¹¹, -N(R¹²)COR¹³, -C(R¹⁴)=N-OR¹⁵, -SO₂NR¹⁶R¹⁷; für jeweils gegebenenfalls einfach oder mehrfach, gleich oder verschieden durch Halogen, Cyano, C₁-C₁₀-Alkoxy und/oder -NR⁸R⁹ substituiertes C₁-C₂₀-Alkyl, C₂-C₂₀-Alkenyl, C₂-C₁₀-Alkinyl, C₁-C₂₀-Alkoxy, C₂-C₂₀-Alkenyloxy; oder für jeweils gegebenenfalls einfach bis vierfach, gleich oder verschieden durch Halogen, Cyano, Nitro, C₁-C₆-Alkyl, C₁-C₆-Halogenalkyl, C₂-C₆-Alkenyl, C₂-C₆-Halogenalkenyl, C₁-C₆-Alkoxy, C₁-C₆-Halogenalkoxy, C₁-C₆ Alkylthio, C₁-C₆-Halogenalkylthio substituiertes C₃-C₁₂-Cycloalkyl, C₃-C₇-Cycloalkyl-C₁-C₄-alkyl, C₃-C₁₂-Cycloalkyloxy, Aryl, Aryl-C₁-C₄-alkyl, Aryloxy, Aryloxy-C₁-C₄-alkyl, gesättigtes oder ungesättigtes, 5- bis 10-gliedriges Heterocyclyl oder Heterocyclyl-C₁-C₄-alkyl mit 1 bis 4 Heteroatomen, welche 0 bis 4 Stickstoffatome, 0 bis 2 nicht benachbarte Sauerstoffatome und/oder 0 bis 2 nicht benachbarte Schwefelatome enthalten (insbesondere Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidino, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Piperidino, Morpholinyl, Thiomorpholinyl, Morpholino, Thiomorpholino, Triazinyl, Triazolyl, Chinolinyl oder Isochinolinyl).
- W¹: steht besonders bevorzugt für Fluor, Chlor, Brom, Cyano, Formyl, Nitro, Tri(C₁-C₆-alkyl)silyl, C₁-C₆-Alkyl, C₁-C₆-Halogenalkyl, C₁-C₆-Alkoxy, C₁-C₆-Halogenalkoxy, C₂-C₆-Alkenyl, C₂-C₆-Halogenalkenyl, C₂-C₆-Alkenyloxy, C₂-C₆-Halogenalkenyloxy, C₁-C₆-Alkylcarbonyl, C₁-C₆-Alkoxycarbonyl, -S(O)_{w}R⁷, -C(R¹⁴)=N-OR¹⁵, -SO₂NR¹⁶R¹⁷, (CH₂)ₚNR¹⁶R¹⁷, -(CH₂)ₚN(R¹⁶)COR¹⁷, -(CH₂)ₚN(R¹⁶)SO₂R¹⁷, -OSO₂R¹⁶, -OSO₂NR¹⁶R¹⁷.
- R⁶: steht besonders bevorzugt für C₁-C₆-Alkyl, C₁-C₆-Halogenalkyl oder für jeweils gegebenenfalls einfach bis vierfach durch Halogen, C₁-C₆-Alkyl, C₁-C₆-Halogenalkyl, C₁-C₆-Alkoxy, C₁-C₆-Halogenalkoxy, C₁-C₆-Alkylthio und/oder C₁-C₆-Halogenalkylthio substituiertes C₃-C₇-Cycloalkyl, C₃-C₆-Cycloalkyl-C₁-C₄-alkyl, Aryl oder Aryl-C₁-C₄-alkyl.
- R⁷: steht besonders bevorzugt für gegebenenfalls einfach oder mehrfach, gleich oder verschieden durch Halogen und/oder NR⁸R⁹ substituiertes C₁-C₂₀-Alkyl, für jeweils gegebenenfalls einfach bis achtfach, gleich oder verschieden durch Halogen, Cyano, C₁-C₆-Alkyl, C₁-C₆-Halogenalkyl, C₁-C₆-Alkoxy, C₁-C₆-Halogenalkoxy, C₁-C₆-Alkylthio und/oder C₁-C₆-Halogenalkylthio substituiertes C₃-C₆-Cycloalkyl, Aryl oder Aryl-C₁-C₄-alkyl.
- R⁸ und R⁹: stehen unabhängig voneinander besonders bevorzugt für Wasserstoff, -SO₂R⁷, -COR⁷, -CO₂R⁷, für jeweils gegebenenfalls einfach oder mehrfach, gleich oder verschieden durch Halogen, C₁-C₆-Alkylcarbonyl, C₁-C₆-Alkylcarbonyloxy, C₁-C₆-Alkylamino, Di-(C₁-C₆-alkyl)amino, C₁-C₆-Alkoxy, C₁-C₆-Halogenalkoxy, C₁-C₆-Alkylthio, C₁-C₆-Halogenalkylthio substituiertes C₁-C₂₀-Alkyl oder C₂-C₂₀-Alkenyl; für jeweils gegebenenfalls einfach bis vierfach, gleich oder verschieden durch Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio substituiertes C₃-C₁₂-Cycloalkyl, C₃-C₇-Cycloalkyl-C₁-C₄-alkyl, Aryl, Aryl-C₁-C₄-alkyl oder gesättigtes oder ungesättigtes, 5- bis 10-gliedriges Heterocyclyl oder Heterocyclyl-C₁-C₄-alkyl mit 1 bis 4 Heteroatomen, welche 0 bis 4 Stickstoffatome, 0 bis 2 nicht benachbarte Sauerstoffatome und/oder 0 bis 2 nicht benachbarte Schwefelatome enthalten (insbesondere Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl).
- R⁸ und R⁹: stehen außerdem gemeinsam besonders bevorzugt für gegebenenfalls einfach oder mehrfach, gleich oder verschieden durch Halogen, Cyano, C₁-C₆-Alkoxy, C₁-C₆-Halogenalkoxy, C₁-C₆-Alkylthio, C₁-C₆-Halogenalkylthio substituiertes C₂-C₁₂-Alkenylen oder für gegebenenfalls im Alkylenteil einfach oder mehrfach, gleich oder verschieden durch Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio substituiertes C₃-C₁₂-Alkylen, wobei die Alkylenkette jeweils durch -O-, -S- oder -NR¹⁸- unterbrochen sein kann.
- R¹⁰ und R¹¹: stehen unabhängig voneinander besonders bevorzugt für Wasserstoff, -SO₂R⁷, für jeweils gegebenenfalls einfach bis dreizehnfach, gleich oder verschieden durch Halogen, C₁-C₆-Alkylamino, Di-(C₁-C₆-alkyl)amino, C₁-C₆-Alkoxy, C₁-C₆-Halogenallcoxy, C₁-C₆-Alkylthio, C₁-C₆-Halogenalkylthio substituiertes C₁-C₆-Alkyl oder C₂-C₆-Alkenyl; für jeweils gegebenenfalls einfach bis vierfach, gleich oder verschieden durch Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio substituiertes C₃-C₇-Cycloalkyl, C₃-C₇-Cycloalkyl-C₁-C₄-alkyl, Aryl, Aryl-C₁-C₄-alkyl oder gesättigtes oder ungesättigtes, 5- bis 10-gliedriges Heterocyclyl oder Heterocyclyl-C₁-C₄-alkyl mit 1 bis 4 Heteroatomen, welche 0 bis 4 Stickstoffatome, 0 bis 2 nicht benachbarte Sauerstoffatome und/oder 0 bis 2 nicht benachbarte Schwefelatome enthalten (insbesondere Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl).
- R¹⁰ und R¹¹: stehen außerdem gemeinsam besonders bevorzugt für jeweils gegebenenfalls im Alkylenteil einfach bis vierfach, gleich oder verschieden durch Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio substituiertes C₃-C₆-Alkylen, -(CH₂)₂-O-(CH₂)₂-, -(CH₂)₂-S-(CH₂)₂- oder -(CH₂)₂-N(R¹⁸)-(CH₂)₂-.
- R¹² und R¹³: stehen unabhängig voneinander besonders bevorzugt für Wasserstoff, für gegebenenfalls einfach bis dreizehnfach, gleich oder verschieden durch Halogen, Cyano, C₁-C₆-Alkoxy, C₁-C₆-Alkylthio substituiertes C₁-C₆-Alkyl, für jeweils gegebenenfalls einfach bis achtfach, gleich oder verschieden durch Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio substituiertes C₃-C₇-Cycloalkyl, C₃-C₇-Cycloalkyl-C₁-C₄-alkyl, Aryl oder Aryl-C₁-C₄-alkyl.
- R¹² und R¹³: stehen außerdem gemeinsam besonders bevorzugt für jeweils gegebenenfalls einfach bis achtfach, gleich oder verschieden, durch Halogen, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio substituiertes C₃-C₁₀-Alkylen oder C₃-C₁₀-Alkenylen.
- R¹⁴ und R¹⁵: stehen unabhängig voneinander besonders bevorzugt für Wasserstoff, für C₁-C₆-Alkyl, C₁-C₆-Halogenalkyl, C₂-C₆-Alkenyl oder C₂-C₆-Halogenalkenyl.
- R¹⁶ und R¹⁷: stehen unabhängig voneinander besonders bevorzugt für Wasserstoff, C₁-C₆ Alkyl, C₁-C₆-Halogenalkyl oder für gegebenenfalls einfach bis achtfach, gleich oder verschieden durch Fluor, Chlor, Brom und/oder C₁-C₆-Alkyl substituiertes C₃-C₇-Cycloalkyl.
- R¹⁶ und R¹⁷: stehen außerdem gemeinsam besonders bevorzugt für jeweils gegebenenfalls einfach bis neunfach, gleich oder verschieden durch Fluor, Chlor, Brom und/oder C₁-C₆-Alkyl substituiertes C₃-C₆-Alkylen, C₁-C₃-Alkoxy-C₁-C₃-alkylen oder C₁-C₃-Alkylthio-C₁-C₃-alkylen.
- R¹⁸: steht besonders bevorzugt für Wasserstoff, -SO₂R⁷, -COR⁷ oder -CO₂R⁷; für jeweils gegebenenfalls einfach oder mehrfach, gleich oder verschieden durch Halogen, Cyano, C₁-C₆-Alkylamino, Di-(C₁-C₆-alkyl)amino, C₁-C₆-Alkoxy, C₁-C₆-Halogenalkoxy, C₁-C₆-Alkylthio, C₁-C₆-Halogenalkylthio substituiertes C₁-C₂₀-Alkyl oder C₂-C₂₀-Alkenyl; für jeweils gegebenenfalls einfach bis vierfach, gleich oder verschieden durch Halogen, Cyano, C₁-C₆-Alkyl, C₁-C₆-Halogenalkyl, C₁-C₆-Alkoxy, C₁-C₆-Halogenallcoxy, C₁-C₆-Alkylthio, C₁-C₆-Halogenalkylthio substituiertes C₃-C₁₂-Cycloalkyl, C₃-C₇-Cycloalkyl-C₁-C₄-alkyl, Aryl, Aryl-C₁-C₄-alkyl, gesättigtes oder ungesättigtes, 5- bis 10-gliedriges Heterocyclyl oder Heterocyclyl-C₁-C₄-alkyl mit 1 bis 4 Heteroatomen, welche 0 bis 4 Stickstoffatome, 0 bis 2 nicht benachbarte Sauerstoffatome und/oder: 0 bis 2 nicht benachbarte Schwefelatome enthalten (insbesondere Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl).
- w: steht besonders bevorzugt für 0,1 oder 2.
- n: steht besonders bevorzugt für 1, 2 oder 3.
- p: steht besonders bevorzugt für 0, 1 oder 2.
- R¹: steht ganz besonders bevorzugt für Fluor, Chlor, Brom, C₁-C₄-Alkyl, C₁-C₄-Halogenalkyl mit 1 bis 9 Fluor-, Chlor- und/oder Bromatomen, C₁-C₄-Alkoxy, C₁-C₄-Halogenalkoxy mit 1 bis 9 Fluor-, Chlor- und/oder Bromatomen oder -S(O)_{w}R⁴.
- R² und R³: stehen unabhängig voneinander ganz besonders bevorzugt für Wasserstoff, Fluor, Chlor, Brom, C₁-C₄-Alkyl, C₁-C₄-Halogenalkyl mit 1 bis 9 Fluor-, Chlor- und/oder Bromatomen, C₁-C₄-Alkoxy oder C₁-C₄-Halogenalkoxy mit 1 bis 9 Fluor-, Chlor- und/oder Bromatomen.
- R⁴: steht ganz besonders bevorzugt für C₁-C₄-Alkyl oder für jeweils durch Fluor oder Chlor substituiertes Methyl oder Ethyl.
- Het: steht ganz besonders bevorzugt für jeweils gegebenenfalls einfach bis dreifach, gleich oder verschieden durch R⁵ substituiertes 2-Thienyl, 3-Thienyl, 2-Benzo[b]thienyl, 2-Furyl, 3-Furyl, 2-Benzo[b]furyl, 2-Indolyl, 2-Thieno[3,2-b]thienyl, 2-Thieno[3,2-b]furyl, 5-Thieno[3,2 b]furyl, 2-Thieno[2,3-f][1]-benzothienyl, 2-Thieno[2,3-f][1]benzofuryl, 6-Thieno[2,3-f][1]benzofuryl, 2-Pyridinyl, 3-Pyridinyl, 2-Pyrimidinyl, 5-Pyrimidinyl, 3-Pyridazinyl, 4-Pyridazinyl, 2-Pyrazinyl, Triazolyl oder Tetrazolyl.
- R⁵: steht ganz besonders bevorzugt für die Gruppierung -X-Y-Z-E mit der Maßgabe, dass Y nicht für eine direkte Bindung steht, wenn X nicht für eine direkte Bindung steht.
- X: steht ganz besonders bevorzugt für eine direkte Bindung, Sauerstoff, -S(O)_{w}-, -NR⁶-, Carbonyl, Carbonyloxy, Oxycarbonyl, Oxysulfonyl (OSO₂), C₁-C₄-Alkylen, C₁-C₄-Halogenalkylen mit 1 bis 8 Fluor-, Chlor- und/oder Bromatomen, C₂-C₄-Alkenylen, C₂-C₄-Halogenalkenylen mit 1 bis 6 Fluor-, Chlor- und/oder Bromatomen, C₂-C₄-Alkinylen, C₁-C₄-Alkylenoxy, Oxy-C₁-C₄-alkylen, Oxy-C₁-C₄-alkylenoxy oder -S(O)_{w}-C₁-C₄-alkylen.
- Y: steht ganz besonders bevorzugt für eine direkte Bindung oder für jeweils gegebenenfalls einfach bis dreifach, gleich oder verschieden durch Reste aus der Liste W¹ substituiertes 1,4-Phenylen, 1,3-Phenylen, 1,2-Phenylen, 2,6-Naphthylen, 2,7-Naphthylen, 1,4-Naphthylen, 2,6-(1,2,3,4-Tetrahydro)-naphthylen, 2,7-(1,2,3,4-Tetrahydro)naphthylen, 1,4-(1,2,3,4-Tetrahydro)-naphthylen, 5,8-(1,2,3,4-Tetrahydro)naphthylen, 2,4-Furylen, 2,4-Thienylen, 2,4-Pyrrolylen, 2,5-Oxazolylen, 2,5-Thiazolylen, 2,5-Pyridinylen, 2,6-Pyridinylen, 2,5-Pyrimidinylen, 3,6-Pyridazinylen oder 2,5-Pyrazinylen.
- Z: steht ganz besonders bevorzugt für eine direkte Bindung oder -(CH₂)ₙ-.
- E: steht ganz besonders bevorzugt für Wasserstoff, Fluor, Chlor, Brom, Hydroxy, Cyano, Formyl, Nitro, Trimethylsilyl, Dimethyl-tert-butylsilyl, -S(O)_{w}R⁷, -OSO₂R⁷, -NR⁸R⁹, -COR⁷, -CO₂R⁷, -OC(O)R⁷, -CONR¹⁰R¹¹, -N(R¹²)COR¹³, -SO₂NR¹⁶R¹⁷; für jeweils gegebenenfalls einfach oder mehrfach, gleich oder verschieden durch Fluor, Chlor, Brom, Cyano, C₁-C₆-Alkoxy und/oder -NR⁸R⁹ substituiertes C₁-C₁₆-Alkyl, C₂-C₁₆-Alkenyl, C₂-C₆-Alkinyl, C₁-C₁₆-Alkoxy, C₂-C₁₆-Alkenyloxy; oder für jeweils gegebenenfalls einfach bis dreifach, gleich oder verschieden, durch Fluor, Chlor, Brom, Cyano, C₁-C₄-Alkyl, C₁-C₄-Halogenalkyl, C₂-C₆-Alkenyl, C₂-C₆-Halogenalkenyl, C₁-C₄-Alkoxy, C₁-C₄-Halogenalkoxy, C₁-C₄-Alkylthio, C₁-C₄-Halogenalkylthio substituiertes C₃-C₁₀-Cycloalkyl, C₃-C₆-Cycloalkyl-C₁-C₄-alkyl, C₃-C₁₀-Cycloalkyloxy, Phenyl, Phenoxy, Benzyl, Phenylethyl, Benzyloxy, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidino, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Piperidino, Morpholinyl, Thiomorpholinyl, Morpholino, Thiomorpholino, Triazinyl, Triazolyl, Chinolinyl oder Isochinolinyl.
- W¹: steht ganz besonders bevorzugt für Fluor, Chlor, Brom, Cyano, Formyl, Trimethylsilyl, Dimethyl-tert-butylsilyl, C₁-C₄-Alkyl, C₁-C₄-Alkoxy, C₂-C₄-Alkenyl, C₂-C₄-Alkenyloxy; für C₁-C₄-Halogenalkyl, C₁-C₄-Halogenalkoxy mit jeweils 1 bis 9 Fluor-, Chlor- und/oder Bromatomen, C₂-C₄-Halogenalkenyl, C₂-C₄-Halogenalkenyloxy mit jeweils 1 bis 8 Fluor, Chlor- und/oder Bromatomen; für C₁-C₄-Alkylcarbonyl, C₁-C₄-Alkoxycarbonyl, -S(O)_{w}R⁷, -SO₂NR¹⁶R¹⁷, -(CH₂)ₚNR¹⁶R¹⁷, -(CH₂)ₚN(R¹⁶)COR¹⁷, -(CH₂)ₚN(R¹⁶)SO₂R¹⁷, -OSO₂R¹⁶, -OSO₂NR¹⁶R¹⁷.
- R⁶: steht ganz besonders bevorzugt für Methyl, Ethyl, n-Propyl, i-Propyl, n-Butyl, i-Butyl, s-Butyl, t-Butyl, Trifluormethyl, Trifluorethyl, oder für jeweils gegebenenfalls einfach bis vierfach, gleich oder verschieden durch Fluor, Chlor, Brom, C₁-C₄-Alkyl, C₁-C₄-Alkoxy, C₁-C₄-Alkylthio, C₁-C₄-Halogenalkyl, C₁-C₄-Halogenalkoxy und/oder C₁-C₄-Halogenalkylthio, mit jeweils 1 bis 9 Fluor-, Chlor- und/oder Bromatomen, substituiertes Cyclopropyl, Cyclopropylmethyl, Cyclopentyl, Cyclopentylmethyl, Cyclohexyl, Cyclohexylmethyl, Phenyl, Benzyl oder Phenylethyl.
- R⁷: steht ganz besonders bevorzugt für gegebenenfalls einfach oder mehrfach, gleich oder verschieden durch Fluor, Chlor, Brom und/oder -NR⁸R⁹ substituiertes C₁-C₁₀-Alkyl, für jeweils gegebenenfalls einfach bis vierfach, gleich oder verschieden durch Fluor, Chlor, Brom, Cyano, C₁-C₄-Alkyl, C₁-C₄-Alkoxy, C₁-C₄-Alkylthio, C₁-C₄-Halogenalkyl, C₁-C₄-Halogenalkoxy, und/oder C₁-C₄-Halogenalkylthio, mit jeweils 1 bis 9 Fluor-, Chlor- und/oder Bromatomen, substituiertes Cyclopropyl, Cyclopentyl, Cyclohexyl, Phenyl oder Benzyl.
- R⁸ und R⁹: stehen unabhängig voneinander ganz besonders bevorzugt für Wasserstoff, -SO₂R⁷, -COR⁷, -CO₂R⁷, für jeweils gegebenenfalls einfach oder mehrfach, gleich oder verschieden, durch Fluor, Chlor, Brom, C₁-C₄-Alkylcarbonyl, C₁-C₄-Alkylcarbonyloxy, C₁-C₄-Alkylamino, Di-(C₁-C₄-alkyl)amino, C₁-C₄-Alkoxy, C₁-C₄-Halogenalkoxy, C₁-C₄-Alkylthio, C₁-C₄-Halogenalkylthio Substituiertes C₁-C₁₆-Alkyl oder C₂-C₁₆-Alkenyl; für jeweils gegebenenfalls einfach bis dreifach, gleich oder verschieden durch Fluor, Chlor, Brom, Cyano, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio substituiertes C₃-C₁₀-Cycloalkyl, C₃-C₆-Cycloalkyl-C₁-C₄-alkyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl.
- R⁸ und R⁹: stehen außerdem gemeinsam ganz besonders bevorzugt für gegebenenfalls einfach oder mehrfach, gleich oder verschieden durch Fluor, Chlor, Brom, C₁-C₄-Alkoxy, C₁-C₄-Halogenalkoxy, C₁-C₄-Alkylthio, C₁-C₄-Halogenalkylthio substituiertes C₂-C₁₀-Alkenylen oder für gegebenenfalls im Alkylenteil einfach oder mehrfach, gleich oder verschieden durch Fluor, Chlor, Cyano, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio substituiertes C₃-C₁₀-Alkylen, wobei die Alkylenkette jeweils durch -O-, -S- oder -NR¹⁸- unterbrochen sein kann.
- R¹⁰ und R¹¹: stehen unabhängig voneinander ganz besonders bevorzugt für Wasserstoff, -SO₂R⁷, für jeweils gegebenenfalls einfach bis neunfach, gleich oder verschieden durch Fluor, Chlor, Brom, C₁-C₄-Alkylamino, Di-(C₁-C₄-alkyl)-amino, C₁-C₄-Alkoxy, C₁-C₄-Halogenalkoxy, C₁-C₄-Alkylthio, C₁-C₄-Halogenalkylthio substituiertes C₁-C₆-Alkyl oder C₂-C₆-Alkenyl; für jeweils gegebenenfalls einfach bis dreifach, gleich oder verschieden durch Fluor, Chlor und/oder Brom, Cyano, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio substituiertes C₃-C₆-Cycloalkyl, C₃-C₆-Cycloalkyl-C₁-C₄-alkyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl.
- R¹⁰ und R¹¹: stehen außerdem gemeinsam ganz besonders bevorzugt für jeweils gegebenenfalls im Alkylenteil einfach bis vierfach, gleich oder verschieden durch Fluor, Chlor, Cyano, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio substituiertes C₄-C₆-Alkylen, -(CH₂)₂-O-(CH₂)₂-, -(CH₂)₂-S-(CH₂)₂-, -(CH₂)₂-N(R¹⁸)-(CH₂)₂-.
- R¹² und R¹³: stehen unabhängig voneinander ganz besonders bevorzugt für Wasserstoff, für gegebenenfalls einfach bis neunfach, gleich oder verschieden durch Fluor, Chlor, Brom, C₁-C₄-Alkoxy, C₁-C₄-Alkylthio, substituiertes C₁-C₆-Alkyl, für jeweils gegebenenfalls einfach bis vierfach, gleich oder verschieden durch Fluor, Chlor, Brom, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio substituiertes C₃-C₆-Cycloalkyl, C₃-C₆-Cycloalkyl-C₁-C₄-alkyl, Phenyl, Benzyl oder Phenylethyl.
- R¹² und R¹³: stehen außerdem gemeinsam ganz besonders bevorzugt für jeweils gegebenenfalls einfach bis vierfach, gleich oder verschieden durch Fluor, Chlor, Brom, C₁-C₄-Alkyl, C₁-C₄-Halogenalkyl, C₁-C₄-Alkoxy, C₁-C₄-Halogenalkoxy, C₁-C₄-Allcylthio, C₁-C₄-Halogena!kyHhiö substituiertes C₃-C₈-Alkylen oder C₃-C₈-Alkenylen.
- R¹⁶ und R¹⁷: stehen unabhängig voneinander ganz besonders bevorzugt für Wasserstoff C₁-C₆-Alkyl, C₁-C₄-Halogenalkyl mit 1 bis 9 Fluor-, Chlor- und/oder Bromatomen oder für gegebenenfalls einfach bis vierfach, gleich oder verschieden durch Fluor, Chlor, Brom und/oder C₁-C₄-Alkyl substituiertes C₃-C₆-Cycloalkyl.
- R¹⁶ und R¹⁷: stehen außerdem gemeinsam ganz besonders bevorzugt für -(CH₂)₃-, -(CH₂)₄-, -(CH₂)₅-, -(CH₂)₂-O-(CH₂)₂- oder -(CH₂)₂-S-(CH₂)₂-.
- R¹⁸: steht ganz besonders bevorzugt für Wasserstoff, -SO₂R⁷, für -COR⁷ oder -CO₂R⁷; für jeweils gegebenenfalls einfach oder mehrfach, gleich oder verschieden durch Fluor, Chlor, Brom, Cyano, Methylamino, Ethylamino, Di(C₁-C₆-alkyl)amino, C₁-C₄-Alkoxy, C₁-C₄-Halogenalkoxy, C₁-C₄-Alkylthio, C₁-C₄-Halogenalkylthio substituiertes C₁-C₁₆-Alkyl oder C₂-C₁₆-Alkenyl; für jeweils gegebenenfalls einfach bis dreifach, gleich oder verschieden durch Fluor, Chlor, Brom, Cyano, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio substituiertes C₃-C₁₀-Cycloalkyl, C₃-C₆-Cycloalkyl-C₁-C₄-alkyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl.
- w: steht ganz besonders bevorzugt für 0, 1 oder 2.
- n: steht ganz besonders bevorzugt für 1 oder 2.
- p: steht ganz besonders bevorzugt für 0 oder 1.
- R¹: steht insbesondere ganz besonders bevorzugt für Fluor, Chlor, Brom, Methyl, Trifluormethyl, Methoxy, Trifluormethoxy, Methylthio oder Trifluormethylthio.
- R² und R³: stehen unabhängig voneinander insbesondere ganz besonders bevorzugt für Wasserstoff, Fluor, Chlor, Brom, Methyl, Trifluormethyl, Methoxy oder Trifluormethoxy.
- Het: steht insbesondere ganz besonders bevorzugt für jeweils gegebenenfalls einfach oder zweifach, gleich oder verschieden durch R⁵ substituiertes 2-Thienyl, 3-Thienyl, 2-Benzo[b]thienyl, 2-Furyl, 3-Furyl, 2-Benzo[b]furyl, 2-Thieno[3,2-b]thienyl, 2-Thieno[3,2-b]furyl, 5-Thieno[3,2-b]furyl, 2-Thieno-[2,3-f][1]benzothienyl, 2-Thieno[2,3-f][1]benzofuryl, 6-Thienop[2,3-f][1]-benzofuryl, 2-Pyridinyl, 3-Pyridinyl, 2-Pyrimidinyl, 5-Pyrimidinyl, 3-Pyridazinyl, 4-Pyridazinyl oder 2-Pyrazinyl.
- R⁵: steht insbesondere ganz besonders bevorzugt für die Gruppierung -X-Y-Z-E mit der Maßgabe, dass Y nicht für eine direkte Bindung steht, wenn X nicht für eine direkte Bindung steht.
- X: steht insbesondere ganz besonders bevorzugt für eine direkte Bindung, Sauerstoff, Schwefel, -SO₂-, -NR⁶-, -CO-, -C(O)-O-, -O-C(O)- -CH₂-, -(CH₂)₂-, -C=C- (E oder Z), -C≡C-, -CH₂O-, -(CH₂)₂O-, -OCH₂-, -O(CH₂)₂-, -O-CH₂-O-, -SCH₂-, -S(CH₂)₂-, -CH₂S- oder -(CH₂)₂S-.
- Y: steht insbesondere ganz besonders bevorzugt für eine direkte Bindung oder für jeweils gegebenenfalls einfach oder zweifach, gleich oder verschieden durch Reste aus der Liste W¹ substituiertes 1,4-Phenylen, 1,3-Phenylen, 2,6-Naphthylen, 2,7-Naphthylen, 2,4-Furylen, 2,4-Thienylen, 2,5-Pyridinylen, 2,5-Pyrimidinylen, 3,6-Pyridazinylen oder 2,5-Pyrazinylen.
- Z: steht insbesondere ganz besonders bevorzugt eine direkte Bindung, Methylen oder Ethylen.
- E: steht insbesondere ganz besonders bevorzugt für Wasserstoff, Fluor, Chlor, Brom, Hydroxy, Cyano, Formyl, -S(O)_{w}R⁷, -OSO₂R⁷, -NR⁸R⁹, -COR⁷, -CO₂R⁷, -OC(O)R⁷, -CONR¹⁰R¹¹, -N(R¹²)COR¹³, -SO₂NR¹⁶R¹⁷; für jeweils gegebenenfalls einfach oder mehrfach, gleich oder verschieden durch Fluor, Chlor, Brom, Cyano, C₁-C₆-Alkoxy und/oder -NR⁸R⁹ substituiertes C₁-C₁₆-Alkyl, C₂-C₁₆-Alkenyl, C₁-C₁₆-Alkoxy, C₂-C₁₆-Alkenyloxy; oder für jeweils gegebenenfalls einfach bis dreifach, gleich oder verschieden, durch Fluor, Chlor, Brom, Cyano, Methyl, Ethyl, n-Propyl, i-Propyl, -CF₃, -CHF₂, -CClF₂, -CF₂CHFCl, -CF₂CH₂F, -CF₂CCl₃, -CH₂CF₃, -CF₂CHFCF₃, -CH₂CF₂H, -CH₂CF₂CF₃, -CF₂CF₂H, -CF₂CHFCF₃, Vinyl, Allyl, 1-Propenyl, Butenyl, -CF=CHF, -CF=CH₂, -CF=CCl₂, -CH=CF₂, -CF₂CF=CF₂, -CH=CFH, -CH₂CF=CF₂, -CF=CF₂, -CF₂CH=CF₂, Methoxy, Ethoxy, n-Propoxy, i-Propoxy, n-Butoxy, i-Butoxy, s-Butoxy, t-Butoxy, Trifluormethoxy, Difluormethoxy, Chlordifluormethoxy, Trifluorethoxy, Methylthio, Ethylthio, n-Propylthio, i-Propylthio, n-Butylthio, i-Butylthio, s-Butylthio, t-Butylthio, Trifluormethylthio, Difluormethylthio, Chlordifluormethylthio, Trifluorethylthio substituiertes Cyclopropyl, Cyclopentyl, Cyclohexyl, Cyclopropyloxy; Cyclopentyloxy, Cyclohexyloxy, Cyclopropylmethyl, Cyclopentylmethyl, Cyclohexylmethyl, Phenyl, Phenoxy; Benzyl, Phenylethyl, Benzyloxy, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidino, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Piperidino, Morpholinyl, Thiomorpholinyl, Morpholino, Thiomorpholino, Triazinyl, Triazolyl, Chinolinyl oder Isochinolinyl.
- W¹: steht insbesondere ganz besonders bevorzugt für Fluor, Chlor, Brom, Cyano, Formyl, Methyl, Ethyl, n-Propyl, i-Propyl, n-Butyl, i-Butyl, s-Butyl, t-Butyl, Methoxy, Ethoxy, n-Propoxy, i-Propoxy, n-Butoxy, i-Butoxy, s-Butoxy, t-Butoxy, Vinyl, Allyl, Trifluormethyl, Trifluorethyl, Trifluormethoxy, Trifluorethoxy, -OCF₂CF₂H, -CH=CF₂, -CH=CCl₂, -OCF=CF₂, -COMe, -COEt, -CO₂Me, -CO₂Et, -CO₂(t-Bu), -SMe, -SOMe, -SO₂Me, -SCF₃, -SOCF₃, -SO₂CF₃, -SCHF₂, -SOCHF₂, -SO₂CHF₂, -SO₂Nme₂, -Nme₂, Net₂, -N(n-Pr)₂, -N(Me)COMe, -N(Me)COEt, -N(Me)COPr, -N(Me)CO(t-Bu), 2-Pyrrolidonyl, 2-Piperidonyl, -N(Me)SO₂Me, -N(Me)SO₂Et, -N(Me)SO₂CF₃, -N(Et)SO₂CF₃, -N(Me)SO₂(CF₂)₃CF₃ oder -OSO₂Nme₂.
- R⁶: steht insbesondere ganz besonders bevorzugt für Methyl, Ethyl, n-Propyl, i-Propyl, n-Butyl, i-Butyl, s-Butyl, t-Butyl, Trifluormethyl, Trifluorethyl, Cyclopropyl, Cyclopropylmethyl, Cyclopentyl, Cyclopentylmethyl, Cyclohexyl, Cyclohexylmethyl, Phenyl, Benzyl oder Phenylethyl.
- R⁷: steht insbesondere ganz besonders bevorzugt für Methyl, Ethyl, n-Propyl, i-Propyl, n-Butyl, i-Butyl, s-Butyl, t-Butyl, Pentyl, Hexyl, -CF₃, -CHF₂, -CCl₃, -CCl₂F, Dimethylaminomethyl, Dimethylaminoethyl, Diethylaminomethyl, Diethylaminoethyl, Cyclopropyl, Cyclopentyl, Cyclohexyl, Phenyl oder Benzyl.
- R⁸ und R⁹: stehen unabhängig voneinander insbesondere ganz besonders bevorzugt für Wasserstoff, -SO₂R⁷, -COR⁷, -CO₂R⁷, für jeweils gegebenenfalls einfach oder mehrfach, gleich oder verschieden, durch Fluor, Chlor, Brom, C₁-C₄-Alkylcarbonyl, C₁-C₄-Alkylcarbonyloxy, C₁-C₄-Alkylamino, Di-(C₁-C₄-alkyl)amino, C₁-C₄-Alkoxy, C₁-C₄-Halogenalkoxy, C₁-C₄-Alkylthio, C₁-C₄-Halogenalkylthio substituiertes C₁-C₁₆-Alkyl, C₂-C₁₆-Alkenyl; für jeweils gegebenenfalls einfach bis dreifach, gleich oder verschieden durch Fluor, Chlor, Brom, Cyano, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio substituiertes C₃-C₈-Cycloalkyl, Cyclopropylmethyl, Cyclopentylmethyl, Cyclohexylmethyl, Cyclopropylethyl, Cyclopentylethyl, Cyclohexylethyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl.
- R⁸ und R⁹: stehen außerdem gemeinsam insbesondere ganz besonders bevorzugt für gegebenenfalls einfach oder mehrfach, gleich oder verschieden durch Fluor, Chlor, Brom, C₁-C₄-Alkoxy, C₁-C₄-Halogenalkoxy, C₁-C₄-Alkylthio, C₁-C₄-Halogenalkylthio substituiertes C₂-C₈-Alkenylen oder für gegebenenfalls im Alkylenteil einfach oder mehrfach, gleich oder verschieden durch Fluor, Chlor, Cyano, Methyl, Ethyl, n-Propyl, i-Propyl, n-Butyl, i-Butyl, s-Butyl, t-Butyl, C₁-C₄-Halogenalkyl, Methoxy, Ethoxy, n-Propoxy, i-Propoxy, n-Butoxy, i-Butoxy, s-Butoxy, t-Butoxy, C₁-C₄-Halogenalkoxy, Methylthio, Ethylthio, n-Propylthio, i-Propylthio, n-Butylthio, i-Butylthio, s-Butylthio, t-Butylthio, C₁-C₄-Halogenalkylthio substituiertes C₃-C₈-Alkylen, wobei die Alkylenkette jeweils durch -O-, -S- oder -NR¹⁸- unterbrochen sein kann.
- R¹⁰ und R¹¹: stehen unabhängig voneinander insbesondere ganz besonders bevorzugt für Wasserstoff, -SO₂CF₃, Methyl, Ethyl, n-Propyl, i-Propyl, n-Butyl, i-Butyl, s-Butyl, t-Butyl, Pentyl, Hexyl, -CF₃, -CH₂CF₃, -(-CF₂)₃CF₃, Methoxymethyl, Methoxyethyl, Cyclopropyl, Cyclopentyl, Cyclohexyl, oder für jeweils gegebenenfalls einfach bis dreifach, gleich oder verschieden durch Fluor, Chlor, Brom, Methyl, Trifluormethyl, Methoxy, Trifluormethoxy substituiertes Phenyl oder Benzyl.
- R¹⁰ und R¹¹: stehen außerdem gemeinsam insbesondere ganz besonders bevorzugt für -(CH₂)₄-, -(CH₂)₅-, -(CH₂)₆-, -CH₂-CH(CH₃)-CH₂-CH(CH₃)-CH₂-, -(CH₂)₂-O-(CH₂)₂-, -(CH₂)₂-S-(CH₂)₂- oder -(CH₂)₂-N(R¹⁸)-CH₂)₂-.
- R¹² und R¹³: stehen unabhängig voneinander insbesondere ganz besonders bevorzugt für Wasserstoff, Methyl, Ethyl, n-Propyl, i-Propyl, n-Butyl, i-Butyl, s-Butyl, t-Butyl, n-Hexyl, Trifluormethyl, Trifluorethyl, Cyclopropyl, Cyclopentyl, Cyclohexyl oder für jeweils gegebenenfalls einfach bis vierfach, gleich oder verschieden durch Fluor, Chlor, Brom, Methyl, Trifluormethyl, Methoxy, Trifluormethoxy substituiertes Phenyl oder Benzyl.
- R¹² und R¹³: stehen außerdem gemeinsam insbesondere ganz besonders bevorzugt für jeweils gegebenenfalls einfach bis vierfach, gleich oder verschieden durch Fluor, Chlor, Brom, Methyl, Ethyl, Methoxy, Ethoxy, Methylthio, Ethylthio, Trifluormethyl, Trifluormethoxy, Trifluormethylthio substituiertes -(CH₂)₃-, -(CH₂)₄-, -(CH₂)₅-, -(CH₂)₆-.
- R¹⁶ und R¹⁷: stehen unabhängig voneinander insbesondere ganz besonders bevorzugt für Wasserstoff, Methyl, Ethyl, n-Propyl, i-Propyl, n-Butyl, i-Butyl, s-Butyl, t-Butyl, n-Hexyl, Trifluormethyl, Trifluorethyl, Cyclopropyl, Cyclopentyl oder Cyclohexyl.
- R¹⁶ und R¹⁷: stehen außerdem gemeinsam insbesondere ganz besonders bevorzugt für -(CH₂)₃-, -(CH₂)₄-, -(CH₂)₅-, -(CH₂)₂-O-(CH₂)₂- oder -(CH₂)₂-S-(CH₂)₂-.
- R¹⁸: steht insbesondere ganz besonders bevorzugt für Wasserstoff, -SO₂R⁷, für -COR⁷ oder -CO₂R⁷; für gegebenenfalls einfach oder mehrfach, gleich oder verschieden durch Fluor, Chlor, Brom, Cyano, Methylamino, Ethylamino, Di-(C₁-C₆-alkyl)amino, C₁-C₄-Alkoxy, C₁-C₄-Halogenalkoxy, C₁-C₄-Alkylthio, C₁-C₄-Halogenalkylthio substituiertes C₁-C₁₆-Alkyl, C₂-C₁₆-Alkenyl; für jeweils gegebenenfalls einfach bis dreifach, gleich oder verschieden durch Fluor, Chlor, Brom, Cyano, Methyl, Ethyl, n-Propyl, i-Propyl, n-Butyl, i-Butyl, s-Butyl, t-Butyl, C₁-C₄-Halogenalkyl, Methoxy, Ethoxy, n-Propoxy, i-Propoxy, n-Butoxy, i-Butoxy, s-Butoxy, t-Butoxy, C₁-C₄-Halogenalkoxy, Methylthio, Ethylthio, n-Propylthio, i-Propylthio, n-Butylthio, i-Butylthio, s-Butylthio, t-Butylthio, C₁-C₄-Halogenalkylthio substituiertes C₃-C₈-Cycloalkyl, Cyclopropylmethyl, Cyclopentylmethyl, Cyclohexylmethyl, Cyclopropylethyl, Cyclopentylethyl, Cyclohexylethyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl.

Weiterhin ganz besonders bevorzugt sind Verbindungen der Formeln (I-1) bis (I-16) 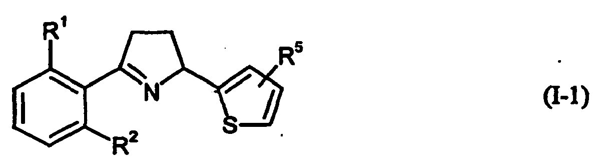 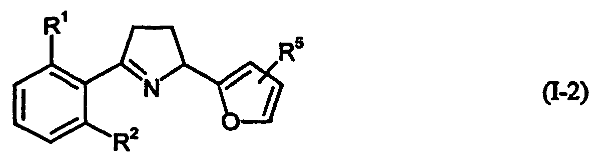 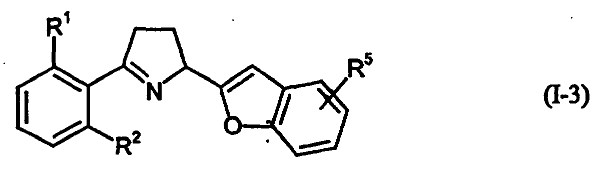 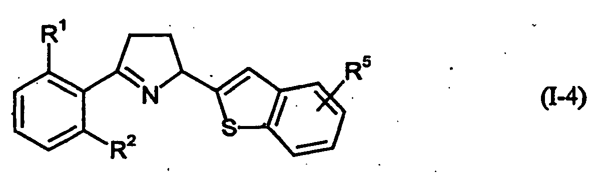 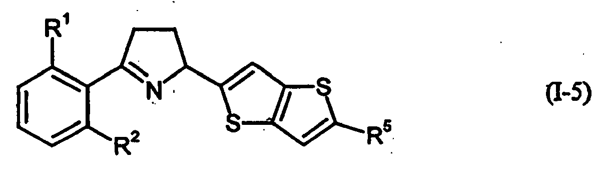 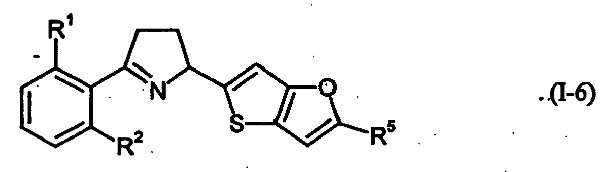 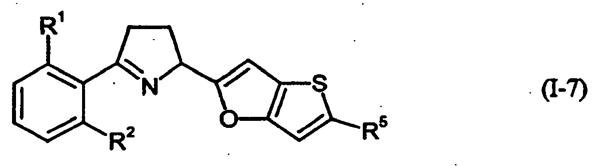 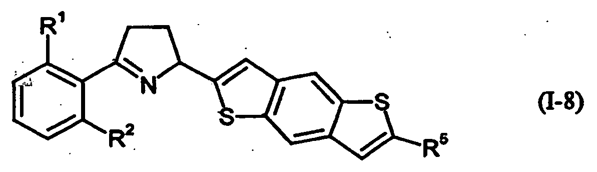 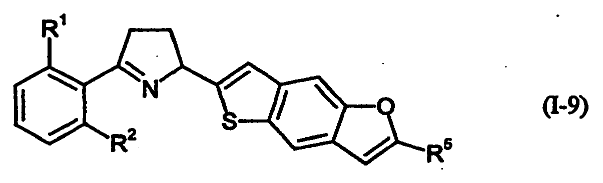 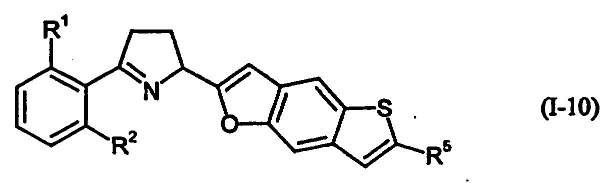 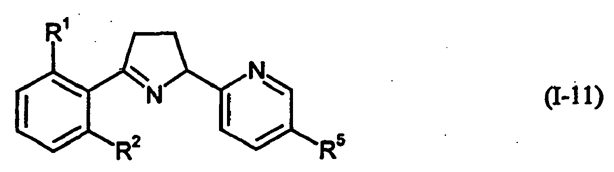 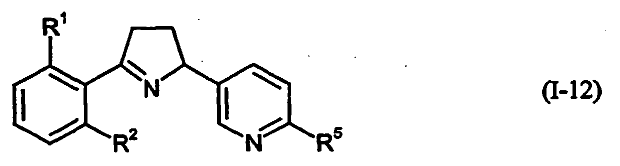 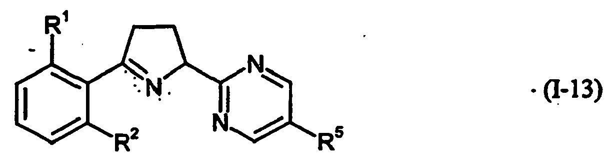 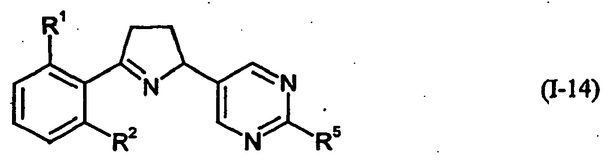 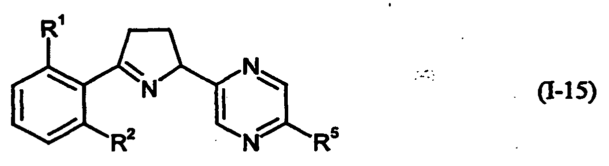 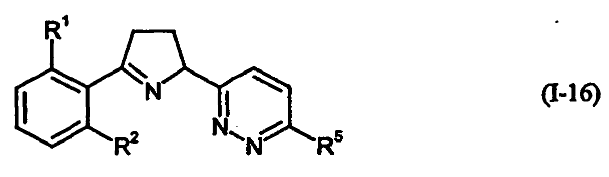 in welchen jeweils
- R¹: für Fluor oder Chlor steht,
- R²: für Wasserstoff oder Fluor steht und
- R⁵: die oben angegebenen Bedeutungen hat.

In den Verbindungen der Formeln (I-1) bis (I-16) stehen R¹, R² und R⁵ bevorzugt, besonders bevorzugt, ganz besonders bevorzugt und insbesondere ganz besonders bevorzugt für diejenigen Bedeutungen, die bereits oben für diese Reste als bevorzugt, besonders bevorzugt etc. genannt wurden.

Weiterhin ganz besonders bevorzugt sind Verbindungen der Formel (I-d) mit (R)-Konfiguration 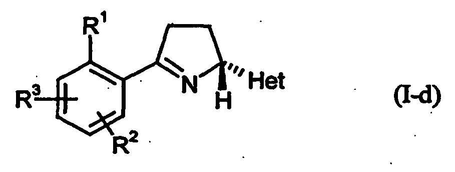 in welcher
R¹, R², R³ und Het die oben angegebenen Bedeutungen haben.

In den Verbindungen der Formel (I-d) stehen R¹, R², R³ und Het bevorzugt, besonders bevorzugt, ganz besonders bevorzugt und insbesondere ganz besonders bevorzugt für diejenigen Bedeutungen, die bereits oben für diese Reste als bevorzugt, besonders bevorzugt etc. genannt wurden.

Weiterhin ganz besonders bevorzugt sind Verbindungen der Formeln (I-17) bis (I-32) mit (R)-Konfiguration 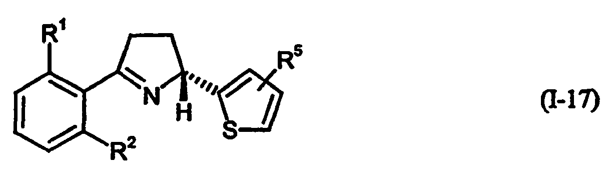 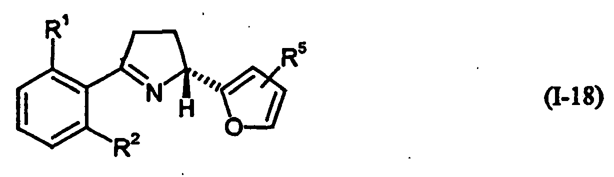 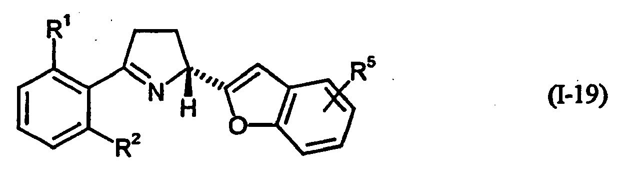 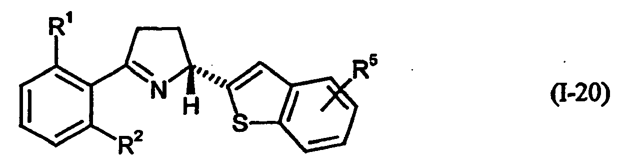 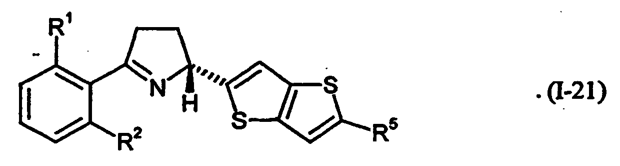 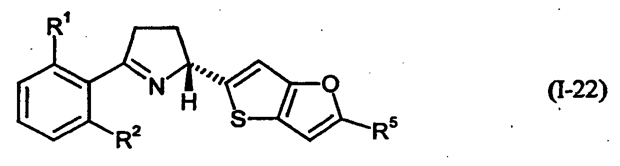 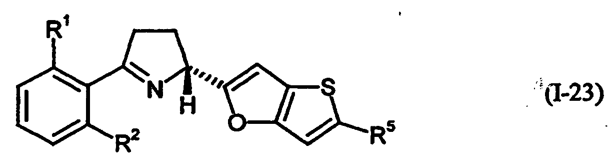 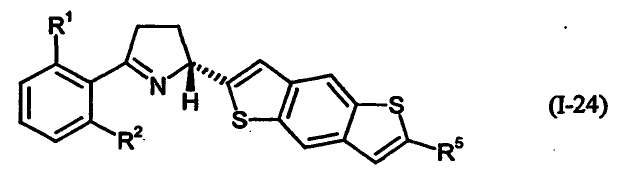 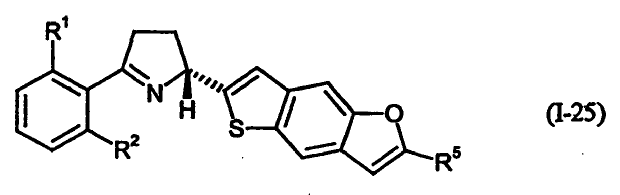 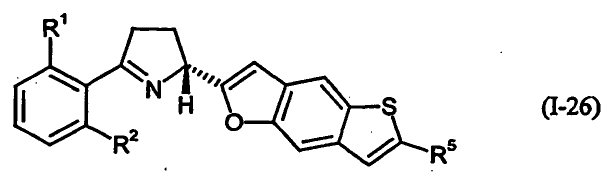 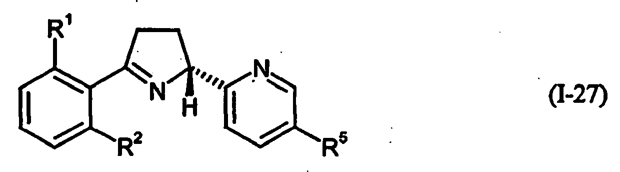 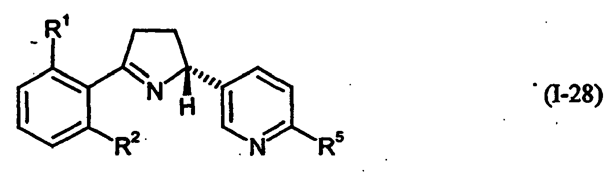 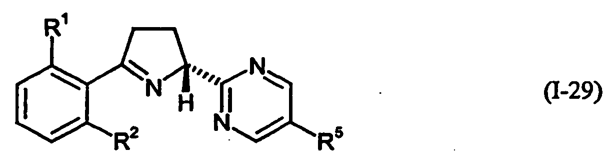 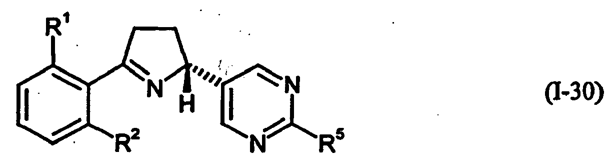 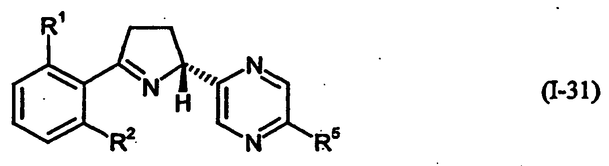 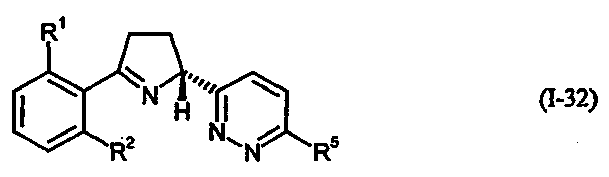 in welchen jeweils
- R¹: für Fluor oder Chlor steht,
- R²: für Wasserstoff oder Fluor steht und
- R⁵: die oben angegebenen Bedeutungen hat.

In den Verbindungen der Formeln (I-17) bis (I-32) stehen R¹, R² und R⁵ bevorzugt, besonders bevorzugt, ganz besonders bevorzugt und insbesondere ganz besonders bevorzugt für diejenigen Bedeutungen, die bereits oben für diese Reste als bevorzugt, besonders bevorzugt etc. genannt wurden.

Verbindungen der Formel (I-d) erhält man durch übliche Verfahren zur Racematspaltung, wie zum Beispiel durch Chromatographie der entsprechenden Racemate an einer chiralen stationären Phase. Es ist möglich, sowohl die racemischen Endprodukte oder racemische Zwischenprodukte auf diese Weise in die beiden Enantiomere zu zerlegen.

Gesättigte Kohlenwasserstoffreste wie Alkyl können, auch in Verbindung mit Heteroatomen, wie z.B. in Alkoxy, soweit möglich, jeweils geradkettig oder verzweigt sein.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend.

Verwendet man tert-Butyl-1-(5-brom-2-thienyl)-4-(2,6-difluorpbenyl)-4-oxobutylcarbamat als Ausgangsstoff und Trifluoressigsäure (TFA), so kann der Verlauf des erfindungsgemäßen Verfahrens (A) durch das folgende Formelschema veranschaulicht werden.

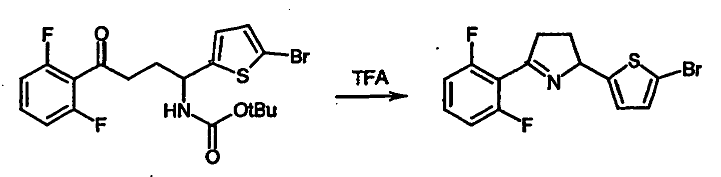

Verwendet man 4-Azido-4-(6-chlor-3-pyridinyl)-1-(2,6-difluorphenyl)-1-butanon als Ausgangsstoff und Triphenylphosphin (PPh₃), so kann der Verlauf des erfindungsgemäßen Verfahrens (B) durch das folgende Formelschema veranschaulicht werden.

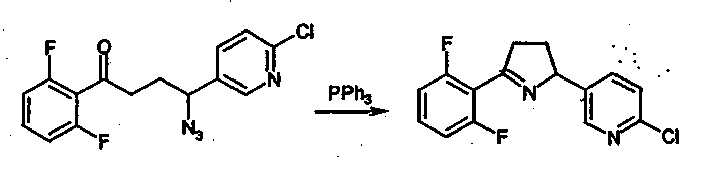

Verwendet man N-[1-(4-Brom-2-thienyl)-4-(2,6-difluorphenyl)-4-oxobutyl]acetamid als Ausgangsstoff und Salzsäure (HCl), so kann der Verlauf des erfindungsgemäßen Verfahrens (©durch das folgende Formelschema veranschaulicht werden.

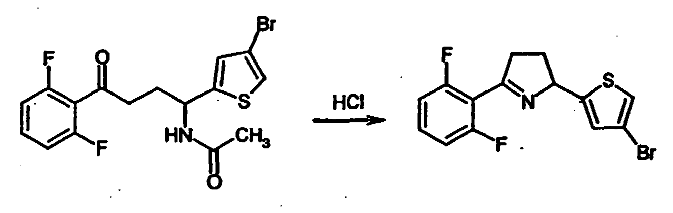

Verwendet man 2-Chlor-5-[5-(2,6-difluorphenyl)-3,4-dihydro-2H-pyrrol-2-yl]pyridin und N-(5-Brom-2-pyrimidinyl)-N,N-diethylamin als Ausgangsstoffe und 4,4,4',4',5,5,5',5'-Octamethyl-2,2'-bi-1,3,2-dioxaborolan sowie einen Palladiumkatalysator, so kann der Verlauf des erfindungsgemäßen Verfahrens (D) durch das folgende Formelschema veranschaulicht werden.

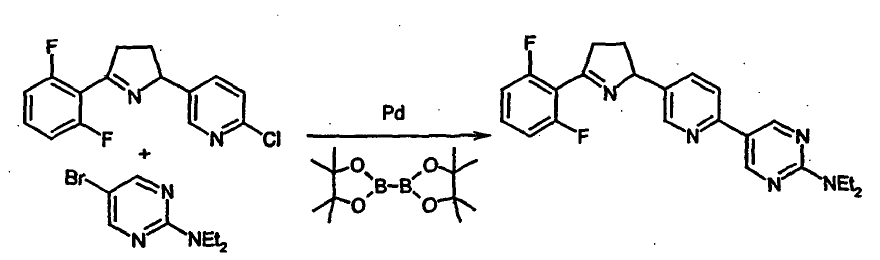

Verwendet man 2-(4,4,5,5-tetramethyl-1,3,2-dioxaborolan-2-yl)-5-[S-(2,6-difluorphenyl)-3,4-dihydro-2H-pyrrol-2yl]pyridin und 5-Brom-2-trifluormethoxypyrimidin als Ausgangsstoffe sowie einen Palladiumkatalysator, so kann der Verlauf des erfindungsgemäßen Verfahrens (E) durch das folgende Formelschema veranschaulicht werden.

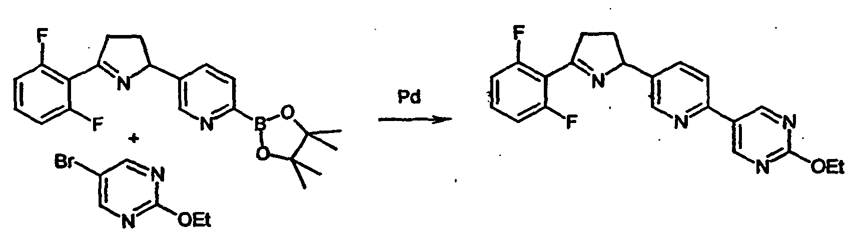

Verwendet man 2-Chlor-5-[5-(2,6-difluorphenyl)-3,4-dihydro-2H-pyrrol-2-yl]pyridin und 4-Trifluormethoxyphenylboronsäure als Ausgangsstoffe sowie einen Palladiumkatalysator, so kann der Verlauf des erfindungsgemäßen Verfahrens (F) durch das folgende Formelschema veranschaulicht werden.

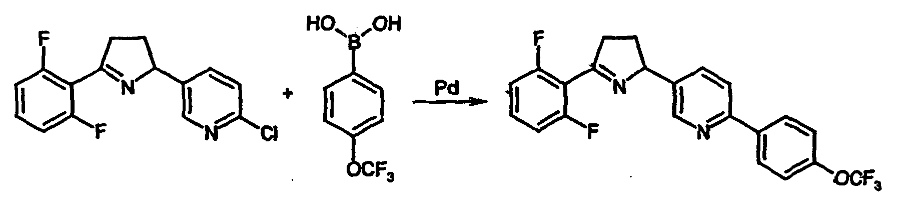

Verwendet man 2-Brom-5-[5-(2,6-difluorphenyl)-3,4-dihydro-2H-pyrrol-2-yl]pyridin und 2-Trifluormethyl-5-(tributylstannyl)pyiridin als Ausgangsstoffe sowie einen Palladiumkatalysator, so kann der Verlauf des erfindungsgemäßen Verfahrens (G) durch das folgende Formelschema veranschaulicht werden.

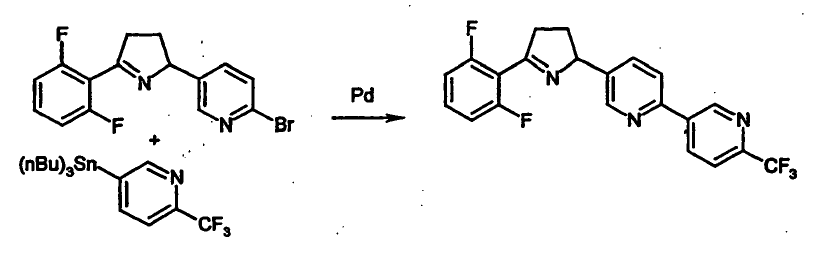

### Erläuterung der Verfahren und Zwischenprodukte

### Verfahren (A)

Die bei der Durchführung des erfindungsgemäßen Verfahrens (A) als Ausgangsstoffe benötigten Aminoketone sind durch die Formel (II) allgemein definiert. In dieser Formel stehen R¹, R², R³ und Het bevorzugt, besonders bevorzugt, ganz besonders bevorzugt bzw. insbesondere ganz besonders bevorzugt für diejenigen Bedeutungen, die bereits in Zusammenhang mit der Beschreibung der erfindungsgemäßen Verbindungen der Formel (I) für diese Reste als bevorzugt, besonders bevorzugt etc. genannt wurden.

Aminoketone der Formel (II) sind neu. Sie lassen sich herstellen, indem man
a) Lactame der Formel (IX) 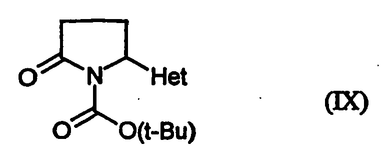 in welcher
   Het die oben angegebenen Bedeutungen hat,
   mit metallierten Aromaten der Formel (X) 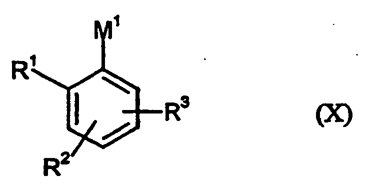 in welcher
   - R¹ R² und R³: die oben angegebenen Bedeutungen haben und
   - M¹: für Li, MgCl, MgBr, MgI oder ZnCl steht,
bei Temperaturen zwischen minus 70°C und plus 70°C gegebenenfalls in Gegenwart eines Verdünnungsmittels (z.B. Tetrahydrofuran) umsetzt.

Die bei der Durchführung des Verfahrens (a) als Ausgangsstoffe benötigten Lactame sind durch die Formel (IX) allgemein definiert. In dieser Formel steht Het bevorzugt, besonders bevorzugt, ganz besonders bevorzugt bzw. insbesondere ganz besonders bevorzugt für diejenigen Bedeutungen, die bereits in Zusammenhang mit der Beschreibung der erfindungsgemäßen Verbindungen der Formel (I) für diesen Rest als bevorzugt, besonders bevorzugt etc. genannt wurden.

Lactame der Formel (IX) sind neu. Sie lassen sich herstellen, indem man
b) Lactame der Formel (XI) 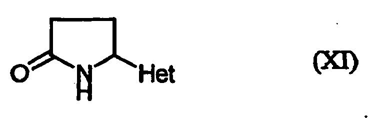 in welcher
   Het die oben angegebenen Bedeutungen hat,
   z.B. mit Di-tert-butyldicarbonat in Anwesenheit einer Base (z.B. Dimethylaminopyridin) und gegebenenfalls in Gegenwart eines Verdünnungsmittels (z.B. Dichlormethan) umsetzt (vgl. Tetrahedron Lett. 1998, 39, 2705-2706).

Die bei der Durchführung des Verfahrens (a) als Ausgangsstoffe benötigten metallierten Aromaten sind durch die Formel (X) allgemein definiert. In dieser Formel stehen R¹, R² und R³ bevorzugt, besonders bevorzugt, ganz besonders bevorzugt bzw. insbesondere ganz besonders bevorzugt für diejenigen Bedeutungen, die bereits in Zusammenhang mit der Beschreibung der erfindungsgemäßen Verbindungen der Formel (I) für diese Reste als bevorzugt, besonders bevorzugt etc. genannt wurden. M¹ steht bevorzugt für Li, MgCl, MgBr, MgI oder ZnCl, besonders bevorzugt für Li, MgCl, MgBr oder MgI, ganz besonders bevorzugt für Li, MgCl oder MgBr.

Metallierte Aromaten der Formel (X) sind bekannt und/oder können nach bekannten Methoden (z.B. Lithiierung oder Grignard-Reaktion) aus den entsprechenden Aromaten oder Halogenaromaten hergestellt werden.

Die bei der Durchführung des Verfahrens (b) als Ausgangsstoffe benötigten Lactame sind durch die Formel (XI) allgemein definiert. In dieser Formel steht Het bevorzugt, besonders bevorzugt, ganz besonders bevorzugt bzw. insbesondere ganz besonders bevorzugt für diejenigen Bedeutungen, die bereits in Zusammenhang mit der Beschreibung der erfindungsgemäßen Verbindungen der Formel (I) für diesen Rest als bevorzugt, besonders bevorzugt etc. genannt wurden.

Lactame der Formel (X1) sind teilweise bekannt. Sie lassen sich z.B. herstellen, indem man
c) Alkoxylactame der Formel (XII) 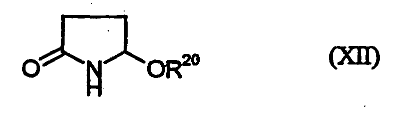 in welcher
   - R²⁰: für Alkyl steht,
   entweder
   α) mit Heteroaromaten der Formel (XIII)

      H-Het⁵ (XIII)

      in welcher
      - Het⁵: für gegebenenfalls einfach bis vierfach, gleich oder verschieden durch R⁵ substituiertes 5- bis 14-gliedriges, 1 bis 3 aroma tische Ringe enthaltendes Heteroaryl mit einem oder mehreren Heteroatomen aus der Reihe Sauerstoff oder Schwefel steht, wobei R⁵ die oben angegebenen Bedeutungen hat,
      in Gegenwart einer Protonensäure (z.B. Schwefelsäure, Essigsäure) oder einer Lewissäure (z.B. Aluminiumchlorid) gegebenenfalls in Gegenwart eines Verdünnungsmittels (z.B. Dichlormethan oder Acetonitril) umsetzt (vgl. Tetrahedron 1976, 32, 1571),
      oder
   β) mit Grignard-Verbindungen der Formel (XTV) 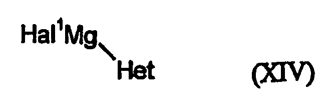 in welcher
      - Het: die oben angegebenen Bedeutungen hat und
      - Hal¹: für Halogen steht,
      in Gegenwart eines Verdünnungsmittels (z.B. Tetrahydrofuran) umsetzt (vgl. Org. Prep. Proced. Int. 1993, 25, 255).

Die bei der Durchführung des Verfahrens © als Ausgangsstoffe benötigten Alkoxylactame sind durch die Formel (XII) allgemein definiert. In dieser Formel steht R²⁰ bevorzugt für C₁-C₆-Alkyl, besonders bevorzugt für C₁-C₄-Alkyl, ganz besonders bevorzugt für Methyl oder Ethyl.

Die Alkoxylactame der Formel (XII) sind bekannt und lassen sich beispielsweise aus den entsprechenden unsubstituierten Imiden durch kathodische oder Natriumboronat-Reduktion oder aus den unsubstituierten Lactamen durch anodische Oxidation herstellen (vgl. J. Org. Chem. 1991, 56, 1822; Synthesis 1980, 315).

Die bei der Durchführung des Verfahrens (α) als Ausgangsstoffe benötigten Heteroaromaten sind durch die Formel (XIII) allgemein definiert. In dieser Formel steht Het⁵ bevorzugt für gegebenenfalls einfach bis vierfach, gleich oder verschieden durch R⁵ substituiertes 5- bis 14-gliedriges, 1 bis 3 aromatische Ringe enthaltendes Heteroaryl mit 1 bis 2 Heteroatomen, welches 0 bis 2 nicht benachbarte Sauerstoffatome und/oder 0 bis 2 nicht benachbarte Schwefelatome enthält (insbesondere Thienyl, Benzothienyl, Furyl, Benzofuryl, Thienothienyl, Thienofuryl, Thienobenzothienyl oder Thienobenzofuryl). Het⁵ steht besonders bevorzugt für jeweils gegebenenfalls einfach bis dreifach, gleich oder verschieden durch R⁵ substituiertes 2-Thienyl, 3-Thienyl, 2-Benzo[b]thienyl, 2-Furyl, 3-Furyl, 2-Benzo[b]furyl, 2-Thieno[3,2-b]thienyl, 2-Thieno[3,2-b]furyl, 5-Thieno[3,2-b]furyl, 2-Thieno[2,3-f][1]benzothienyl, 2-Thieno[2,3-f][1]benzofuryl oder 6-Thieno[2,3-f][1]benzofuryl. Het⁵ steht -ganz besonders bevorzugt für jeweils gegebenenfalls einfach oder zweifach, gleich oder verschieden durch R⁵ substituiertes 2-Thienyl, 3-Thienyl, 2-Benzo[b]thienyl, 2-Furyl, 3-Furyl, 2-Benzo[b]furyl, 2-Thieno[3,2-b]thienyl, 2-Thieno[3,2 b]furyl, 5-Thieno-[3,2-b]furyl, 2-Thieno[2,3-f][1]benzothienyl, 2-Thieno[2,3-f][1]benzofuryl oder 6-Thieno[2,3-f][1]benzofuryl. R⁵ steht dabei bevorzugt, besonders bevorzugt, ganz besonders bevorzugt bzw. insbesondere ganz besonders bevorzugt für diejenigen Bedeutungen, die bereits in Zusammenhang mit der Beschreibung der erfindungsgemäßen Verbindungen der Formel (I) für diese Reste als bevorzugt, besonders bevorzugt etc. genannt wurden.

Die Heteroaromaten der Formel (XIII) sind bekannt und/oder können nach bekannten Methoden hergestellt werden.

Die bei der Durchführung des Verfahrens (β) als Ausgangsstoffe benötigten Grignard-Verbindungen sind durch die Formel (XIV) allgemein definiert. In dieser Formel steht Het bevorzugt, besonders bevorzugt, ganz besonders bevorzugt bzw. insbesondere ganz besonders bevorzugt für diejenigen Bedeutungen, die bereits in Zusammenhang mit der Beschreibung der erfindungsgemäßen Verbindungen der Formel (I) für diesen Rest als bevorzugt, besonders bevorzugt etc. genannt wurden. Hal¹ steht bevorzugt für Chlor, Brom oder Iod.

Die Grignard-Verbindungen der Formel (XIV) sind bekannt und/oder können nach bekannten Verfahren hergestellt werden.

Als Verdünnungsmittel kommen bei der Durchführung des erfindungsgemäßen Verfahrens (A) jeweils alle üblichen inerten, organischen Solventien in Frage. Vorzugsweise verwendbar sind gegebenenfalls halogenierte aliphatische, alicyclische oder aromatische Kohlenwasserstoffe, wie Petrolether, Hexan, Heptan, Cyclohexan, Methylcyclohexan, Benzol, Toluol, Xylol oder Decalin; Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäutetnamid; Ester wie Essigsäuremethylester oder Essigsäureethylester, Sulfoxide, wie Dimethylsulfoxid oder Sulfone, wie Sulfolan. Besonders bevorzugt verwendet man Methylenchlorid, Chloroform, Toluol, Methanol oder Ethanol.

Bei der Durchführung des erfindungsgemäßen Verfahrens (A) kommen jeweils alle üblichen Lewissäuren bzw. Protonsäuren in Frage. Methoden zur Boc-Abspaltung sind allgemein bekannt (vgl. z.B. T. W. Greene, P. G. M. Wuts, Protective Groups in Organic Synthesis, Ed. 3, New York, Wiley & Sons, 1999, S. 520-525). Bevorzugt setzt man Trifluoressigsäure, HCl oder HBr zur Abspaltung der Boc-Schutzgruppe ein.

Die Reaktionstemperaturen können bei der Durchführung des erfindungsgemäßen Verfahrens (A) jeweils in einem größeren Bereich variiert werden. Im allgemeinen arbeitet man bei Temperaturen zwischen -20°C und +120°C, vorzugsweise zwischen -10°C und 60°C.

Bei der Durchführung des erfindungsgemäßen Verfahrens (A) setzt man auf 1 Mol an Verbindung der Formel (II) im allgemeinen 100 Mol einer Protonsäure ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch einengt, in einem geeigneten Lösungsmittel aufnimmt, mit Natriumhydroxid auf pH 12 einstellt und die organische Phase mit Wasser wäscht, über Natriumsulfat trocknet, filtriert und einengt. Der Rückstand wird gegebenenfalls nach üblichen Methoden, wie Chromatographie oder Umkristallisation, von eventuell noch vorhandenen Verunreinigungen befreit.

### Verfahren (B)

Die bei der Durchführung des erfindungsgemäßen Verfahrens (B) als Ausgangsstoffe benötigten Azide sind durch die Formel (III) allgemein definiert. In dieser Formel stehen R¹, R², R³ und Het bevorzugt, besonders bevorzugt, ganz besonders bevorzugt bzw. insbesondere ganz besonders bevorzugt für diejenigen Bedeutungen, die bereits in Zusammenhang mit der Beschreibung der erfindungsgemäßen Verbindungen der Formel (I) für diese Reste als bevorzugt, besonders bevorzugt etc. genannt wurden.

Azide der Formel (III) sind neu. Sie lassen sich herstellen, indem man
d) Halogenide der Formel (XV) 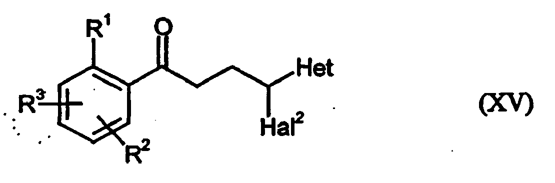 in welcher
   - R¹, R², R³ und Het: die oben angegebenen Bedeutungen haben und
   - Hal²: für Halogen steht,
   mit Aziden der Formel (XVI)

   Q-N₃ (XVI)

   in welcher
   - Q: für ein Kation steht,
   in Gegenwart eines Verdünnungsmittels (z.B. Wasser-Aceton Gemische oder Wasser-Ethanol Gemische) und gegebenenfalls in Gegenwart eines Katalysators (z.B. Methyltrioctylammoniumchlorid = Aliquat 336, vgl. M. Es-Sayed, Dissertation, Universität Göttingen, 1992) umsetzt

Die bei der Durchführung des Verfahrens (d) als Ausgangsstoffe benötigten Halogenide sind durch die Formel (XV) allgemein definiert. In dieser Formel steht R¹, R², R³ und Het bevorzugt, besonders bevorzugt, ganz besonders bevorzugt bzw. insbesondere ganz besonders bevorzugt für diejenigen Bedeutungen, die bereits in Zusammenhang mit der Beschreibung der erfindungsgemäßen Verbindungen der Formel (I) für diese Reste als bevorzugt, besonders bevorzugt etc. genannt wurden. Hal² steht bevorzugt für Chlor, Brom oder Iod, besonders bevorzugt für Chlor oder Brom, ganz besonders bevorzugt für Chlor.

Halogenide der Formel (XV) sind neu. Sie lassen sich herstellen, indem man
e) Cyclopropane der Formel (XVII) 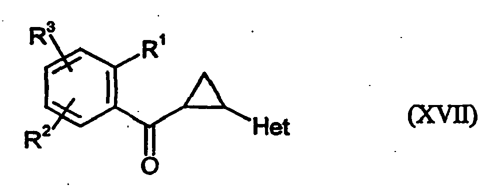 in welcher
   R¹, R², R³ und Het die oben angegebenen Bedeutungen haben,
   mit einer Protonsäure (z.B. HCl) gegebenenfalls in Gegenwart eines Verdünnungsmittels (z.B. Wasser) umsetzt.

Die bei der Durchführung des Verfahrens (d) als Ausgangsstoffe benötigten Azide sind durch die Formel (XVI) allgemein definiert. In dieser Formel steht Q bevorzugt für Alkalimetallionen, Trialkylsilyl, Tetraalkylammonium, Tetraalkylguanidinium oder polymergebundenes Trialkylammonium. Besonders bevorzugt steht Q für Natrium, Lithium, Trimethylsilyl, Tetraethylammonium, Tetra-n-butylammonium oder Tetramethylguanidinium, ganz besonders bevorzugt für Natrium oder Lithium.

Azide der Formel (XVI) sind kommerziell erhältlich bzw. lassen sich nach bekannten Methoden herstellen (vgl. Houben-Weyl: Methoden der Organischen Chemie, Vierte Auflage, Organo-Stickstoff Verbindungen I, Seiten 1243-1290; Herausgeber: D. Klamann).

Die bei der Durchführung des Verfahrens (e) als Ausgangsstoffe benötigten Cyclopropane sind durch die Formel (XVII) allgemein definiert. In dieser Formel stehen R¹, R², R³ und Het bevorzugt, besonders bevorzugt, ganz besonders bevorzugt bzw. insbesondere ganz besonders bevorzugt für diejenigen Bedeutungen, die bereits in Zusammenhang mit der Beschreibung der erfindungsgemäßen Verbindungen der Formel (I) für diese Reste als bevorzugt, besonders bevorzugt etc. genannt wurden.

Cyclopropane der Formel (XVII) sind teilweise bekannt Sie lassen sich z.B. herstellen, indem man
f) α,β-ungesättigte Ketone der Formel (XVIII) 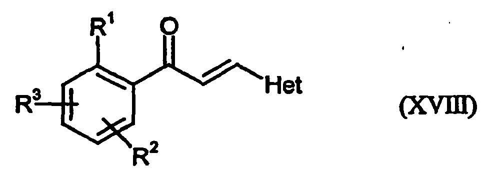 in welcher
   R¹, R², R³ und Het die oben angegebenen Bedeutungen haben,
   mit einem Trialkylsulfoxoniumhalogenid (z.B. Trimethylsulfoxoniumiodid) in Gegenwart einer Base (z.B. Natriumhydrid) und gegebenenfalls in Gegenwart eines Verdünnungsmittels (z.B. Dimethylsulfoxid) umsetzt (vgl. Tetrahedron Asymmetry 1998, 9, 1035).

Die bei der Durchführung des Verfahrens (f) als Ausgangsstoffe benötigten α,β-ungesättigten Ketone sind durch die Formel (XVIII) allgemein definiert. In dieser Formel stehen R¹, R², R³ und Het bevorzugt, besonders bevorzugt, ganz besonders bevorzugt bzw. insbesondere ganz besonders bevorzugt für diejenigen Bedeutungen, die bereits in Zusammenhang mit der Beschreibung der erfindungsgemäßen Verbindungen der Formel (I) für diese Reste als bevorzugt, besonders bevorzugt etc. genannt wurden.

α,β-ungesättigte Ketone der Formel (XVIII) sind bekannt. Sie lassen sich z.B. herstellen, indem man
g) Aldehyde der Formel (XIX)

   Het-CHO (XIX)

   in welcher
   Het die oben angegebenen Bedeutungen hat
   mit Acetophenonen der Formel (XX) 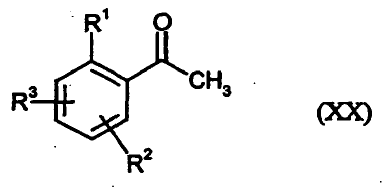 in welcher
   R¹, R² und R³ die oben angegebenen Bedeutungen haben,
   in Gegenwart eine Base (z.B. Natriumhydroxid) und in Gegenwart eines Verdünnungsmittels (z.B. Methanol) umsetzt.

Die bei der Durchführung des Verfahrens (g) als Ausgangsstoffe benötigten Aldehyde sind durch die Formel (XIX) allgemein definiert. In dieser Formel steht Het bevorzugt, besonders bevorzugt, ganz besonders bevorzugt bzw. insbesondere ganz besonders bevorzugt für diejenigen Bedeutungen, die bereits in Zusammenhang mit der Beschreibung der erfindungsgemäßen Verbindungen der Formel (I) für diese Reste als bevorzugt, besonders bevorzugt etc. genannt wurden.

Aldehyde der Formel (XIX) sind bekannt und/oder lassen sich nach bekannten Methoden herstellen.

Die bei der Durchführung des Verfahrens (g) als Ausgangsstoffe benötigten Acetophenone sind durch die Formel (XX) allgemein definiert. In dieser Formel stehen R¹, R² und R³ bevorzugt, besonders bevorzugt, ganz besonders bevorzugt bzw. insbesondere ganz besonders bevorzugt für diejenigen Bedeutungen, die bereits in Zusammenhang mit der Beschreibung der erfindungsgemäßen Verbindungen der Formel (I) für diese Reste als bevorzugt, besonders bevorzugt etc. genannt wurden.

Acetophenone der Formel (XX) sind' bekannt und/oder lassen sich nach bekannten Methoden herstellen.

Cyclopropane der Formel (XVII-a) 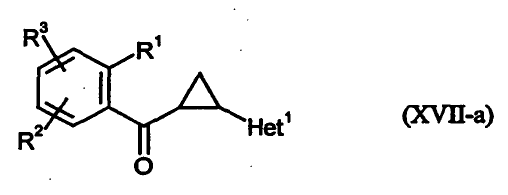 in welcher
R¹, R², R³ und Het¹ die oben angegebenen Bedeutungen haben,
lassen sich auch herstellen, indem man
h) Cyclopropane der Formel (XVII-b) 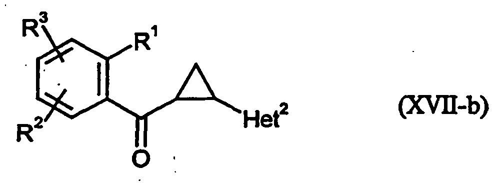 in welcher
   R¹, R², R³ und Het² die oben angegebenen Bedeutungen haben,
   mit Boronsäure-Derivaten der Formel (VII)

   A²-Y¹-E (VII)

   in welcher
   Y¹, E und A² die oben angegebenen Bedeutungen haben,
   in Gegenwart eines Katalysators, gegebenenfalls in Gegenwart eines Säurebindemittels und gegebenenfalls in Gegenwart eines Verdünnungsmittels umsetzt.

Dieses Verfahren entspricht dem erfindungsgemäßen Verfahren (F), in dem Δ¹-Pyrroline der Formel (I-b) umgesetzt werden. Die Kupplungsreaktionen entsprechend den Verfahren (D) und (E) können ebenso auf Cyclopropane der Formel (XVII-a) angewendet werden.

Die in Verfahren (h) als Ausgangsstoffe eingesetzten Cyclopropane der Formel (XVII-b) sind eine Teilmenge der Verbindungen der Formel (XVII) und können analog Verfahren (f) hergestellt werden.

Boronsäure-Derivate der Formel (VII) werden unten bei der Beschreibung des erfindungsgemäßen Verfahrens (F) beschrieben.

Für Verfahren (h) können dieselben Reaktionsbedingungen, Verdünnungsmittel, Reaktionshilfsmittel, Katalysatoren wie für Verfahren (F) unten beschrieben verwendet werden.

Bei der Durchführung des erfindungsgemäßen Verfahrens (B) können alle üblicherweise für diesen Zweck einsetzbaren Trialkylphosphine, Triarylphosphine und Trialkylphosphite (vgl. Tetrahedron Lett. 1999, 40, 4825; Tetrahedron 1997, 53, 3693; Tetrahedron 1997, 55, 8353; J. Chem. Soc. Chem. Commun. 1982, 1224; Synthesis 1996, 123) eingesetzt werden. Bevorzugt verwendet man Organophosphor-Verbindungen wie Triphenylphosphin, Tri-n-butylphosphin oder Trimethylphosphit, besonders bevorzugt Triphenylphosphin.

Weiterhin lassen sich Azide der Formel (III) durch katalytische Hydrierung, z.B. mit PtO₂ als Katalysator, entsprechend dem erfindungsgemäßen Verfahren zu Verbindungen der Formel (I) umsetzen (vgl. J. Am. Chem. Soc. 1954, 76, 1231).

Weitere Möglichkeiten zur Reduktion von Azido-Verbindungen sind in der Literatur beschrieben (vgl. Houben-Weyl: Methoden der Organischen Chemie, Vierte Auflage, Organo-Stickstoff Verbindungen II, Seiten 956-975; Herausgeber: D. Klamann).

Als Verdünnungsmittel kommen bei der Durchführung des erfindungsgemäßen Verfahrens (B) aliphatische oder aromatische Kohlenwasserstoffe, halogenierte Kohlenwasserstoffe oder Ether in Betracht. Vorzugsweise verwendbar sind Pentan, Hexan, Heptan, Benzol, Toluol, Tetrahydrofuran, Diethylether, Dioxan oder Acetonitril, besonders bevorzugt Pentan, Hexan oder Heptan.

Die Reaktionstemperaturen zur Durchführung des erfindungsgemäßen Verfahrens (B) können in einem größeren Bereich variiert werden. Im allgemeinen arbeitet man bei Temperaturen zwischen -10°C und +60°C, vorzugsweise zwischen 0°C und 40°C, besonders bevorzugt bei Raumtemperatur.

Bei der Durchführung des erfindungsgemäßen Verfahrens (B) setzt man im allgemeinen auf 1 Mol an Azid der Formel (III) 1 Mol Trialkylphosphin oder Triarylphosphin oder Trialkylphosphit und ein geeignetes Verdünnungsmittel ein. Es können aber auch andere Verhältnisse der Reaktionskomponenten gewählt werden. Die Aufarbeitung erfolgt nach üblichen Methoden. Im allgemeinen geht man in der Weise vor, dass man das Reaktionsgemisch in Anwesenheit von Florisil einengt und anschließend mit einem Gemisch aus n-Hexan und Essigsäureethylester chromatographiert.

### Verfahren ©

Die bei der Durchführung des erfindungsgemäßen Verfahrens © als Ausgangsstoffe benötigten Amide sind durch die Formel (IV) allgemein definiert. In dieser Formel stehen R¹, R², R³ und Het bevorzugt, besonders bevorzugt, ganz besonders bevorzugt bzw. insbesondere ganz besonders bevorzugt für diejenigen Bedeutungen, die bereits in Zusammenhang mit der Beschreibung der erfindungsgemäßen Verbindungen der Formel (I) für diese Reste als bevorzugt, besonders bevorzugt etc. genannt wurden. R¹⁹ steht bevorzugt für C₁-C₄-Alkyl, C₁-C₄-Halogenalkyl, Phenyl oder Arylalkyl, besonders bevorzugt für Methyl, Ethyl, Phenyl oder Benzyl, ganz besonders bevorzugt für Methyl, Phenyl oder Benzyl.

Amide der Formel (IV) sind neu. Sie lassen sich herstellen, indem man
i) Cyclopropane der Formel (XVII) 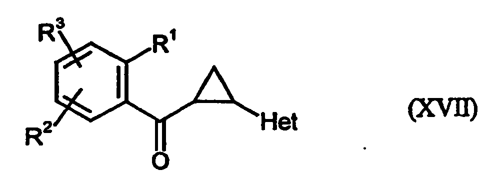 in welcher
   R¹, R², R³ und Het die oben angegebenen Bedeutungen haben,
   mit einem Nitril der Formel (XXI) 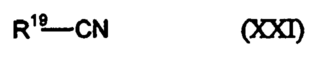 in welcher
   R¹⁹ die oben angegebenen Bedeutungen hat,
   in- Gegenwart einer Protonsäure (z.B. Schwefelsäure) gegebenenfalls in Gegenwart eines Verdünnungsmittels umsetzt.

Die bei der Durchführung des Verfahrens (i) als Ausgangsstoffe benötigten Cyclopropane der Formel (XVII) wurden bereits in Zusammenhang mit der Erläuterung des erfindungsgemäßen Verfahrens (B) beschrieben.

Die bei der Durchführung des Verfahrens (i) als Ausgangsstoffe benötigten Nitrile sind durch die Formel (XXI) allgemein definiert. In dieser Formel steht R¹⁹ bevorzugt, besonders bevorzugt, ganz besonders bevorzugt bzw. insbesondere -ganz besonders bevorzugt für diejenigen Bedeutungen, die bereits in Zusammenhang mit der Beschreibung der Verbindungen der Formel (IV) für diese Reste als bevorzugt, besonders bevorzugt etc. genannt wurden.

Bei der Durchführung des erfindungsgemäßen Verfahrens © werden zur N-Entacylierung der Amide der Formel (IV) bei der Umsetzung zu Pyrrolinen der Formel (I) Protonsäuren (vgl. J. Org. Chem. 1978, 43, 4593), anorganische Basen (vgl. J. Chem. Soc. 1964, 4142), Hydrazine (vgl. J. Org. Chem. 1978, 43, 3711) oder Biotransformationen mit Enzymen (vgl. Appl. Microbiol. Biotechnol. 1997,47,650) verwendet. Andere übliche Verfahren zur Entacylierung von Amiden sind beschrieben bei T. W. Greene, P. G. M. Wuts, Protective Groups in Organic Synthesis (Ed. 3, New York, Wiley 1999, S. 553-555).

Als N-Entacylierungsmittel werden bevorzugt Protonsäuren oder organische Säuren, besonders bevorzugt wässrige Salzsäure, wässrige Bromwasserstoffsäure oder Trifluoressigsäure, ganz besonders bevorzugt wässrige Salzsäure; bevorzugt anorganische Basen, besonders bevorzugt Bariumhydroxid [Ba(OH)₂] und Natriumhydroxid (NaOH) und bevorzugt Biotransformationen, besonders bevorzugt unter Einsatz von Acylasen, verwendet.

Bei der N-Entacylierung mittels Biotransformationen erhält man die Verbindungen der Formel (I) mit einer der beiden Enantiomeren im Überschuss.

Als Verdünnungsmittel kommen bei der Durchführung des erfindungsgemäßen Verfahrens (©Wasser oder Alkohole und Gemische von diesen in Betracht. Bevorzugt verwendet man Wasser, Methanol oder Ethanol oder Gemische aus zwei oder drei dieser drei Verdünnungsmittel.

Die Reaktionstemperaturen bei der Durchführung des erfindungsgemäßen Verfahrens (©können in einem größeren Bereich variiert werden. Im allgemeinen arbeitet man bei Temperaturen zwischen 20°C und 200°C, vorzugsweise zwischen 60°C und 140°C, besonders bevorzugt zwischen 80°C und 120°C. Wird die N-Entacylierung enzymatisch unter Einsatz von Acylasen durchgeführt, arbeitet man im allgemeinen zwischen 20°C und 60°C, vorzugsweise zwischen 20°C und 40°C.

Bei der Durchführung des erfindungsgemäßen Verfahrens © setzt man im allgemeinen auf 1 Volumenanteil einer 10 %-igen (w/v) alkoholischen Lösung an Amid der Formel (IV) 2 Volumenanteile einer Protonsäure ein. Es können aber auch andere Verhältnisse der Reaktionskomponenten gewählt werden. Die Aufarbeitung erfolgt nach üblichen Methoden. Im allgemeinen geht man in der Weise vor, dass man mit Natronlauge neutralisiert und anschließend mit Essigsäureethylester extrahiert, die organische Phase trocknet, filtriert und einengt

### Verfahren D

In einem ersten Reaktionsschritt wird eine Verbindung der Formel (I-b) mit einem Diboronsäureester in Gegenwart eines Palladium-Katalysators, gegebenenfalls in Gegenwart eines Säurebindemittels und gegebenenfalls in Gegenwart eines Lösungsmittels gekuppelt. Ohne Isolierung des Zwischenprodukts wird in demselben Reaktionsgefäß in einem zweiten Reaktionsschritt eine Verbindung der Formel (V) in Gegenwart eines Katalysators, gegebenenfalls in Gegenwart eines Säurebindemittels und gegebenenfalls in Gegenwart eines Lösungsmittels gekuppelt (vgl. zz.B.Tetrahedron Lett. 1997, 38, 3841).

Das erfindungsgemäße Verfahren (D) kann in zwei Varianten durchgeführt werden. Es kann entweder eine Verbindung der Formel (I-b) oder eine Verbindung der Formel (V) vorgelegt werden. Verfahren (D) ist als Tandem-Reaktion der nachfolgend beschriebenen Verfahren (E) und (F) anzusehen.

Die bei der Durchführung des erfindungsgemäßen Verfahrens (D) als Ausgangsstoffe benötigten Δ¹-Pyrroline sind durch die Formel (I-b) allgemein definiert. In dieser Formel stehen R¹, R² und R³ bevorzugt, besonders bevorzugt, ganz besonders bevorzugt bzw. insbesondere ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden. Het² steht bevorzugt für einfach durch R⁵⁻² substituiertes 5- bis 14-gliedriges, 1 bis 3 aromatische Ringe enthaltendes Heteroaryl mit einem oder mehreren Heteroatomen aus der Reihe Stickstoff, Sauerstoff und Schwefel. Het² steht besonders bevorzugt für einfach durch R⁵⁻² substituiertes 5- bis 14-gliedriges, 1 bis 3 aromatische Ringe enthaltendes Heteroaryl mit 1 bis 4 Heteroatomen, welches 0 bis 4 Stickstoffatome, 0 bis 2 nicht benachbarte Sauerstoffatome und/oder 0 bis 2 nicht benachbarte Schwefelatome enthält (insbesondere Thienyl, Benzothienyl, Furyl, Benzofuryl, Indolyl, Thienothienyl, Thienofuryl, Thienobenzothienyl, Thienobenzofuryl, Pyridinyl, Pyrimidinyl, Pyridazinyl, pyrazinyl, Triazolyl oder Tetrazolyl). Het² steht ganz besonders bevorzugt für einfach durch R⁵⁻² substituiertes 2-Thienyl, 3-Thienyl, 2-Benzo[b]thienyl, 2-Furyl, 3-Furyl, 2-Benzo[b]furyl, 2-Indolyl, 2-Thieno[3,2-b]thienyl, 2-Thieno[3,2-b]furyl, 5-Thieno[3,2-b]furyl, 2-Thieno[2,3-f][1]benzothienyl, 2-Thieno[2,3-f][1]benzofuryl, 6-Thieno[2,3-f][1]benzofuryl, 2-Pyridinyl, 3-Pyridinyl, 2-Pyrimidinyl, 5-Pyrimidinyl, 3-Pyridazinyl, 4-Pyridazinyl, 2-Pyrazinyl, Triazolyl oder Tetrazolyl. Het² steht insbesondere ganz besonders bevorzugt für einfach durch R⁵⁻² substituiertes 2-Thienyl, 3-Thienyl, 2-Benzo[b]thienyl, 2-Furyl, 3-Furyl, 2-Benzo[b]furyl, 2-Thieno[3,2-b]thienyl, 2-Thieno[3,2-b]furyl, 5-Thieno[3,2-b]furyl, 2-Thieno[2,3-f][1]benzothienyl, 2-Thieno[2,3-f][1]benzofuryl, 6-Thieno[2,3-f][1]benzofuryl, 2-Pyridinyl, 3-Pyridinyl, 2-Pyrimidinyl, 5-Pyrimidinyl, 3-Pyridazinyl, 4-Pyridazinyl oder 2-Pyrazinyl. R⁵⁻² steht bevorzugt für Brom, Iod, -OSO₂CF₃ oder -SO₂(CF₂)₃CF₃, besonders bevorzugt für Brom, -OSO₂CF₃ oder -OSO₂(CF₂)₃CF₃, ganz besonders bevorzugt für Brom oder -OSO₂CF₃.

Δ¹-Pyrroline der Formel (I-b) sind Gegenstand dieser Erfindung und lassen sich nach einem der Verfahren (A), (B) oder (C) herstellen.

Die bei der Durchführung des erfindungsgemäßen Verfahrens (D) als Ausgangsstoffe benötigten Heterocyclen sind durch die Formel (V) allgemein definiert. In dieser Formel steht E bevorzugt, besonders bevorzugt, ganz besonders bevorzugt bzw. insbesondere ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden. Y¹ steht bevorzugt für jeweils gegebenenfalls einfach bis vierfach, gleich oder verschieden durch Reste aus der Liste W¹ substituiertes Phenylen oder 5- bis 10-gliedriges, gesättigtes oder ungesättigtes Heterocyclylen mit einem oder mehreren Heteroatomen aus der Reihe Stickstoff, Sauerstoff und Schwefel. Y¹ steht besonders bevorzugt für jeweils gegebenenfalls einfach bis vierfach, gleich oder verschieden durch Reste aus der Liste W¹ substituiertes 1,4-Phenylen, 1,3-Phenylen, 1,2-Phenylen; oder für jeweils gegebenenfalls einfach bis vierfach, gleich oder verschieden durch Reste aus der Liste W¹ substituiertes 5- bis 6-gliedriges, gesättigtes oder ungesättigtes Heterocyclylen mit 1 bis 3 Heteroatomen, welches 0 bis 3 Stickstoffatome, 0 bis 2 nicht benachbarte Sauerstoffatome und/oder 0 bis 2 nicht benachbarte Schwefelatome enthält (insbesondere Furylen, Thienylen, Pyrrolylen, Oxazolylen, Thiazolylen, Pyridinylen, Pyrimidinylen, Pyridazinylen oder Pyrazinylen). Y¹ steht ganz besonders bevorzugt für jeweils gegebenenfalls einfach bis dreifach, gleich oder verschieden durch Reste aus der Liste W¹ substituiertes 1,4-Phenylen, 1,3-Phenylen, 1,2-Phenylen, 2,4-Furylen, 2,4-Thienylen, 2,4-Pyrrolylen, 2,5-Oxazolylen, 2,5-Thiazolylen, 2,5-Pyridinylen, 2,6-Pyridinylen, 2,5-Pyrimidinylen, 3,6-Pyridazinylen oder 2,5-Pyrazinylen. Y¹ steht insbesondere ganz besonders bevorzugt für jeweils gegebenenfalls einfach oder zweifach, gleich oder verschieden durch Reste aus der Liste W¹ substituiertes 1,4-Phenylen; 1,3-Phenylen, 2,4-Furylen, 2,4-Thienylen, 2,5-Pyridinylen, 2,5-Pyrimidinylen, 3,6-Pyridazinylen oder 2,5-Pyrazinylen. W¹ steht bevorzugt, besonders bevorzugt, ganz besonders bevorzugt bzw. insbesondere ganz besonders bevorzugt für diejenigen Bedeutungen, die bereits in Zusammenhang mit der Beschreibung der erfindungsgemäßen Verbindungen der Formel (I) für diese Reste als bevorzugt, besonders bevorzugt etc. genannt wurden. A¹ steht bevorzugt für Brom, Chlor, Iod oder -SO₂CF₃, besonders bevorzugt für Brom, Chlor oder Iod, ganz besonders bevorzugt für Brom oder Chlor.

Die Heterocyclen der Formel (V) sind bekannt oder können nach bekannten Verfahren hergestellt werden (vgl. Aust. J. Chem. 1964, 17, 794; Chem. Ber. 1992, 125, 1169; Chem. Pharm. Bull. 1995, 43, 247; Eur. J. Med. Chem. 1989, 24, 249; J. Chem. Soc. C 1971, 1889; J. Chem. Soc. Perkin Trans, 1 1995, 2497; J. Med. Chem. 1991, 34, 315; J. Org. Chem. 1984, 49, 2240; J. Org. Chem. 1990, 55, 69; Org. Prep. Proced. Int. 1998, 30, 433; Synthesis 1999, 1163; Tetrahedron 1999, 40, 7975; Tetrahedron Lett. 1996, 37, 4447; Tetrahedron Lett. 2000, 41, 4335).

Als Diboronsäureester kommen bei der Durchführung des erfindungsgemäßen Verfahrens (D) 4,4,4',4',5,5,5',5'-Octamethyl-2,2'-bi-1,3,2-dioxaborolan, 5,5,5',5'-Tetramethyl-2,2'-bi-1,3,2-dioxaborinan, 4,4,4',4',6,6'-Hexamethyl-2,2'-bi-1,3,2-dioxaborinan oder 2,2'-Bi-1,3,2-benzodioxaborol in Frage. Bevorzugt verwendet man 4,4,4',4',5,5,5',5'-Octamethyl-2,2'-bi-1,3,2-dioxaborolan, 5,5,5',5'-Tetramethyl-2,2'-bi-1,3,2-dioxaborinan oder 4,4,4',4,6,6'-Hexamethyl-2,2'-bi-1,3,2-dioxaborinan, besonders bevorzugt 4,4,4',4',5,3,5',5'-Octamethyl-2,2'-bi-1,3,2-dioxaborolan oder 5,5,5',5'-Tetramethyl-2,2'-bi-1,3,2-dioxaborinan, ganz besonders bevorzugt 4,4,4',4',5,5,5',5'-Octamethyl-2,2'-bi-1,3,2-dioxaborolan.

Bei der Durchführung des erfindungsgemäßen Verfahrens (D) setzt man auf 1 Mol an Verbindung der Formel (I-b) im allgemeinen 1 Mol oder einen leichten Überschuss eines Diboronesters und 1 Mol oder einen leichten Überschuss einer Verbindung der Formel (V), sowie 3 % eines Palladiumkatalysators ein. Es ist jedoch auch möglich, die Reaktionskomponenten in anderen Verhältnissen einzusetzen. Es kann wahlweise die Verbindung der Formel (I-b) oder die Verbindung der Formel (V) zuerst vorgelegt werden. Die Aufarbeitung erfolgt nach üblichen Methoden. Im allgemeinen verfährt man in der Weise, dass man das Reaktionsgemisch mit Wasser verdünnt und mit Essigsäureethylester extrahiert. Die organische Phase wird gewaschen, getrocknet, filtriert und eingeengt. Der Rückstand wird gegebenenfalls nach üblichen Methoden, wie Chromatographie oder Umkristallisation, von eventuell noch vorhandenen Verunreinigungen befreit.

### Verfahren (E)

Die bei der Durchführung des erfindungsgemäßen Verfahrens (E) als Ausgangsstoffe benötigten Δ¹-Pyrroline sind durch die Formel (VI) allgemein definiert. In dieser Formel stehen R¹, R² und R³ bevorzugt, besonders bevorzugt, ganz besonders bevorzugt bzw. insbesondere ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden. Het³ steht bevorzugt für einfach durch A² substituiertes 5- bis 14-gliedriges, 1 bis 3 aromatische Ringe enthaltendes Heteroaryl mit einem oder mehreren Heteroatomen aus der Reihe Stickstoff, Sauerstoff und Schwefel. Het³ steht besonders bevorzugt für einfach durch A² substituiertes 5- bis 14-gliedriges, 1 bis 3 aromatische Ringe enthaltendes Heteroaryl mit 1 bis 4 Heteroatomen, welches 0 bis 4 Stickstoffatome, 0 bis 2 nicht benachbarte Sauerstoffatome und/oder 0 bis 2 nicht benachbarte Schwefelatome enthält (insbesondere Thienyl, Benzothienyl, Furyl, Benzofuryl, Indolyl, Thienothienyl, Thienofuryl, Thienobenzothienyl, Thienobenzofuryl, Pyridinyl, Pyrimidinyl, Pyridazinyl, Pyrazinyl, Triazolyl oder Tetrazolyl). Het³ steht ganz besonders bevorzugt für einfach durch A² substituiertes 2-Thienyl, 3-Thienyl, 2-Benzo[b]thienyl, 2-Furyl, 3-Furyl, 2-Benzo[b]furyl, 2-Indolyl, 2-Thieno-[3,2-b]thienyl, 2-Thieno[3,2-b]furyl, 5-Thieno[3,2-b]furyl, 2-Thieno[2,3-f][1]benzothienyl, 2-Thieno[2,3-f][1]benzofuryl, 6-Thieno[2,3-f][1]benzofuryl, 2-Pyridinyl, 3-Pyridinyl, 2-Pyrimidinyl, 5-Pyrimidinyl, 3-Pyridazinyl, 4-Pyridazinyl, 2-Pyrazinyl, Triazolyl oder Tetrazolyl. Het³ steht insbesondere ganz besonders bevorzugt für einfach durch A² substituiertes 2-Thienyl, 3-Thienyl, 2-Benzo[b]thienyl, 2-Furyl, 3-Furyl, 2-Benzo[b]furyl, 2-Thieno[3,2-b]thienyl, 2-Thieno[3,2-b]furyl, 5-Thieno[3,2-b]furyl, 2-Thieno[2,3-f][1]benzothienyl, 2-Thieno[2,3-f][1]benzofuryl, 6-Thieno[2,3-f][1]benzofuryl, 2-Pyridinyl, 3-Pyridinyl, 2-Pyrimidinyl, 5-Pyrimidinyl, 3-Pyridazinyl, 4-Pyridazinyl oder 2-Pyrazinyl. A² steht bevorzugt für -B(OH)₂, (4,4,5,5-Tetramethyl-1,3,2-dioxaborolan)-2-yl, (5,5-Dimethyl-1,3,2-dioxaborinan)-2-yl, (4,4,6-Trimethyl-1,3,2-dioxaborinan)-2-yl oder 1,3,2-Benzodioxaborol-2-yl, besonders bevorzugt für -(OH)₂, (4,4,5,5-Tetramethyl-1,3,2-dioxaborolan)-2-yl, (5,5-Dimethyl-1,3,2-dioxaborinan)-2-yl oder (4,4,6-Trimethyl-1,3,2-dioxaborinan)-2-yl, ganz besonders bevorzugt für -B(OH)₂, (4,4,5,5-Tetramethyl-1,3,2-dioxaborolan)-2-yl, (5,5-Dimethyl-1,3,2-dioxaborinan)2-yl.

Δ¹-Pyrroline der Formel (VI) lassen sich herstellen, indem man
k) Verbindungen der Formel (I-b) 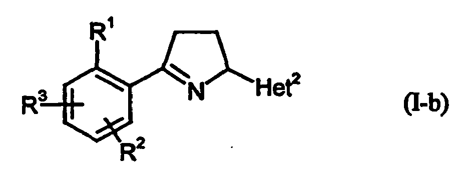 in welcher
   R¹, R², R³ und Het² die oben angegebenen Bedeutungen haben,
   mit einem Diboronsäureester in Gegenwart eines Katalysators, gegebenenfalls in Gegenwart eines Säurebindemittels und gegebenenfalls in Gegenwart eines Verdünnungsmittels umsetzt (vgl. J. Org. Chem. 1995, 60, 7508; Tetrahedron Lett. 1997, 38, 3447).

Geeignete Diboronsäureester zur Durchführung des Verfahrens (k) sind bei der Beschreibung des erfindungsgemäßen Verfahrens (D) bereits genannt worden.

Die bei der Durchführung des erfindungsgemäßen Verfahrens (E) als Ausgangsstoffe benötigten Heterocyclen der Formel (V) wurden bereits oben bei der Beschreibung des Verfahrens (D) beschrieben.

Bei der Durchführung des erfindungsgemäßen Verfahrens (E) setzt man auf 1 Mol an Verbindung der Formel (VI) im allgemeinen 1 Mol oder einen leichten Überschuss an einer Verbindung der Formel (V) ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch in Essigsäureethylester aufnimmt und die organische Phase mit Wasser wäscht, über Natriumsulfat trocknet, filtriert und einengt. Der Rückstand wird gegebenenfalls nach üblichen Methoden, wie Chromatographie oder Umkristallisation, von eventuell noch vorhandenen Verunreinigungen befreit.

### Verfahren (F)

Die bei der Durchführung des erfindungsgemäßen Verfahrens (F) als Ausgangsstoffe benötigten Δ¹-Pyrroline der Formel (I-b) wurden bereits bei der Beschreibung des Verfahrens (D) beschrieben.

Die bei der Durchführung des erfindungsgemäßen Verfahrens (F) als Ausgangsstoffe benötigten Boronsäure-Derivate sind durch die Formel (VII) allgemein definiert. In dieser Formel steht E bevorzugt besonders bevorzugt, ganz besonders bevorzugt bzw, insbesondere ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden. Y¹ steht bevorzugt, besonders bevorzugt, ganz besonders bevorzugt bzw. insbesondere ganz besonders bevorzugt für diejenigen Bedeutungen, die bereits in Zusammenhang mit der Beschreibung der Stoffe der Formel (V) für diese Reste als bevorzugt, besonders bevorzugt etc. genannt wurden. A² steht bevorzugt, besonders bevorzugt, ganz besonders bevorzugt bzw. insbesondere ganz besonders bevorzugt für diejenigen Bedeutungen, die bereits in Zusammenhang mit der Beschreibung der Stoffe der Formel (VI) für diese Reste als bevorzugt, besonders bevorzugt etc. genannt wurden.

Die Verbindungen der Formel (VII) sind bekannt oder lassen sich nach bekannten Verfahren herstellen (vgl. J. Org. Chem. 1995, 60, 7508, Tetrahedron Lett. 1997, 38, 3447).

Bei der Durchführung des erfindungsgemäßen Verfahrens (F) setzt man auf 1 Mol an Verbindung der Formel (I-b) im allgemeinen 1 Mol oder einen leichten Überschuss einer Verbindung der Formel (VII) ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch in Essigsäureethylester aufnimmt und die organische Phase mit Wasser wäscht, über Natriumsulfat trocknet, filtriert und einengt. Der Rückstand wird gegebenenfalls nach üblichen Methoden, wie Chromatographie oder Umkristallisation, von eventuell noch vorhandenen Verunreinigungen befreit.

### Verfahren (G)

Die bei der Durchführung des erfindungsgemäßen Verfahrens (G) als Ausgangsstoffe benötigten Δ¹-Pyrroline sind durch die Formel (I-c) allgemein definiert. In dieser Formel stehen R¹, R² und R³ bevorzugt, besonders bevorzugt, ganz besonders bevorzugt bzw, insbesondere ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden. Het⁴ steht bevorzugt für einfach durch R⁵⁻³ substituiertes 5- bis 14-gliedriges, 1 bis 3 aromatische Ringe enthaltendes Heteroaryl mit einem oder mehreren Heteroatomen aus der Reihe Stickstoff, Sauerstoff und Schwefel. Het⁴ steht besonders bevorzugt für einfach durch R⁵⁻³ substituiertes 5- bis 14-gliedriges, 1 bis 3 aromatische Ringe enthaltendes Heteroaryl mit 1 bis 4 Heteroatomen, welches 0 bis 4 Stickstoffatome, 0 bis 2 nicht benachbarte Sauerstoffatome und/oder 0 bis 2 nicht benachbarte Schwefelatome enthält (insbesondere Thienyl, Benzothienyl, Furyl, Benzofuryl, Indolyl, Thienothienyl, Thienofuryl Thienobenzothienyl, Thienobenzofuryl Pyridinyl, Pyrimidinyl, Pyridazinyl, Pyrazinyl, Triazolyl oder Tetrazolyl). Het⁴ steht ganz besonders bevorzugt für einfach durch R⁵⁻³ substituiertes 2-Thienyl, 3-Thienyl, 2-Benzo[b]thienyl, 2-Indolyl, 2-Furyl, 3-Furyl, 2-Benzo[b]furyl, 2-Thieno[3,2-b]-thienyl, 2-Thieno[3,2-b]furyl, 5-Thieno[3,2-b]furyl, 2-Thieno[2,3-f][1]benzothienyl, 2-Thieno[2,3-f][1]benzofuryl, 6-Thieno[2,3-f][1]benzofuryl, 2-Pyridinyl, 3-Pyridinyl, 2-Pyrimidinyl, 5-Pyrimidinyl, 3-Pyridazinyl, 4-Pyridazinyl, 2-Pyrazinyl, Triazolyl oder Tetrazolyl. Het⁴ steht insbesondere ganz besonders bevorzugt für einfach durch R⁵⁻³ substituiertes 2-Thienyl, 3-Thienyl, 2-Benzo[b]thienyl, 2-Furyl, 3-Furyl, 2-Benzo[b]furyl, 2-Thieno[3,2-b]thienyl, 2-Thieno[3,2-b]furyl, 5-Thieno[3,2-b]furyl, 2-Thieno[2,3-f][1]benzothienyl, 2-Thieno[2,3-f][1]benzofuryl, 6-Thieno[2,3-f][1]benzofuryl, 2-Pyridinyl, 3-Pyridinyl, 2-Pyrimidinyl, 5-Pyrimidinyl, 3-Pyridazinyl, 4-Pyridazinyl oder 2-Pyrazinyl. R⁵⁻³ steht bevorzugt für Brom oder Iod.

Δ¹-Pyrroline der Formel (I-c) sind Gegenstand dieser Erfindung und lassen sich nach einem der Verfahren (A), (B) oder (C) herstellen.

Die bei der Durchführung des erfindungsgemäßen Verfahrens (G) als Ausgangsstoffe benötigten metallorganischen Verbindungen sind durch die Formel (VIII) allgemein definiert. In dieser Formel steht E bevorzugt, besonders bevorzugt, ganz besonders bevorzugt bzw. insbesondere ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt etc. genannt wurden. In dieser Formel steht Y¹ bevorzugt, besonders bevorzugt, ganz besonders bevorzugt bzw. insbesondere ganz besonders bevorzugt für diejenigen Bedeutungen, die bereits in Zusammenhang mit der Beschreibung der erfindungsgemäßen Stoffe der Formel (V) für diese Reste als bevorzugt, besonders bevorzugt etc. genannt wurden. M steht bevorzugt für ZnCl, Sn(Me)₃ oder Sn(n-Bu)₃.

Metallorganische Verbindungen der Formel (VIII) sind teilweise bekannt und/oder können nach bekannten Methoden hergestellt werden. Es ist z.B. möglich, Verbindungen der Formel (VIII) aus den entsprechenden Verbindungen der Formel (V), in welchen A¹ für -OSO₂CF₃ steht, in situ herzustellen (vgl. Tetrahedron Lett. 1995, 36, 9085).

Bei der Durchführung des erfindungsgemäßen Verfahrens (G) setzt man auf 1 Mol an Verbindung der Formel (I-c) im allgemeinen 1 Mol oder einen leichten Überschuss einer Verbindung der Formel (VIII) ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch in Essigsäureethylester aufnimmt und die organische Phase mit Wasser wäscht, über Natriumsulfat trocknet, filtriert und einengt. Der Rückstand wird gegebenenfalls nach üblichen Methoden, wie Chromatographie oder Umkristallisation, von eventuell noch vorhandenen Verunreinigungen befreit.

Bei der Durchführung der erfindungsgemäßen Verfahren (D), (E), (F) und (G) setzt man jeweils einen Palladium-Katalysator ein, der wiederum mit oder ohne Zusatz von weiteren Liganden verwendet werden kann. Vorzugsweise verwendet man als Katalysator PdCl₂(dppf) [dppf = 1,1'-Bis(diphenylphosphino)ferrocene], Pd(PPh₃)₄, PdCl₂(PPh₃)₂, PdCl₂(CH₃CN)₂, Pd₂(dba)₃ [dba = Dibenzylidenaceton] oder Pd(Oac)₂, besonders bevorzugt PdCl₂(dppf), Pd(PPh₃)₄, PdCl₂(PPh₃)₂, oder Pd(Oac)₂, ganz besonders bevorzugt PdCl₂(dppf) oder PdCl₂(PPh₃)₂.

Als Liganden kommen Triarylphosphine, Trialkylphosphine oder Arsine in Frage. Vorzugsweise verwendet man dppf, PPh₃, P(t-Bu)₃, Pcy₃ oder AsPh₃, besonders bevorzugt dppf.

Als Verdünnungsmittel kommen bei der Durchführung der erfindungsgemäßen Verfahren (D), (E) und (F) jeweils alle üblichen inerten, organischen Solventien in Frage. Vorzugsweise verwendbar sind gegebenenfalls halogenierte aliphatische, alicyclische oder aromatische Kohlenwasserstoffe, wie Petrolether, Hexan, Heptan, Cyclohexan, Methylcyclohexan, Benzol, Toluol, Xylol oder Decalin; Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid oder Sulfone, wie Sulfolan. Besonders bevorzugt verwendet man Aceton, Dimethoxyethan, Dioxan, Tetrahydrofuran, Dimethylformamid, Dimethylacetamid, Dimethylsulfoxid, Ethanol, Toluol oder gegebenenfalls Gemische dieser genannten Verdünnungsmittel mit Wasser.

Als Verdünnungsmittel kommen bei der Durchführung des erfindungsgemäßen Verfahrens (G) jeweils alle üblichen inerten, organischen Solventien in Frage. Vorzugsweise verwendbar sind gegebenenfalls halogenierte aliphatische, alicyclische oder aromatische Kohlenwasserstoffe, wie Petrolether, Hexan, Heptan, Cyclohexan, Methylcyclohexan, Benzol, Toluol, Xylol oder Decalin; Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol. Besonders bevorzugt verwendet man Dioxan, Tetrahydrofuran oder Toluol.

Als Säurebindemittel kommen bei der Durchführung der erfindungsgemäßen Verfahrens (D), (E), (F) und (G) jeweils alle für derartige Reaktionen üblichen anorganischen und organischen Basen in Betracht Vorzugsweise verwendbar sind Erdalkali- oder Alkalimetallhydroxide, wie Natriumhydroxid, Calciumhydroxid, Kaliumhydroxid, oder auch Ammoniumhydroxid, Alkalimetallcarbonate, wie Natnumcarbonat, Kaliumcarbonat, Kaliumhydrogencarbonat, Natriumhydrogencarbonat, Alkali- oder Erdalkalimetallacetate wie Natriumacetat, Kaliumacetat, Calciumacetat, Alkalimetallfluoride, sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU). Es ist jedoch auch möglich, ohne zusätzliches Säurebindemittel zu arbeiten, oder die Aminkomponente in einem Überschuss einzusetzen, so dass sie gleichzeitig als Säurebindemittel fungiert. Besonders bevorzugt verwendet man Bariumhydroxid, Natriumhydroxid, Kaliumhydroxid, Trikaliumphosphat, Caesiumcarbonat, Kaliumcarbonat, Natriumcarbonat, Kaliumacetat, Triethylamin, Kaliumtert-butanolat, Caesiumfluorid oder Kaliumfluorid.

Die Reaktionstemperaturen können bei der Durchführung der erfindungsgemäßen Verfahren (D), (E) und (F) jeweils in einem größeren Bereich variiert werden. Im allgemeinen arbeitet man bei Temperaturen zwischen 0°C und 140°C, vorzugsweise zwischen 20°C und 120°C, besonders bevorzugt zwischen 60°C und 100°C.

Die Reaktionstemperaturen können bei der Durchführung des erfindungsgemäßen Verfahrens (G) jeweils in einem größeren Bereich variiert werden. Im allgemeinen arbeitet man bei Temperaturen zwischen 0°C und 140°C, vorzugsweise zwischen 20°C und 120°C.

### Chirale Verbindungen der Formel (I-d)

Zur Herstellung chiraler Verbindungen der Formel (I-d) können beispielsweise Δ¹-Pyrroline der Formel (I-e) 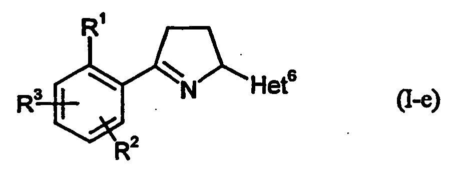 in welcher
- R¹, R² und R³: die oben angegebenen Bedeutungen haben,
- Het⁶: für einfach durch R⁵⁻⁴ substituiertes Heteroaryl steht und
- R⁵⁻⁴: für Chlor, Brom oder Iod steht,
einer Racematspaltung unterzogen werden. Es können aber auch andere erfindungsgemäße Verbindungen der Formel (I) eingesetzt werden. Dabei arbeitet man beispielsweise nach Methoden der präparativen Chromatographie, vorzugsweise nach der Methode der High Performance Liquid Chromatography (HPLC). Dabei wird eine chirale stationäre Kieselgelphase verwendet. Als besonders geeignet für die Trennung der Verbindungen der Formel (I-e) in die beiden Enantiomere hat sich ein mit Tris(3,5-dimethylphenylcarbamat)-cellulose modifiziertes Kieselgel erwiesen. Dieses Trennmaterial ist kommerziell erhältlich. Es ist aber auch möglich, andere stationäre Phasen zu verwenden. Als Eluenten kommen alle üblichen inerten, organischen Solventien sowie Gemische von diesen in Frage. Vorzugsweise verwendbar sind gegebenenfalls halogenierte aliphatische, alicyclische oder aromatische Kohlenwasserstoffe, wie Petrolether, Hexan, Heptan, Cyclohexan; Dichlormethan, Chloroform; Alkohole, wie Methanol, Ethanol, Propanol; Nitrile, wie Acetonitril; Ester wie Essigsäuremethylester oder Essigsäureethylester. Besonders bevorzugt verwendet man aliphatische Kohlenwasserstoffe, wie Hexan oder Heptan, und Alkohole, wie Methanol oder Propanol, ganz besonders bevorzugt n-Heptan und Isopropanol bzw. Gemische von diesen. Im allgemeinen arbeitet man bei Temperaturen zwischen 10°C und 60°C, vorzugsweise zwischen 10°C und 40°C, besonders bevorzugt bei Raumtemperatur.

Δ¹-Pyrroline der Formel (I-e) sind Gegenstand dieser Erfindung und lassen sich nach einem der Verfahren (A), (B) oder (C) herstellen. Die auf diesem Wege erhaltenen (R)-konfigurierten Enantiomere werden dann als Ausgangsstoffe für die Verfahren (D), (F) oder (G) eingesetzt.

Bei der Durchführung aller erfindungsgemäßen Verfahren arbeitet man im allgemeinen unter Atmosphärendruck. Es ist aber auch möglich, jeweils unter erhöhtem oder vermindertem Druck zu arbeiten.

Die erfindungsgemäßen Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spp.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Acheta domesticus, Gryllotalpa spp., Locusta migratoria migratorioides, Melanoplus spp., Schistocerca gregaria.
Aus der Ordnung der Blattaria z.B. Blatta orientalis, Periplaneta americana, Leucophaea maderae, Blattella germanica.
Aus der Ordnung der Dermaptera z.B. Forficula auricularia.
Aus der Ordnung der Isoptera z.B. Reticulitermes spp..
Aus der Ordnung der Phthiraptera z.B. Pediculus humanus corporis, Haematopinus spp., Linognathus spp., Trichodectes spp., Damalinia spp..
Aus der Ordnung der Thysanoptera z.B. Hercinothrips femoralis, Thrips tabaci, Thrips palmi, Frankliniella accidentalis.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.
Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura furniferana, Clysia ambiguella, Homona magnanima, Tortrix viridana, Cnaphalocerus spp., Oulema oryzae.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp..
Aus der Ordnung der Siphonaptera z.B. Xenopsylla cheopis, Ceratophyllus spp..
Aus der Klasse der Arachnida z.B. Scorpio maurus, Latrodectus mactans, Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp..

Die erfindungsgemäßen Verbindungen der Formel (I) zeichnen sich insbesondere durch eine hervorragende Wirkung gegen Raupen, Käferlarven, Spinnmilben, Blattläuse und Minierfliegen aus.

Die erfindungsgemäßen Verbindungen können gegebenenfalls in bestimmten Konzentrationen bzw. Aufwandmengen auch als Herbizide und Mikrobizide, beispielsweise als Fungizide, Antimykotika und Bakterizide verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die erfindungsgemäßen Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der erfindungsgemäßen Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.Z.B.rdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängeln;
als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate;
als Dispergiermittel kommen in Frage: z.B. Lignin-Sulfitablaugen und Methylcellulose.
Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können in handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:
**Fungizide:**
   Aldimorph, Ampropylfos, Ampropylfos-Kalium, Andoprim, Anilazin, Azaconazol, Azoxystrobin,
   Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat, Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyptodinil, Cyproluram,
   Debacarb, Dichlorophen, Diclobutrazol, Diclofluanid, Diclomezin, Dicloran, Diethofencarb, Difenoconazol, Dimethirimol, Dimethomorph, Duniconazol, Diniconazol-M, Dinocap, Diphenylamin, Dipyrithione, Ditalimfos, Dithianon, Dodemorph, Dodine, Drazoxolon,
   Ediphenphos, Epoxiconazol, Etaconazol, Ethirimol, Etridiazol,
   Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox, Guazatin, Hexachlorobenzol, Hexaconazol, Hymexazol,
   Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
   Kasugamycin, Kresoxim-triethyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
   Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
   Nickel-dimethyldithiocarbamat, Nitrothal-isopropyl, Nuarimol,
   Ofurace, Oxadixyl, Oxamocarb, Oxolinicacid, Oxycarboxim, Oxyfenthiin,
   Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,
   Quinconazol, Quintozen (PCNB),
   Schwefel und Schwefel-Zubereitungen,
   Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,
   Uniconazol,
   Validamycin A, Vinclorolin, Viniconazol,
   Zarilamid, Zineb, Ziram sowie
   Dagger G, OK-8705, OK-8801,
   α-(1,1-Dimethylethyl)-β-(2-phenoxyethyl)-1H-1,2,4-triazol-1-ethanol,
   α-(2,4-Dichlolphenyl)-β-fluor-β-propyl-1H-1,2,4-triazol-1-ethanol,
   α-(2,4-Dichlorphenyl)-β-methoxy-α-methyl-1H-1,2,4-triazol-1-ethanol,
   α-(5-Methyl-1,3-dioxan-5-yl)-β-[[4-(trifluormethyl)-phenyl]-methylen]-1H-1,2,4-triazol-1-ethanol,
   (5RS,6RS)-6-Hydroxy-2,2,7,7-tetramethyl-5-(1H-1,2,4-triazol-1-yl)-3-octanon,
   (E)-α-(Methoxyimino)-N-methyl-2-phenoxy-phenylacetamid,
   {2-Methyl-1-[[[1-(4-methylphenyl)-ethyl]-amino]-carbonyl]-propyl}-carbaminsäure-1-isopropylester
   1-(2,4-Dichlorphenyl)-2-(1H-1,2,4-triazol-1-yl)-ethanon-O-(phenylmethyl)-oxim,
   1-(2-Methyl-1-naphthalenyl)-1H-pyrrol-2,5-dion,
   1-(3,5-Dichlorphenyl)-3-(2-propenyl)-2,5-pyrrolidindion,
   1-[(Diiodmethyl)-sulfonyl]-4-methyl-benzol,
   1-[[2-(2,4-Dichlorphenyl)-1,3-dioxolan-2-yl]-methyl]-1H-imidazol,
   1-[[2-(4-Chlorphenyl)-3-phenyloxiranyl]-methyl]-1H-1,2,4-triazol,
   1-[1-[2-[(2,4-Dichlorphenyl)-methoxy]-phenyl]-ethenyl]-1H-imidazol,
   1-Methyl-5-nonyl-2-(phenylmethyl)-3-pyrrolidinol,
   2',6'-Dibrom-2-methyl-4'-trifluormethoxy-4'-trifluor-methyl-1,3-thiazol-5-carboxanilid,
   2,2-Dichlor-N-[1-(4-chlorphenyl)-ethyl]-1-ethyl-3-methyl-cyclopropancarboxamid,
   2,6-Dichlor-5-(methylthio)-4-pyrimidinyl-thiocyanat,
   2,6-Dichlor-N-(4-trifluormethylbenzyl)-benzamid,
   2,6-Dichlor-N-[[4-(trifluormethyl)-phenyl]-methyl]-benzamid,
   2-(2,3,3-Triiod-2-propenyl)-2H-tetrazol,
   2-[(1-Methylethyl)-sulfonyl]-5-(trichlormethyl)-1,3,4-thiadiazol,
   2-[[6-Deoxy-4-O-(4-O-methyl-β-D-glycopyranosyl)-α-D-glucopyranosyl]-amino]-4-methoxy-1H-pyrrolo[2,3-d]pyrimidin-5-carbonitril,
   2-Aminobutan,
   2-Brom-2-(brommethyl)-pentandinitril,
   2-Chlor-N-(2,3-dihydro-1,1,3-trimethyl-1H-inden-4-yl)-3-pyridincarboxamid,
   2-Chlor-N-(2,6-dimethylphenyl)-N-(isothiocyanatomethyl)-acetamid,
   2-Phenylphenol(OPP),
   3,4-Dichlor-1-[4-(difluormethoxy)-phenyl]-1H-pyrrol-2,5-dion,
   3,5-Dichlor-N-[cyan[(1-methyl-2-propynyl)-oxy]-methyl]-benzamid,
   3-(1,1-Dimethylpropyl-1-oxo-1H-inden-2-carbonitril,
   3-[2-(4-Chlorphenyl)-5-ethoxy-3-isoxazolidinyl]-pyridin,
   4-Chlor-2-cyan-N,N-dimethyl-5-(4-methylphenyl)-1H-imidazol-1-sulfonamid,
   4-Methyl-tetrazolo[1,5-a]quinazolin-5(4H)-on,
   8-(1,1-Dimethylethyl)-N-ethyl-N-propyl-1,4-dioxaspiro[4.5]decan-2-medianamin,
   8-Hydroxychinolinsulfat,
   9H-Xanthen-9-carbonsäure-2-[(phenylamino)carbonyl]-hydrazid,
   bis-(1-Methylethyl)-3-methyl-4-[(3-methylbenzoyl)-oxy]-2,5 thiophendicarboxylat,
   cis-1-(4-Chlorphenyl)-2-(1H-1,2,4-triazol-1-yl)-cycloheptanol,
   cis-4-[3-[4-(1,1-Dimethylpropyl)-phenyl-2-methylpropyl]-2,6-dimethyl-morpholinhydrochlorid,
   Ethyl-[(4-chlorphenyl)-azo]-cyanoacetat,
   Kaliumhydrogencarbonat,
   Methantetrathiol-Natriumsalz,
   Methyl-1-(2,3-dihydro-2,2-dimethyl-1H-inden-1-yl)-1H-imidazol-5-carboxylat,
   Methyl-N-(2,6-dimethylphenyl)-N-(5-isoxazolylcarbonyl)-DL-alaninat,
   Methyl-N-(chloracetyl)-N-(2,6-dimethylphenyl)-DL-alaninat,
   N-(2,3-Dichlor-4-hydroxyphenyl)-1-methyl-cyclohexancarboxamid.
   N-(2,6-Dimethyphenyl)-2-methoxy-N-(tetrahydro-2-oxo-3-furanyl)-acetamid,
   N-(2,6-Dimethylphenyl)-2-methoxy-N-(tetrahydro-2-oxo-3-thienyl)-acetamid,
   N-(2-Chlor-4-nitrophenyl)-4-methyl-3-nitro-benzolsulfonamid,
   N-(4-Cyclohexylphenyl)-1,4,5,6-tetrahydro-2-pyrimidinamin,
   N-(4-Hexylphenyl)-1,4,5,6-tetrahydro-2-pyrimidinamin,
   N-(5-Chlor-2-methylphenyl)-2-methoxy N-(2-oxo-3-oxazolidinyl)-acetamid,
   N-(6-Methoxy)-3-pyridinyl)-cyclopropancarboxamid,
   N-[2,2,2-Trichlor-1-[(chloracetyl)-amino]-ethyl]-benzamid,
   N-[3-Chlor-4,5-bis-(2-propinyloxy)-phenyl]-N'-methoxy-methanimidamid,
   N-Formyl-N-hydroxy-DL-alanin -Natriumsalz,
   O,O-Diethyl-[2-(dipropylamino)-2-oxoethyl]-ethylphosphoramidothioat,
   O-Methyl-S-phenyl-phenylpropylphosphoramidothioat,
   S-Methyl-1,2,3-benzothiadiazol-7-carbothioat,
   spiro[2H]-1-Benzopyran-2,1'(3'H)-isobenzofuran]-3'-on,
**Bakterizide:**
   Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, Acephate, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
   Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, BPMC, Bromophos A, Bufencarb, Buprofezin, Butathiofos, Butocarboxim, Butylpyridaben,
   Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Cis-Resmethrin, Cispermethrin, Clocythrin, Cloethocarb, Clofentezine, Cyanophos, Cycloprene, Cycloprothrin, Cyfluthrin, Cyhalothrin, Cyhexatin, Cypermethrin, Cyromazine,
   Deltamethrin, Demeton M, Demeton S, Demeton-S-methyl, Diafenthiuron, Diazinon, Dichlorvos, Diflubenzuron, Dimethoat, Dimethylvinphos, Diofenolan, Disulfoton, Docusat-sodium, Dofenapyn,
   Eflusilanate, Emamectin, Empenthrin, Endosulfan, Entomopfthora spp., Eprinomectin, Esfenvalerate, Ethiofencarb, Ethion, Ethoprophos, Etofenprox, Etoxazole, Etrimfos,
   Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazuron, Flubrocythrinate, Flucycloxuron, Flucythrinate, Flufenoxuron, Flutenzine, Fluvalinate, Fonophos, Fosmethilan, Fosthiazate, Fubfenprox, Furathiocarb,
   Granuloseviren
   Halofenozide, HCH, Heptenophos, Hexaflumuron, Hexythiazox, Hydroprene,
   Imidacloprid, Isazofos, Isofenphos, Isoxathion, Ivermectin,
   Kernpolyederviren
   Lambda-cyhalothrin, Lufenuron
   Malathion, Mecarbam, Metaldehyd, Methamidophos, Metharhizium anisopliae, Metharhizium flavoviride, Methidathion, Methiocarb, Methomyl, Methoxyfenozide, Metolcarb, Metoxadiazone, Mevinphos, Milbemectin, Monocrotophos,
   Naled, Nitenpyram, Nithiazine, Novaluron
   Omethoat, Oxamyl, Oxydemethon M
   Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propoxur, Prothiofos, Prothoat, Pymetrozine, Pyraclofos, Pyresmethrin, Pyrethrum, Pyridaben, Pyridathion, Pyrimidifen, Pyriproxyfen,
   Quinalphos,
   Ribavirin
   Salithion, Sebufos, Selamectin, Silafluofen, Spinosad, Sulfotep, Sulprofos,
   Tau-fluvalinate, Tebufenozide, Tebufenpyrad, Tebupirimiphos, Teflubenzuron, Tefluthrin, Temephos, Temivinphos, Terbufos, Tetrachlorvinphos, Thetacypermethrin, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
   Vamidothion, Vaniliprole, Verticillium lecanii
   YI 5302
   Zeta-cypermethrin, Zolaprofos
   (1R-cis)-[5-(Phenylmethyl)-3-furanyl]-methyl-3-[(dihydro-2-oxo-3(2H)-furanyliden)-methyl]-2,2-dimethylcyclopropancarboxylat
   (3-Phenoxyphenyl)-methyl-2,2,3,3-tetramethylcyclopropanecarboxylat
   1-[(2-Chlor-5-thiazolyl)methyl]tetrahydro-3,5-dimethyl-N-nitro-1,3,5-triazin-2(1H)-imin
   2-(2-Chlor-6-fluorphenyl)-4-[4-(1,1-dimethylethyl)phenyl]-4,5-dihydro-oxazol
   2-(Acetlyoxy)-3-dodecyl-1,4-naphthalindion
   2-Chlor-N-[[[4-(1-phenylethoxy)-phenyl]-amino]-carbonyl]-benzamid
   2-Chlor-N-[[[4-(2,2-dichlor-1,1-difluorethoxy)-phenyl]-amino]-carbonyl]-benzamid
   3-Methylphenyl-propylcarbamat
   4-[4-(4-Ethoxyphenyl)-4-methylpentyl]-1-fluor-2-phenoxy-benzol
   4-Chlor-2-(1,1-dimethylethyl)-5-[[2-(2,6-dimethyl-4-phenoxyphenoxy)ethyl]thio]-3(2H)-pyridazinon
   4-Chlor-2-(2-chlor-2-methylpropyl)-5-[(6-iod-3-pyridinyl)methoxy]-3(2H)-pyridazinon
   4-Chlor-5-[(6-chlor-3-pyridinyl)methoxy]-2-(3,4-dichlorphenyl)-3(2H)-pyridazinon Bacillus thuringiensis strain EG-2348
   Benzoesäure [2-benzoyl-1-(1,1-dimethylethyl)-hydrazid
   Butansäure 2,2-dimethyl-3-(2,4-dichlorphenyl)-2-oxo-1-oxaspiro[4.5]dec-3-en-4-yl-ester
   [3-[(6-Chlor-3-pyridinyl)methyl]-2-thiazolidinyliden]-cyanamid
   Dihydro-2-(nitromethylen)-2H-1,3-thiazine-3(4H)-carboxaldehyd
   Ethyl-[2-[[1,6-dihydro-6-oxo-1-(phenylmethyl)-4-pyrindazinyl]oxy]ethyl]-carbamat N-(3,4,4-Trifluor-1-oxo-3-butenyl)-glycin
   N-(4-Chlorphenyl)-3-[4-(difluormethoxy)phenyl]-4,5-Mydro-4-phenyl-1H-pyrazol-1-carboxamid
   -N-[(2-Chlor-5-thiazolyl)methyl]-N'-methyl-N"-nitro-guanidin
   N-Methyl-N'-(1-methyl-2-propenyl)-1,2-hydrazindicarbothioamid
   N-Methyl-N'-2-propenyl-1,2-hydrazindicarbothioamid
   O,O-Diethyl-[2-(dipropylamino)-2-oxoethyl]-ethylphosphoramidothioat
   Auch eine Mischung mit anderen bekannten Wirkstoffen, wie Herbiziden oder mit Düngemitteln und Wachstumsregulatoren ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der erfindungsgemäßen Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepassten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit bestimmten Eigenschaften ("Traits"), die sowohl durch konventionelle Züchtung, durch Mutagenese oder durch rekombinante DNA-Techniken erhalten worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch über additive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen. Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der. Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus thuringiensis (zZ.B.durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD^{®} (z.B. Mais, Baumwolle, Soja), KnockOut^{®} (z.B. Mais), StarLink^{®} (z.B. Mais), Bollgard^{®} (Baumwolle), Nucotn^{®} (Baumwolle) und NewLeaf^{®} (Kartoffel) vertrieben werden. Als Beispiele für Herbizid tolerante Pflanzen seien Maissorten, Baumwollsorten und Sojasorten genannt, die unter den Handelsbezeichnungen Roundup Ready^{®} (Toleranz gegen Glyphosate z.B. Mais, Baumwolle, Soja), Liberty Link^{®} (Toleranz gegen Phosphinotricin, z.B. Raps), IMI^{®} (Toleranz gegen Imidazolinone) und STS^{®} (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung ClearField^{®} vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoff mischungen behandelt werden. Die bei den Wirkstoffen bzw. Mischungen oben angegebenen Vorzugsbereiche gelten auch für die Behandlung dieser Pflanzen Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der. Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..

Aus der Unterklasse der Acaria (Acarida) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Stemostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

Beispielsweise zeigen sie eine hervorragende Wirksamkeit gegen die Entwicklungsstadien von Zecken wie zum Beispiel Amblyomma hebraeum, gegen parasitierende Fliegen wie zum Beispiel gegen Lucilia cuprina, gegen Flöhe wie zum Beispiel Ctenocephalides felis.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), AufgieBens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die erfindungsgemäßen Wirkstoffe der Formel (I) als Formulierungen (beispielsweise Pulver, Emulsionen, fließfähige Mittel), die die erfindungsgemäßen Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie
   Hylotrupes bajulus, Chlorophorus pilosis, Anobium punctatum, Xestobium rufovillosum, Ptilinus pecticornis, Dendrobium pertinex, Emobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.
Hautflügler wie
   Sirex juvencus, Urocerus gigas, Urocerus gigas taignus, Urocerus augur.
Termiten wie
   Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:

Bauholz, Holzbalken, Eisenbahnschwellen, Brückenteile, Bootsstege, Holzfahrzeuge, Kisten, Paletten, Container, Telefonmasten, Holzverkleidungen, Holzfenster und -t-ren, Sperrholz, Spanplatten, Tischlerarbeiten oder Holzprodukte, die ganz allgemein beim Hausbau oder in der Bautischlerei Verwendung finden.

Die erfindungsgemäßen Wirkstoffe können als solche, in Form von Konzentraten oder allgemein üblichen Formulierungen wie Pulver, Granulate, Lösungen, Suspensionen, Emulsionen oder Pasten angewendet werden.
Die genannten Formulierungen können in an sich bekannter Weise hergestellt werden, z.B. durch Vermischen der erfindungsgemäßen Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige. Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindelöl und/oder Monochlornaphthalin, vorzugswiese α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30°C, vorzugsweise oberhalb 45°C, aufweist und dass das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, zZ.B.Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällen vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, zZ.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.
Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron, Transfluthrin, Thiacloprid, Methoxyphenoxid und Triflumuron, sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflusskrebse) zusammengefasst werden, besondere Bedeutung zu.

Es wurde nun überraschenderweise gefunden, dass die erfindungsgemäßen Verbindungen allein oder in Kombination mit anderen Wirkstoffen, eine hervorragende Antifouling (Antibewuchs)-Wirkung aufweisen.

Durch Einsatz von erfindungsgemäßen Verbindungen allein oder in Kombination mit anderen Wirkstoffen, kann auf den Einsatz von Schwermetallen wie z.B. in Bis-(trialkylzinn)-sulfiden, Tri-*n*-butylzinnlaurat, Tri-*n*-butylzinnchlorid, Kupfer(I)-oxid, Triethylzinnchlorid, Tri-*n*-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-*n*-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoyizinkethylenbisthiocarbamat, Zinkoxid, Kupfer(I)-ethylen-bisdithiocarbamat, Kupferthiocyanat, Kupfemaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:
Algizide wie
   2-*tert*.-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;
Fungizide wie
   Benzo[*b*]thiophencarbonsäurecyclohexylamid-S,S-dioxid, Dichlofluanid, Fluorfolpet, 3-Iod-2-propinyl-butylcarbamat, Tolylfluanid und Azole wie Azaconazole, Cyproconazole, Epoxyconazole, Hexaconazole, Metconazole, Propiconazole und Tebuconazole;
Molluskizide wie
   Fentinacetat, Metaldehyd, Methiocarb, Niclosamid, Thiodicarb und Trimethacarb; oder herkömmliche Antifouling-Wirkstoffe wie 4,5-Dichlor-2-octyl-4-isothiazolin-3-on, Diiodmethylparatrylsulfon, 2-(N,N-Dimethylthiocarbamoylthio)-5-nitrothiazyl, Kalium-, Kupfer-, Natrium- und Zinksalze von 2-Pyridinthiol-1-oxid, Pyridin-triphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinimid.

Die verwendeten Antifouling-Mittel enthalten die erfindungsgemäßen Wirkstoff der erfindungsgemäßen Verbindungen in einer Konzentration von 0,001 bis 50 Gew.-%, insbesondere von 0,01 bis 20 Gew: %.

Die erfindungsgemäßen Antifouling-Mittel enthalten des weiteren die üblichen Bestandteile wie z.B. in Ungerer, Chem. Ind. 1985, 37, 730-732 und Williams, Antifouling Marine Coatings, Noyes, Park Ridge, 1973 beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wässrigen System, Vinylchlorid/Vinylacetat-Copolymersysteme in Form wässriger Dispersionen oder in Form von organischen Lösungsmittelsystemen, Butadien/Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die erfindungsgemäßen Wirkstoffe eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp..
Aus der Ordnung der Chilopoda z.B. Geophilus spp..
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta sustralasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die Herstellung und die Verwendung der erfindungsgemäßen Stoffe geht aus den folgenden Beispielen hervor.

### Herstellungsbeispiele

### Beispiel 1

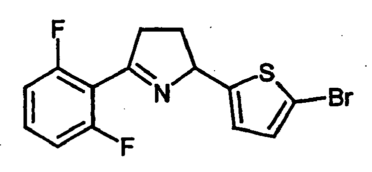

Trifluoressigsäure (5 ml) wird bei 5°C vorgelegt. Eine Lösung aus tert-Butyl-1-(5-brom-2-thienyl)-4-(2,6-difluorphenyl)-4-oxobutylcarbamat (II-1) (1.50 g, 3.1 mmol) in Dichlormethan (10 ml) wird zugetropft. Den Ansatz lässt man auf Raumtemperatur kommen und rührt 3 Stunden bei dieser Temperatur nach. Die flüssige Phase wird unter vermindertem Druck eingeengt und der Rückstand in Essigsäureethylester (50 ml) aufgenommen. Die organische Lösung wird mit 1N Natronlauge (50 ml) gewaschen, filtriert und eingeengt.

Man erhält 0.85 g (75 % d. Th.) an 2-(5-Brom-2-thienyl)-5-(2,6-difluorphenyl)-3,4-dihydro-2H-pyrrol.
- HPLC:: Log P (pH 2.3) = 3.45 (93 %ig)
- NMR (CDCl₃):: δ = 2.05 (1H, m), 2.57 (1H, m), 3.08 (2H, m), 5.50 (1H, m), 6.76 (1H, d), 6.92 (1H, d), 6.97 (2H, m), 7.35 (1H, m) ppm.

Analog Beispiel 1 erhält man folgende Verbindungen:

| Nr. | Verbindung | Log P (pH 2.3) | ¹H-NMR (δ in ppm) |
|---|---|---|---|
| 2 | 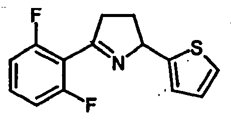 | 1.95 | (CDCl₃) 1.90 (1H, m), 2.55 (1H, m), 3.01 (2H, m), 5.51 (1H, m), 7.02 (2H, d), 7.22 (2H, m), 7.42 (1H, m), 7.56 (1H, m) |
| 3 | 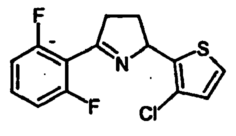 | 3.34 | (CD₃CN) 1.87 (1H, m), 2.69 (1H, m), 3.08 (2H, m), 5.57 (1H, m), 6.98 (1H, d), 7.08 (2H, m), 7.34 (1H, d), 7.48 (1H, m) |
| 4 | 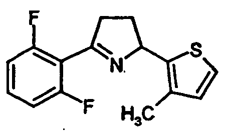 | 2.34 | |
| 5 | 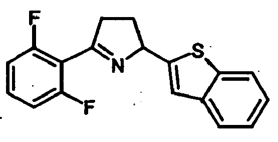 | 3.11 | (CDCl₃) 2.00 (1H, m), 2.63 (1H, m), 3.07 (2H, m), 5.70 (1H, m), 6.94 (2H, m), 7.19-7.37 (4H, m), 7.76 (1H, d), 7.83 (1H, d) |

### Beispiel 6

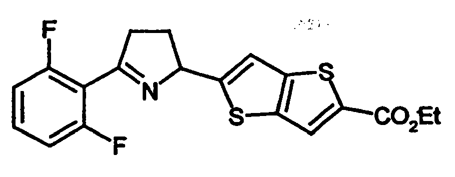

1.1 g (2.16 mmol) Ethyl-5-[1-[(tert-butoxycarbonyl)amino]-4-(2,6-difluorophenyl)-4-oxobutyl]thieno[3,2-b]thiophen-2-carboxylat (II-6) werden in 5 ml Dichlormethan gelöst und bei 0°C in 15 ml Trifluoressigsäure getropft. Man rührt 30 min bei 0°C nach und gibt anschließend 100 ml Wasser zu. Es wird 3 mal mit Dichlormethan extrahiert Die vereinigten organischen Phasen werden 2 mal mit Wasser, 1 mal mit konzentrierter Natriumchloridlösung gewaschen und über Natriumsulfat getrocknet. Anschließend wird über eine 3 g Kieselgelkartusche mit Cyclohexan/Essigsäureethylester (2:1) filtriert und das Lösungsmittel unter vermindertem Druck entfernt. Das orangefarbene Öl wird aus ca. 20 ml Methyl-tert.-butylketon umkristallisiert.

Man erhält 0.26 g (38 % d. Th.) an Ethyl-5-[5-(2,6-difluorophenyl)-3,4-dihydro-2H-pyrrol-2-yl]thieno[3,2-b]thiophene-2-carboxylat in Form von schwach gelben Kristallen.
- Fp.:: 107°C

### Beispiel 7

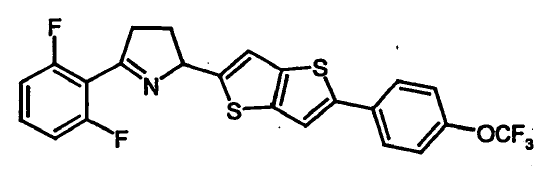

350 mg (0.58 mmol) tert-Butyl-4-(2,6-difluorophenyl)-4-oxo-1-{5-[4-(trifluormethoxy)-phenyl]thieno[3,2-b]thien-2-yl}butylcarbamat (II-7) werden in 5 ml Dichlormethan gelöst und bei 0°C in eine Lösung aus 15 ml Dichlormethan und 5 ml Trifluoressigsäure getropft. Das Reaktionsgemisch wird 5 min nachgerührt und anschließend in 100 ml Eiswasser eingerührt. Die organische Phase wird abgetrennt, die wässrige Phase einmal mit Dichlormethan extrahiert und die organischen Phasen vereinigt. Die organische Phase wird erneut einmal mit Wasser gewaschen, über Natriumsulfat getrocknet und eingeengt. Das Rohprodukt wird anschließend zweimal durch Kartuschen-Chromatographie (1. Cyclohexan / Essigsäureethylester -2. Dichlormethan / Cyclohexan - Gradienten) gereinigt.

Man erhält 0.077g (28% d. Th.) an 5-(2,6-Difluorophenyl)-2-{5-[4-(trifluormethoxy)phenyl]thieno[3,2-b]thien-2-yl}-3,4-dihydro-2H-pyrrol in Form eines gelben Feststoffes.
- Fp.:: 135°C

### Ausgangsstoffe der Formel (II)

### Beispiel (II-I)

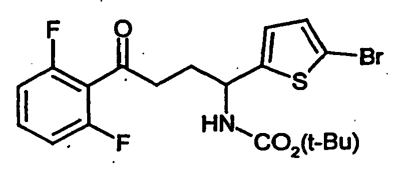

Unter Argonatmosphäre wird -1,3-Difluorbenzol (18.20 g, 0.16 mol) in Tetrahydrofuran (100 ml) bei -65°C vorgelegt. Man tropft n-Butyllithium (100 ml, 1.6 M in Hexan, 0.16 mol) zu und rührt das Reaktionsgemisch bei -65°C für 30 Minuten nach. Eine Lösung aus tert-Butyl-2-(5-brom-2-thienyl)-5-oxo-1-pyrrolidincarboxylat (IX-1) (41.50 g, 0.12 mol) in Tetrahydrofuran (250 ml) wird zugetropft, worauf man das Reaktionsgemisch innerhalb von16 Stunden auf Raumtemperatur kommen lässt. Das Reaktionsgemisch wird in Wasser (500 ml) eingerührt und mit Essigsäureethylester (2 x 400 ml) extrahiert. Die kombinierten organischen Phasen werden über Natriumsulfat getrocknet, filtriert und eingeengt.

Man erhält 45.80 g (76 % d. Th.) an tert-Butyl-1-(5-brom-2-thienyl)-4-(2,6-difluorphenyl)-4-oxobutylcarbamat.
- HPLC:: Log P (pH 2.3) = 4.29 (95 %ig)
- NMR (CDCl₃):: δ = 1.42 (9H, s), 2.26 (2H, m), 2.99 (2H, m), 4.84 (2H, br), 6.72 (1H, d), 6.90 (1H, d), 6.96 (2H, m), 7.39 (1H, m) ppm.

Analog Beispiel (II-1) erhält man folgende Verbindungen:

| Nr. | Verbindung | Log P (pH 2.3) | ¹H-NMR (δ in ppm) |
|---|---|---|---|
| II-2 | 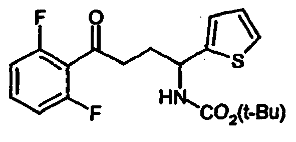 | 4.48 | (CDCl₃) 1.42 (9H, s), 2.31 (2H, m), 2.99 (2H, m), 4.86 (1H, br), 4.98 (1H, br), 6.80-6.93 (3H, m), 7.15-7.25 (3H, m) |
| II-3 | 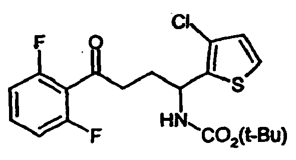 | 4.28 | (CD₃CN) 1.36 (9H, s), 2.15 (2H, m), 2.96 (2H, m), 4.95 (1H, m), 5.85 (1H, br), 6.90 (1H, d), 7.08 (2H, m), 7.35 (1H, d), 7.51 (1H, m) |
| II-4 | 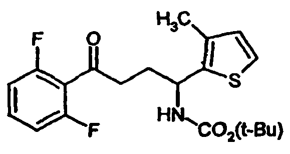 | 4.27 | |
| II-5 | 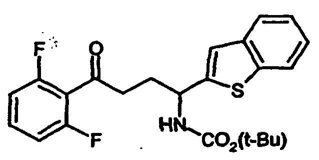 | 4.20 | (CDCl₃) 1.41 (9H, s), 2.42 (2H, m), 3.07 (2H, m), 4.80 (1H, br), 5.15 (1H, br), 6.94 (2H, m), 7.39 (4H, m), 7.89 (2H, m) |

### Beispiel (II-6)

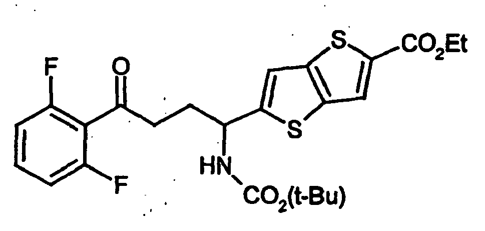

0.35 g (3.06 mmol/1.1 eq.) 1,3-Difluorbenzol werden unter Argon in 20 ml Tetrahydrofuran gelöst. Bei -65°C werden 1.2 ml Butyllithiumlösung (23 %ig in Hexan) zugetropft. Das Reaktionsgemisch wird 10 min bei -30°C -gerührt. Anschließend tropft man eine Lösung aus 1.10 g (2.78 mmol) tert-Butyl-2-[5-(ethoxycarbonyl)-thieno[3,2-b]thien-2-yl]-5-oxo-1-pyrrolidincarboxylat (IX-6) in 20 ml Tetrahydrofuran zu und rührt 30 min bei 20°C nach. Der Ansatz wird auf Raumtemperatur erwärmt, nach 30 min in 120 ml kaltes Wasser eingerührt und mit Methyl-tert.-butylketon extrahiert. Die organische Phase wird jeweils einmal mit Wasser und konzentrierter Natriumchloridlösung gewaschen, über Natriumsulfat getrocknet und eingeengt. Das Rohprodukt wird über eine 3 g-Kieselgelkartusche (Laufmittel Dichlormethan) filtiert.

Man erhält 1.15 g (81 % d. Th.) an Ethyl 5-[1-[(tert-butoxycarbonyl)amino]-4-(2,6-difluorophenyl)-4-oxobutyl]thieno[3,2-b]thiophene-2-carboxylat als braunes Gel.
- Fp.:: 104°C

### Beispiel (II-7)

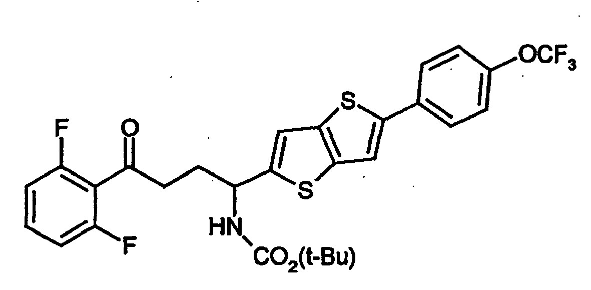

184 mg (1.62 mmol/1.1 eq.) 1,3-Difluorbenzol werden unter Argon in 20 ml Tetrahydrofuran gelöst und bei -65°C 0.7 ml Butyllithiumlösung (23 %-ig in Hexan) zugetropft. Der Ansatz wird 10 min bei -30°C gerührt. Anschließend tropft man eine Lösung aus 710 mg (1.47 mmol) tert-Butyl-2-oxo-5-{5-[4-(trifluormethoxy)phenyl]-thieno[3,2-b]thien-2-yl}-1-pyrrolidincarboxylat (IX-7) in 5 ml Tetrahydrofuran zu und rührt 30 min bei -20°C. Der Ansatz wird auf Raumtemperatur erwärmt und nach 30 min unter Argon mit 60 ml Wasser verdünnt und mit Methyl-tert.-butylketon extrahiert. Die organische Phase wird jeweils einmal mit Wasser und konzentrierter Natriumchloridlösung gewaschen, über Natriumsulfat getrocknet und eingeengt. Das Rohprodukt wird über eine 3 g-Kieselgelkartusche mit Dichlormethan als Laufmittel filtiert.

Man erhält 0.24 g (28 % d. Th.) an tert-Butyl-4-(2,6-difluorphenyl)-4-oxo-1-{5-[4-(trifluormethoxy)phenyl]thieno[3,2-b]thien-2-yl}butylcarbamat als gelben Feststoff.
- Fp.:: 128-130°C

### Ausgangsstoffe der Formel (IX)

### Beispiel (IX-1)

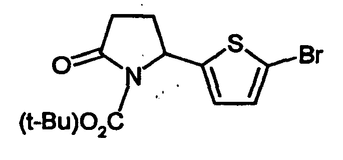

5-(5-Brom-2-thienyl)-2-pyrrolidinon (XI-1) (3.26 g, 94 %ig, ≈ 0.012 mol) wird in Dichlormethan (65 ml) bei 0°C vorgelegt. Nacheinander werden Di-tert-butyldicarbonat (3.00 g, 0.014 mol) und 4-Dimethylaminopyridin (92 mg, 0.75 mmol) zugegeben. Der Ansatz wird 24 Stunden bei Raumtemperatur nachgerührt, worauf man erneut Di-tert-butyldicarbonat (0.65 g, 3.0 mmol) und 4-Dimethylaminopyridin (10 mg, 0.08 mmol) zugibt. Der Ansatz wird weitere 4 Stunden nachgerührt. Die organische Lösung wird nacheinander mit 1N Salzsäure, gesättigter wässriger Natriumhydrogencarbonatlösung, Wasser und Kochsalzlösung gewaschen, über Natriumsulfat getrocknet, filtriert und eingeengt.

Man erhält 4.10 g (95 % d. Th.) an tert-Butyl-2-(5-brom-2-thienyl)-5-oxo-1-pyrrolidincarboxylat.
- HPLC:: Log P (pH 2.3) = 3.03 (96 %ig)
- Fp.:: 93°C
- NMR (CDCl₃):: δ = 1.43 (9H, s), 2.03-2.09 (1H, m), 2.43-2.58 (2H, m), 2.75 (1H, m) 5.37 (1H, m), 6.72 (1H, d), 6.92 (1H, d) ppm.

Analog Beispiel (IX-1) erhält man folgende Verbindungen:

| Nr. | Verbindung | Fp. | Log P | ¹H-NMR (δ in ppm) |
|---|---|---|---|---|
| IX-2 | 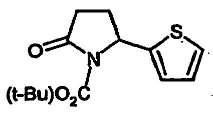 | | 2.39 | (CDCl₃) 1.39 (9H, s), 2.05-2.12 (1H, m), 2.48-2.58 (2H, m), 2.77 (1H, m) 5.47 (1H, m), 6.95 (2H, m), 7.23 (1H, m) |
| | | | (pH 2.3) | |
| IX-3 | 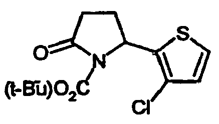 | | 2.77 | (CDCl₃) 1.36 (9H, s), 2.00 (1H, m), 2.47-2.60 (2H, m), 2.70-2.80 (1H, m) 5.55 (1H, m), 6.91 (1H, d), 7.21 (1H, d) |
| | | | (pH 7.5) | |
| IX-4 | 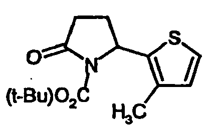 | | 2.65 | (CDCl₃) 1.36 (9H, s), 1.98 (1H, m), 2.48-2.59 (2H, m), 2.77 (1H, m) 5.55 (1H, m), 6.91 (1H, d), 7.21 (1H, d) |
| | | | (pH 2.3) | |
| IX-5 | 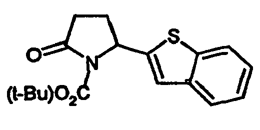 | 171°C | 3.03 | (CD₃CN) 1.20 (9H, s), 1.93 (1H, m), 2.45-2.63 (3H, m), 5.60 (1H, m), 7.30 (1H, s) 7.42 (2H, m), 7.82 (1H, d), 7.95 (1H, d) |
| | | | (pH 2.3) | |

### Beispiel (IX-6)

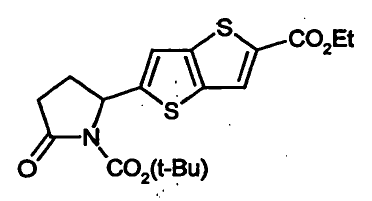

Man löst 2.00 g (6.77 mmol) Ethyl 5-(5-oxo-2-pyrrolidinyl)thieno[3,2-b]thiophene-2-carboxylat (XI-6) unter Argon in 40 ml Dichlormethan und gibt bei 0°C nacheinander 3.00 g (13.7 mmol/2 eq.) Di-tert.-butyldicarbonat, 0.84 g (6.9 mmol/ 1,02 eq.) 4-Dimethylaminopyridin und 9.0 ml Triethylamin zu. Es wird 60 min bei 0°C nachgerührt. Der Ansatz wird mit 60 ml Dichlormethan verdünnt, zweimal mit je 100 ml wässriger Salzsäure (0.25 N), einmal mit Wasser, einmal mit konzentrierter Natriumchloridlösung gewaschen, über Natriumsulfat getrocknet und eingeengt. Anschließend wird das Rohprodukt in 10 ml Methyl-tert.-butylketon bei ca. 40°C verrührt, abgesaugt und getrocknet.

Man erhält 2.3 g (86 % d. Th.) an tert-Butyl-2-[5-(ethoxycarbonyl)thieno[3,2-b]thien-2-yl]-5-oxo-1-pyrrolidin-carboxylat als hellbraunen Feststoff.
- Fp.:: 178°C

### Beispiel (IX-7)

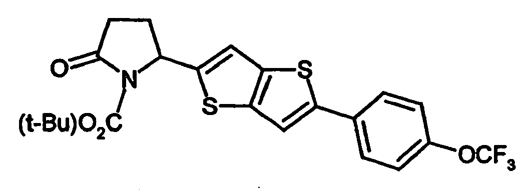

Man löst 6.6 g (8.94 mmol, 52 %ig) 5-{5-[4-(Trifluoromethoxy)phenyl]thieno[3,2-b]thien-2-yl}-2-pyrrolidinon (XI-7) unter Argon in 120 ml Dichlormethan und gibt bei 0°C nacheinander 7.5 g (34.2 mmol/2 eq.) Di-tert.-butyldicarbonat, 2.2 g (17.5 mmol/1.02 eq.) 4-Dimethylaminopyridin und 23 ml Triethylamin zu. Es wird 2 Stunden bei 0°C nachgerührt. Der Ansatz wird mit 60 ml Dichlormethan verdünnt, zweimal mit je 50 ml wässriger Salzsäure (0.25 N), einmal mit Wasser, einmal mit konzentrierter Natriumchloridlösung gewaschen und anschließend über Natriumsulfat getrocknet. Die so erhaltene Lösung wird über Kieselgel mit Dichlormethan filtriert und das Lösungsmittels anschließend entfernt.

Man erhält 4.1 g (95 % d. Th.) an tert-Butyl-2-oxo-5-{5-[4-(trifluormethoxy)phenyl]-thieno[3,2-b]thien-2-yl}-1-pyrrolidincarboxylat in Form eines grauen Feststoffs.
- Fp.:: 164°C

### Ausgangsstoffe der Formel (XI)

### Beispiel (XI-1)

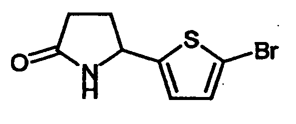

Ein Gemisch aus Eisessig (15 ml) und konzentrierter Schwefelsäure (5 ml) wird vorgelegt und auf 0°C gekühlt. Nacheinander werden 5-Ethoxy-pyrrolidin-2-on (3.04 g, 85 %ig, ≈ 0.02 mol) und 2-Bromthiophen (13.12 g, 0.08 mol) zugegeben. Man lässt das Reaktionsgemisch langsam auf Raumtemperatur erwärmen und rührt anschließend 60 Stunden bei dieser Temperatur nach. Der Ansatz wird in Eiswasser eingegossen und mit Essigsäureethylester extrahiert. Die organische Phase wird nacheinander mit gesättigter wässriger Natriumhydrogencarbonatlösung, Wasser und Kochsalzlösung gewaschen, über Natriumsulfat getrocknet, filtriert und eingeengt.

Man erhält 3.94 g (75 % d. Th.) an 5-(5-Brom-2-thienyl)-2-pyrrolidinon.
- HPLC:: Log P (pH 2.3) =1.62 (94 %ig)
- Fp.:: 104°C
- NMR (CDCl₃):: δ = 2.10 (1H, m), 2.34-2.66 (3H, m), 4.94 (1H, m), 6.01 (1H, br), 6.75 (1H, d), 6.91 (1H, d) ppm.

Analog Beispiel (IX-1) erhält man folgende Verbindungen:

| Nr. | Verbindung | Fp. (°C) | Log P (pH 2.3) | ¹H-NMR (δ in ppm) |
|---|---|---|---|---|
| XI-2 | 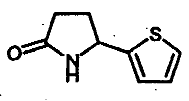 | 111: | 1.03 | (CDCl₃) 2.14 (1H, m), 2.36-2.63 (3H, m), 5.04 (1H, m), 6.15 (1H, br), 6.98 (2H, m), 7.26 (1H, m) |
| XI-3 | 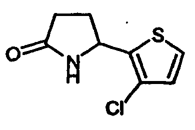 | 122 | 1.41 | (CDCl₃) 2.14 (1H, m), 2.38-2.73 (3H, m), 5.16 (1H, m), 6.00 (1H, br), 6.91 (1H, d), 7.25 (1H, d) |
| XI-4 | 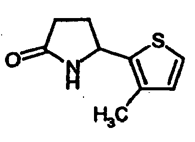 | 104 | 1.33 | (CDCl₃) 2.12 (1H, m), 2.44-2.60 (3H, m), 5.10 (1H, m), 6.15 (1H, br), 6.81 (1H, d), 7.25 (1H, d) |
| XI-5 | 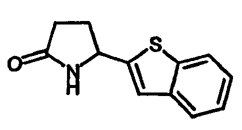 | 115 | 1.73 | (CD₃CN) 2.03 (1H, m), 2.33 (2H, m), 2.66 (1H, m), 5.14 (1H, m), 6.45 (1H, br), 7.42 (3H, m), 7.83 (1H, d), 7.94 (1H, d) |

### Beispiel (XI-6)

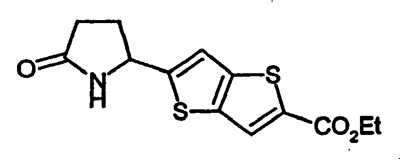

1.2 g (9.42 mmol) 5-Ethoxy-2-pyrrolidinon werden in 15 ml Eisessig und 5 ml konzentrierter Schwefelsäure vorgelegt. Bei einer Temperatur kleiner als 10°C werden 1-0 g (4.71 mmol) Ethyl-thieno[3,2-b]thiophen-2-carboxylat zugegeben und 40 h bei Raumtemperatur nachgerührt. Der Ansatz wird, in 150 ml kaltes Wasser eingerührt und dreimal mit je 25 ml Dichlormethan extrahiert, Die vereinigten organischen Phasen werden zweimal mit Wasser und einmal mit konzentrierter Natriumchloridlösung gewaschen, getrocknet und eingeengt. Das gelbe Rohprodukt wird in ca. 20 ml Methyl-tert.-butylketon verrührt. Der Rückstand wird abgesaugt und getrocknet.

Man erhält 1.0 g (72 % d. Th.) an Ethyl-5-(5-oxo-2-pyrrolidinyl)thieno[3,2-b]thiophen-2-carboxylat in Form von cremefarbenen Kristallen.
- Fp.:: 140°C

### Beispiel (XI-7)

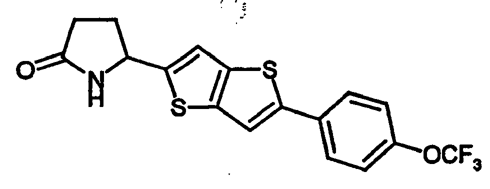

5.3 g (40.6 mmol) 5-Ethoxy-2-pyrrolidinon werden in 80 ml Eisessig und 27 ml konzentrierter Schwefelsäure vorgelegt. Man gibt bei einer Temperatur kleiner als 10°C 6.1 g (20.3 mmol) 2-[4-(Trifluormethoxy)phenyl]thieno[3,2-b]thiophen (XIII-1) zu und rührt für 16 Stunden bei Raumtemperatur nach. Der Ansatz wird in 300 ml kaltes Wasser eingerührt und dreimal mit je 60 ml Dichlormethan extrahiert. Die organischen Phasen werden vereinigt, zweimal mit Wasser und einmal mit konzentrierter Natriumchloridlösung gewaschen, über Natriumsulfat getrocknet und eingeengt. Anschließend wird das Rohprodukt in 60 ml Methyl-tert.-butylketon bei ca. 40°C verrührt, abgekühlt, abgesaugt und getrocknet.

Man erhält 6.8 g (87 % d. Th.) an 5-{5-[4-(Trifluormethoxy)phenyl]thieno[3,2-b]-thien-2-yl}-2-pyrrolidinon als hellgrauen Feststoff.
- Fp.:: 220°C (Zersetzung)

### Ausgangsstoffe der Formel (XIII)

### Beispiel (XIII-1)

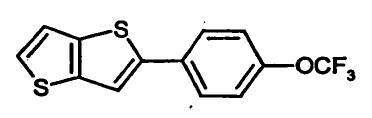

Man löst 29.0 g (25.5 mmol) 2-Bromthieno[3,2-b]thiophen unter Argon in 150 ml Tetrahydrofuran, gibt bei Raumtemperatur 10.0 g (48.5 mmol) 4-Trifluormethoxyphenylboronsäure und 0.8 g (0.7 mmol) Tetrakis-(triphenylphosphan)-palladium(0) zu und erhitzt für 1 Stunde unter Rückfluss. Anschließend werden weitere 2.0 g 4-Trifluormethoxyphenylboronsäure und 0.2 g Tetrakis-(triphenylphosphan)-palladium(0) nachdosiert und für weitere 16 Stunden unter Rückfluss gerührt. Anschließend werden bei dieser Temperatur insgesamt 100 ml einer 20 %-igen wässrigen Natriumcarbonatlösung zugetropft und 1 Stunde unter Rückfluss erhitzt. Das Reaktionsgemisch wird mit 400 ml Wasser verdünnt und dreimal mit je 100 ml Methyl-tert.-butylketon extrahiert. Die organischen Phasen werden vereinigt, je einmal mit Wasser und konzentrierter Natriumchloridlösung gewaschen, über Natriumsulfat getrocknet und eingeengt Das Rohprodukt wird auf ca. 25 g Kieselgel aufgezogen und durch Flash-Chromatographie mit n-Hexan als Laufmittel aufgereinigt.

Man erhält 6.2 g (81 % d. Th.) an 2-[4-(Trifluormethoxy)phenyl]thieno[3,2-b]thiophen in Form eines weißen Feststoffs.
- Fp.:: 162°C

### Beispiel 8

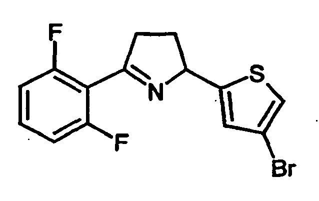

4-Azido-4-(4-brom-2-thienyl)-1-(2,6-difluorphenyl)-1-butanon (III-1) (1.63 g, 42 %ig, ≈ 1.79 mmol) wird in n-Pentan (100 ml) vorgelegt. Bei Raumtemperatur gibt man Triphenylphosphin (0.65 g, 2.50 mmol) portionsweise zu. Der Ansatz wird 16 Stunden bei Raumtemperatur nachgerührt. Nach Zugabe von Florisil (5 g) wird der Ansatz zur Trockne eingeengt. Das Rohprodukt wird mittels Chromatographie an Kieselgel (Laufmittel: Cyclohexan / Essigsäureethylester 9:1 **→** 7:3) aufgereinigt.

Man erhält 0.15 g (37 % d. Th.) an 2-(4-Brom-2-thienyl)-5-(-2,6-difluorphenyl)-3,4-dihydro-2H-pyrrol.
- HPLC:: Log P (pH 2.3) = 3.41 (100 %ig)
- NMR (CD₃CN):: δ = 1.95 (1H, m), 2.60 (1H, m), 3.05-3.08 (2H, m), 5.47 (1H, m), 6.96 (1H, s), 7.07 (2H, m), 7.27 (1H, s), 7.44-7.47 (1H, m) ppm.

Eine weitere Charge (0.15 g, 78 %ig) wurde ebenfalls isoliert.

### Beispiel 9

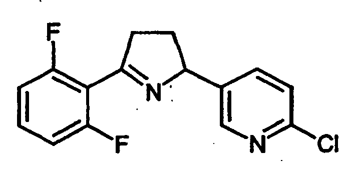

4-Azido-4-(6-chlor-3-pyridinyl)-1-(2,6-difluorphenyl)-1-butanon (III-2) (1.20 g, 54.4 %ig, 1.94 mmol) wird in n-Hexan (100 ml) vorgelegt. Bei Raumtemperatur gibt man Triphenylphosphin (0.94 g, 3.56 mmol) portionsweise zu. Der Ansatz wird 16 Stunden bei Raumtemperatur nachgerührt. Nach Zugabe von Florisil (7 g) wird das Reaktionsgemisch zur Trockne eingeengt. Das Rohprodukt wird mittels Chromatographie an Kieselgel (Laufmittel: Cyclohexan / Essigsäureethylester 9:1 → 7:3) aufgereinigt.

Man erhält 0.47 g (83 % d. Th.) an 2-(6-Chlor-3-pyridinyl)-5-(2,6-difluorphenyl)-3,4-dihydro-2H-pyrrol.
- HPLC:: Log P (pH 2.3) =1.93 (100 %ig)
- NMR (CD₃CN):: δ = 1.77-1.84 (1H, m), 2.60-2.66 (1H, m), 3.05-3.09 (2H, m), 5.32 (1H, m), 7.07 (2H, m), 7.38 (1H, d), 7.47 (1H, m), 7.69 (1H, dd), 8.37 (1H, d) ppm.

### Beispiel 10

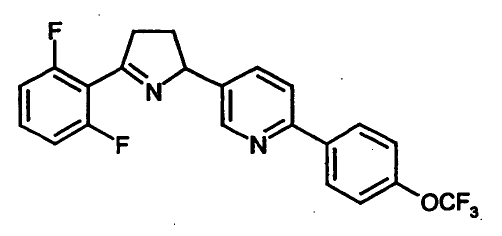

4-Azido-1-(2,6-difluorphenyl)-4-{6-[4-(trifluormethoxy)phenyl]-3-pyridinyl}-1-butanon (III-3) (0.80 g, 1.73 mmol) wird in n-Hexan (100 ml) vorgelegt. Man gibt man Triphenylphosphin (0.45 g, 1.73 mmol) zu. Der Ansatz wird 16 Stunden bei Raumtemperatur nachgerührt. Nach Zugabe von Florisil (5 g) wird das Reaktionsgemisch eingeengt. Das Rohprodukt wird mittels Chromatographie an Kieselgel (Laufmittel: Hexan / Essigsäureethylester 9:1 → 4:1) aufgereinigt.

Man erhält 0.42 g (50 % d. Th.) an 5-[5-(2,6-Difluorphenyl)-3,4-dihydro-2H-pyrrol-2-yl]-2-[4-(trifluormethoxy)phenyl]pyridin.
- HPLC:: Log P (pH 2.3) = 3.50 (86 %ig)

### Ausgangsstoffe der Formel (III)

### Beispiel (III-1)

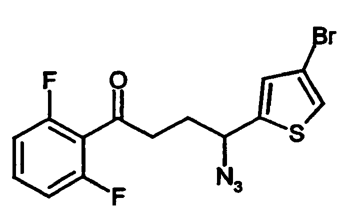

4-(4-Brom-2-thienyl)-4-chlor-1-(2,6-difluorphenyl)-1-butanon (XV-1) (0.95 g, 84.65 %ig, ≈ 2.1 mmol) wird in Aceton (4 ml) vorgelegt. Nacheinander gibt man eine Lösung von Natriumazid (0.25 g, 3.75 mmol) in Wasser (10 ml) und Methyl-tri-n-octylammoniumchlorid (0.30 g, 0.74 mmol) zu. Der Ansatz wird 16 Stunden bei 50°C nachgerührt. Das Reaktionsgemisch wird mit Wasser (30 ml) versetzt und mit Essigsäureethylester (2 x 50 ml) extrahiert. Die kombinierten organischen Phasen werden über Natriumsulfat getrocknet, filtriert und eingeengt. Das Rohprodukt wird ohne Reinigung weiter umgesetzt.

Man erhält 1.63 g (85 % d. Th.) an 4-Azido-4-(4-brom-2-thienyl)-1-(2,6-difluorphenyl)-1-butanon
- HPLC:: Log P (pH 2.3) = 3.00 (42 %ig)
- ν̃ Azid:: 2090 cm⁻¹

### Beispiel (III-21)

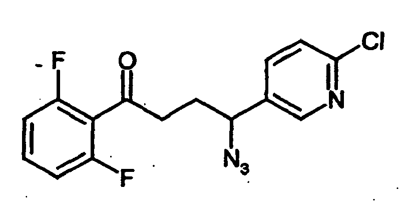

4-(6-Chlor-3-pyridinyl)-4-chlor-1-(2,6-difluorphenyl)-1-butanon (XV-2) (1.30g, 57.6 %ig, 2.27 mmol) wird in Aceton (7.5 ml) vorgelegt. Nacheinander gibt man eine Lösung von Natriumazid (0.38 g, 5.90 mmol) in Wasser (15 ml) und Methyl-tri-n-octylammoniumchlorid (0.30 g, 0.74 mmol) zu. Der Ansatz wird 16 Stunden bei 50°C nachgerührt. Man versetzt das Reaktionsgemisch mit Wasser (30 ml) und extrahiert mit Essigsäureethylester (2 × 50 ml). Die kombinierten organischen Phasen werden über Natriumsulfat getrocknet, filtriert und eingeengt. Das Rohprodukt wird ohne Reinigung weiter umgesetzt.

Man erhält 1.20 g (85 % d. Th.) an 4-Azido-4-(6-chlor-3-pyridinyl)-1-(2,6-difluorphenyl)-1-butanon.
- HPLC:: Log P (pH 2.3) = 3.33 (54 %ig)
- ν̃ Azid:: 2080 cm⁻¹

### Beispiel (III-3)

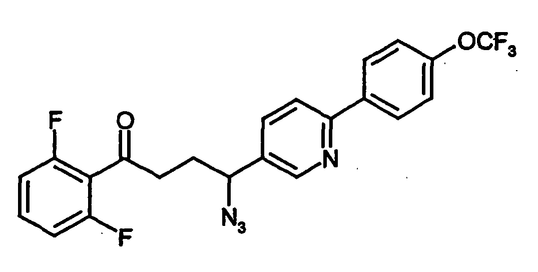

4-Chlor-1-(2,6-difluorophenyl)-4-{6-[4-(trifluormethoxy)phenyl]-3-pyiridinyl}-1-butanon (XV-3) (0.81 g, 1.78 mmol) wird in Aceton (5 ml) vorgelegt. Nacheinander gibt man eine Lösung von Natriumazid (0.17 g, 2.67 mmol) in Wasser (10 ml) und Methyl-tri-n-octylammoniumchlorid zu. Der Ansatz wird 16 Stunden bei 50°C nachgerührt. Man versetzt das Reaktionsgemisch mit Wasser und extrahiert zweimal mit Essigsäureethylester. Die kombinierten organischen Phasen werden über Natriumsulfat getrocknet, filtriert und eingeengt.

Man erhält 0.80 g (61 % d. Th.) an 4-Azido-1-(2,6-difluorphenyl-4-{6-[4-(trifluormethoxy)phenyl]-3-pyridinyl}-1-butanon.
- HPLC:: Log P (pH 2.3) = 4.78 (63 %ig)

### Ausgangsstoffe der Formel (XV)

### Beispiel (XV-1)

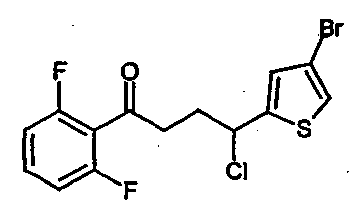

[2-(4-Brom-2-thienyl)cyclopropyl](2,6-difluorphenyl)methanon (XVII-1) (1.40 g, 82 %ig, ≈ 3.4 mmol) wird mit konzentrierter Salzsäure (10 ml) 72 Stunden bei Raumtemperatur gerührt. Dichlormethan wird zugegeben und die organische Phase mit Wasser gewaschen, über Natriumsulfat getrocknet, filtriert und eingeengt. Das Rohprodukt wird ohne Reinigung weiter umgesetzt.

Man erhält 1.27 g (83% d. Th.) an 4-(4-Brom-2-thienyl)-4-chlor-1-(2,6-difluorphenyl)-1-butanon.
- HPLC:: Log P (pH 2.3) = 3.00 (85 %ig)
- NMR (CD₃CN):: δ = 2.48 (2H, m), 3.08 (2H, m), 5.36 (1H, m), 7.00-7.12 (3H, m), 7.40 (1H, s), 7.47-7.57 (1H, m) ppm.

### Beispiel (XV-2)

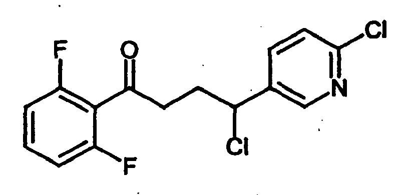

[2-(6-Chlor-3-pyridinyl)cyclopropyl](2,6-difluorphenyl)methanon (XVII-2) (2.20 g, 7.50 mmol) wird mit konzentrierter Salzsäure (22 ml) 60 Stunden bei Raumtemperatur gerührt. Das Reaktionsgemisch wird unter vermindertem Druck eingeengt. Das Rohprodukt wird ohne Reinigung weiter umgesetzt.

Man erhält 2.10 g (49 % d. Th.) an 4-(6-Chlor-3-pyridinyl)-4-chlor-1-(2,6-difluorphenyl)-1-butanon.
- HPLC:: Log P (pH 2.3) = 3.37 (58 %ig)

### Beispiel (XV-3)

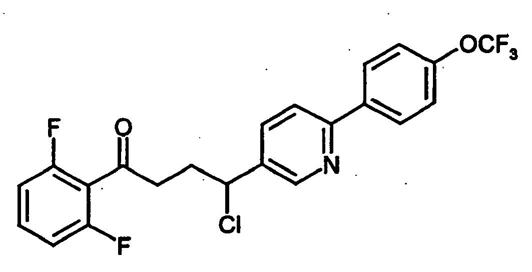

(2,6-Difluorphenyl)(2-{6-[4-(trifluormethoxy)phenyl]-3-pyridinyl}cyclopropyl)methanon (XVII-3) (0.75 g, 1.79 mmol) wird mit konzentrierter Salzsäure (8.00 ml) 60 Stunden bei Raumtemperatur gerührt. Das Reaktionsgemisch wird unter vermindertem Druck eingeengt.

Man erhält 0.81 g (85 % d. Th.) an 4-Chlor-1-(2,6-difluorphenyl)-4-{6-[4-(trifluormethoxy)phenyl]-3-pyridinyl}-1-butanon.
- HPLC:: Log P (pH 2.3) = 4.88 (86 %ig)

### Ausgangsstoffe der Formel (XVII)

### Beispiel (XVII-1)

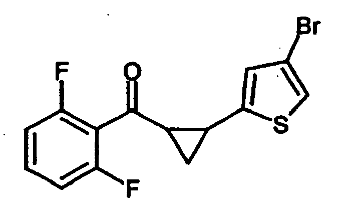

Natriumhydrid (0.40 g, 0.01 mol, 60 %ige Suspension in Parafinöl) wird in DMSO (15 ml) suspendiert. Trimethylsulfoxoniumiodid (2.20 g, 0.01 mol) wird bei Raumtemperatur portionsweise zugegeben. Das Reaktionsgemisch wird anschließend 0.5 Stunden bei Raumtemperatur nachgerührt. Eine Lösung aus 3-(4-Brom-2-thienyl)-1-(2,6-difluorphenyl)-2-propen-1-on (XVIII-1) (2.30 g, 7.0 mmol) in Dimethylsulfoxid (15 ml) wird bei Raumtemperatur langsam zugetropft, worauf für weitere 16 Stunden bei Raumtemperatur nachgerührt wird. Der Ansatz wird in Eiswasser gegossen und zweimal mit Essigsäureethylester extrahiert. Die kombinierten organischen Phasen werden über Natriumsulfat getrocknet, filtriert und eingeengt. Das Rohprodukt wird ohne Reinigung weiter umgesetzt.

Man erhält 1.86 g (63 % d. Th.) an [2-(4-Brom-2-thienyl)cyclopropyl](2,6-difluorphenyl)methanon.
- HPLC:: Log P (pH 2.3) = 4.14 (82.0 %ig)
- NMR (CD₃CN):: δ = 1.59 (1H, m), 1.87 (1H, m), 2.73 (1H, m), 2.84 (1H, m), 6.88 (1H, s), 7.08 (2H, m), 7.19 (1H, s), 7.54 (1H, m) ppm.

### Beispiel (XVII-2)

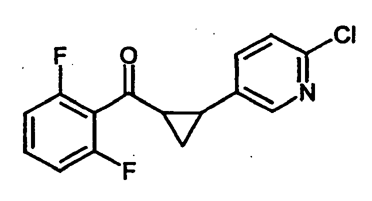

Natriumhydrid (3.19 g, 79.84 mmol, 60 %ige Suspension in Parafinöl) wird in Dimethylsulfoxid (100 ml) suspendiert. Trimethylsulfoxoniumiodid (17.57 g, 79.84-mmol) wird portionsweise bei einer Temperatur von kleiner oder gleich 32°C zugegeben. Das Reaktionsgemisch wird anschließend 1.5 Stunden bei Raumtemperatur nachgerührt. Eine Lösung aus 3-(6-Chlor-3-pyndinyl)-1-(2,6-difluorphenyl)-2-propen-1-on (XVIII-2) (20.30 g, 72.59 mmol) in DMSO (120 ml) wird bei einer Temperatur von kleiner oder gleich 36°C langsam zugetropft. Der Ansatz wird anschließend für 16 Stunden bei Raumtemperatur nachgerührt. Man gießt das Reaktionsgemisch in Eiswasser und saugt den Niederschlag ab.

Man erhält 22.70 g (97 % d. Th.) an [2-(6-Chlor-3-pyridinyl)cyclopropyl](2,6-difluorphenyl)methanon.
- HPLC:: Log P (pH 2.3) = 2.92 (91 %ig)
- NMR (CD₃CN):: δ = 1.63 (1H, m), 1.91 (1H, m), 2.73 (2H, m), 7.07 (2H, m), 7.32 (2H, d), 7.50-7.55 (3H, m), 8.28 (1H, d) ppm.

### Beispiel (XVII-3)

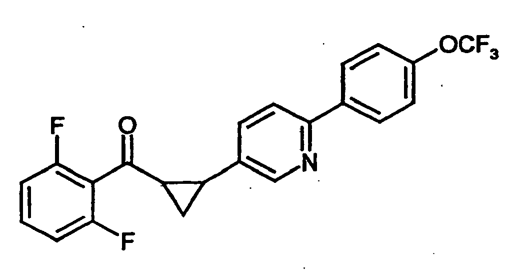

Zu einer Lösung von [2-(6-Chlor-3-pyridinyl)cyclopropyl](2,6-(difluorphenyl)-methanon (XVII-2) (1.47g, 5.00 mmol) in 20 ml Dimethoxyethan gibt man nacheinander 4,4,5;5-Tetramethyl-2-[4-(trifluormethoxy)phenyl]-1,3,2-dioxaborolan (1.73g, 6.00 mmol), Bis(diphenylmethylphosphin)palladium(II)chlorid (0.11 g, 0.15 mmol) und Natriumcarbonat-Lösung (7.50 ml, 2 M) zu und lässt das Reaktionsgemisch anschließend für 16 Stunden bei 80°C reagieren. Zur Aufarbeitung wird das Reaktionsgemisch mit Wasser versetzt und der entstandene Niederschlag abgesaugt. Der Niederschlag wird in Dichlormethan aufgenommen, 10g Florisil zugesetzt und anschließend durch Chromatographie an Kieselgel (Laufmittel: Hexan / Essigsäureethylester 9:1, v/v) aufgereinigt.

Man erhält 1.09 g (51 % d. Th.) an (2,6-Difluorphenyl)(2-{6-[4-(trifluormethoxy)-phenyl]-3-pyridinyl}cyclopropyl)methanon.
- HPLC:: Log P (pH 2.3) = 4.40 (98 %ig)

### Ausgangsstoffe der Formel (XVIII)

### Beispiel (XVIII-1)

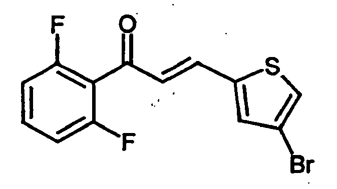

2,6-Difluoracetophenon (1.56 g, 0.01 mol) und 4-Bromthiophen-2-carboxaldehyd. (1.91 g, 0.01 mol) werden in Methanol (70 ml) und Wasser (16ml) vorgelegt. Bei Raumtemperatur wird Natronlauge (3 ml, 10 % w/v) langsam zugetropft, worauf man für 16 Stunden bei Raumtemperatur nachrührt. Nach Abkühlen auf 0°C wird der Niederschlag abgesaugt und mit eiskaltem Methanol-Wasser (3:1)-Gemisch nachgewaschen.

Man erhält 2.49 g (76 % d. Th.) an 3-(4-Brom-2-thienyl)-1-(2,6-difluorphenyl)-2-propen-1-on.
- HPLC:: Log P (pH 2.3) = 3.78 (100 %ig)
- NMR (CD₃CN):: δ = 6.89 (1H, d), 7.11 (2H, m), 7.40 (1H, s), 7.54-7.59 (3H, m) ppm.

### Beispiel (XVIII-2)

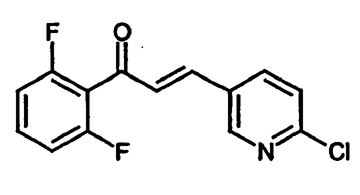

2,6-Difluoracetophenon (15.60g, 0.10 mol) und 2-Chlorpyridin-5-carboxaldehyd (14.20 g, 0.10 mol) werden in Methanol (300 ml) und Wasser (100 ml) vorgelegt.

Bei 5-8°C wird Natronlauge (30 ml, 10 % w/v) langsam zugetropft. Das Reaktionsgemisch wird anschließend eine Stunde bei 8°C nachgerührt. Der Niederschlag wird abgesaugt

Man erhält 20.30 g (73 % d. Th.) an 3-(6-Chlor-3-pyridinyl)-1-(2,6-difluorphenyl)-2-propen-1-on.
- HPLC:: Log P (pH 2.3) = 2.87 (98.54 %ig)
- NMR(CD₃CN):: δ = 7.09-7.20 (3H, m), 7.45-7.61 (3H, m), 8.04 (1H, dd), 8.58 (1H, d)ppm.

### Beispiel 11

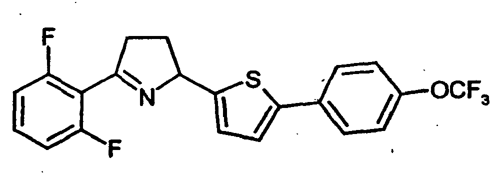

Unter Argonatmosphäre werden 2-(5-Brom-2-thienyl)-(2,6-difIuorphenyl)-3,4-dihydro-2H-pyrrol (Bsp. 1) (0.65 g, 1.90 mmol) und 4-Trifluormethoxyphenylboronsäure (0.50 g, 2.4 mmol) in Aceton (10 ml) vorgelegt. Nacheinander gibt man eine Lösung von Kaliumcarbonat (0.70 g, 5.07 mmol) in Wasser (10 ml) und eine Lösung von Palladiumacetat (13 mg, 0.058 mmol) in Aceton (2 ml) zu. Nach 4 Stunden Rühren bei 60°C gibt man nochmals 4-Trifluormethoxyphenylboronsäure (0.25 g, 1.2 mmol) eine Lösung von Kaliumcarbonat (0.35 g, 2.37 mmol) in Wasser (5 ml) und eine Lösung von Palladiumacetat (6 mg, 0.027 mmol) in Aceton (1 ml) zu und rührt weitere 6 Stunden bei 60°C. Nach Abkühlen auf Raumtemperatur wird der Ansatz filtriert und eingeengt. Das Filtrat wird mit Essigsäureethylester und Wasser verdünnt, und die Phasen werden getrennt. Die organische Phase wird über Natriumsulfat getrocknet, filtriert und eingeengt. Das Rohprodukt wird mittels Chromatographie an Kieselgel (Laufmittel: Cyclohexan / Essigsäureethylester 4:1) aufgereinigt.

Man erhält 0.40 g (47 % d. Th.) an 5-(2,6-Difluorphenyl)-2-{5-[4-(trifluormethoxy)-phenyl]-2-thienyl}-3,4-dihydro-2H-pyrrol.
- HPLC:: Log P (pH 2.3) = 5.16 (95 %ig)
- NMR (CD₃CN):: δ = 2.12 (1H, m), 2.62 (1H, m), 3.08 (1H, m), 3.14 (1H, m), 5.58 (1H, m), 6.97 (3H, m), 7.15 (1H, s), 7.20 (2H, d), 7.34 (1H, m), 7.58 (2H, d) ppm.

### Beispiel 12

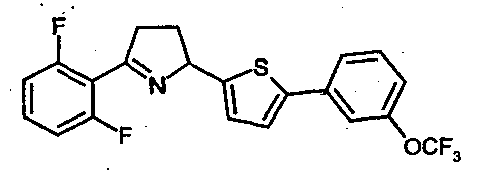

Unter Argonatmosphäre werden 2-(5-Brom-2-thienyl)-(2,6-difluorphenyl)-3,4-dihydro-2H-pyrrol (Bsp. 1) (0.65g, 1.90 mmol) und 3-Trifluormethoxyphenylboronsäure (0.50 g, 2.4 mmol) in Aceton (10 ml) vorgelegt. Nacheinander gibt man eine Lösung von Kaliumcarbonat (0.70 g, 5.07 mmol) in Wasser (10 ml) und eine Lösung von Palladiumacetat (26 mg, 0.12 mmol) in Aceton (3 ml) zu. Das Reaktionsgemisch wird 16 Stunden bei 60°C gerührt. Nach Abkühlen auf Raumtemperatur wird der Ansatz mit Essigsäureethylester und Wasser verdünnt, und die Phasen werden getrennt. Die organische Phase wird über Natriumsulfat getrocknet, filtriert und eingeengt. Das Rohprodukt wird mittels Chromatographie an Kieselgel (Laufmittel: Cyclohexan / Essigsäureethylester 9:1) aufgereinigt.

Man erhält 0.45 g (53 % d. Th.) an 5-(2,6-Difluorphenyl)2-{5-[3-(trifluormethoxy)-phenyl]-2-thienyl}-3,4-dihydro-2H-pyrrol.
- HPLC:: Log P (pH 2.3) = 4:84 (94 %ig)
- NMR (CD₃CN):: δ = 2.15 (1H, m), 2.65 (1H, m), 3.13 (2H, m), 5.61 (1H, m), 6.99 (3H, m), 7.11 (1H, m), 7.22 (1H, d), 7.33-7.49 (3H, m), 7:51 (1H, m) ppm.

### Beispiel 13

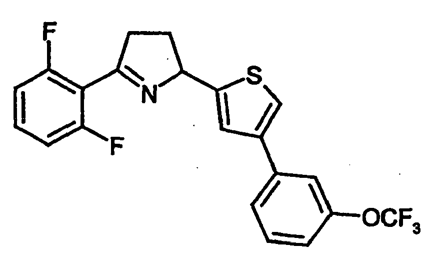

Unter Argonatmosphäre werden 2-(4-Brom-2-thienyl)-5-(2,6-difluorphenyl)-3,4-dihydro-2H-pyrrol (Bsp. 8). (0.56 g, 1.70 mmol) und 3-Trifluormethoxyphenylboronsäure (0.50 g, 2.4 mmol) in Aceton (10 ml) vorgelegt. Nacheinander gibt man eine Lösung von Kaliumcarbonat (0.70 g, 5.07 mmol) in Wasser (10 ml) und eine Lösung von Palladiumacetat (24 mg, 0.11 mmol) in Aceton (3 ml) zu. Das Reaktionsgemisch wird 16 Stunden bei 60°C gerührt. Nach Abkühlen auf Raumtemperatur wird der Ansatz mit Essigsäureethylester und Wasser verdünnt, und die Phasen werden getrennt. Die organische Phase wird über Natriumsulfat getrocknet, filtriert und eingeengt. Das Rohprodukt wird mittels Chromatographie an Kieselgel (Laufmittel: Toluol / Essigsäureethylester 1:0 → 9:1) aufgereinigt.

Man erhält 0.41 g (53 % d. Th.) an 5-(2,6-Difluorphenyl)-2-{4-[3-(trifluormethoxy)-phenyl]-2-thienyl}-3,4-dihydro-2H-pyrrol.
- HPLC:: Log P (pH 2.3) = 4.08 (93.6 %ig)
- NMR (CD₃CN):: δ = 2.05 (1H, m), 2.64 (1H, m), 3.07 (2H, m), 5.54 (1H, m), 7.07 (2H, m), 7.23 (1H, d), 7.39 (1H, s), 7.51 (2H, m), 7.58 (2H, m), 7.67 (1H, d) ppm.

### Beispiel 14

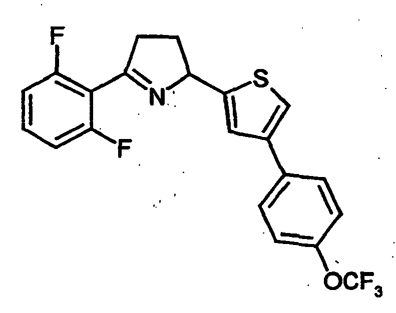

Unter Argonatmosphäre werden 2-(4-Brom-2-thienyl)-5-(2,6-difluorphenyl)-3,4-dihydro-2H-pyrrol (Bsp. 8) (0.75 g, 2.30 mmol) und 4-Trifluormethoxyphenylboronsäure (0.71 g, 3.45 mmol) in Aceton (10 ml) vorgelegt. Nacheinander gibt man eine Lösung von Kaliumcarbonat (0.75 g, 5.43 mmol) in Wasser (10 ml) und eine Lösung von Palladiumacetat (25 mg, 0.11 mmol) in Aceton (3 ml) zu. Das Reaktionsgemisch wird 16 Stunden bei 60°C gerührt. Nach Abkühlen auf Raumtemperatur wird der Ansatz mit Essigsäureethylester und Wasser verdünnt, und die Phasen werden getrennt. Die organische Phase wird über Natriumsulfat getrocknet, filtriert und eingeengt. Das Rohprodukt wird mittels Chromatographie an Kieselgel (Laufmittel: n-Hexan / Essigsäureethylester 9:1) aufgereinigt.

Man erhält 0.15 g (15 % d. Th.) an 5-(2,6-Difluorphenyl)-2-{4-[4-trifluormethoxy)-phenyl]-2-thienyl}-3,4-dihydro-2H-pyrrol.
- HPLC:: Log P (pH 2.3) = 4.66 (96.3 %ig)
- NMR (CD₃CN):: δ = 2.05 (1H, m), 2.65 (1H, m), 3.07(2H, m), 5.54 (1H, m), 7.08 (2H, m), 7.33 (2H, d), 7.37 (1H, s), 7.47 (1H, m), 7.49 (1H, s), 7.73 (2H,d)ppm.

Die Bestimmung der angegebenen IogP-Werte erfolgte gemäß EEC-Directive 79/831 Annex V.A8 durch HPLC (High Performance Liquid Chromatography) an einer Phasenumkehrsäule (C 18). Temperatur: 43°C.

Die Bestimmung erfolgt im sauren Bereich bei pH 2.3 mit 0,1 % wässriger Phosphorsäure und Acetonitril als Eluenten; linearer Gradient von 10 % Acetonitril bis 90 % Acetonitril.

Die Bestimmung erfolgt im neutralen Bereich bei pH 7.5 mit 0,01-molare wässriger Phosphatpuffer-Lösung und Acetonitril als Eluenten; linearer Gradient von 10% Acetonitril bis 90 % Acetonitril.

Die Eichung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Anwendungsbeispiele

### Beispiel A

### Meloidogyne-Test

| | | |
|---|---|---|
| Lösungsmittel: | 30 | Gewichtsteile Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Gefäße werden mit Sand, Wirkstofflösung, Meloidogyne incognita-Ei-Larvensuspension und Salatsamen gefüllt. Die Salatsamen keimen und die Pflänzchen entwickeln sich. An den Wurzeln entwickeln sich die Gallen.

Nach der gewünschten Zeit wird die nematizide Wirkung an Hand der Gallenbildung in % bestimmt. Dabei bedeutet 100 %, dass keine Gallen gefunden wurden; 0 % bedeutet, dass die Zahl der Gallen an den behandelten pflanzen der der unbehandelten Kontrolle entspricht.

Wirkstoffe und Versuchsergebnisse gehen aus der folgenden Tabelle hervor.

**Tabelle A**

| pflanzenschädigende Nematoden | | |
|---|---|---|
| **Meloidogyne-Test** | | |
| Wirkstoffe | Wirkstoffkonzentration in ppm | Abtötungsgrad in % nach 14d |
| 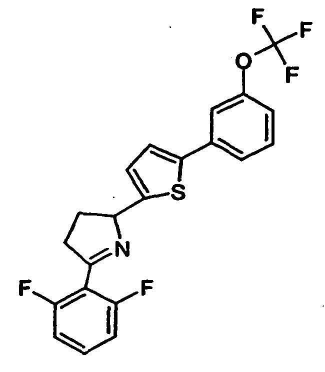 | 20 | 98 |

### Beispiel B

### Myzus-Test

| | |
|---|---|
| Lösungsmittel: | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea), die stark von der Pfirsichblattlaus (Myzus persicae) befallen sind, werden durch Tauchen in die Wirkstoffzuber-eitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Wirkstoffe und Versuchsergebnisse gehen aus der folgenden Tabelle hervor.

**Tabelle B**

| pflanzenschädigende Insekten | | |
|---|---|---|
| **Myzus-Test** | | |
| Wirkstoffe | Wirkstoffkonzentration in ppm | Abtötungsgrad in % nach 6^{d} |
| 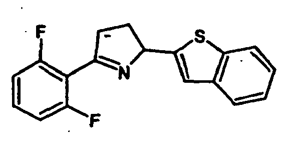 | 1000 | 95 |

### Beispiel C

### Phaedon-Larven-Test

| | | |
|---|---|---|
| Lösungsmittel: | 30 | Gewichtsteile Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Larven des Meerrettichblattkäfers (Phaedon cochleariae) besetzt, solange die Blätter noch feucht sind.

Nach der gewünschten Zeit wird die Abtötung in % bestimmt. Dabei bedeutet 100 %, dass alle Käferlarven abgetötet wurden; 0 % bedeutet, dass keine Käferlarven abgetötet wurden.

Wirkstoffe und Versuchsergebnisse gehen aus der folgenden Tabelle hervor.

**Tabelle C**

| pflanzenschädigende Insekten | | |
|---|---|---|
| **Phaedon-Larven -Test** | | |
| Wirkstoffe | Wirkstoffkonkonzentration in ppm | Abtötungsgrad in % nach 7^{d} |
| 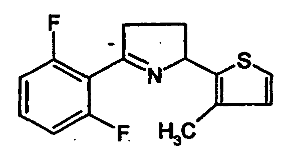 | 1000 | 100 |
| 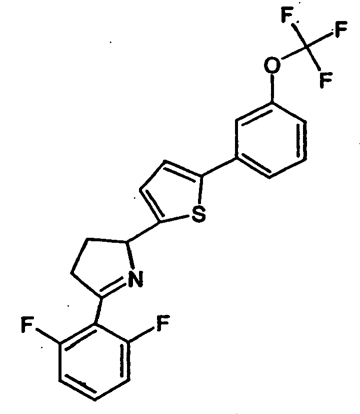 | 1000 | 100 |
| 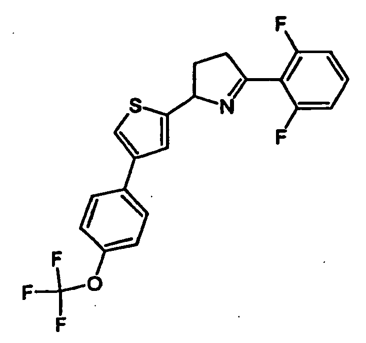 | 1000 | 100 |

### Beispiel D

### Plutella-Test

| | | |
|---|---|---|
| Lösungsmittel: | 30 | Gewichtsteile Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella xylostella) besetzt, solange die Blätter noch feucht sind.

Nach der gewünschten Zeit wird die Abtötung in % bestimmt. Dabei bedeutet 100 %, dass alle Raupen abgetötet wurden; 0% bedeutet, dass keine Raupen abgetötet wurden.

Wirkstoffe und Versuchsergebnisse gehen aus der folgenden Tabelle hervor.

**Tabelle D**

| pflanzenschädigende Insekten | | |
|---|---|---|
| **Plutella-test** | | |
| Wirkstoffe | Wirkstoffkonzentration in ppm | Abtötungsgrad in % nach 6^{d} |
| 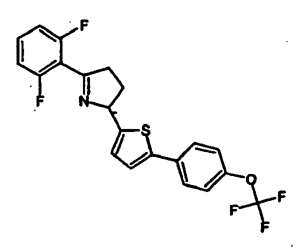 | 8 | 90 |

### Beispiel E

### Spodoptera frugiperda-Test

| | | |
|---|---|---|
| Lösungsmittel: | 30 | Gewichtsteile Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen des Heerwurms (Spodoptera frugiperda) besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Wirkstoffe und Versuchsergebnisse gehen aus der folgenden Tabelle hervor.

**Tabelle E**

| pflanzenschädigende Insekten | | |
|---|---|---|
| **Spodoptera frugiperda-Test** | | |
| Wirkstoffe | Wirkstoffkon-Zentration in ppm | Abtötungsgrad in % nach 7^{d} |
| 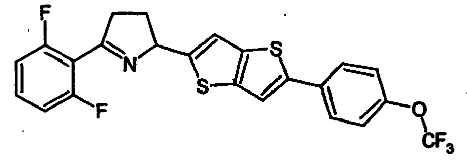 | 500 | 100 |
| 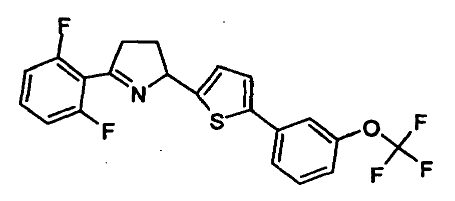 | 1000 | 100 |
| 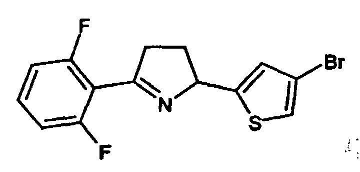 | 1000 | 100 |
| 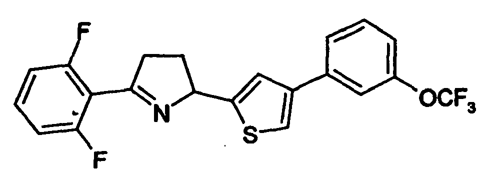 | 1000 | 100 |
| 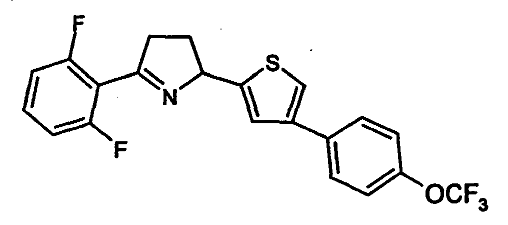 | 1000 | 100 |
| 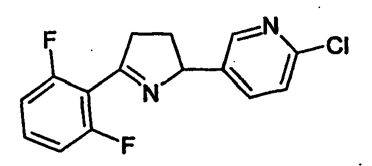 | 500 | 100 |

### Beispiel F

### Tetranychus-Test (OP-resistent/Tauchbehandlung)

| | | |
|---|---|---|
| Lösungsmittel: | 30 | Gewichtsteile Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenpflanzen (Phaseolus vulgaris), die stark von allen Stadien der gemeinen Spinnmilbe (Tetranychus urticae) befallen sind, werden in eine Wirkstoffzubereitung der gewünschten Konzentration getaucht.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Wirkstoffe und Versuchsergebnisse gehen aus der folgenden Tabelle hervor.

**Tabelle F**

| pflanzenschädigende Milben | | |
|---|---|---|
| **Tetranychus-Test** (OP-resistent/Tauchbehandung) | | |
| Wirkstoffe | Wirkstoffkonzentration in ppm | Abtötungsgrad in % nach 7^{d} |
| 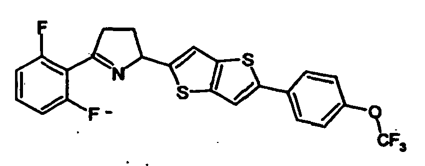 | 500 | 80 |

### Beispiel G

### Diabrotica balteata - Test (Larven im Boden)

Grenzkonzentrations-Test / Bodeninsekten - Behandlung transgener Pflanzen

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird auf den Boden gegossen. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/l) angegeben wird. Man füllt den Boden in 0,251 Töpfe und lässt diese bei 20°C stehen.

Sofort nach dem Ansatz werden je Topf 5 vorgekeimte Maiskörner der Sorte YIELD GUARD (Warenzeichen von Monsanto Comp., USA) gelegt. Nach 2 Tagen werden in den behandelten Boden die entsprechenden Testinsekten gesetzt. Nach weiteren 7 Tagen wird der Wirkungsgrad des Wirkstoffs durch Auszählen der aufgelaufenen Maispflanzen bestimmt (1 Pflanze = 20 % Wirkung).

### Beispiel H

### Heliothis virescens - Test (Behandlung transgener Pflanzen)

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) der Sorte Roundup Ready (Warenzeichen der Monsanto Comp. USA) werden durch Tauchen in die Wirkstoffzubereitung der gewünschten Konzentration behandelt und mit der Tabakknospenraupe Heliothis virescens besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

## Patentansprüche

1. Δ¹-Pyrroline der Formel (I) 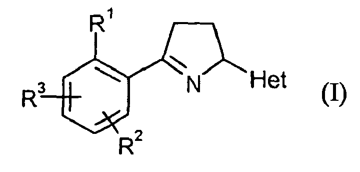 in welcher
R¹ für Halogen, jeweils gegebenenfalls substituiertes Alkyl, Alkoxy oder -S(O)_{w}R⁴ steht,
R² und R³ unabhängig voneinander für Wasserstoff, Halogen oder für jeweils gegebenenfalls substituiertes Alkyl, Alkoxy oder Alkoxyalkyl stehen,
R⁴ für gegebenenfalls substituiertes Alkyl steht,
Het für gegebenenfalls einfach oder mehrfach, gleich oder verschieden durch R⁵ substituiertes Heteroaryl steht,
R⁵ für die Gruppierung -X-Y-Z-E steht, mit der Maßgabe, dass Y nicht für eine direkte Bindung steht, wenn X nicht für eine direkte Bindung steht,
X für eine direkte Bindung, Sauerstoff, -S(O)_{w}-, -NR⁶-, Carbonyl, Carbonyloxy, Oxycarbonyl, Oxysulfonyl (OSO₂), Alkylen, Halogenalkylen, Alkenylen, Halogenalkenylen, Alkinylen, Alkylenoxy, Oxyalkylen, Oxyalkylenoxy, -S(O)_{w}-akylen, Cyclopropylen oder Oxiranylen steht,
Y für eine direkte Bindung oder für jeweils gegebenenfalls substituiertes Phenylen, Naphthylen, Tetrahydronaphthylen oder Heterocyclylen steht,
Z für eine direkte Bindung oder -(CH₂)ₙ- steht,
E für Wasserstoff, Halogen, Hydroxy, Cyano, Formyl, Nitro, Trialkylsilyl, Pentafluorthio, -S(O)_{w}R⁷, -OSO₂R⁷, -NR⁸R⁹, -COR⁷, -CO₂R⁷, -OC(O)R⁷, -CONR¹⁰R¹¹, -N(R¹²)COR¹³, -C(R¹⁴)=N-OR¹⁵, -SO₂NR¹⁶R¹⁷, für jeweils gegebenenfalls substituiertes Alkyl, Alkenyl, Alkinyl, Alkoxy, Alkenyloxy, Cycloalkyl, Cycloalkylalkyl, Cycloalkyloxy, Aryl, Arylalkyl, Aryloxy, Aryloxyalkyl, gesättigtes oder ungesättigtes Heterocyclyl oder Heterocyclylalkyl steht,
R⁶ für jeweils gegebenenfalls substituiertes Alkyl, Cycloalkyl, Cycloalkylalkyl, Aryl oder Arylalkyl steht,
R⁷ für jeweils gegebenenfalls substituiertes Alkyl, Cycloalkyl, Aryl oder Arylalkyl steht,
R⁸ und R⁹ unabhängig voneinander für Wasserstoff, -SO₂R⁷, -COR⁷, -CO₂R⁷, für jeweils gegebenenfalls substituiertes Alkyl, Alkenyl, Cycloalkyl, Cycloalkylalkyl, Aryl, Arylalkyl oder gesättigtes oder ungesättigtes Heterocyclyl oder Heterocyclylalkyl stehen,
R⁸ und R⁹ außerdem gemeinsam für jeweils gegebenenfalls substituiertes Alkenylen oder Alkylen stehen, wobei die Alkylenkette jeweils durch -O-, -S- oder -NR¹⁸- unterbrochen sein kann,
R¹⁰ und R¹¹ unabhängig voneinander für Wasserstoff, -SO₂R⁷, für jeweils gegebenenfalls substituiertes Alkyl, Alkenyl, Cycloalkyl, Cycloalkylalkyl, Aryl, Arylalkyl oder gesättigtes oder ungesättigtes Heterocyclyl oder Heterocyclylalkyl stehen,
R¹⁰ und R¹¹ außerdem gemeinsam für gegebenenfalls substituiertes Alkylen stehen, wobei die Alkylenkette jeweils durch -O-, -S- oder -NR¹⁸-unterbrochen sein kann,
R¹² und R¹³ unabhängig voneinander für Wasserstoff, für jeweils gegebenenfalls substituiertes Alkyl, Cycloalkyl, Cycloalkylalkyl, Aryl oder Arylalkyl stehen,
R¹² und R¹³ außerdem gemeinsam für jeweils gegebenenfalls substituiertes Alkylen oder Alkenylen stehen,
R¹⁴ und R¹⁵ unabhängig voneinander für Wasserstoff, für jeweils gegebenenfalls substituiertes Alkyl oder Alkenyl stehen,
R¹⁶ und R¹⁷ unabhängig voneinander für Wasserstoff, für gegebenenfalls substituiertes Alkyl oder Cycloalkyl stehen,
R¹⁶ und R¹⁷ außerdem gemeinsam für gegebenenfalls substituiertes Alkylen, Alkoxyalkylen oder Alkylthioalkylen stehen,
R¹⁸ für Wasserstoff, -SO₂R⁷, -COR⁷ oder -CO₂R⁷; für jeweils gegebenenfalls substituiertes Alkyl, Alkenyl, Cycloalkyl, Cycloalkylalkyl, Aryl, Arylalkyl oder gesättigtes oder ungesättigtes Heterocyclyl oder Heterocyclylalkyl steht,
w für 0, 1 oder 2 steht,
n für 1, 2, 3 oder 4 steht.

2. Δ¹-Pyrroline der Formel (I) gemäß Anspruch 1, in welcher
R¹ für Halogen, Alkyl, Halogenalkyl, Alkoxy, Halogenalkoxy oder -S(O)_{w}R⁴ steht,
R² und R³ unabhängig voneinander für Wasserstoff, Halogen, Alkyl, Halogenalkyl, Alkoxy, Halogenalkoxy oder Alkoxyalkyl stehen,
R⁴ für Alkyl oder Halogenalkyl steht,
Het für gegebenenfalls einfach bis vierfach, gleich oder verschieden durch R⁵ substituiertes 5- bis 14-gliedriges, 1 bis 3 aromatische Ringe enthaltendes Heteroaryl mit einem oder mehreren Heteroatomen aus der Reihe Stickstoff, Sauerstoff und Schwefel steht,
R⁵ für die Gruppierung -X-Y-Z-E steht mit der Maßgabe, dass Y nicht für eine direkte Bindung steht, wenn X nicht für eine direkte Bindung steht,
X für eine direkte Bindung, Sauerstoff, -S(O)_{w}-, -NR⁶-, Carbonyl, Carbonyloxy, Oxycarbonyl, Oxysulfonyl (OSO₂), Alkylen, Halogenalkylen, Alkenylen, Halogenalkenylen, Alkinylen, Alkylenoxy, Oxyalkylen, Oxyalkylenoxy, -S(O)_{w}-alkylen, Cyclopropylen oder Oxiranylen steht,
Y für eine direkte Bindung oder für jeweils gegebenenfalls einfach bis vierfach, gleich oder verschieden durch Reste aus der Liste W¹ substituiertes Phenylen, Naphthylen, Tetrahydronaphthylen oder 5- bis 10-gliedriges, gesättigtes oder ungesättigtes Heterocyclylen mit einem oder mehreren Heteroatomen aus der Reihe Stickstoff, Sauerstoff und Schwefel steht,
Z für eine direkte Bindung oder -(CH₂)ₙ- steht,
E für Wasserstoff, Halogen, Hydroxy, Cyano, Formyl, Nitro, Trialkylsilyl, Pentafluorthio, -S(O)_{w}R⁷, -OSO₂R⁷, -NR⁸R⁹, -COR⁷, -CO₂R⁷, -OC(O)R⁷, -CONR¹⁰R¹¹, -N(R¹²)COR¹³, -C(R¹⁴)=N-OR¹⁵, -SO₂NR¹⁶R¹⁷; für jeweils gegebenenfalls einfach oder mehrfach, gleich oder verschieden durch Halogen, Cyano, Alkoxy und/oder -NR⁸R⁹ substituiertes Alkyl, Alkenyl, Alkinyl, Alkoxy, Alkenyloxy; oder für jeweils gegebenenfalls einfach oder mehrfach, gleich oder verschieden durch Halogen, Cyano, Nitro, Alkyl, Halogenalkyl, Alkenyl, Halogenalkenyl, Alkoxy, Halogenalkoxy, Alkylthio und/oder Halogenalkylthio substituiertes Cycloalkyl, Cycloalkylalkyl, Cycloalkyloxy, Aryl, Arylalkyl, Aryloxy, Aryloxyalkyl, gesättigtes oder ungesättigtes 5- bis 10-gliedriges Heterocyclyl oder Heterocyclylalkyl mit einem oder mehreren Heteroatomen aus der Reihe Stickstoff, Sauerstoff und Schwefel steht,
W¹ für Halogen, Cyano, Formyl, Nitro, Trialkylsilyl, Alkyl, Halogenalkyl, Alkoxy, Halogenalkoxy, Alkenyl, Halogenalkenyl, Alkenyloxy, Halogenalkenyloxy, Alkylcarbonyl, Alkoxycarbonyl, -S(O)_{w}R⁷, -C(R¹⁴)=N-OR¹⁵, -SO₂NR¹⁶R¹⁷, -(CH₂)ₚNR¹⁶R¹⁷, -(CH₂)ₚN(R¹⁶)COR¹⁷, -(CH₂)ₚN(R¹⁶)SO₂R¹⁷, -OSO₂R¹⁶, -OSO_{Z}NR¹⁶R¹⁷ steht,
R⁶ für Alkyl, Halogenalkyl oder für jeweils gegebenenfalls einfach oder mehrfach, gleich oder verschieden durch Halogen, Alkyl; Halogenalkyl, Alkoxy, Halogenalkoxy, Alkylthio und/oder Halogenalkylthio substituiertes Cycloalkyl, Cycloalkylalkyl, Aryl oder Arylalkyl steht,
R⁷ für gegebenenfalls einfach oder mehrfach, gleich oder verschieden durch Halogen und/oder -NR⁸R⁹ substituiertes Alkyl, für jeweils gegebenenfalls einfach oder mehrfach, gleich oder verschieden durch Halogen, Cyano, Alkyl, Halogenalkyl, Alkoxy, Halogenalkoxy, Alkylthio, Halogenalkylthio substituiertes Cycloalkyl, Aryl oder Arylalkyl steht,
R⁸ und R⁹ unabhängig voneinander für Wasserstoff, -SO₂R⁷, -COR⁷, -CO₂R⁷, für jeweils gegebenenfalls einfach oder mehrfach, gleich oder verschieden durch Halogen, Alkylcarbonyl, Alkylcarbonyloxy, Alkylamino, Dialkylamino, Alkoxy, Halogenalkoxy, Alkylthio und/oder Halogenalkylthio substituiertes Alkyl oder Alkenyl; für jeweils gegebenenfalls einfach oder mehrfach, gleich oder verschieden durch Halogen, Cyano, Alkyl, Halogenalkyl, Alkoxy, Halogenalkoxy, Alkylthio und/oder Halogenalkylthio substituiertes Cycloalkyl, Cycloalkylalkyl, Aryl, Arylalkyl oder gesättigtes oder ungesättigtes, 5- bis 10-gliedriges Heterocyclyl oder Heterocyclylalkyl mit einem oder mehreren Heteroatomen aus der Reihe Stickstoff, Sauerstoff und Schwefel stehen,
R⁸ und R⁹ außerdem gemeinsam für gegebenenfalls einfach oder mehrfach, gleich oder verschieden durch Halogen, Cyano, Alkoxy, Halogenalkoxy, Alkylthio und/oder Halogenalkylthio substituiertes Alkenylen oder für gegebenenfalls einfach oder mehrfach, gleich oder verschieden durch Halogen, Cyano, Alkyl, Halogenalkyl, Alkoxy, Halogenalkoxy, Alkylthio und/oder Halogenalkylthio substituiertes Alkylen, wobei die Alkylenkette jeweils durch -O-, -S- oder -NR¹⁸- unterbrochen sein kann, stehen,
R¹⁰ und R¹¹ unabhängig voneinander für Wasserstoff, -SO₂R⁷, für jeweils gegebenenfalls einfach oder mehrfach, gleich oder verschieden durch Halogen, Alkylamino, Dialkylamino, Alkoxy und/oder Alkylthio substituiertes Alkyl oder Alkenyl; für jeweils gegebenenfalls einfach oder mehrfach, gleich oder verschieden durch Halogen, Cyano, Alkyl, Halogenalkyl, Alkoxy, Halogenalkoxy, Alkylthio und/oder Halogenalkykthio substituiertes Cycloalkyl, Cycloalkylalkyl, Aryl, Arylalkyl oder gesättigtes oder ungesättigtes, 5- bis 10-gliedriges Heterocyclyl oder Heterocyclylalkyl mit einem oder mehreren Heteroatomen aus der Reihe Stickstoff, Sauerstoff und Schwefel stehen,
R¹⁰ und R¹¹ außerdem gemeinsam für gegebenenfalls einfach oder mehrfach, gleich oder verschieden durch Halogen, Cyano, Alkyl, Halogenalkyl, Alkoxy, Halogenalkoxy, Alkylthio und/oder Halogenalkylthio substituiertes Alkylen, wobei die Alkylenkette jeweils durch -O-, -S- oder -NR¹⁸- unterbrochen sein kann, stehen,
R¹² und R¹³ unabhängig voneinander für Wasserstoff, für gegebenenfalls einfach oder mehrfach, gleich oder verschieden durch Halogen, Cyano, Alkoxy und/oder Alkylthio substituiertes Alkyl, für jeweils gegebenenfalls einfach oder mehrfach, gleich oder verschieden durch Halogen, Cyano, Alkyl, Halogenalkyl, Alkoxy, Halogenalkoxy, Alkylthio und/oder Halogenalkylthio substituiertes Cycloalkyl, Cycloalkylalkyl, Aryl oder Arylalkyl stehen,
R¹² und R¹³ außerdem gemeinsam für jeweils gegebenenfalls einfach oder mehrfach, gleich oder verschieden durch Halogen, Alkyl, Halogenalkyl, Alkoxy, Halogenalkoxy, Alkylthio und/oder Halogenalkylthio substituiertes Alkylen oder Alkenylen stehen,
R¹⁴ und R¹⁵ unabhängig voneinander für Wasserstoff, für Alkyl, Halogenalkyl, Alkenyl oder Halogenalkenyl stehen,
R¹⁶ und R¹¹ unabhängig voneinander für Wasserstoff, Alkyl, Halogenalkyl oder für gegebenenfalls einfach oder mehrfach, gleich oder verschieden durch Halogen oder Alkyl substituiertes Cycloalkyl stehen,
R¹⁶ und R¹⁷ außerdem gemeinsam für jeweils gegebenenfalls einfach oder mehrfach, gleich oder verschieden durch Halogen oder Alkyl substituiertes Alkylen, Alkoxyalkylen oder Alkylthioalkylen stehen,
R¹⁸ für Wasserstoff, -SO₂R⁷, -COR⁷ oder -CO₂R⁷; für jeweils gegebenenfalls einfach oder mehrfach, gleich oder verschieden durch Halogen, Cyano, Alkylamino, Dialkylamino, Alkoxy, Halogenalkoxy, Alkylthio und/oder Halogenalkylthio substituiertes Alkyl oder Alkenyl; für jeweils gegebenenfalls einfach oder mehrfach, gleich oder verschieden durch Halogen, Cyano, Alkyl, Halogenalkyl, Alkoxy, Halogenalkoxy, Alkylthio und/oder Halogenalkylthio substituiertes Cycloalkyl, Cycloalkylalkyl, Aryl, Arylalkyl, gesättigtes oder ungesättigtes, 5- bis 10-gliedriges Heterocyclyl oder Heterocyclylalkyl mit einem oder mehreren Heteroatomen aus der Reihe Stickstoff, Sauerstoff und Schwefel steht,
w für 0, 1 oder 2 steht,
n für 1, 2, 3 oder 4 steht,
p für 0, 1 oder 2 steht.

3. Δ¹-Pyrroline der Formel (I) gemäß Anspruch 1, in welcher
R¹ für Halogen, C₁-C₆-Alkyl, C₁-C₆-Halogenalkyl, C₁-C₆-Alkoxy, C₁-C₆-Halogenalkoxy oder -S(O)_{w}R⁴ steht,
R² und R³ unabhängig voneinander für Wasserstoff, Halogen, C₁-C₆-Alkyl, C₁-C₆-Halogenakyl, C₁-C₆-Alkoxy, C₁-C₆-Halogenalkoxy oder C₁-C₆-Alkoxy-C₁-C₆-alkyl stehen,
R⁴ für C₁-C₆-Alkyl oder C₁-C₆-Halogenalkyl steht,
Het für gegebenenfalls einfach bis vierfach, gleich oder verschieden durch R⁵ substituiertes 5- bis 14-gliedriges, 1 bis 3 aromatische Ringe enthaltendes Heteroaryl mit 1 bis 4 Heteroatomen, welches 0 bis 4 Stickstoffatome, 0 bis 2 nicht benachbarte Sauerstoffatome und/oder 0 bis 2 nicht benachbarte Schwefelatome enthält (insbesondere Thienyl, Benzothienyl, Furyl, Benzofuryl, Indolyl, Thienothienyl, Thienofuryl Thienobenzothienyl, Thienobenzofuryl, Pyridinyl, Pyrimidinyl, Pyridazinyl, Pyrazinyl, Triazolyl oder Tetrazolyl) steht,
R⁵ für die Gruppierung -X-Y-Z-E steht mit der Maßgabe, dass Y nicht für eine direkte Bindung steht, wenn X nicht für eine direkte Bindung steht,
X für eine direkte Bindung, Sauerstoff, -S(O)_{w}-, -NR⁶-, Carbonyl, Carbonyloxy, Oxycarbonyl, Oxysulfonyl (OSO₂), C₁-C₆-Alkylen, C₁-C₆-Halogenalkylen, C₂-C₆-Alkenylen, C₂-C₆-Halogenalkenylen, C₂-C₆-Alkinylen, C₁-C₆-Alkylenoxy, C₁-C₆-Oxyalkylen, Oxy-C₁-C₆-alkylenoxy, -S(O)_{w}-C₁-C₆-alkylen, Cyclopropylen oder Oxiranylen steht,
Y für eine direkte Bindung oder für jeweils gegebenenfalls einfach bis vierfach, gleich oder verschieden durch Reste aus der Liste W¹ substituiertes 1,4-Phenylen, 1,3-Phenylen, 1,2-Phenylen, 2,6-Naphthylen, 2,7-Naphthylen, 1,4-Naphthylen, 2,6-(1,2,3,4-Tetrahydro)naphthylen, 2,7-(1,2,3,4-Tetrahydro)naphthylen, 1,4-(1,2,3,4-Tetrahydro)naphthylen, 5,8-(1,2,3,4-Tetrahydro)naphthylen; oder für jeweils gegebenenfalls einfach bis vierfach, gleich oder verschieden durch Reste aus der Liste W¹ substituiertes 5- oder 6-gliedriges, gesättigtes oder ungesättigtes Heterocyclylen mit 1 bis 3 Heteroatomen, welches 0 bis 3 Stickstoffatome, 0 bis 2 nicht benachbarte Sauerstoffatome und/oder 0 bis 2 nicht benachbarte Schwefelatome enthält (insbesondere Furylen, Thienylen, Pyrrolylen, Oxazolylen, Thiazolylen, Pyridinylen, Pyrimidinylen, Pyridazinylen oder Pyrazinylen) steht,
Z für eine direkte Bindung oder -(CH₂)n- steht,
E für Wasserstoff, Fluor, Chlor, Brom, Hydroxy, Cyano, Formyl, Nitro, Tri-(C₁-C₆-alkyl)silyl, Pentafluorthio, -S(O)_{w}R⁷, -OSO₂R⁷, -NR⁸R⁹, -COR⁷, -CO₂R⁷, -OC(O)R⁷, -CONR¹⁰R¹¹, -N(R¹²)COR¹³, -C(R¹⁴)=N-OR¹⁵, -SO₂NR¹⁶R¹⁷; für jeweils gegebenenfalls einfach oder mehrfach, gleich oder verschieden durch Halogen, Cyano, C₁-C₁₀-Alkoxy und/oder -NR⁸R⁹ substituiertes C₁-C₂₀-Alkyl, C₂-C₁₀-Alkenyl, C₂-C₁₀-Alkinyl, C₁-C₂₀-Alkoxy, C₂-C₂₀-Alkenyloxy; oder für jeweils gegebenenfalls einfach bis vierfach, gleich oder verschieden durch Halogen, Cyano, Nitro, C₁-C₆-Alkyl, C₁-C₆-Halogenalkyl, C₂-C₆-Alkenyl, C₂-C₆-Halogenalkenyl, C₁-C₆-Alkoxy, C₁-C₆-Halogenalkoxy, C₁-C₆-Alkylthio, C₁-C₆-Halogenalkylthio substituiertes C₃-C₁₂-Cycloalkyl, C₃-C₇-Cycloalkyl-C₁-C₄-alkyl, C₃-C₁₂-Cycloalkyloxy, Aryl, Aryl-C₁-C₄-alkyl, Aryloxy, Aryloxy-C₁-C₄-alkyl, gesättigtes oder ungesättigtes, 5- bis 10-gliedriges Heterocyclyl oder Heterocyclyl-C₁-C₄-alkyl mit 1 bis 4 Heteroatomen, welche 0 bis 4 Stickstoffatome, 0 bis 2 nicht benachbarte Sauerstoffatome und/oder 0 bis 2 nicht benachbarte Schwefelatome enthalten (insbesondere Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidino, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Piperidino, Morpholinyl, Thiomorpholinyl, Morpholino, Thiomorpholino, Triazinyl, Triazolyl, Chinolinyl oder Isochinolinyl) steht,
W¹ für Fluor, Chlor, Brom, Cyano, Formyl, Nitro, Tri-(C₁-C₆-akyl)silyl, C₁-C₆-Alkyl, C₁-C₆-Halogenalkyl, C₁-C₆-Alkoxy, C₁-C₆-Halogenalkoxy, C₂-C₆-Alkenyl, C₂-C₆-Halogenalkenyl, C₂-C₆-Alkenyloxy, C₂-C₆-Halogenalkenyloxy, C₁-C₆-Alkylcarbonyl, C₁-C₆-Alkoxycarbonyl, -S(O)_{w}R⁷, -C(R¹⁴)=N-OR¹⁵, -SO₂NR¹⁶R¹⁷, -(CH₂)ₚNR¹⁶R¹⁷, -(CH₂)ₚN(R¹⁶)COR¹⁷, -(CH₂)ₚN(R¹⁶)SO₂R¹⁷, -OSO₂R¹⁶, -OSO₂NR¹⁶R¹⁷ steht,
R⁶ für C₁-C₆-Alkyl, C₁-C₆-Halogenalkyl oder für jeweils gegebenenfalls einfach bis vierfach durch Halogen, C₁-C₆-Alkyl, C₁-C₆-Halogenalkyl, C₁-C₆-Alkoxy, C₁-C₆-Halogenalkoxy, C₁-C₆-Alkylthio und/oder C₁-C₆-Halogenalkylthio substituiertes C₃-C₇-Cycloakyl, C₃-C₆-Cycloalkyl-C₁-C₄-alkyl, Aryl oder Aryl-C₁-C₄-alkyl steht,
R⁷ für gegebenenfalls einfach oder mehrfach, gleich oder verschieden durch Halogen und/oder -NR⁸R⁹ substituiertes C₁-C₂₀-Alkyl, für jeweils gegebenenfalls einfach bis achtfach, gleich oder verschieden durch Halogen, Cyano, C₁-C₆-Alkyl, C₁-C₆-Halogenalkyl, C₁-C₆-Alkoxy, C₁-C₆-Halogenalkoxy, C₁-C₆-Alkylthio und/oder C₁-C₆-Halogenalkylthio substituiertes C₃-C₆-Cycloalkyl, Aryl oder Aryl-C₁-C₄-alkyl steht,
R⁸ und R⁹ unabhängig voneinander für Wasserstoff, -SO₂R⁷, -COR⁷, -CO₂R⁷, für jeweils gegebenenfalls einfach oder mehrfach, gleich oder verschieden durch Halogen, C₁-C₆-Alkylcarbonyl, C₁-C₆-Alkylcarbonyloxy, C₁-C₆-Alkylamino, Di-(C₁-C₆-alkyl)amino, C₁-C₆-Alkoxy, C₁-C₆-Halogenalkoxy, C₁-C₆-Alkylthio, C₁-C₆-Halogenalkylthio substituiertes C₁-C₂₀-Alkyl oder C₂-C₂₀-Alkenyl; für jeweils gegebenenfalls einfach bis vierfach, gleich oder verschieden durch Halogen, Cyano, C₁-C₆-Alkyl, C₁-C₆-Halogenakyl, C₁-C₆-Akoxy, C₁-C₆-Halogenalkoxy, C₁-C₆-Alkylthio, C₁-C₆-Halogenalkylthio substituiertes C₃-C₁₂-Cycloalkyl, C₃-C₇-Cycloalkyl-C₁-C₄-alkyl, Aryl, Aryl-C₁-C₄-alkyl oder gesättigtes oder ungesättigtes, 5- bis 10-gliedriges Heterocyclyl oder Heterocyclyl C₁-C₄-alkyl mit is 4 Heteroatomen, welche 0 bis 4 Stickstoffatome, 0 bis 2 nicht benachbarte Sauerstoffatome und/oder 0 bis 2 nicht benachbarte Schwefelatome enthalten (insbesondere Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl) stehen,
R⁸ und R⁹ außerdem gemeinsam für gegebenenfalls einfach oder mehrfach, gleich oder verschieden durch Halogen, Cyano, C₁-C₆-Alkoxy, C₁-C₆-Halogenalkoxy, C₁-C₆-Alkylthio, C₁-C₆-Halogenalkylthio substituiertes C₂-C₁₂-Alkenylen oder für gegebenenfalls im Alkylenteil einfach oder mehrfach, gleich oder verschieden durch Halogen, Cyano, C₁-C₆-Alkyl, C₁-C₆-Halogenakyl, C₁-C₆-Alkoxy, C₁-C₆-Halogenalkoxy, C₁-C₆-Alkylthio, C₁-C₆-Halogenalkylthio substituiertes C₃-C₁₂-Alkylen, wobei die Alkylenkette jeweils durch -O-, -S- oder -NR¹⁸- unterbrochen sein kann, stehen,
R¹⁰ und R¹¹ unabhängig voneinander für Wasserstoff, -SO₂R⁷, für jeweils gegebenenfalls einfach bis dreizehnfach, gleich oder verschieden durch Halogen, C₁-C₆-Alkylamino, Di-(C₁-C₆-alkyl)amino, C₁-C₆-Alkoxy, C₁-C₆-Halogenalkoxy, C₁-C₆-Alkylthio, C₁-C₆-Halogenalkylthio substituiertes C₁-C₆-Alkyl oder C₂-C₆-Alkenyl; für jeweils gegebenenfalls einfach bis vierfach, gleich oder verschieden durch Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio substituiertes C₃-C₇-Cycloalkyl, C₃-C₇-Cycloalkyl-C₁-C₄-Alkyl, Aryl, Aryl-C₁-C₄-alkyl oder gesättigtes oder ungesättigtes, 5- bis 10-gliedriges Heterocyclyl öder Heterocyclyl-C₁-C₄-alkyl mit 1 bis 4 Heteroatomen, welche 0 bis 4 Stickstoffatome, 0 bis 2 nicht benachbarte Sauerstoffatome und/oder 0 bis 2 nicht benachbarte Schwefelatome enthalten (insbesondere Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl) stehen,
R¹⁰ und R¹¹ außerdem gemeinsam für jeweils gegebenenfalls im Alkylenteil einfach bis vierfach, gleich oder verschieden durch Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio substituiertes C₃-C₆-Alkylen, -(CH₂)₂-O-(CH₂)₂-, -(CH₂)₂-S-(CH₂)₂- oder -(CH₂)₂-N(R¹⁸)-(CH₂)₂- stehen,
R¹² und R¹³ unabhängig voneinander für Wasserstoff, für gegebenenfalls einfach bis dreizehnfach, gleich oder verschieden durch Halogen, Cyano, C₁-C₆-Alkoxy, C₁-C₆-Alkylthio substituiertes C₁-C₆-Alkyl, für jeweils gegebenenfalls einfach bis achtfach, gleich oder verschieden durch Halogen, Cyano, C₁-C₆-Alkyl, C₁-C₆-Halogenalkyl, C₁-C₆-Alkoxy, C₁-C₆-Halogenakoxy, C₁-C₆-Alkylthio, C₁-C₆-Halogenalkylthio substituiertes C₃-C₇-Cycloalkyl, C₃-C₇-Cycloalkyl-C₁-C₄-alkyl, Aryl oder Aryl-C₁-C₄-alkyl stehen,
R¹² und R¹³ außerdem gemeinsam für jeweils gegebenenfalls einfach bis achtfach, gleich oder verschieden, durch Halogen, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio substituiertes C₃-C₁₀-Alkylen oder C₃-C₁₀-Alkenylen stehen,
R¹⁴ und R¹⁵ unabhängig voneinander für Wasserstoff, für C₁-C₆-Alkyl, C₁-C₆-Halogenalkyl, C₂-C₆-Alkenyl oder C₂-C₆-Halogenalkenyl stehen,
R¹⁶ und R¹⁷ unabhängig voneinander für Wasserstoff, C₁-C₆-Alkyl, C₁-C₆-Halogenalkyl oder für gegebenenfalls einfach bis achtfach, gleich oder verschieden durch Fluor, Chlor, Brom und/oder C₁-C₆-Alkyl substituiertes C₃-C₇-Cycloakyl stehen,
R¹⁶ und R¹⁷ außerdem gemeinsam für jeweils gegebenenfalls einfach bis neunfach, gleich oder verschieden durch Fluor, Chlor, Brom und/oder C₁-C₆-Alkyl substituiertes C₃-C₆-Alkylen, C₁-C₃-Alkoxy-C₁-C₃-alkylen oder C₁-C₃-Allcylthio-C₁-C₃-alkylen stehen,
R¹⁸ für Wasserstoff, -SO₂R⁷, -COR⁷ oder -CO₂R⁷; für jeweils gegebenenfalls einfach oder mehrfach, gleich oder verschieden durch Halogen, Cyano, C₁-C₆-Alkylamino, Di-(C₁-C₆-akyl)amino, C₁-C₆-Alkoxy, C₁-C₆-Halogenalkoxy, C₁-C₆-Alkylthio, C₁-C₆-Halogenalkylthio substituiertes C₁-C₂₀-Alkyl oder C₂-C₂₀-Alkenyl; für jeweils gegebenenfalls einfach bis vierfach, gleich oder verschieden durch Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio substituiertes C₃-C₁₂-Cycloalkyl, C₃-C₇-Cycloalkyl-C₁-C₄-alkyl, Aryl, Aryl-C₁-C₄-alkyl, gesättigtes oder ungesättigtes, 5- bis 10-gliedriges Heterocyclyl oder Heterocyclyl-C₁-C₄-alkyl mit 1 bis 4 Heteroatomen, welche 0 bis 4 Stickstoffatome, 0 bis 2 nicht benachbarte Sauerstoffatome und/oder 0 bis 2 nicht benachbarte Schwefelatome enthalten (insbesondere Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl) steht,
w für 0, 1 oder 2 steht,
n für 1, 2 oder 3 steht,
p für 0, 1 oder 2 steht.

4. Δ¹-Pyrroline der Formel (I) gemäß Anspruch 1, in welcher
R¹ für Fluor, Chlor, Brom, C₁-C₄-Alkyl, C₁-C₄-Halogenalkyl mit 1 bis 9 Fluor-, Chlor- und/oder Bromatomen, C₁-C₄-Alkoxy, C₁-C₄-Halogenalkoxy mit 1 bis 9 Fluor-, Chlor- und/oder Bromatomen oder -S(O)_{w}R⁴ steht,
R² und R³ unabhängig voneinander für Wasserstoff, Fluor, Chlor, Brom, C₁-C₄-Alkyl, C₁-C₄-Halogenalkyl mit 1 bis 9 Fluor-, Chlor- und/oder Bromatomen, C₁-C₄-Akoxy oder C₁-C₄-Halogenalkoxy mit 1 bis 9 Fluor-, Chlor- und/oder Bromatomen stehen,
R⁴ für C₁-C₄-Alkyl oder für jeweils durch Fluor oder Chlor substituiertes Methyl oder Ethyl steht,
Het steht für jeweils gegebenenfalls einfach bis dreifach, gleich oder verschieden durch R⁵ substituiertes 2-Thienyl, 3-Thienyl, 2-Benzo[b]-thienyl, 2-Furyl, 3-Furyl, 2-Benzo[b]furyl, 2-Indolyl, 2-Thieno[3,2-b]-thienyl, 2-Thieno[3,2-b]furyl, 5-Thieno[3,2-b]furyl, 2-Thieno[2,3-f][1]-benzothienyl, 2-Thieno[2,3-f][1]benzofuryl, 6-Thieno[2,3-f][1]benzofuryl, 2-Pyridinyl, 3-Pyridinyl, 2-Pyrimidinyl, 5-Pyrimidinyl, 3-Pyridazinyl, 4-Pyridazinyl, 2-Pyrazinyl, Triazolyl oder Tetrazolyl steht,
R⁵ für die Gruppierung -X-Y-Z-E steht mit der Maßgabe, dass Y nicht für eine direkte Bindung steht, wenn X nicht für eine direkte Bindung steht,
X für eine direkte Bindung, Sauerstoff, -S(O)_{w}-, -NR⁶-, Carbonyl, Carbonyloxy, Oxycarbonyl, Oxysulfonyl (OSO₂), C₁-C₄-Alkylen, C₁-C₄-Halogenalkylen mit 1 bis 8 Fluor-, Chlor- und/oder Bromatomen, C₂-C₄-Alkenylen, C₂-C₄-Halogenalkenylen mit 1 bis 6 Fluor-, Chlor- und/oder Bromatomen, C₂-C₄-Alkinylen, C₁-C₄-Alkylenoxy, Oxy-C₁-C₄-alkylen, Oxy-C₁-C₄-akylenoxy oder -S(O)_{w}-C₁-C₄-alkylen steht,
Y für eine direkte Bindung oder für jeweils gegebenenfalls einfach bis dreifach, gleich oder verschieden durch Reste aus der Liste W¹ substituiertes 1,4-Phenylen, 1,3-Phenylen, 1,2-Phenylen, 2,6-Naphthylen, 2,7-Naphthylen, 1,4-Naphthylen; 2,6-(1,2,3,4-Tetrahydro)-naphthylen, 2,7-(1,2,3,4-Tetrahydro)naphthylen, 1,4-(1,2,3,4-Tetrahydro)naphthylen, 5,8-(1,2,3,4-Tetrahydro)naphthylen, 2,4-Furylen, 2,4-Thienylen, 2,4-Pyrrolylen, 2,5-Oxazolylen, 2,5-Thiazolylen, 2,5-Pyridinylen, 2,6-Pyridinylen, 2,5-Pyrimidinylen, 3,6-Pyridazinylen oder 2,5-Pyrazinylen steht,
Z für eine direkte Bindung oder -(CH₂)ₙ- steht,
E für Wasserstoff, Fluor, Chlor, Brom, Hydroxy, Cyano, Formyl, Nitro, Trimethylsilyl, Dimethyl-tert-butylsilyl, -S(O)_{w}R⁷, -OSO₂R⁷, -NR⁸R⁹, -COR⁷, -CO₂R⁷, -OC(O)R⁷, -CONR¹⁰R¹¹, -N(R¹²)COR¹³, -SO₂NR¹⁶R¹⁷; für jeweils gegebenenfalls einfach oder mehrfach, gleich oder verschieden durch Fluor, Chlor, Brom, Cyano, C₁-C₆-Alkoxy und/oder -NR⁸R⁹ substituiertes C₁-C₁₆-Alkyl, C₂-C₁₆-Alkenyl, C₂-C₆-Alkinyl, C₁-C₁₆-Alkoxy, C₂-C₁₆-Alkenyloxy; oder für jeweils gegebenenfalls einfach bis dreifach, gleich oder verschieden, durch Fluor, Chlor, Brom, Cyano, C₁-C₄-Akyl, C₁-C₄-Halogenalkyl, C₂-C₆-Alkenyl, C₂-C₆-Halogenalkenyl, C₁-C₄-Alkoxy, C₁-C₄-Halogenakoxy, C₁-C₄-Alkylthio, C₁-C₄-Halogenalkylthio substituiertes C₃-C₁₀-Cycloalkyl, C₃C₆-Cycloakyl-C₁-C₄-alkyl, C₃-C₁₀-Cycloalkyloxy, Phenyl, Phenoxy, Benzyl, Phenylethyl, Benzyloxy, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidino, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Piperidino, Morpholinyl, Thiomorpholinyl, Morpholino, Thiomorpholino, Triazinyl, Triazolyl, Chinolinyl oder Isochinolinyl steht,
W¹ für Fluor, Chlor, Brom, Cyano, Formyl, Trimethylsilyl, Dimethyl-tert-butylsilyl, C₁-C₄-Alkyl, C₁-C₄-Alkoxy, C₂-C₄-Alkenyl, C₂-C₄-Alkenyloxy; für C₁-C₄-Halogenalkyl, C₁-C₄-Halogenalkoxy mit jeweils 1 bis 9 Fluor-, Chlor- und/oder Bromatomen, C₂-C₄-Halogenalkenyl, C₂-C₄-Halogenalkenyloxy mit jeweils 1 bis 8 Fluor, Chlor- und/oder Bromatomen; für C₁-C₄-Alkylcarbonyl, C₁-C₄-Alkoxycarbonyl, -S(O)_{w}R⁷, -SO₂NR¹⁶R¹⁷, -(CH₂)ₚNR¹⁶R¹⁷, -(CH₂)ₚN(R¹⁶)COR¹⁷, -(CH₂)ₚN(R¹⁶)SO₂R¹⁷, -OSO₂R¹⁶, -OSO₂NR¹⁶R¹⁷ steht,
R⁶ für Methyl, Ethyl, n-Propyl, i-Propyl, n-Butyl, i-Butyl, s-Butyl, t-Butyl, Trifluormethyl, Trifluorethyl, oder für jeweils gegebenenfalls einfach bis vierfach, gleich oder verschieden durch Fluor, Chlor, Brom, C₁-C₄-Alkyl, C₁-C₄-Alkoxy, C₁-C₄-Alkylthio, C₁-C₄-Halogenalkyl, C₁-C₄-Halogenalkoxy und/oder C₁-C₄-Halogenalkylthio, mit jeweils 1 bis 9 Fluor-, Chlor- und/oder Bromatomen, substituiertes Cyclopropyl, Cyclopropylmethyl, Cyclopentyl, Cyclopentylmethyl, Cyclohexyl, Cyclohexylmethyl, Phenyl, Benzyl oder Phenylethyl steht,
R⁷ für gegebenenfalls einfach oder mehrfach, gleich oder verschieden durch Fluor, Chlor, Brom und/oder -N-R⁸R⁹ substituiertes C₁-C₁₀-Alkyl, für jeweils gegebenenfalls einfach bis vierfach, gleich oder verschieden durch Fluor, Chlor, Brom, Cyano, C₁-C₄-Alkyl, C₁-C₄-Alkoxy, C₁-C₄-Akylthio, C₁-C₄-Halogenalkyl, C₁-C₄-Halogenalkoxy, und/oder C₁-C₄-Halogenalkylthio, mit jeweils 1 bis 9 Fluor-, Chlor und/oder Bromatomen, substituiertes Cyclopropyl, Cyclopentyl, Cyclohexyl, Phenyl oder Benzyl steht,
R⁸ und R⁹ unabhängig voneinander für Wasserstoff, -SO₂R⁷, -COR⁷, -CO₂R⁷, für jeweils gegebenenfalls einfach oder mehrfach, gleich oder verschieden, durch Fluor, Chlor, Brom, C₁-C₄-Alkylcarbonyl, C₁-C₄-Akylcarbonyloxy, C₁-C₄-Alkylamino, Di-(C₁-C₄-alkyl)amino, C₁-C₄-Alkoxy, C₁-C₄-Halogenalkoxy, C₁-C₄-Alkylthio, C₁-C₄-Halogenalkylthio substituiertes C₁-C₁₆-Akyl oder C₂-C₁₆-Alkenyl; für jeweils gegebenenfalls einfach bis dreifach, gleich oder verschieden durch Fluor, Chlor, Brom, Cyano, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenakylthio substituiertes C₃-C₁₀-Cycloalkyl, C₃-C₆-Cycloalkyl-C₁-C₄-alkyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl stehen,
R⁸ und R⁹ außerdem gemeinsam für gegebenenfalls einfach oder mehrfach, gleich oder verschieden durch Fluor, Chlor, Brom, C₁-C₄-Alkoxy, C₁-C₄-Halogenalkoxy, C₁-C₄-Alkylthio, C₁-C₄-Halogenalkylthio substituiertes C₂-C₁₀-Alkenylen oder für gegebenenfalls im Alkylenteil einfach oder mehrfach, gleich oder verschieden durch Fluor, Chlor, Cyano, C₁-C₄-Akyl, C₁-C₄-Halogenalkyl, C₁-C₄-Alkoxy, C₁-C₄-Halogenalkoxy, C₁-C₄-Alkylthio, C₁-C₄-Halogenalkylthio substituiertes C₃-C₁₀-Alkylen, wobei die Alkylenkette jeweils durch -O-, -S- oder -NR¹⁸- unterbrochen sein kann, stehen,
R¹⁰ und R¹¹ unabhängig voneinander für Wasserstoff, -SO₂R⁷, für jeweils gegebenenfalls einfach bis neunfach, gleich oder verschieden durch Fluor, Chlor, Brom, C₁-C₄-Alkylamino, Di-(C₁-C₄-alkyl)amino, C₁-C₄-Alkoxy, C₁-C₄-Halogenalkoxy, C₁-C₄-Akylthio, C₁-C₄-Halogenalkylthio substituiertes C₁-C₆-Alkyl oder C₂-C₆-Alkenyl; für jeweils gegebenenfalls einfach bis dreifach, gleich oder verschieden durch Fluor, Chlor, Brom, Cyano, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio substituiertes C₃-C₆-Cycloalkyl, C₃-C₆-Cycloakyl-C₁-C₄-alkyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl stehen,
R¹⁰ und R¹¹ außerdem gemeinsam für jeweils gegebenenfalls im Alkylenteil einfach bis vierfach, gleich oder verschieden durch Fluor, Chlor, Cyano, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio substituiertes C₄-C₆-Alkylen, -(CH₂)₂-O-(CH₂)₂-, -(CH₂)₂-S-(CH₂)₂-, -(CH₂)₂-N(R¹⁸)-(CH₂)₂- stehen,
R¹² und R¹³ unabhängig voneinander für Wasserstoff, für gegebenenfalls einfach bis neunfach, gleich oder verschieden durch Fluor, Chlor, Brom, C₁-C₄-Alkoxy, C₁-C₄-Alkylthio, substituiertes C₁-C₆-Alkyl, für jeweils gegebenenfalls einfach bis vierfach, gleich oder verschieden durch Fluor, Chlor, Brom, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio substituiertes C₃-C₆-Cycloalkyl, C₃-C₆-Cycloalkyl-C₁-C₄-alkyl, Phenyl, Benzyl oder Phenylethyl stehen,
R¹² und R¹³ außerdem gemeinsam für jeweils gegebenenfalls einfach bis vierfach, gleich oder verschieden durch Fluor; Chlor, Brom, C₁-C₄-Alkyl, C₁-C₄-Halogenalkyl, C₁-C₄-Akoxy, C₁-C₄-Halogenalkoxy, C₁-C₄-Alkylthio, C₁-C₄-Halogenalkylthio substituiertes C₃-C₈-Alkylen oder C₃-C₈-Alkenylen stehen,
R¹⁶ und R¹⁷ unabhängig voneinander für Wasserstoff, C₁-C₆-Alkyl, C₁-C₄-Halogenalkyl mit 1 bis 9 Fluor-, Chlor- und/oder Bromatomen oder für gegebenenfalls einfach bis vierfach, gleich oder verschieden durch Fluor, Chlor, Brom und/oder C₁-C₄-Akyl substituiertes C₃-C₆-Cycloalkyl stehen,
R¹⁶ und R¹⁷ außerdem gemeinsam für -(CH₂)₃-, -(CH₂)₄-, -(CH₂)₅-, -(CH₂)₂-O-(CH₂)₂- oder -(CH₂)₂-S-(CH₂)₂- stehen,
R¹⁸ für Wasserstoff, -SO₂R⁷, für -COR⁷ oder -CO₂R⁷; für jeweils gegebenenfalls einfach oder mehrfach, gleich oder verschieden durch Fluor, Chlor, Brom, Cyano, Methylamino, Ethylamino, Di-(C₁-C₆-alkyl)amino, C₁-C₄-Alkoxy, C₁-C₄-Halogenalkoxy, C₁-C₄-Alkylthio, C₁-C₄-Halogenalkylthio substituiertes C₁-C₁₆-Akyl oder C₂-C₁₆-Alkenyl; für jeweils gegebenenfalls einfach bis dreifach, gleich oder verschieden durch Fluor, Chlor, Brom, Cyano, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio substituiertes C₃-C₁₀-Cycloalkyl, C₃-C₆-Cycloakyl-C₁-C₄-alkyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl steht,
w für 0, 1 oder 2 steht,
n für 1 oder 2 steht,
p für 0 oder 1 steht.

5. Δ¹-Pyrroline der Formel (I) gemäß Anspruch 1, in welcher
R¹ für Fluor, Chlor, Brom, Methyl, Trifluormethyl, methoxy, Trifluormethoxy, Methylthio oder Trifluormethylthio steht,
R² und R³ unabhängig voneinander für Wasserstoff, Fluor, Chlor, Brom, Methyl, Trifluormethyl, Methoxy oder Trifluormethoxy stehen,
Het für jeweils gegebenenfalls einfach oder zweifach, gleich oder verschieden durch R⁵ substituiertes. 2-Thienyl, 3-Thienyl, 2-Benzo[b]thienyl, 2-Furyl, 3-Furyl, 2-Benzo[b]furyl, 2-Thieno[3,2-b]-thienyl, 2-Thieno[3,2-b]furyl, 5-Thieno[3,2-b]furyl, 2-Thieno[2,3-f][1]-benzothienyl, 2-Thieno[2,3-f][1]benzofuryl, 6-Thieno[2,3-f][1]benzofuryl, 2-Pyridinyl, 3-Pyridinyl, 2-Pyrimidinyl, 5-Pyrimidinyl, 3-Pyridazinyl, 4-Pyridazinyl oder 2-Pyrazinyl steht,
R⁵ für die Gruppierung -X-Y-Z-E steht mit der Maßgabe; dass Y nicht für eine direkte Bindung steht, wenn X nicht für eine direkte Bindung steht,
X für eine direkte Bindung, Sauerstoff Schwefel, -SO₂-, -NR⁶-, -CO-, -C(O)-O-, -O-C(O)-, -CH₂-, -(CH₂)₂-, -C=C- (E oder Z), -C≡C-, -CH₂O-, -(CH₂)₂O-, -OCH₂-, -O(CH₂)₂-, -O-CH₂-O-, -SCH₂-, -S(CH₂)₂-, -CH₂S- oder -(CH₂)₂S- steht,
Y für eine direkte Bindung oder für jeweils gegebenenfalls einfach oder zweifach, gleich oder verschieden durch Reste aus der Liste W¹ substituiertes 1,4-Phenylen, 1,3-Phenylen, 2,6-Naphthylen, 2,7-Naphthylen, 2,4-Furylen, 2,4-Thienylen, 2,5-Pyridinylen, 2,5-Pyrimidinylen, 3,6-Pyridazinylen oder 2,5-Pyrazinylen steht,
Z eine direkte Bindung, Methylen oder Ethylen steht,
E für Wasserstoff, Fluor, Chlor, Brom, Hydroxy, Cyano, Formyl, -S(O)_{w}R⁷, -OSO₂R⁷, -NR⁸R⁹, -COR⁷, -CO₂R⁷, -OC(O)R⁷, -CONR¹⁰R¹¹, -N(R¹²)COR¹³, -SO₂NR¹⁶R¹⁷; für jeweils gegebenenfalls einfach oder mehrfach, gleich oder verschieden durch Fluor, Chlor, Brom, Cyano, C₁-C₆-Alkoxy und/oder -NR⁸R⁹ substituiertes C₁-C₁₆-Alkyl, C₂-C₁₆-Alkenyl, C₁-C₁₆-Alkoxy, C₂-C₁₆-Alkenyloxy; oder für jeweils gegebenenfalls einfach bis dreifach, gleich oder verschieden, durch Fluor, Chlor, Brom, Cyano, Methyl, Ethyl, n-Propyl, i-Propyl, -CF₃, -CHF₂, -CClF₂, -CF₂CHFCl, -CF₂CH₂F, -CF₂CCl₃, -CH₂CF₃, -CF₂CHFCF₃, -CH₂CF₂H, -CH₂CF₂CF₃, -CF₂CF₂H, -CF₂CHFCF₃, Vinyl, Allyl, 1-Propenyl, Butenyl, -CF=CHF, -CF=CH₂, -CF=CCl₂, -CH=CF₂, -CF₂CF=CF₂, -CH=CFH, -CH₂CF=CF₂, -CF=CF₂, -CF₂CH=CF₂, Methoxy, Ethoxy, n-Propoxy, i-Propoxy, n-Butoxy, i-Butoxy, s-Butoxy, t-Butoxy, Trifluormethoxy, Difluormethoxy, Chlordifluormethoxy, Trifluorethoxy, Methylthio, Ethylthio, n-Propylthio, i-Propylthio, n-Butylthio, i-Butylthio, s-Butylthio, t-Butylthio, Trifluormethylthio, Difluormethylthio, Chlordifluormethylthio, Trifluorethylthio substituiertes Cyclopropyl, Cyclopentyl, Cyclohexyl, Cyclopropyloxy, Cyclopentyloxy, Cyclohexyloxy, Cyclopropylmethyl, Cyclopentylmethyl, Cyclohexylmethyl, Phenyl, Phenoxy, Benzyl, Phenylethyl, Benzyloxy, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidino, Pyrrolidinyl, Oxazolyl, Benzoxazolyl, Isoxazolyl, Imidazolyl Pyrazolyl, Thiazolyl, Benzothiazolyl, Thiazolidinyl, Pyridinyl, Pyrimidinyl, Pyridazyl, Pyrazinyl, Piperidinyl, Piperidino, Morpholinyl, Thiomorpholinyl, Morpholino, Thiomorpholino, Triazinyl, Triazolyl, Chinolinyl oder Isochinolinyl steht,
W¹ für Fluor, Chlor, Brom, Cyano, Formyl, Methyl, Ethyl, n-Propyl, i-Propyl, n-Butyl, i-Butyl, s-Butyl, t-Butyl, Methoxy, Ethoxy, n-Propoxy, i-Propoxy, n-Butoxy, i-Butoxy, s-Butoxy, t-Butoxy, Vinyl, Allyl, Trifluormethyl, Trifluorethyl, Trifluormethoxy, Trifluorethoxy, -OCF₂CF₂H, -CH=CF₂, -CH=CCl₂, -OCF=CF₂, -COMe, -COEt, -CO₂Me, -CO₂Et, -CO₂(t-Bu), -SMe, -SOMe, -SO₂Me, -SCF₃, -SOCF₃, -SO₂CF₃, -SCHF₂, -SOCHF₂, -SO₂CHF₂, -SO₂NMe₂, -NMe₂, -NEt₂, -N(n-Pr)₂, -N(Me)COMe, -N(Me)COEt, -N(Me)COPr, -N(Me)CO(t-Bu), 2-Pyrrolidonyl, 2-Piperidonyl, -N(Me)SO₂Me, -N(Me)SO₂Et, -N(Me)SO₂CF₃, -N(Et)SO₂CF₃, -N(Me)SO₂(CF₂)₃CF₃ oder -OSO₂NMe₂ steht,
R⁶ für Methyl, Ethyl, n-Propyl, i-Propyl, n-Butyl, i-Butyl, s-Butyl, t-Butyl, Trifluormethyl, Trifluorethyl, Cyclopropyl, Cyclopropylmethyl, Cyclopentyl, Cyclopentylmethyl, Cyclohexyl, Cyclohexylmethyl, Phenyl, Benzyl oder Phenylethyl steht,
R⁷ für Methyl, Ethyl, n-Propyl, i-Propyl, n-Butyl, i-Butyl, s-Butyl, t-Butyl, Pentyl, Hexyl, -CF₃, -CHF₂, -CCl₃, -CCl₂F, Dimethylaminomethyl, Dimethylaminoethyl, Diethylaminomethyl, Diethylaminoethyl, Cyclopropyl, Cyclopentyl, Cyclohexyl, Phenyl oder Benzyl steht,
R⁸ und R⁹ unabhängig voneinander für Wasserstoff, -SO₂R⁷, -COR⁷, -CO₂R⁷, für jeweils gegebenenfalls einfach oder mehrfach, gleich oder verschieden, durch Fluor, Chlor, Brom, C₁-C₄-Alkylcarbonyl, C₁-C₄-Alkylcarbonyloxy, C₁-C₄-Alkylamino, Di-(C₁-C₄-alkyl)amino, C₁-C₄-Alkoxy, C₁-C₄-Halogenalkoxy, C₁-C₄-Alkylthio, C₁-C₄-Halogenalkylthio substituiertes C₁-C₁₆-Alkyl, C₂-C₁₆-Alkenyl; für jeweils gegebenenfalls einfach bis dreifach, gleich oder verschieden durch Fluor, Chlor, Brom, Cyano, C₁-C₄-Alkyl, C₁-C₄-Halogenakyl, C₁-C₄-Alkoxy, C₁-C₄-Halogenalkoxy, C₁-C₄-Alkylthio, C₁-C₄-Halogenalkylthio substituiertes C₃-C₈-Cycloalkyl, Cyclopropylmethyl, Cyc1opentylmethyl, Cyclohexylmethyl, Cyclopropylethyl, Cyclopentylethyl, Cyclohexylethyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl stehen,
R⁸ und R⁹ außerdem gemeinsam für gegebenenfalls einfach oder mehrfach, gleich oder verschieden durch Fluor, Chlor, Brom, C₁-C₄-Alkoxy, C₁-C₄-Halogenalkoxy, C₁-C₄-Alkylthio, C₁-C₄-Halogenalkylthio substituiertes C₂-C₈-Alkenylen oder für gegebenenfalls im Alkylenteil einfach oder mehrfach, gleich oder verschieden durch Fluor, Chlor, Cyano, Methyl, Ethyl, n-Propyl, i-Propyl, n-Butyl, i-Butyl, s-Butyl, t-Butyl, C₁-C₄-Halogenalkyl, Methoxy, Ethoxy, n-Propoxy, i-Propoxy, n-Butoxy, i-Butoxy, s-Butoxy, t-Butoxy, C₁-C₄-Halogenalkoxy, Methylthio, Ethylthio, n-Propylthio, i-Propylthio, n-Butylthio, i-Butylthio, s-Butylthio, t-Butylthio, C₁-C₄-Halogenalkylthio substituiertes C₃-C₈-Alkylen, wobei die Alkylenkette jeweils durch -O-, -S- oder -NR¹⁸-unterbrochen sein kann, stehen,
R¹⁰ und R¹¹ unabhängig voneinander für Wasserstoff, -SO₂CF₃, Methyl, Ethyl, n-Propyl, i-Propyl, n-Butyl, i-Butyl, s-Butyl, t-Butyl, Pentyl, Hexyl, -CF₃, -CH₂CF₃, -(CF₂)₃CF₃, Methoxymethyl, Methoxyethyl, Cyclopropyl, Cyclopentyl, Cyclohexyl, oder für jeweils gegebenenfalls einfach bis dreifach, gleich oder verschieden durch Fluor, Chlor, Brom, Methyl, Trifluormethyl, Methoxy, Trifluormethoxy substituiertes Phenyl oder Benzyl stehen,
R¹⁰ und R¹¹ außerdem gemeinsam für -(CH₂)₄-, -(CH₂)₅-, -(CH₂)₆-, -CH₂-CH(CH₃)-CH₂-CH(CH₃)-CH₂-, -(CH₂)₂-O-(CH₂)₂-, -(CH₂)₂-S-(CH₂)₂- oder -(CH₂)₂-N(R¹⁸)-(CH₂)₂- stehen,
R¹² und R¹³ unabhängig voneinander für Wasserstoff, Methyl, Ethyl, n-Propyl, i-Propyl, n-Butyl, i-Butyl, s-Butyl, t-Butyl, n-Hexyl, Trifluormethyl, Trifluorethyl, Cyclopropyl, Cyclopentyl, Cyclohexyl oder für jeweils gegebenenfalls einfach bis vierfach, gleich oder verschieden durch Fluor, Chlor, Brom, Methyl, Trifluormethyl, Methoxy, Trifluormethoxy substituiertes Phenyl oder Benzyl stehen,
R¹² und R¹³ außerdem gemeinsam für jeweils gegebenenfalls einfach bis vierfach, gleich oder verschieden durch Fluor, Chlor, Brom, Methyl, Ethyl, Methoxy, Ethoxy, Methylthio, Ethylthio, Trifluormethyl, Trifluormethoxy, Trifluormethylthio substituiertes -(CH₂)₃-, -(CH₂)₄-, -(CH₂)₅-, -(CH₂)₆- stehen,
R¹⁶ und R¹⁷ unabhängig voneinander für Wasserstoff, Methyl, Ethyl, n-Propyl, i-Propyl, n-Butyl, i-Butyl, s-Butyl, t-Butyl, n-Hexyl, Trifluormethyl, Trifluorethyl, Cyclopropyl, Cyclopentyl oder Cyclohexyl stehen,
R¹⁶ und R¹⁷ außerdem gemeinsam für -(CH₂)₃-, -(CH₂)₄-, -(CH₂)₅-, -(CH₂)₂-O-(CH₂)₂- oder -(CH₂)₂-S-(CH₂)₂- stehen,
R¹⁸ für Wasserstoff, -SO₂R⁷, für -COR⁷ oder -CO₂R⁷; für gegebenenfalls einfach oder mehrfach, gleich oder verschieden durch Fluor, Chlor, Brom, Cyano, Methylamino, Ethylamino, Di-(C₁-C₆-alkyl)amino, C₁-C₄-Alkoxy, C₁-C₄-Halogenalkoxy, C₁-C₄-Alkylthio, C₁-C₄-Halogenalkylthio-substituiertes C₁-C₁₆-Alkyl, C₂-C₁₆-Alkenyl; für jeweils gegebenenfalls einfach bis dreifach, gleich oder verschieden durch Fluor, Chlor, Brom, Cyano, Methyl, Ethyl, n-Propyl, i-Propyl, n-Butyl, i-Butyl, s-Butyl, t-Butyl, C₁-C₄-Halogenalkyl, Methoxy, Ethoxy, n-Propoxy, i-Propoxy, n-Butoxy, i-Butoxy, s-Butoxy, t-Butoxy, C₁-C₄-Halogenalkoxy, Methylthio, Ethylthio, n-Propylthio, i-Propylthio, n-Butylthio, i-Butylthio, s-Butylthio, t-Butylthio, C₁-C₄-Halogendkylthio substituiertes C₃-C₈-Cycloalkyl, Cyclopropylmethyl, Cyclopentylmethyl, Cyclohexylmethyl, Cyclopropylethyl, Cyclopentylethyl, Cyclohexylethyl, Phenyl, Benzyl, Phenylethyl, Tetrazolyl, Furyl, Furfuryl, Benzofuryl, Tetrahydrofuryl, Thienyl, Thenyl, Benzothienyl, Thiolanyl, Pyrrolyl, Indolyl, Pyrrolinyl, Pyrrolidinyl, Oxazolyl, Benzoxazolyl, Isoxazolyl, Imidazolyl, Pyrazolyl, Thiazolyl, Benzothiazolyl, Thiazolidinyl, Pyridinyl, Pyrimidinyl, Pyridazyl, Pyrazinyl, Piperidinyl, Morpholinyl, Thiomorpholinyl, Triazinyl, Triazolyl, Chinolinyl oder Isochinolinyl steht.

6. Δ¹-Pyrroline der Formel 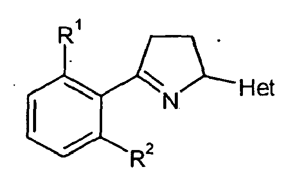 gemäß Anspruch 1, in welcher
R¹ für Fluor oder Chlor steht,
R² für Wasserstoff oder Fluor steht und
Het die in einem oder mehreren der Ansprüche 1 bis 5 angegebenen Bedeutungen hat.

7. Verbindungen der Formeln (I-1) bis (I-16) 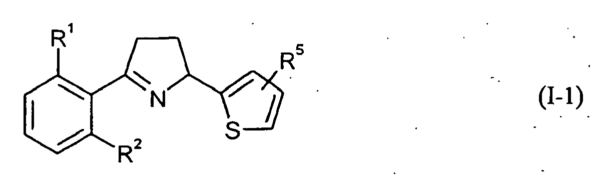 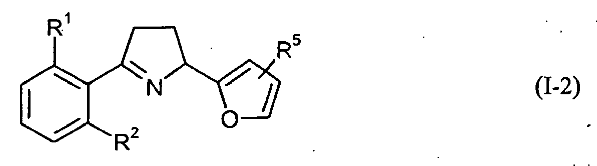 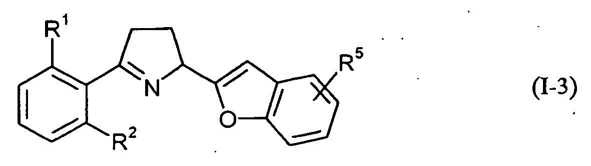 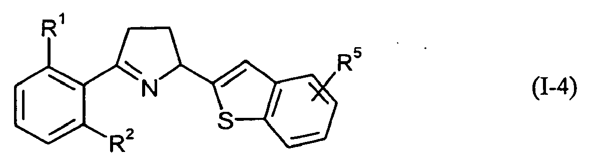 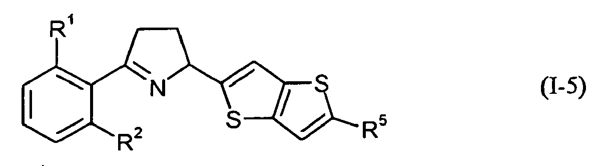 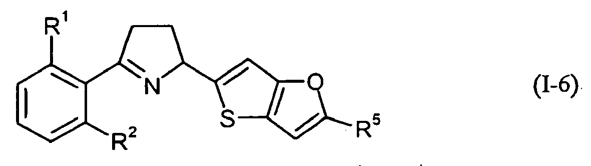 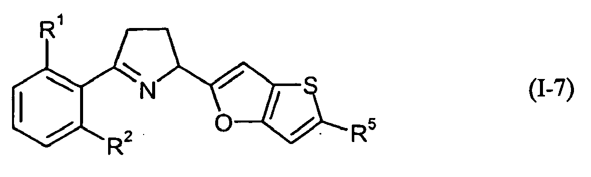 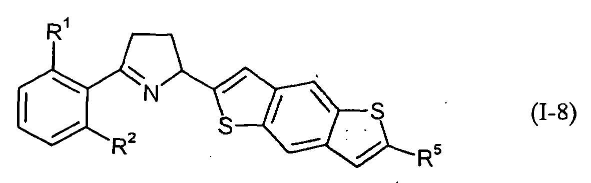 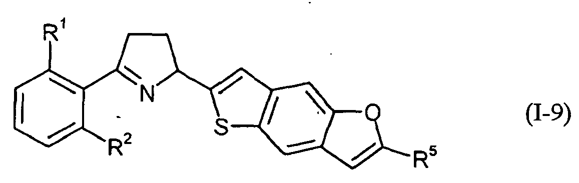 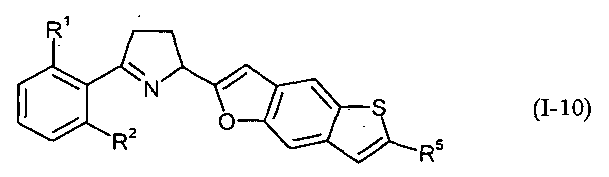 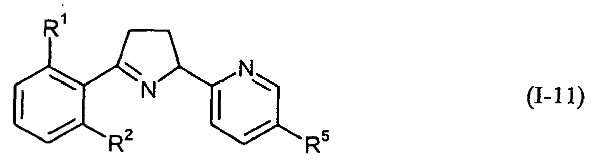 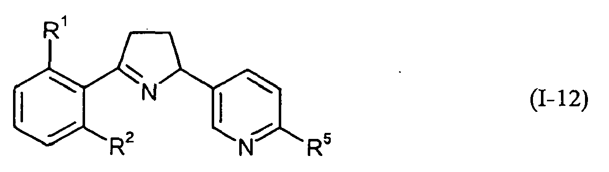 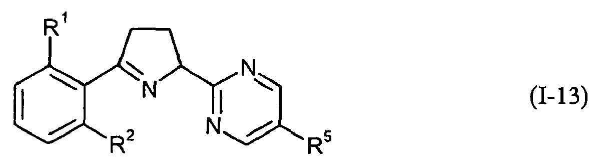 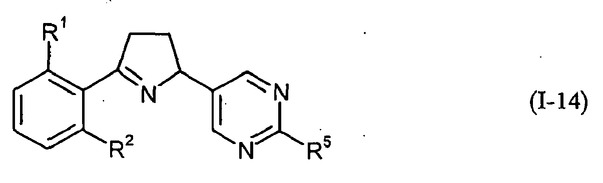 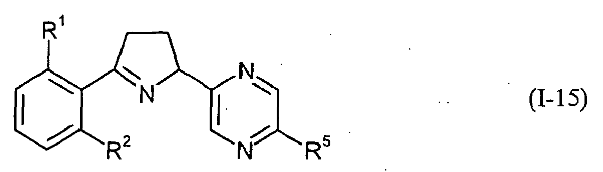 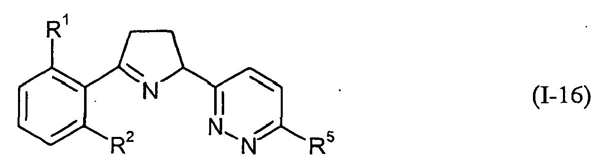 gemäß Anspruch 1, in welchen jeweils
R¹ für Fluor oder Chlor steht,
R² für Wasserstoff oder Fluor steht und
R⁵ die in einem oder mehreren der Ansprüche 1 bis 5 angegebenen Bedeutungen hat.

8. Verbindungen der Formel (I-d) 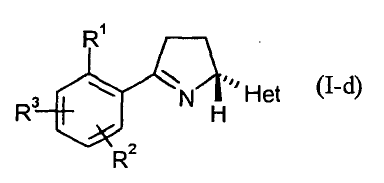 mit (R)-Konfiguration gemäß Anspruch 1, in welcher
R¹, R², R³ und Het die in einem oder mehreren der Ansprüche 1 bis 5 angegebenen Bedeutungen haben.

9. Δ¹-Pyrroline der Formel (I) gemäß Anspruch 1, in welcher Het für jeweils gegebenenfalls einfach oder zweifach, gleich oder verschieden durch R⁵ substituiertes 2-Thienyl, 3-Thienyl, 2-Thieno[3,2-b]-thienyl, 2-Pyridinyl oder 3-Pyridinyl steht.

10. Δ¹-Pyrroline der Formel (I) gemäß Anspruch 1, in welcher R¹ für Fluor oder Chlor, R² für Wasserstoff oder Fluor und R³ für Wasserstoff steht.

11. Δ¹-Pyrroline der Formel (I) gemäß Anspruch 1, in welcher R² für Wasserstoff oder Fluor steht, wobei R² die 6-Position des Phenylrings, an das es gebunden ist, einnimmt.

12. Δ¹-Pyrroline der Formel (I) gemäß Anspruch 1, in welcher X für eine direkte Bindung und Y für Phenylen, bevorzugt 1,4-Phenylen, steht.

13. Δ¹-Pyrroline der Formel (I) gemäß Anspruch 9, in welcher X für eine direkte Bindung und Y für Phenylen, bevorzugt 1,4-Phenylen, steht.

14. Verfahren zur Herstellung von Verbindungen der Formel (I) gemäß Anspruch 1, **dadurch gekennzeichnet,**
A) **dass** man Aminoketone der Formel (II) 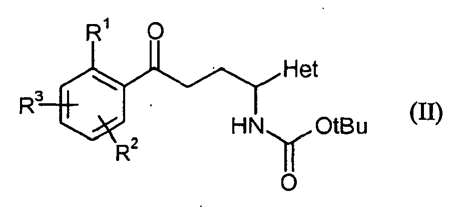 in welcher
R¹, R², R³ und Het die in Anspruch 1 angegebenen Bedeutungen haben,
mit einer Lewissäure oder einer Protonsäure gegebenenfalls in Gegenwart eines Verdünnungsmittels behandelt
oder
B) **dass** man Azide der Formel (III) 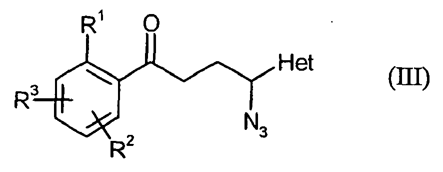 in welcher
R¹, R², R³ und Het die in Anspruch 1 angegebenen Bedeutungen haben,
mit einem Trialkylphosphin oder einem Triarylphosphin oder einem Trialkylphosphit oder einem Reduktionsmittel in Gegenwart eines Verdünnungsmittels und gegebenenfalls in Gegenwart eines Katalysators umsetzt,
oder
C) **dass** man Amide der Formel (IV) 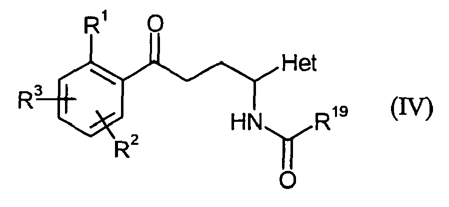 in welcher
R¹⁹ für Alkyl, Halogenalkyl, Aryl oder Aralkyl steht und
R¹, R², R³, Het die in Anspruch 1 angegebenen Bedeutungen haben,
mit einem N-Entacylierungsmittel gegebenenfalls in Gegenwart eines Verdünnungsmittels umsetzt,
oder
**dass** man Δ¹-Pyrroline der Formel (I-a) 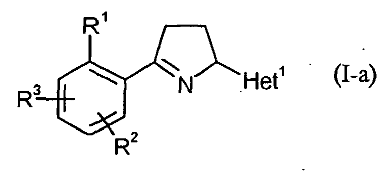 in welcher
Het¹ für einfach durch R⁵⁻¹ substituiertes Heteroaryl steht,
R⁵⁻¹ für die Gruppierung -Y¹-E steht,
Y¹ für jeweils gegebenenfalls substituiertes Phenylen oder Heterocyclylen steht und
R¹, R², R³ und E die in Anspruch 1 angegebenen Bedeutungen haben,
erhält, indem man
D) Δ¹-Pyrroline der Formel (I-b) 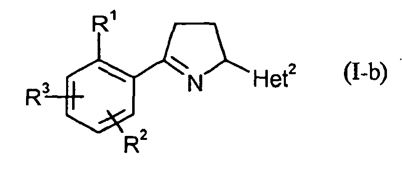 in welcher
Het² für einfach durch R⁵⁻² substituiertes Heteroaryl steht,
R⁵⁻² für Chlor, Brom, Iod, -OSO₂CF₃ oder -OSO₂(CF₂)₃CF₃ steht,
R¹, R² und R³ die in Anspruch 1 angegebenen Bedeutungen haben,
mit (Hetero)cyclen der Formel (V) 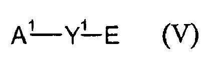 in welcher
E die in Anspruch 1 angegebenen Bedeutungen hat,
Y¹ die oben angegebenen Bedeutungen hat,
A¹ für Chlor, Brom, Iod, -OSO₂CF₃ oder -OSO₂(CF₂)₃CF₃ steht,
in Gegenwart eines Katalysators, in Gegenwart eines Diboronsäureesters und gegebenenfalls in Gegenwart eines Säurebindemittels und gegebenenfalls in Gegenwart eines Verdünnungsmittels in einer Tandem-Reaktion umsetzt,
oder
E) Δ¹-Pyrroline der Formel (VI) 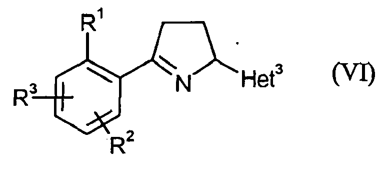 in welcher
Het³ für einfach durch A² substituiertes Heteroaryl steht,
A² für -B(OH)₂, (4,4,5,5-Tetramethyl-1,3,2-dioxaborolan)-2-yl, (5,5-Dimethyl-1,3,2-dioxaborinan)-2-yl, (4,4,6-Trimethyl-1,3,2-dioxaborinan)-2-yl oder 1,3,2-Benzodioxaborol-2-yl steht und
R¹, R² und R³ die in Anspruch 1 angegebenen Bedeutungen haben,
mit Heterocyclen der Formel (V) 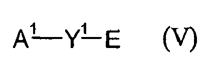 in welcher
E die in Anspruch 1 angegebenen Bedeutungen hat,
Y¹ und A¹ die oben angegebenen Bedeutungen haben,
in Gegenwart eines Katalysators, gegebenenfalls in Gegenwart eines Säurebindemittels und gegebenenfalls in Gegenwart eines Verdünnungsmittels umsetzt,
oder
F) Δ¹-Pyrroline der Formel (I-b) 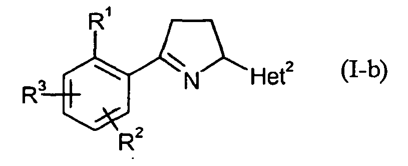 in welcher
R¹, R² und R³ die in Anspruch 1 angegebenen Bedeutungen haben,
Het² die oben angegebenen Bedeutungen hat,
mit Boronsäure-Derivaten der Formel (VII) 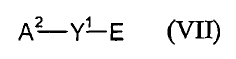 in welcher
E die in Anspruch 1 angegebenen Bedeutungen hat,
Y¹ und A² die oben angegebenen Bedeutungen haben,
in Gegenwart eines Katalysators, gegebenenfalls in Gegenwart eines Säurebindemittels und gegebenenfalls in Gegenwart eines Verdünnungsmittels umsetzt,
oder
G) Δ¹-Pyrroline der Formel (I-c) 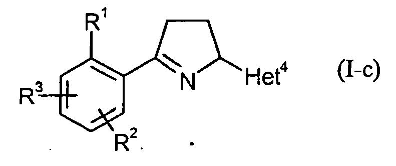
in welcher
Het⁴ für einfach durch R⁵⁻³ substituiertes Heteroaryl steht,
R⁵⁻³ für Brom oder Iod steht und
R¹, R² und R³ die in Anspruch 1 angegebenen Bedeutungen haben,
mit metallorganischen Verbindungen der Formel (VIII) 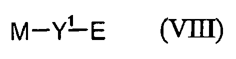 in welcher
E die in Anspruch 1 angegebenen Bedeutungen hat,
Y¹ die oben angegebenen Bedeutungen hat,
M für ZnCl, Sn(Me)₃ oder Sn(n-Bu)₃ steht,
in Gegenwart eines Katalysators, gegebenenfalls in Gegenwart eines Säurebindemittels und gegebenenfalls in Gegenwart eines Verdünnungsmittels umsetzt.

15. Δ¹-Pyrroline der Formel (I-d) 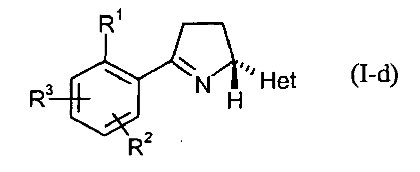 gemäß Anspruch 1, in welcher
R¹, R², R³ und Het die in Anspruch 1 angegebenen Bedeutungen haben.

16. Aminoketone der Formel (II) 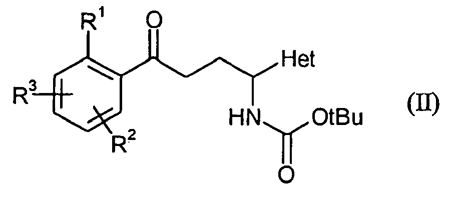 in welcher
R¹, R², R³ und Het die in Anspruch 1 angegebenen Bedeutungen haben.

17. Azide der Formel (III) 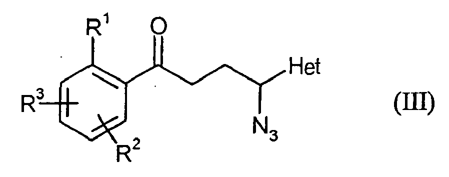 in welcher
R¹, R², R³ und Het die in Anspruch 1 angegebenen Bedeutungen haben.

18. Amide der Formel (IV) 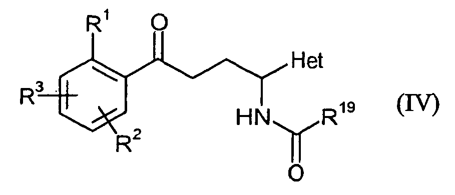 in welcher
R¹⁹ für Alkyl, Halogenalkyl, Aryl oder Arylalkyl steht und
R¹, R², R³ und Het die in Anspruch 1 angegebenen Bedeutungen haben.

19. Lactame der Formel (IX) 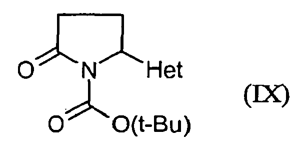 in welcher
Het die in Anspruch 1 angegebenen Bedeutungen hat.

20. Halogenide der Formel (XV) 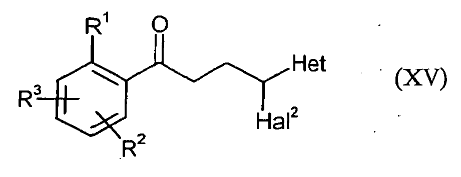 in welcher
R¹, R², R³ und Het die in Anspruch 1 angegebenen Bedeutungen haben und
Hal² für Halogen steht.

21. Schädlingsbekämpfungsmittel, **gekennzeichnet durch** einen Gehalt an mindestens einer Verbindung gemäß irgendeinem der Ansprüche 1 bis 13 und 15 neben Streckmitteln und/oder oberflächenaktiven Stoffen.

22. Verwendung von Verbindungen gemäß irgendeinem der Ansprüche 1 bis 13 und 15 zur Bekämpfung von Schädlingen, ausgenommen zur therapeutischen Behandlung des menschlichen oder tierischen Körpers.

23. Verfahren zur Bekämpfung von Schädlingen, **dadurch gekennzeichnet, dass** man Verbindungen gemäß irgendeinem der Ansprüche 1 bis 13 und 15 auf Schädlinge und/oder ihren Lebensraum einwirken lässt, ausgenommen zur therapeutischen Behandlung des menschlichen oder tierischen Körpers.

24. Verwendung von Verbindungen gemäß irgendeinem der Ansprüche 1 bis 13 und 15 zur Herstellung eines Tierarzneimittels gegen tierische Parasiten, insbesondere Ektoparasiten.

25. Verfahren zur Herstellung von Schädlingsbekämpfungsmitteln, **dadurch gekennzeichnet, dass** man Verbindungen gemäß irgendeinem der Ansprüche 1 bis 13 und 15 mit Streckmitteln und/oder oberflächenaktiven Stoffen vermischt.

## Claims

1. Δ¹-pyrrolines of the formula (I) 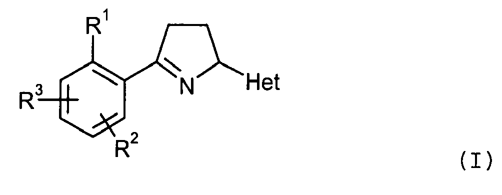 in which
R¹ represents halogen, in each case optionally substituted alkyl or alkoxy or -S(O)_{w}R⁴,
R² and R³ independently of one another represent hydrogen, halogen or in each case optionally substituted alkyl, alkoxy or alkoxyalkyl,
R⁴ represents optionally substituted alkyl,
Het represents heteroaryl which is optionally mono- or polysubstituted by identical or different R⁵,
R⁵ represents the grouping -X-Y-Z-E with the proviso that Y does not represent a direct bond if X does not represent a direct bond,
X represents a direct bond, oxygen, -S(O)_{w}-, -NR⁶-, carbonyl, carbonyloxy, oxycarbonyl, oxysulfonyl (OSO₂), alkylene, halogenalkylene, alkenylene, halogenoalkenylene, alkinylene, alkyleneoxy, oxyalkylene, oxyalkyleneoxy, -S(O)_{w}-alkylene, cyclopropylene or oxiranylene,
Y represents a direct bond or represents in each case optionally substituted phenylene, naphthylene, tetrahydronaphthylene or heterocyclylene,
Z represents a direct bond or -(CH₂)ₙ-,
E represents hydrogen, halogen, hydroxyl, cyano, formyl, nitro, trialkylsilyl, pentafluorothio, -S(O)_{w}R⁷, -OSO₂R⁷, -NR⁸R⁹, -COR⁷, -CO₂R⁷, -OC(O)R⁷, -CONR¹⁰R¹¹, -N(R¹²)COR¹³, -C(R¹⁴)=N-OR¹⁵, -SO₂NR¹⁶R¹⁷; represents in each case optionally substituted alkyl, alkenyl, alkinyl, alkoxy, alkenyloxy, cycloalkyl, cycloalkylalkyl, cycloalkyloxy, aryl, arylalkyl, aryloxy, aryloxyalkyl, saturated or unsaturated heterocyclyl or heterocyclylalkyl,
R⁶ represents in each case optionally substituted alkyl, cycloalkyl, cycloalkylalkyl, aryl or arylalkyl,
R⁷ represents in each case optionally substituted alkyl, cycloalkyl, aryl or arylalkyl,
R⁸ and R⁹ independently of one another represent hydrogen, -SO₂R⁷, -COR⁷, -CO₂R⁷, represent in each case optionally substituted alkyl, alkenyl, cycloalkyl, cycloalkylalkyl, aryl, arylalkyl or saturated or unsaturated heterocyclyl or heterocyclylalkyl,
R⁸ and R⁹ furthermore together represent in each case optionally substituted alkenylene or alkylene, where the alkylene chain may in each case be interrupted by -O-, -S- or -NR¹⁸-,
R¹⁰ and R¹¹ independently of one another represent hydrogen, -SO₂R⁷, represent in each case optionally substituted alkyl, alkenyl, cycloalkyl, cycloalkylalkyl, aryl, arylalkyl or saturated or unsaturated heterocyclyl or heterocyclylalkyl,
R¹⁰ and R¹¹ furthermore together represent optionally substituted alkylene, where the alkylene chain may in each case be interrupted by -O-, -S- or -NR¹⁸-,
R¹² and R¹³ independently of one another represent hydrogen, represent in each case optionally substituted alkyl, cycloalkyl, cycloalkylalkyl, aryl or arylalkyl,
R¹² and R¹³ furthermore together represent in each case optionally substituted alkylene or alkenylene,
R¹⁴ and R¹⁵ independently of one another represent hydrogen, represent in each case optionally substituted alkyl or alkenyl,
R¹⁶ and R¹⁷ independently of one another represent hydrogen, represent optionally substituted alkyl or cycloalkyl,
R¹⁶ and R¹⁷ furthermore together represent optionally substituted alkylene, alkoxyalkylene or alkylthioalkylene,
R¹⁸ represents hydrogen, -SO₂R⁷, -COR⁷ or -CO₂R⁷; represents in each case optionally substituted alkyl, alkenyl, cycloalkyl, cycloalkylalkyl, aryl, arylalkyl or saturated or unsaturated heterocyclyl or heterocyclylalkyl,
w represents 0, 1 or 2,
n represents 1, 2, 3 or 4.

2. Δ¹-pyrrolines of the formula (I) according to Claim 1, in which
R¹ represents halogen, alkyl, halogenoalkyl, alkoxy, halogenoalkoxy or -S(O)_{w}R⁴,
R² and R³ independently of one another represent hydrogen, halogen, alkyl, halogenoalkyl, alkoxy, halogenoalkoxy or alkoxyalkyl,
R⁴ represents alkyl or halogenoalkyl,
Het represents 5- to 14-membered heteroaryl which comprises 1 to 3 aromatic rings and one or more heteroatoms from the group consisting of nitrogen, oxygen and sulfur and is optionally mono- to tetrasubstituted by identical or different R⁵,
R⁵ represents the grouping -X-Y-Z-E, with the proviso that Y does not represent a direct bond if X does not represent a direct bond,
X represents a direct bond, oxygen, -S(O)_{w}-, -NR⁶-, carbonyl, carbonyloxy, oxycarbonyl, oxysulfonyl (OSO₂), alkylene, halogenoalkylene, alkenylene, halogenoalkenylene, alkinylene, alkyleneoxy, oxyalkylene, oxyalkyleneoxy, -S(O)_{w}-alkylene, cyclopropylene or oxiranylene,
Y represents a direct bond or represents phenylene, naphthylene, tetrahydronaphthylene or 5- to 10-membered saturated or unsaturated heterocyclylene having one or more heteroatoms from the group consisting of nitrogen, oxygen and sulfur, each of which radicals is optionally mono- to tetrasubstituted by identical or different radicals from the list W¹,
Z represents a direct bond or -(CH₂)ₙ-,
E represents hydrogen, halogen, hydroxyl, cyano, formyl, nitro, trialkylsilyl, pentafluorothio, -S(O)_{w}R⁷, -OSO₂R⁷, -NR⁸R⁹, -COR⁷, -CO₂R⁷, -OC(O)R⁷, -CONR¹⁰R¹¹, -N(R¹²) COR¹³, -C(R¹⁴)=N-OR¹⁵, -SO₂NR¹⁶R¹⁷; represents alkyl, alkenyl, alkinyl, alkoxy, alkenyloxy, each of which is optionally mono- or polysubstituted by identical or different substituents from the group consisting of halogen, cyano, alkoxy and/or -NR⁸R⁹; or represents cycloalkyl, cycloalkylalkyl, cycloalkyloxy, aryl, arylalkyl, aryloxy, aryloxyalkyl, saturated or unsaturated 5- to 10-membered heterocyclyl or heterocyclylalkyl having one or more heteroatoms from the group consisting of nitrogen, oxygen and sulfur, each of which radicals is optionally mono- or polysubstituted by identical or different substituents from the group consisting of halogen, cyano, nitro, alkyl, halogenoalkyl, alkenyl, halogenoalkenyl, alkoxy, halogenoalkoxy, alkylthio and halogenoalkylthio,
W¹ represents halogen, cyano, formyl, nitro, trialkylsilyl, alkyl, halogenoalkyl, alkoxy, halogenoalkoxy, alkenyl, halogenoalkenyl, alkenyloxy, halogenoalkenyloxy, alkylcarbonyl, alkoxycarbonyl, -S(O)_{w}R⁷, -C(R¹⁴)=N-OR¹⁵, -SO₂NR¹⁶R¹⁷, -(CH₂)ₚNR¹⁶R¹⁷, -(CH₂)ₚN(R¹⁶)COR¹⁷, -(CH₂)ₚN(R¹⁶) SO₂R¹⁷, -OSO₂R¹⁶, -OSO₂NR¹⁶R¹⁷,
R⁶ represents alkyl, halogenoalkyl or represents cycloalkyl, cycloalkylalkyl, aryl or arylalkyl, each of which is optionally mono- or polysubstituted by identical or different substituents from the group consisting of halogen, alkyl, halogenoalkyl, alkoxy, halogenoalkoxy, alkylthio and/or halogenoalkylthio,
R⁷ represents alkyl which is optionally mono- or polysubstituted by identical or different substituents from the group consisting of halogen and -NR⁸R⁹, represents cycloalkyl, aryl or arylalkyl, each of which is optionally mono- or polysubstituted by identical or different substituents from the group consisting of halogen, cyano, alkyl, halogenoalkyl, alkoxy, halogenoalkoxy, alkylthio and halogenoalkylthio,
R⁸ and R⁹ independently of one another represent hydrogen, -SO₂R⁷, -COR⁷, -CO₂R⁷, represent alkyl or alkenyl, each of which is optionally mono- or polysubstituted by identical or different substituents from the group consisting of halogen, alkylcarbonyl, alkylcarbonyloxy, alkylamino, dialkylamino, alkoxy, halogenoalkoxy, alkylthio and halogenoalkylthio; represents cycloalkyl, cycloalkylalkyl, aryl, arylalkyl or saturated or unsaturated 5- to 10-membered heterocyclyl or heterocyclylalkyl having one or more heteroatoms from the group consisting of nitrogen, oxygen and sulfur, each of which radicals is optionally mono- or polysubstituted by identical or different substituents from the group consisting of halogen, cyano, alkyl, halogenoalkyl, alkoxy, halogenoalkoxy, alkylthio and halogenoalkylthio,
R⁸ and R⁹ furthermore together represent alkenylene which is optionally mono- or polysubstituted by identical or different substituents from the group consisting of halogen, cyano, alkoxy, halogenoalkoxy, alkylthio and halogenoalkylthio or represent alkylene which is optionally mono- or polysubstituted by identical or different substituents from the group consisting of halogen, cyano, alkyl, halogenoalkyl, alkoxy, halogenoalkoxy, alkylthio and halogenoalkylthio, where the alkylene chain may in each case be interrupted by -O-, -S- or -NR¹⁸-,
R¹⁰ and R¹¹ independently of one another represent hydrogen, -SO₂R⁷, represent alkyl or alkenyl, each of which is optionally mono- or polysubstituted by identical or different substituents from the group consisting of halogen, alkylamino, dialkylamino, alkoxy and alkylthio; represent cycloalkyl, cycloalkylalkyl, aryl, arylalkyl or saturated or unsaturated 5- to 10-membered heterocyclyl or heterocyclylalkyl having one or more heteroatoms from the group consisting of nitrogen, oxygen and sulfur, each of which radicals is optionally mono- or polysubstituted by identical or different substituents from the group consisting of halogen, cyano, alkyl, halogenoalkyl, alkoxy, halogenoalkoxy, alkylthio and halogenoalkylthio,
R¹⁰ and R¹¹ furthermore together represent alkylene which is optionally mono- or polysubstituted by identical or different substituents from the group consisting of halogen, cyano, alkyl, halogenoalkyl, alkoxy, halogenoalkoxy, alkylthio and halogenoalkylthio, where the alkylene chain may in each case be interrupted by -O-, -S- or -NR¹⁸-,
R¹² and R¹³ independently of one another represent hydrogen, represent alkyl which is optionally mono- or polysubstituted by identical or different substituents from the group consisting of halogen, cyano, alkoxy and alkylthio, represent cycloalkyl, cycloalkylalkyl, aryl or arylalkyl, each of which is optionally mono- or polysubstituted by identical or different substituents from the group consisting of halogen, cyano, alkyl, halogenoalkyl, alkoxy, halogenoalkoxy, alkylthio and halogenoalkylthio,
R¹² and R¹³ furthermore together represent alkylene or alkenylene, each of which is optionally mono- or polysubstituted by identical or different substituents from the group consisting of halogen, alkyl, halogenoalkyl, alkoxy, halogenoalkoxy, alkylthio and halogenoalkylthio,
R¹⁴ and R¹⁵ independently of one another represent hydrogen, represent alkyl, halogenoalkyl, alkenyl or halogenoalkenyl,
R¹⁶ and R¹⁷ independently of one another represent hydrogen, alkyl, halogenoalkyl or represent cycloalkyl which is optionally mono- or polysubstituted by identical or different substituents from the group consisting of halogen and alkyl,
R¹⁶ and R¹⁷ furthermore together represent alkylene, alkoxyalkylene or alkylthioalkylene, each of which is optionally mono- or polysubstituted by identical or different substituents from the group consisting of halogen and alkyl,
R¹⁸ represents hydrogen, -SO₂R⁷, -COR⁷ or -CO₂R⁷; represents alkyl or alkenyl, each of which is optionally mono- or polysubstituted by identical or different substituents from the group consisting of halogen, cyano, alkylamino, dialkylamino, alkoxy, halogenoalkoxy, alkylthio and halogenoalkylthio; represents cycloalkyl, cycloalkylalkyl, aryl, arylalkyl, saturated or unsaturated 5- to 10-membered heterocyclyl or heterocyclylalkyl having one or more heteroatoms from the group consisting of nitrogen, oxygen and sulfur, each of which radicals is optionally mono- or polysubstituted by identical or different substituents from the group consisting of halogen, cyano, alkyl, halogenoalkyl, alkoxy, halogenoalkoxy, alkylthio and halogenoalkylthio,
w represents 0, 1 or 2,
n represents 1, 2, 3 or 4,
p represents 0, 1 or 2.

3. Δ¹-pyrrolines of the formula (I) according to Claim 1, in which
R¹ represents halogen, C₁-C₆-alkyl, C₁-C₆-halogenoalkyl, C₁-C₆-alkoxy, C₁-C₆-halogenoalkoxy or -S(O)_{w}R⁴,
R² and R³ independently of one another represent hydrogen, halogen, C₁-C₆-alkyl, C₁-C₆-halogenoalkyl, C₁-C₆-alkoxy, C₁-C₆-halogenoalkoxy or C₁-C₆-alkoxy-C₁-C₆-alkyl,
R⁴ represents C₁-C₆-alkyl or C₁-C₆-halogenoalkyl,
Het represents 5- to 14-membered heteroaryl which comprises 1 to 3 aromatic rings and 1 to 4 heteroatoms including 0 to 4 nitrogen atoms, 0 to 2 nonadjacent oxygen atoms and/or 0 to 2 nonadjacent sulfur atoms (in particular thienyl, benzothienyl, furyl, benzofuryl, indolyl, thienothienyl, thienofuryl, thienobenzothienyl, thienobenzofuryl, pyridinyl, pyrimidinyl, pyridazinyl, pyrazinyl, triazolyl or tetrazolyl), each of which radicals is optionally mono- to tetrasubstituted by identical or different R⁵,
R⁵ represents the grouping -X-Y-Z-E, with the proviso that Y does not represent a direct bond if X does not represent a direct bond.
X represents a direct bond, oxygen, -S(O)_{w}-, -NR⁶-, carbonyl, carbonyloxy, oxycarbonyl, oxysulfonyl (OSO₂), C₁-C₆-alkylene, C₁-C₆-halogenoalkylene, C₂-C₆-alkenylene, C₂-C₆-halogenoalkenylene, C₂-C₆-alkinylene, C₁-C₆-alkyleneoxy, C₁-C₆-oxyalkylene, oxy-C₁-C₆-alkyleneoxy, -S (O)_{w}-C₁-C₆-alkylene, cyclopropylene or oxiranylene,
Y represents a direct bond or represents 1,4-phenylene, 1,3-phenylene, 1,2-phenylene, 2,6-naphthylene, 2,7-naphthylene, 1,4-naphthylene, 2,6-(1,2,3,4-tetrahydro)naphthylene, 2,7-(1,2,3,4-tetrahydro)naphthylene, 1,4-(1,2,3,4-tetrahydro)naphthylene, 5,8-(1,2,3,4-tetrahydro)-naphthylene, each of which is optionally mono- to tetrasubstituted by identical or different radicals from the list W¹; or represents 5- or 6-membered saturated or unsaturated heterocyclylene which comprises 1 to 3 heteroatoms including 0 to 3 nitrogen atoms, 0 to 2 nonadjacent oxygen atoms and/or 0 to 2 nonadjacent sulfur atoms (in particular furylene, thienylene, pyrrolylene, oxazolylene, thiazolylene, pyridinylene, pyrimidinylene, pyridazinylene or pyrazinylene), each of which radicals is optionally mono- to tetrasubstituted by identical or different radicals from the list W¹,
Z represents a direct bond or -(CH₂)ₙ-,
E represents hydrogen, fluorine, chlorine, bromine, hydroxyl, cyano, formyl, nitro, tri-(C₁-C₆-alkyl) silyl, pentafluorothio, -S(O)_{w}R⁷, -OSO₂R⁷, -NR⁸R⁹, -COR⁷, -CO₂R⁷, -OC(O)R⁷, -CONR¹⁰R¹¹, -N (R¹²) COR¹³, -C(R¹⁴) =N-OR¹⁵, -SO₂NR¹⁶R¹⁷; represents C₁-C₂₀-alkyl, C₂-C₂₀-alkenyl, C₂-C₁₀-alkinyl, C₁-C₂₀-alkoxy, C₂-C₂₀-alkenyloxy, each of which is optionally mono- or polysubstituted by identical or different radicals from the group consisting of halogen, cyano, C₁-C₁₀-alkoxy and -NR⁸R⁹; or represents C₃-C₁₂-cycloalkyl, C₃-C₇-cycloalkyl-C₁-C₄-alkyl, C₃-C₁₂-cycloalkyloxy, aryl, aryl-C₁-C₄-alkyl, aryloxy, aryloxy-C₁-C₄-alkyl, saturated or unsaturated 5- to 10-membered heterocyclyl or heterocyclyl-C₁-C₄-alkyl which comprises 1 to 4 heteroatoms including 0 to 4 nitrogen atoms, 0 to 2 nonadjacent oxygen atoms and/or 0 to 2 nonadjacent sulfur atoms (in particular tetrazolyl, furyl, furfuryl, benzofuryl, tetrahydrofuryl, thienyl, thenyl, benzothienyl, thiolanyl, pyrrolyl, indolyl, pyrrolinyl, pyrrolidino, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, piperidino, morpholinyl, thiomorpholinyl, morpholino, thiomorpholino, triazinyl, triazolyl, quinolinyl or isoquinolinyl), each of which radicals is optionally mono- to tetrasubstituted by identical or different substituents from the group consisting of halogen, cyano, nitro, C₁-C₆-alkyl, C₁-C₆-halogenoalkyl, C₂-C₆-alkenyl, C₂-C₆-halogenoalkenyl, C₁-C₆-alkoxy, C₁-C₆-halogenoalkoxy, C₁-C₆-alkylthio and C₁-C₆-halogenoalkylthio,
W¹ represents fluorine, chlorine, bromine, cyano, formyl, nitro, tri- (C₁-C₆-alkyl) silyl, C₁-C₆-alkyl, C₁-C₆-halogenoalkyl, C₁-C₆-alkoxy, C₁-C₆-halogenoalkoxy, C₂-C₆-alkenyl, C₂-C₆-halogenoalkenyl, C₂-C₆-alkenyloxy, C₂-C₆-halogenoalkenyloxy, C₁-C₆-alkylcarbonyl, C₁-C₆-alkoxycarbonyl, -S(O)_{w}R⁷, -C(R¹⁴)=N-OR¹⁵, -SO₂NR¹⁶R¹⁷, -(CH₂)ₚNR¹⁶R¹⁷, (CH₂)ₚN (R¹⁶) COR¹⁷, -(CH₂) ₚN(R¹⁶)SO₂R¹⁷, -OSO₂R¹⁶, -OSO₂NR¹⁶R¹⁷,
R⁶ represents C₁-C₆-alkyl, C₁-C₆-halogenoalkyl or represents C₃-C₇-cycloalkyl, C₃-C₆-cycloalkyl-C₁-C₄-alkyl, aryl or aryl-C₁-C₄-alkyl, each of which is optionally mono- to tetrasubstituted by halogen, C₁-C₆-alkyl, C₁-C₆-halogenoalkyl, C₁-C₆-alkoxy, C₁-C₆-halogenoalkoxy, C₁-C₆-alkylthio and C₁-C₆-halogenoalkylthio,
R⁷ represents C₁-C₂₀-alkyl which is optionally mono- or polysubstituted by identical or different substituents from the group consisting of halogen and -NR⁸R⁹, represents C₃-C₆-cycloalkyl, aryl or aryl-C₁-C₄-alkyl, each of which is optionally mono- to octasubstituted by identical or different substituents from the group consisting of halogen, cyano, C₁-C₆-alkyl, C₁-C₆-halogenoalkyl, C₁-C₆-alkoxy, C₁-C₆-halogenoalkoxy, C₁-C₆-alkylthio and C₁-C₆-halogenoalkylthio,
R⁸ and R⁹ independently of one another represent hydrogen, -SO₂R⁷, -COR⁷, -CO₂R⁷, represent C₁-C₂₀-alkyl or C₂-C₂₀-alkenyl, each of which is optionally mono- or polysubstituted by identical or different substituents from the group consisting of halogen, C₁-C₆-alkylcarbonyl, C₁-C₆-alkylcarbonyloxy, C₁-C₆-alkylamino, di-(C₁-C₆-alkyl)amino, C₁-C₆-alkoxy, C₁-C₆-halogenoalkoxy, C₁-C₆-alkylthio, C₁-C₆-halogenoalkylthio; represent C₃-C₁₂-cycloalkyl, C₃-C₇-cycloalkyl-C₁-C₄-alkyl, aryl, aryl-C₁-C₄-alkyl or saturated or unsaturated 5- to 10-membered heterocyclyl or heterocyclyl-C₁-C₄-alkyl which comprises 1 to 4 heteroatoms including 0 to 4 nitrogen atoms, 0 to 2 nonadjacent oxygen atoms and/or 0 to 2 nonadjacent sulfur atoms (in particular tetrazolyl, furyl, furfuryl, benzofuryl, tetrahydrofuryl, thienyl, thenyl, benzothienyl, thiolanyl, pyrrolyl, indolyl, pyrrolinyl, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, morpholinyl, thiomorpholinyl, triazinyl, triazolyl, quinolinyl or isoquinolinyl), each of which radicals is optionally mono- to tetrasubstituted by identical or different substituents from the group consisting of halogen, cyano, C₁-C₆-alkyl, C₁-C₆-halogenoalkyl, C₁-C₆-alkoxy, C₁-C₆-halogenoalkoxy, C₁-C₆-alkylthio or C₁-C₆-halogenoalkylthio,
R⁸ and R⁹ furthermore together represent C₂-C₁₂-alkenylene which is optionally mono- or polysubstituted by identical or different substituents from the group consisting of halogen, cyano, C₁-C₆-alkoxy, C₁-C₆-halogenoalkoxy, C₁-C₆-alkylthio and C₁-C₆-halogenoalkylthio or represent C₃-C₁₂-alkylene which is optionally mono- or polysubstituted in the alkylene moiety by identical or different substituents from the group consisting of halogen, cyano, C₁-C₆-alkyl, C₁-C₆-halogenoalkyl, C₁-C₆-alkoxy, C₁-C₆-halogenoalkoxy, C₁-C₆-alkylthio and C₁-C₆-halogenoalkylthio, where the alkylene chain may in each case be interrupted by -O-, -S- or -NR¹⁸-,
R¹⁰ and R¹¹ independently of one another represent hydrogen, -SO₂R⁷, represent C₁-C₆-alkyl or C₂-C₆-alkenyl, each of which is optionally mono- to tridecasubstituted by identical or different substituents from the group consisting of halogen, C₁-C₆-alkylamino, di-(C₁-C₆-alkyl) amino, C₁-C₆-alkoxy, C₁-C₆-halogenoalkoxy, C₁-C₆-alkylthio and C₁-C₆-halogenoalkylthio; represent C₃-C₇-cycloalkyl, C₃-C₇-cycloalkyl-C₁-C₄-alkyl, aryl, aryl-C₁-C₄-alkyl or saturated or unsaturated 5- to 10-membered heterocyclyl or heterocyclyl-C₁-C₄-alkyl which comprises 1 to 4 heteroatoms including 0 to 4 nitrogen atoms, 0 to 2 nonadjacent oxygen atoms and/or 0 to 2 nonadjacent sulfur atoms (in particular tetrazolyl, furyl, furfuryl, benzofuryl, tetrahydrofuryl, thienyl, thenyl, benzothienyl, thiolanyl, pyrrolyl, indolyl, pyrrolinyl, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, morpholinyl, thiomorpholinyl, triazinyl, triazolyl, quinolinyl or isoquinolinyl), each of which radicals is optionally mono- to tetrasubstituted by identical or different substituents from the group consisting of halogen, cyano, C₁-C₆-alkyl, C₁-C₆-halogenoalkyl, C₁-C₆-alkoxy, C₁-C₆-halogenoalkoxy, C₁-C₆-alkylthio and C₁-C₆-halogenoalkylthio,
R¹⁰ and R¹¹ furthermore together represent C₃-C₆-alkylene, -(CH₂)₂-O-(CH₂)₂-, -(CH₂)₂-S-(CH₂)₂- or -(CH₂)₂-N(R¹⁸)-(CH₂)₂-, each of which is optionally mono- to tetrasubstituted in the alkylene moiety by identical or different substituents from the group consisting of halogen, cyano, C₁-C₆-alkyl, C₁-C₆-halogenoalkyl, C₁-C₆-alkoxy, C₁-C₆-halogenoalkoxy, C₁-C₆-alkylthio and C₁-C₆-halogenoalkylthio,
R¹² and R¹³ independently of one another represent hydrogen, represent C₁-C₆-alkyl which is optionally mono- to tridecasubstituted by identical or different substituents from the group consisting of halogen, cyano, C₁-C₆-alkoxy and C₁-C₆-alkylthio, represent C₃-C₇-cycloalkyl, C₃-C₇-cycloalkyl-C₁-C₄-alkyl, aryl or aryl-C₁-C₄-alkyl, each of which is optionally mono- to octasubstituted by identical or different substituents from the group consisting of halogen, cyano, C₁-C₆-alkyl, C₁-C₆-halogenoalkyl, C₁-C₆-alkoxy, C₁-C₆-halogenoalkoxy, C₁-C₆-alkylthio and C₁-C₆-halogenoalkylthio,
R¹² and R¹³ furthermore together represent C₃-C₁₀-alkylene or C₃-C₁₀-alkenylene, each of which is optionally mono- to octasubstituted by identical or different substituents from the group consisting of halogen, C₁-C₆-alkyl, C₁-C₆-halogenoalkyl, C₁-C₆-alkoxy, C₁-C₆-halogenoalkoxy, C₁-C₆-alkylthio and C₁-C₆-halogenoalkylthio,
R¹⁴ and R¹⁵ independently of one another represent hydrogen, represent C₁-C₆-alkyl, C₁-C₆-halogenoalkyl, C₂-C₆-alkenyl or C₂-C₆ -halogenoalkenyl,
R¹⁶ and R¹⁷ independently of one another represent hydrogen, C₁-C₆-alkyl, C₁-C₆-halogenoalkyl or represent C₃-C₇-cycloalkyl which is optionally mono- to octasubstituted by identical or different substituents from the group consisting of fluorine, chlorine, bromine and/or C₁-C₆-alkyl,
R¹⁶ and R¹⁷ furthermore together represent C₃-C₆-alkylene, C₁-C₃-alkoxy-C₁-C₃-alkylene or C₁-C₃-alkylthio-C₁-C₃-alkylene, each of which is optionally mono- to nonasubstituted by identical or different substituents from the group consisting of fluorine, chlorine, bromine and C₁-C₆-alkyl,
R¹⁸ represents hydrogen, -SO₂R⁷, -COR⁷ or -CO₂R⁷; represents C₁-C₂₀-alkyl or C₂-C₂₀-alkenyl, each of which is optionally mono- or polysubstituted by identical or different substituents from the group consisting of halogen, cyano, C₁-C₆-alkylamino, di-(C₁-C₆-alkyl)amino, C₁-C₆-alkoxy, C₁-C₆-halogenoalkoxy, C₁-C₆-alkylthio and C₁-C₆-halogenoalkylthio; represents C₃-C₁₂-cycloalkyl, C₃-C₇-cycloalkyl-C₁-C₄-alkyl, aryl, aryl-C₁-C₄-alkyl, saturated or unsaturated 5- to 10-membered heterocyclyl or heterocyclyl-C₁-C₄-alkyl which comprises 1 to 4 heteroatoms including 0 to 4 nitrogen atoms, 0 to 2 nonadjacent oxygen atoms and/or 0 to 2 nonadjacent sulfur atoms (in particular tetrazolyl, furyl, furfuryl, benzofuryl, tetrahydrofuryl, thienyl, thenyl, benzothienyl, thiolanyl, pyrrolyl, indolyl, pyrrolinyl, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, morpholinyl, thiomorpholinyl, triazinyl, triazolyl, quinolinyl or isoquinolinyl), each of which radicals is optionally mono- to tetrasubstituted by identical or different substituents from the group consisting of halogen, cyano, C₁-C₆-alkyl, C₁-C₆-halogenoalkyl, C₁-C₆-alkoxy, C₁-C₆-halogenoalkoxy, C₁-C₆-alkylthio and C₁-C₆-halogenoalkylthio,
w represents 0, 1 or 2,
n represents 1, 2 or 3,
p represents 0, 1 or 2.

4. Δ¹-pyrrolines of the formula (I) according to Claim 1, in which
R¹ represents fluorine, chlorine, bromine, C₁-C₄-alkyl, C₁-C₄-halogenoalkyl having 1 to 9 fluorine, chlorine and/or bromine atoms, C₁-C₄-alkoxy, C₁-C₄-halogenoalkoxy having 1 to 9 fluorine, chlorine and/or bromine atoms or -S(O)_{w}R⁴,
R² and R³ independently of one another represent hydrogen, fluorine, chlorine, bromine, C₁-C₄-alkyl, C₁-C₄-halogenoalkyl having 1 to 9 fluorine, chlorine and/or bromine atoms, C₁-C₄-alkoxy or C₁-C₄-halogenoalkoxy having 1 to 9 fluorine, chlorine and/or bromine atoms,
R⁴ represents C₁-C₄-alkyl or represents in each case fluorine- or chlorine-substituted methyl or ethyl,
Het represents 2-thienyl, 3-thienyl, 2-benzo [b] thienyl, 2-furyl, 3-furyl, 2-benzo[b]furyl, 2-indolyl, 2-thieno[3,2-b]thienyl, 2-thieno[3,2-b]furyl, 5-thieno[3,2-b]furyl, 2-thieno[2,3-f][1]benzothienyl, 2-thieno[2,3-f] [1] benzofuryl, 6-thieno[2,3-f][1]benzofuryl, 2-pyridinyl, 3-pyridinyl, 2-pyrimidinyl, 5-pyrimidinyl, 3-pyridazinyl, 4-pyridazinyl, 2-pyrazinyl, triazolyl or tetrazolyl, each of which is optionally mono- to trisubstituted by identical or different R⁵,
R⁵ represents the grouping -X-Y-Z-E, with the proviso that Y does not represent a direct bond if X does not represent a direct bond,
X represents a direct bond, oxygen -S(O)_{w}-, -NR⁶-, carbonyl, carbonyloxy, oxycarbonyl, oxysulfonyl (OSO₂), C₁-C₄-alkylene, C₁-C₄-halogenoalkylene having 1 to 8 fluorine, chlorine and/or bromine atoms, C₂-C₄-alkenylene, C₂-C₄-halogenoalkenylene having 1 to 6 fluorine, chlorine and/or bromine atoms, C₂-C₄-alkinylene, C₁-C₄-alkyleneoxy, oxy-C₁-C₄-alkylene, oxy-C₁-C₄-alkyleneoxy or -S(O)_{w}-C₁-C₄-alkylene,
Y represents a direct bond or represents 1,4-phenylene, 1,3-phenylene, 1,2-phenylene, 2,6-naphthylene, 2,7-naphthylene, 1,4-naphthylene, 2,6-(1,2,3,4-tetrahydro)naphthylene, 2,7-(1,2,3,4-tetrahydro)naphthylene, 1,4-(1,2,3,4-tetrahydro)naphthylene, 5,8-(1,2,3,4-tetrahydro)-naphthylene, 2,4-furylene, 2,4-thienylene, 2,4-pyrrolylene, 2,5-oxazolylene, 2,5-thiazolylene, 2,5-pyridinylene, 2,6-pyridinylene, 2,5-pyrimidinylene, 3,6-pyridazinylene or 2,5-pyrazinylene, each of which is optionally mono- to trisubstituted by identical or different radicals from the list W¹
Z represents a direct bond or -(CH₂)ₙ-,
E represents hydrogen, fluorine, chlorine, bromine, hydroxyl, cyano, formyl, nitro, trimethylsilyl, dimethyl-tert-butylsilyl, -S(O)_{w}R⁷, -OSO₂R⁷, -NR⁸R⁹, -COR⁷, -CO₂R⁷, -OC(O)R⁷, -CONR¹⁰R¹¹, -N(R¹²)COR¹³, -SO₂NR¹⁶R¹⁷; represents C₁-C₁₆-alkyl, C₂-C₁₆-alkenyl, C₂-C₆-alkinyl, C₁-C₁₆-alkoxy, C₂-C₁₆-alkenyloxy, each of which is optionally mono- or polysubstituted by identical or different substituents from the group consisting of fluorine, chlorine, bromine, cyano, C₁-C₆-alkoxy and -NR⁸R⁹; or represents C₃-C₁₀-cycloalkyl, C₃-C₆-cycloalkyl-C₁-C₄-alkyl, C₃-C₁₀-cycloalkyloxy, phenyl, phenoxy, benzyl, phenylethyl, benzyloxy, tetrazolyl, furyl, furfuryl, benzofuryl, tetrahydrofuryl, thienyl, thenyl, benzothienyl, thiolanyl, pyrrolyl, indolyl, pyrrolinyl, pyrrolidino, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, piperidino, morpholinyl, thiomorpholinyl, morpholino, thiomorpholino, triazinyl, triazolyl, quinolinyl or isoquinolinyl, each of which is optionally mono- to trisubstituted by identical or different substituents from the group consisting of fluorine, chlorine, bromine, cyano, C₁-C₄-alkyl, C₁-C₄-halogenoalkyl, C₂-C₆-alkenyl, C₂-C₆-halogenoalkenyl, C₁-C₄-alkoxy, C₁-C₄-halogenoalkoxy, C₁-C₄-alkylthio and C₁-C₄-halogenoalkylthio,
W¹ represents fluorine, chlorine, bromine, cyano, formyl, trimethylsilyl, dimethyl-tert-butylsilyl, C₁-C₄-alkyl, C₁-C₄-alkoxy, C₂-C₄-alkenyl, C₂-C₄-alkenyloxy; represents C₁-C₄-halogenoalkyl, C₁-C₄-halogenoalkoxy having in each case 1 to 9 fluorine, chlorine and/or bromine atoms, C₂-C₄-halogenoalkenyl, C₂-C₄-halogenoalkenyloxy having in each case 1 to 8 fluorine, chlorine and/or bromine atoms; represents C₁-C₄-alkylcarbonyl, C₁-C₄-alkoxycarbonyl, -S(O)_{w}R⁷, -SO₂NR¹⁶R¹⁷, -(CH₂)ₚNR¹⁶R¹⁷, -(CH₂)ₚN(R¹⁶)COR¹⁷, -(CH₂)ₚN(R¹⁶)SO₂R¹⁷, -OSO₂R¹⁶, -OSO₂NR¹⁶R¹⁷,
R⁶ represents methyl, ethyl, n-propyl, isopropyl, n-butyl, isobutyl, sec-butyl, tert-butyl, trifluoromethyl, trifluoroethyl, or represents cyclopropyl, cyclopropylmethyl, cyclopentyl, cyclopentylmethyl, cyclohexyl, cyclohexylmethyl, phenyl, benzyl or phenylethyl, each of which is optionally mono- to tetrasubstituted by identical or different substituents from the group consisting of fluorine, chlorine, bromine, C₁-C₄-alkyl, C₁-C₄-alkoxy, C₁-C₄-alkylthio, C₁-C₄-halogenoalkyl, C₁-C₄-halogenoalkoxy and C₁-C₄-halogenoalkylthio having in each case 1 to 9 fluorine, chlorine and/or bromine atoms,
R⁷ represents C₁-C₁₀-alkyl which is optionally mono- or polysubstituted by identical or different substituents from the group consisting of fluorine, chlorine, bromine and -NR⁸R⁹, represents cyclopropyl, cyclopentyl, cyclohexyl, phenyl or benzyl, each of which is optionally mono- to tetrasubstituted by identical or different substituents from the group consisting of fluorine, chlorine, bromine, cyano, C₁-C₄-alkyl, C₁-C₄-alkoxy, C₁-C₄-alkylthio, C₁-C₄-halogenoalkyl, C₁-C₄-halogenoalkoxy and C₁-C₄-halogenoalkylthio having in each case 1 to 9 fluorine, chlorine and/or bromine atoms,
R⁸ and R⁹ independently of one another represent hydrogen, -SO₂R⁷, -COR⁷, -CO₂R⁷, represent C₁-C₁₆-alkyl or C₂-C₁₆-alkenyl, each of which is optionally mono- or polysubstituted by identical or different substituents from the group consisting of fluorine, chlorine, bromine, C₁-C₄-alkylcarbonyl, C₁-C₄-alkylcarbonyloxy, C₁-C₄-alkylamino, di-(C₁-C₄-alkyl)amino, C₁-C₄-alkoxy, C₁-C₄-halogenoalkoxy, C₁-C₄-alkylthio and C₁-C₄-halogenoalkylthio; represent C₃-C₁₀-cycloalkyl, C₃-C₆-cycloalkyl-C₁-C₄-alkyl, phenyl, benzyl, phenylethyl, tetrazolyl, furyl, furfuryl, benzofuryl, tetrahydrofuryl, thienyl, thenyl, benzothienyl, thiolanyl, pyrrolyl, indolyl, pyrrolinyl, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, morpholinyl, thiomorpholinyl, triazinyl, triazolyl, quinolinyl or isoquinolinyl, each of which is optionally mono- to trisubstituted by identical or different substituents from the group consisting of fluorine, chlorine, bromine, cyano, C₁-C₄-alkyl, C₁-C₄-halogenoalkyl, C₁-C₄-alkoxy, C₁-C₄-halogenoalkoxy, C₁-C₄-alkylthio and C₁-C₄-halogenoalkylthio,
R⁸ and R⁹ furthermore together represent C₂-C₁₀-alkenylene which is optionally mono- or polysubstituted by identical or different substituents from the group consisting of fluorine, chlorine, bromine, C₁-C₄-alkoxy, C₁-C₄-halogenoalkoxy, C₁-C₄-alkylthio and C₁-C₄-halogenoalkylthio or represent C₃-C₁₀-alkylene, which is optionally mono- or polysubstituted in the alkylene moiety by identical or different substituents from the group consisting of fluorine, chlorine, cyano, C₁-C₄-alkyl, C₁-C₄-halogenoalkyl, C₁-C₄-alkoxy, C₁-C₄-halogenoalkoxy, C₁-C₄-alkylthio and C₁-C₄-halogenoalkylthio, where the alkylene chain may in each case be interrupted by -O-, -S- or -NR¹⁸-,
R¹⁰ and R¹¹ independently of one another represent hydrogen, -SO₂R⁷, represent C₁-C₆-alkyl or C₂-C₆-alkenyl, each of which is optionally mono- to nonasubstituted by identical or different substituents from the group consisting of fluorine, chlorine, bromine, C₁-C₄-alkylamino, di-(C₁-C₄-alkyl) amino, C₁-C₄-alkoxy, C₁-C₄-halogenoalkoxy, C₁-C₄-alkylthio and C₁-C₄-halogenoalkylthio; represent C₃-C₆-cycloalkyl, C₃-C₆-cycloalkyl-C₁-C₄-alkyl, phenyl, benzyl, phenylethyl, tetrazolyl, furyl, furfuryl, benzofuryl, tetrahydrofuryl, thienyl, thenyl, benzothienyl, thiolanyl, pyrrolyl, indolyl, pyrrolinyl, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, morpholinyl, thiomorpholinyl, triazinyl, triazolyl, quinolinyl or isoquinolinyl, each of which is optionally mono- to trisubstituted by identical or different substituents from the group consisting of fluorine, chlorine, bromine, cyano, C₁-C₄-alkyl, C₁-C₄-halogenoalkyl, C₁-C₄-alkoxy, C₁-C₄-halogenoalkoxy, C₁-C₄-alkylthio and C₁-C₄-halogenoalkylthio,
R¹⁰ and R¹¹ furthermore together represent C₄-C₆-alkylene, -(CH₂)₂-O-(CH₂)₂-, -(CH₂)₂-S-(CH₂)₂- or -(CH₂)₂-N(R¹⁸)-(CH₂)₂-, each of which is optionally mono- to tetrasubstituted in the alkylene moiety by identical or different substituents from the group consisting of fluorine, chlorine, cyano, C₁-C₄-alkyl, C₁-C₄-halogenoalkyl, C₁-C₄-alkoxy, C₁-C₄-halogenoalkoxy, C₁-C₄-alkylthio and C₁-C₄-halogenoalkylthio,
R¹² and R¹³ independently of one another represent hydrogen, represent C₁-C₆-alkyl which is optionally mono- to nonasubstituted by identical or different substituents from the group consisting of fluorine, chlorine, bromine, C₁-C₄-alkoxy and C₁-C₄-alkylthio, represent C₃-C₆-cycloalkyl, C₃-C₆-cycloalkyl-C₁-C₄-alkyl, phenyl, benzyl or phenylethyl, each of which is optionally mono- to tetrasubstituted by identical or different substituents from the group consisting of fluorine, chlorine, bromine, C₁-C₄-alkyl, C₁-C₄-halogenoalkyl, C₁-C₄-alkoxy, C₁-C₄-halogenoalkoxy, C₁-C₄-alkylthio and C₁-C₄-halogenoalkylthio,
R¹² and R¹³ furthermore together represent C₃-C₈-alkylene or C₃-C₈-alkenylene, each of which is optionally mono- to tetrasubstituted by identical or different substituents from the group consisting of fluorine, chlorine, bromine, C₁-C₄-alkyl, C₁-C₄-halogenoalkyl, C₁-C₄-alkoxy, C₁-C₄-halogenoalkoxy, C₁-C₄-alkylthio and C₁-C₄-halogenoalkylthio,
R¹⁶ and R¹⁷ independently of one another represent hydrogen, C₁-C₆-alkyl, C₁-C₄-halogenoalkyl having 1 to 9 fluorine, chlorine and/or bromine atoms or represent C₃-C₆-cycloalkyl which is optionally mono- to tetrasubstituted by identical or different substituents from the group consisting of fluorine, chlorine, bromine and C₁-C₄-alkyl,
R¹⁶ and R¹⁷ furthermore together represent -(CH₂)₃-, -(CH₂)₄-, -(CH₂)₅-, -(CH₂)₂-O-(CH₂)₂- or -(CH₂)₂-S-(CH₂)₂-,
R¹⁸ represents hydrogen, -SO₂R⁷, represents -COR⁷ or -CO₂R⁷; represents C₁-C₁₆-alkyl or C₂-C₁₆-alkenyl, each of which is optionally mono- or polysubstituted by identical or different substituents from the group consisting of fluorine, chlorine, bromine, cyano, methylamino, ethylamino, di-(C₁-C₆-alkyl)amino, C₁-C₄-alkoxy, C₁-C₄-halogenoalkoxy, C₁-C₄-alkylthio and C₁-C₄-halogenoalkylthio; represents C₃-C₁₀-cycloalkyl, C₃-C₆-cycloalkyl-C₁-C₄-alkyl, phenyl, benzyl, phenylethyl, tetrazolyl, furyl, furfuryl, benzofuryl, tetrahydrofuryl, thienyl, thenyl, benzothienyl, thiolanyl, pyrrolyl, indolyl, pyrrolinyl, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, morpholinyl, thiomorpholinyl, triazinyl, triazolyl, quinolinyl or isoquinolinyl, each of which is optionally mono- to trisubstituted by identical or different substituents from the group consisting of fluorine, chlorine, bromine, cyano, C₁-C₄-alkyl, C₁-C₄-halogenoalkyl, C₁-C₄-alkoxy, C₁-C₄-halogenoalkoxy, C₁-C₄-alkylthio and C₁-C₄-halogenoalkylthio,
w represents 0, 1 or 2,
n represents 1 or 2,
p represents 0 or 1.

5. Δ¹-pyrrolines of the formula (I) according to Claim 1, in which
R¹ represents fluorine, chlorine, bromine, methyl, trifluoromethyl, methoxy, trifluoromethoxy, methylthio or trifluoromethylthio,
R² and R³ independently of one another represent hydrogen, fluorine, chlorine, bromine, methyl, trifluoromethyl, methoxy or trifluoromethoxy,
Het represents 2-thienyl, 3-thienyl, 2-benzo[b]thienyl, 2-furyl, 3-furyl, 2-benzo[b]furyl, 2-thieno[3,2-b]thienyl, 2-thieno[3,2-b]furyl, 5-thieno[3,2-b]furyl, 2-thieno[2,3-f][1]benzothienyl, 2-thieno-[2,3-f] [1] benzofuryl, 6-thieno[2,3-f][1]benzofuryl, 2-pyridinyl, 3-pyridinyl, 2-pyrimidinyl, 5-pyrimidinyl, 3-pyridazinyl, 4-pyridazinyl or 2-pyrazinyl, each of which is optionally mono- or disubstituted by identical or different R⁵,
R⁵ represents the grouping -X-Y-Z-E, with the proviso that Y does not represent a direct bond if X does not represent a direct bond,
X represents a direct bond, oxygen, sulfur, -SO₂-, -NR⁶-, -CO-, -C (O) -O-, -O-C (O) -, -CH₂-, (CH₂)₂-, -C=C- (E or Z), -C≡C-, -CH₂O-, -(CH₂)₂O-, -OCH₂-, -O(CH₂)₂-, -O-CH₂-O-, -SCH₂-, -S(CH₂)₂-, -CH₂S- or -(CH₂)₂S-,
Y represents a direct bond or represents 1,4-phenylene, 1,3-phenylene, 2,6-naphthylene, 2,7-naphthylene, 2,4-furylene, 2,4-thienylene, 2,5-pyridinylene, 2,5-pyrimidinylene, 3,6-pyridazinylene or 2,5-pyrazinylene, each of which is optionally mono- or disubstituted by identical or different radicals from the list W¹,
Z represents a direct bond, methylene or ethylene,
E represents hydrogen, fluorine, chlorine, bromine, hydroxyl, cyano, formyl, -S(O)_{w}R⁷, -OSO₂R⁷, -NR⁸R⁹, -COR⁷, -CO₂R⁷, -OC(O)R⁷, -CONR¹⁰R¹¹, -N(R¹²)COR¹³, -SO₂NR¹⁶R¹⁷; represents C₁-C₁₆-alkyl, C₂-C₁₆-alkenyl, C₁-C₁₆-alkoxy, C₂-C₁₆-alkenyloxy, each of which is optionally mono- or polysubstituted by identical or different substituents from the group consisting of fluorine, chlorine, bromine, cyano, C₁-C₆-alkoxy and -NR⁸R⁹; or represents cyclopropyl, cyclopentyl, cyclohexyl, cyclopropyloxy, cyclopentyloxy, cyclohexyloxy, cyclopropylmethyl, cyclopentylmethyl, cyclohexylmethyl, phenyl, phenoxy, benzyl, phenylethyl, benzyloxy, tetrazolyl, furyl, furfuryl, benzofuryl, tetrahydrofuryl, thienyl, thenyl, benzothienyl, thiolanyl, pyrrolyl, indolyl, pyrrolinyl, pyrrolidino, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, piperidino, morpholinyl, thiomorpholinyl, morpholino, thiomorpholino, triazinyl, triazolyl, quinolinyl or isoquinolinyl, each of which is optionally mono- to trisubstituted by identical or different substituents from the group consisting of fluorine, chlorine, bromine, cyano, methyl, ethyl, n-propyl, isopropyl, -CF₃, -CHF₂, -CClF₂, -CF₂CHFCl, -CF₂CH₂F, -CF₂CCl₃, -CH₂CF₃, -CF₂CHFCF₃, -CH₂CF₂H, -CH₂CF₂CF₃, -CF₂CF₂H, -CF₂CHFCF₃, vinyl, allyl, 1-propenyl, butenyl, -CF=CHF, -CF=CH₂, -CF=CCl₂ -CH=CF₂, -CF₂CF=CF₂, -CH=CFH, -CH₂CF=CF₂, -CF=CF₂, -CF₂CH=CF₂, methoxy, ethoxy, n-propoxy, isopropoxy, n-butoxy, isobutoxy, sec-butoxy, tert-butoxy, trifluoromethoxy, difluoromethoxy, chlorodifluoromethoxy, trifluoroethoxy, methylthio, ethylthio, n-propylthio, isopropylthio, n-butylthio, isobutylthio, sec-butylthio, tert-butylthio, trifluoromethylthio, difluoromethylthio, chlorodifluoromethylthio and trifluoroethylthio,
W¹ represents fluorine, chlorine, bromine, cyano, formyl, methyl, ethyl, n-propyl, isopropyl, n-butyl, isobutyl, sec-butyl, tert-butyl, methoxy, ethoxy, n-propoxy, isopropoxy, n-butoxy, isobutoxy, sec-butoxy, tert-butoxy, vinyl, allyl, trifluoromethyl, trifluoroethyl, trifluoromethoxy, trifluoroethoxy, -OCF₂CF₂H, -CH=CF₂, -CH=CCl₂, -OCF=CF₂, -COMe, -COEt, -CO₂Me, -CO₂Et, -CO₂(t-Bu), -SMe, -SOMe, -SO₂Me, -SCF₃, -SOCF₃, -SO₂CF₃, -SCHF₂, -SOCHF₂, -SO₂CHF₂, -SO₂NMe₂, -NMe₂, -NEt₂, -N(n-Pr)₂, -N (Me) COMe, -N(Me)COEt, -N(Me)COPr, -N(Me)CO(t-Bu), 2-pyrrolidonyl, 2-piperidonyl, -N(Me)SO₂Me, -N(Me)SO₂Et, -N(Me)SO₂CF₃, -N(Et)-SO₂CF₃, -N(Me)SO₂(CF₂)₃CF₃ or -OSO₂NMe₂,
R⁶ represents methyl, ethyl, n-propyl, isopropyl, n-butyl, isobutyl, sec-butyl, tert-butyl, trifluoromethyl, trifluoroethyl, cyclopropyl, cyclopropylmethyl, cyclopentyl, cyclopentylmethyl, cyclohexyl, cyclohexylmethyl, phenyl, benzyl or phenylethyl,
R⁷ represents methyl, ethyl, n-propyl, isopropyl, n-butyl, isobutyl, sec-butyl, tert-butyl, pentyl, hexyl, -CF₃, -CHF₂, -CCl₃, -CCl₂F, dimethylaminomethyl, dimethylaminoethyl, diethylaminomethyl, diethylaminoethyl, cyclopropyl, cyclopentyl, cyclohexyl, phenyl or benzyl,
R⁸ and R⁹ independently of one another represent hydrogen, -SO₂R⁷, -COR⁷, -CO₂R⁷, represent C₁-C₁₆-alkyl or C₂-C₁₆-alkenyl, each of which is optionally mono- or polysubstituted by identical or different substituents from the group consisting of fluorine, chlorine, bromine, C₁-C₄-alkylcarbonyl, C₁-C₄-alkylcarbonyloxy, C₁-C₄-alkylamino, di-(C₁-C₄-alkyl)amino, C₁-C₄-alkoxy, C₁-C₄-halogenoalkoxy, C₁-C₄-alkylthio and C₁-C₄-halogenoalkylthio; represent C₃-C₈-cycloalkyl, cyclopropylmethyl, cyclopentylmethyl, cyclohexylmethyl, cyclopropylethyl, cyclopentylethyl, cyclohexylethyl, phenyl, benzyl, phenylethyl, tetrazolyl, furyl, furfuryl, benzofuryl, tetrahydrofuryl, thienyl, thenyl, benzothienyl, thiolanyl, pyrrolyl, indolyl, pyrrolinyl, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, morpholinyl, thiomorpholinyl, triazinyl, triazolyl, quinolinyl or isoquinolinyl, each of which is optionally mono- to trisubstituted by identical or different substituents from the group consisting of fluorine, chlorine, bromine, cyano, C₁-C₄-alkyl, C₁-C₄-halogenoalkyl, C₁-C₄-alkoxy, C₁-C₄-halogenoalkoxy, C₁-C₄-alkylthio and C₁-C₄-halogenoalkylthio,
R⁸and R⁹ furthermore together represent C₂-C₈-alkenylene which is optionally mono- or polysubstituted by identical or different substituents from the group consisting of fluorine, chlorine, bromine, C₁-C₄-alkoxy, C₁-C₄-halogenoalkoxy, C₁-C₄-alkylthio and C₁-C₄-halogenoalkylthio or represent C₃-C₈-alkylene, which is optionally mono- or polysubstituted in the alkylene moiety by identical or different substituents from the group consisting of fluorine, chlorine, cyano, methyl, ethyl, n-propyl, isopropyl, n-butyl, isobutyl, sec-butyl, tert-butyl, C₁-C₄-halogenoalkyl, methoxy, ethoxy, n-propoxy, isopropoxy, n-butoxy, isobutoxy, sec-butoxy, tert-butoxy, C₁-C₄-halogenoalkoxy, methylthio, ethylthio, n-propylthio, isopropylthio, n-butylthio, isobutylthio, sec-butylthio, tert-butylthio and C₁-C₄-halogenoalkylthio, where the alkylene chain may in each case be interrupted by -O-, -S- or -NR¹⁸-,
R¹⁰ and R¹¹ independently of one another represent hydrogen, -SO₂CF₃, methyl, ethyl, n-propyl, isopropyl, n-butyl, isobutyl, sec-butyl, tert-butyl, pentyl, hexyl, -CF₃, -CH₂CF₃, -(CF₂)₃CF₃, methoxymethyl, methoxyethyl, cyclopropyl, cyclopentyl, cyclohexyl, or represent phenyl or benzyl, each of which is optionally mono- to trisubstituted by identical or different substituents from the group consisting of fluorine, chlorine, bromine, methyl, trifluoromethyl, methoxy and trifluoromethoxy,
R¹⁰ and R¹¹ furthermore together represent -(CH₂)₄-, -(CH₂)₅-, -(CH₂)₆-, -CH₂-CH(CH₃)-CH₂-CH(CH₃)-CH₂-, -(CH₂)₂-O-(CH₂)₂, -(CH₂)₂-S-(CH₂)₂- or -(CH₂)₂-N(R¹⁸)-(CH₂)₂-,
R¹² and R¹³ independently of one another represent hydrogen, methyl, ethyl, n-propyl, isopropyl, n-butyl, isobutyl, sec-butyl, tert-butyl, n-hexyl, trifluoromethyl, trifluoroethyl, cyclopropyl, cyclopentyl, cyclohexyl or represent phenyl or benzyl, each of which is optionally mono- to tetrasubstituted by identical or different substituents from the group consisting of fluorine, chlorine, bromine, methyl, trifluoromethyl, methoxy and trifluoromethoxy,
R¹² and R¹³ furthermore together represent -(CH₂)₃-, -(CH₂)₄-, -(CH₂)₅- or -(CH₂)₆-, each of which is optionally mono- to tetrasubstituted by identical or different substituents from the group consisting of fluorine, chlorine, bromine, methyl, ethyl, methoxy, ethoxy, methylthio, ethylthio, trifluoromethyl, trifluoromethoxy and trifluoromethylthio,
R¹⁶ and R¹⁷ independently of one another represent hydrogen, methyl, ethyl, n-propyl, isopropyl, n-butyl, isobutyl, sec-butyl, tert-butyl, n-hexyl, trifluoromethyl, trifluoroethyl, cyclopropyl, cyclopentyl or cyclohexyl,
R¹⁶ and R¹⁷ furthermore together represent - (CH₂)₃-, - (CH₂)₄-, -(CH₂)₅-, (CH₂)₂-O- (CH₂)₂-or - (CH₂)₂-S-(CH₂)2-,
R¹⁸ represents hydrogen, -SO₂R⁷, represents -COR⁷ or -CO₂R⁷; represents C₁-C₁₆-alkyl or C₂-C₁₆-alkenyl, each of which is optionally mono- or polysubstituted by identical or different substituents from the group consisting of fluorine, chlorine, bromine, cyano, methylamino, ethylamino, di-(C₁-C₆-alkyl)amino, C₁-C₄-alkoxy, C₁-C₄-halogenoalkoxy, C₁-C₄-alkylthio and C₁-C₄-halogenoalkylthio; represents C₃-C₈-cycloalkyl, cyclopropylmethyl, cyclopentylmethyl, cyclohexylmethyl, cyclopropylethyl, cyclopentylethyl, cyclohexylethyl, phenyl, benzyl, phenylethyl, tetrazolyl, furyl, furfuryl, benzofuryl, tetrahydrofuryl, thienyl, thenyl, benzothienyl, thiolanyl, pyrrolyl, indolyl, pyrrolinyl, pyrrolidinyl, oxazolyl, benzoxazolyl, isoxazolyl, imidazolyl, pyrazolyl, thiazolyl, benzothiazolyl, thiazolidinyl, pyridinyl, pyrimidinyl, pyridazyl, pyrazinyl, piperidinyl, morpholinyl, thiomorpholinyl, triazinyl, triazolyl, quinolinyl or isoquinolinyl, each of which is optionally mono- to trisubstituted by identical or different substituents from the group consisting of fluorine, chlorine, bromine, cyano, methyl, ethyl, n-propyl, isopropyl, n-butyl, isobutyl, sec-butyl, tert-butyl, C₁-C₄-halogenoalkyl, methoxy, ethoxy, n-propoxy, isopropoxy, n-butoxy, isobutoxy, sec-butoxy, tert-butoxy, C₁-C₄-halogenoalkoxy, methylthio, ethylthio, n-propylthio, isopropylthio, n-butylthio, isobutylthio, sec-butylthio, tert-butylthio and C₁-C₄-halogenoalkylthio.

6. Δ¹-pyrrolines of the formula 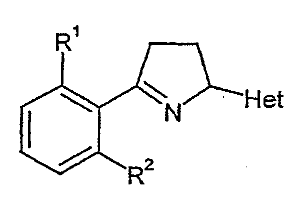 according to Claim 1
in which
R¹ represents fluorine or chlorine,
R² represents hydrogen or fluorine and
Het has the meanings given in one or more of claims 1 to 5.

7. Compounds of the formulae (I-1) to (I-16) 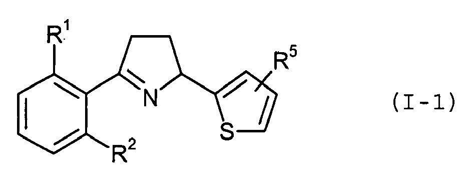 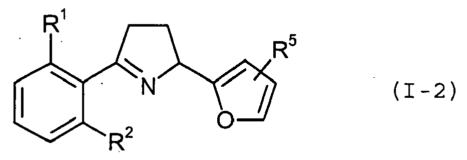 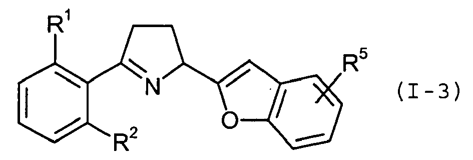 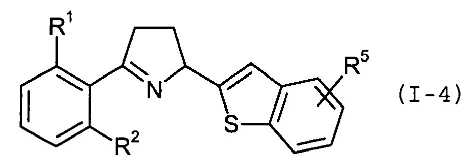 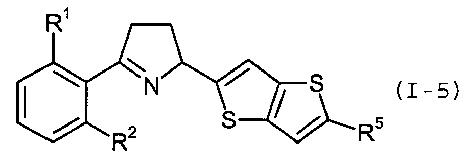 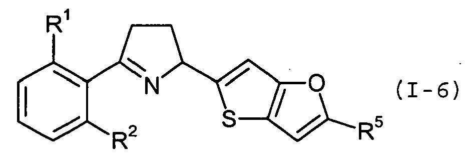 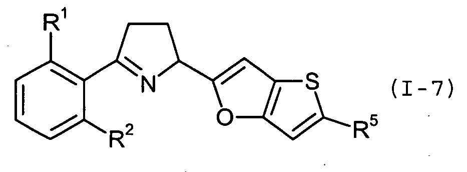 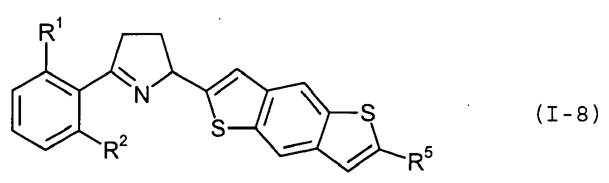 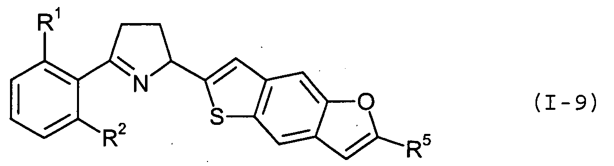 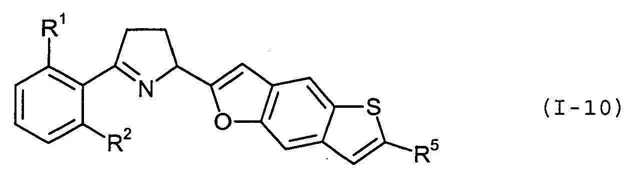 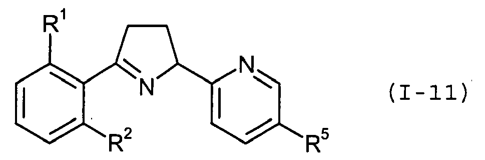 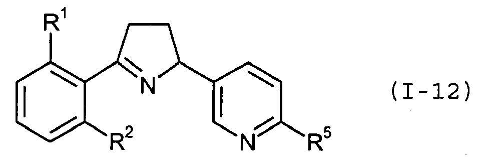 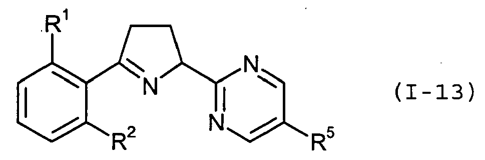 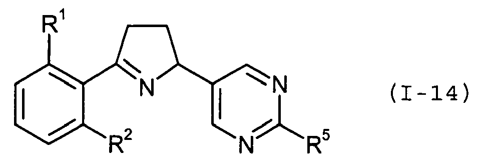 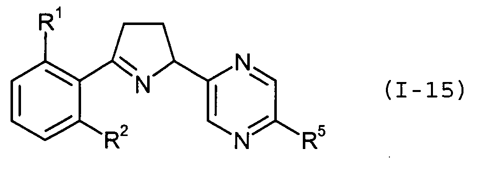 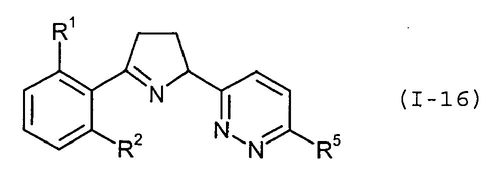 according to Claim 1
in which in each case
R¹ represents fluorine or chlorine,
R² represents hydrogen or fluorine and
R⁵ has the meanings given in one or more of claims 1 to 5.

8. Compounds of the formula (I-d) 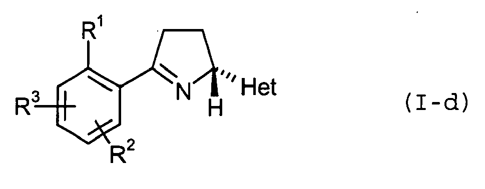 having the (R) configuration according to Claim 1,
in which
R¹, R², R³ and Het have the meanings given in one or more of claims 1 to 5.

9. Δ¹-pyrrolines of the formula (I) according to Claim 1 in which Het represents 2-thienyl, 3-thienyl, 2-thieno[3,2-b]thienyl, 2-pyridinyl or 3-pyridinyl, each of which is optionally mono- or disubstituted by identical or different radicals R⁵.

10. Δ¹-pyrrolines of the formula (I) according to Claim 1 in which R¹ represents fluorine or chlorine, R² represents hydrogen or fluorine and R³ represents hydrogen.

11. Δ¹-pyrrolines of the formula (I) according to Claim 1 in which R² represents hydrogen or fluorine, where R² is located in the 6-position of the phenyl ring to which it is attached.

12. Δ¹-pyrrolines of the formula (I) according to Claim 1 in which X represents a direct bond and Y represents phenylene, preferably 1,4-phenylene.

13. Δ¹-pyrrolines of the formula (I) according to Claim 9 in which X represents a direct bond and Y represents phenylene, preferably 1,4-phenylene.

14. Process for preparing compounds of the formula (I) according to Claim 1, **characterized in that**
A) amino ketones of the formula (II) 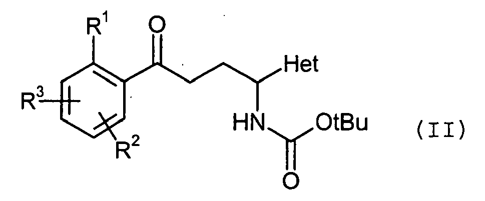 in which
R¹, R², R³ and Het have the meanings given in Claim 1 are treated with a Lewis acid or a protonic acid, if appropriate in the presence of a diluent,
or
B) azides of the formula (III) 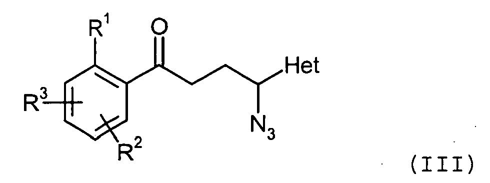 in which
R¹, R², R³ and Het have the meanings given in Claim 1 are reacted with a trialkylphosphine or a triarylphosphine or a trialkyl phosphite or a reducing agent in the presence of a diluent and, if appropriate, in the presence of a catalyst,
or
C) amides of the formula (IV) 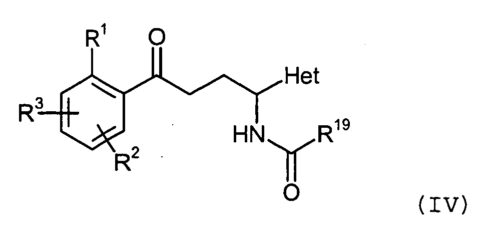 in which
R¹⁹ represents alkyl, halogenoalkyl, aryl or aralkyl and
R¹, R², R³, Het have the meanings given in Claim 1 are reacted with a N-deacetylating agent, if appropriate in the presence of a diluent,
or
Δ¹-pyrrolines of the formula (I-a) 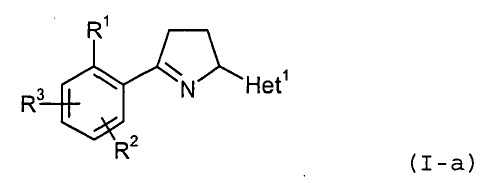 in which
Het¹ represents heteroaryl which is monosubstituted by R⁵⁻¹,
R⁵⁻¹ represents the grouping -Y¹-E,
Y¹ represents in each case optionally substituted phenylene or heterocyclylene and
R¹, R², R³ and E have the meanings given in Claim 1 are obtained by
D) reacting Δ¹-pyrrolines of the formula (I-b) 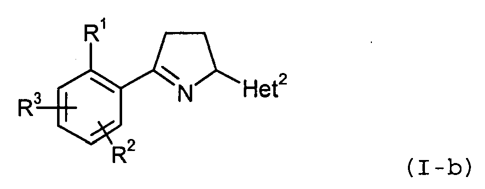 in which
Het² represents heteroaryl which is monosubstituted by R⁵⁻²,
R⁵⁻² represents chlorine, bromine, iodine, -OSO₂CF₃ or -OSO₂(CF₂)₃CF₃,
R¹, R² and R³ have the meanings given in Claim 1
with (hetero)cycles of the formula (V) 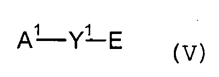 in which
E has the meanings given in Claim 1,
Y¹ has the meanings given above,
A¹ represents chlorine, bromine, iodine, -OSO₂CF₃ or -OSO₂(CF₂)₃CF₃,
in the presence of a catalyst, in the presence of a diboronic ester and, if appropriate, in the presence of an acid binder and, if appropriate, in the presence of a diluent, in a tandem reaction,
or
E) reacting Δ¹-pyrrolines of the formula (VI) 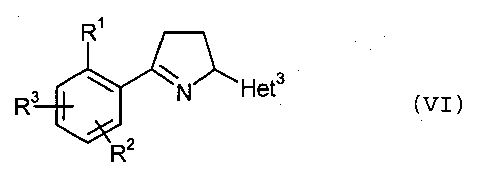 in which
Het³ represents heteroaryl which is monosubstituted by A²,
A² represents -B(OH)₂, (4,4,5,5-tetramethyl-1,3,2-dioxaborolan)-2-yl, (5,5-dimethyl-1,3,2-dioxaborinan)-2-yl, (4,4,6-trimethyl-1,3,2-dioxaborinan)-2-yl or 1,3,2-benzodioxaborol-2-yl and
R¹, R² and R³ have the meanings given in Claim 1
with heterocycles of the formula (V) 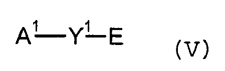 in which
E has the meanings given in Claim 1,
Y¹ and A¹ have the meanings given above,
in the presence of a catalyst, if appropriate in the presence of an acid binder and if appropriate in the presence of a diluent,
or
F) reacting Δ¹-pyrrolines of the formula (I-b) 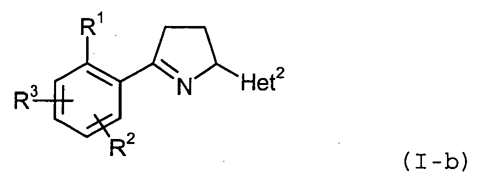 in which
R¹, R² and R³ have the meanings given in Claim 1,
Het² has the meanings given above
with boronic acid derivatives of the formula (VII) 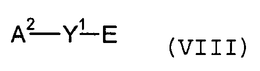 in which
E has the meanings given in Claim 1,
Y¹ and A² have the meanings given above
in the presence of a catalyst, if appropriate in the presence of an acid binder and if appropriate in the presence of a diluent,
or
G) reacting Δ¹-pyrrolines of the formula (I-c) 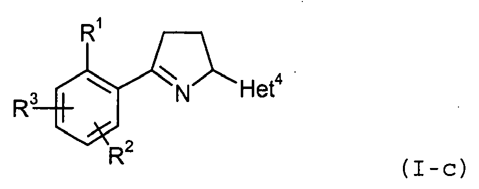 in which
Het⁴ represents heteroaryl which is monosubstituted by R⁵⁻³,
R⁵⁻³ represents bromine or iodine and
R¹, R² and R³ have the meanings given in Claim 1
with organometallic compounds of the formula (VIII) 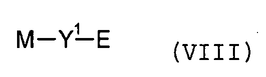 in which
E has the meanings given in Claim 1,
Y¹ has the meanings given above,
M represents ZnCl, Sn(Me)₃ or Sn(n-Bu)₃
in the presence of a catalyst, if appropriate in the presence of an acid binder and if appropriate in the presence of a diluent.

15. Δ¹-pyrrolines of the formula (I-d) 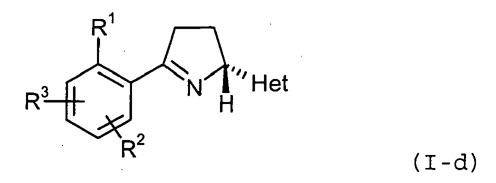 in which
R¹, R², R³ and Het have the meanings given in Claim 1.

16. Aminoketones of the formula (II) 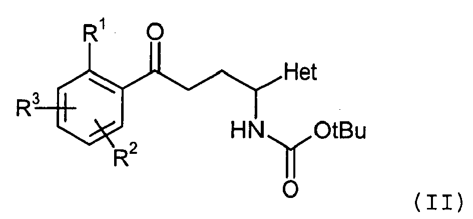 in which
R¹, R², R³ and Het have the meanings given in Claim 1.

17. Azides of the formula (III) 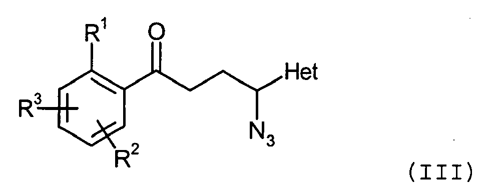 in which
R¹, R², R³ and Het have the meanings given in Claim 1.

18. Amides of the formula (IV) 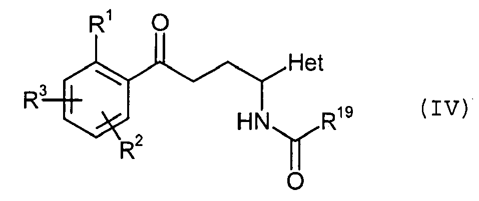 in which
R¹⁹ represents alkyl, halogenoalkyl, aryl or arylalkyl and
R¹, R², R³ and Het have the meanings given in Claim 1.

19. Lactams of the formula (IX) 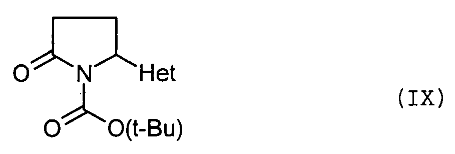 in which
Het has the meanings given in Claim 1.

20. Halides of the formula (XV) 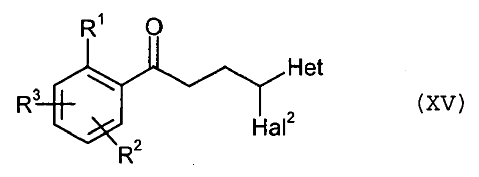 in which
R¹, R², R³ and Het have the meanings given in Claim 1 and
Hal² represents halogen.

21. Pesticide, **characterized in that** it comprises at least one compound according to any of Claims 1 to 13 and 15 in addition to extenders and/or surfactants.

22. Use of compounds according to any of Claims 1 to 13 and 15 for controlling pests, except for the therapeutic treatment of the human or animal body.

23. Method for controlling pests, **characterized in that** compounds according to any of Claims 1 to 13 and 15 are allowed to act on pests and/or their habitat, except for the therapeutic treatment of the human or animal body.

24. Use of compounds according to any of Claims 1 to 13 and 15 for producing an animal medicament against animal parasites, in particular ectoparasites.

25. Process for preparing pesticides, **characterized in that** compounds according to any of Claims 1 to 13 and 15 are mixed with extenders and/or surfactants.

## Revendications

1. Δ¹-pyrrolines de la formule (I) 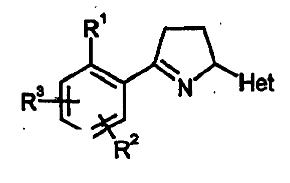 dans laquelle
R¹ représente un halogène, un groupe alkyle, alcoxy ou -S(O)_{w}R⁴, respectivement le cas échéant substitué,
R² et R³ représentent indépendamment l'un de l'autre l'hydrogène, un halogène ou un groupe alkyle, alcoxy ou alcoxyalkyle, respectivement le cas échéant substitué,
R⁴ représente un groupe alkyle, le cas échéant substitué,
Het représente un groupe hétéroaryle, le cas échéant substitué une fois ou plusieurs fois, de manière identique ou différente par R⁵,
R⁵ représente le groupement -X-Y-Z-E, avec la condition que Y ne représente pas une liaison directe si X ne représente pas une liaison directe,
X représente une liaison directe, l'oxygène, -S(O)_{w}-, -NR⁶-, un groupe carbonyle, carbonyloxy, oxycarbonyle, oxysulfonyle (OSO₂), alkylène, halogénoalkylène, alcénylène, halogénoalcénylène, alcynylène, alkylénoxy, oxyalkylène, oxyalkylénoxy, -S(O)_{w}-alkylène, cyclopropylène ou oxiranylène,
Y représente une liaison directe ou un groupe phénylène, naphtylène, tétrahydronaphtylène ou hétérocyclylène, respectivement le cas échéant substitué,
Z représente une liaison directe ou -(CH₂)ₙ-,
E représente l'hydrogène, un halogène, un groupe hydroxy, cyano, formyle, nitro, trialkylsilyle, pentafluorothio, -S(O)_{w}R⁷, -OSO₂R⁷, -NR⁸R⁹, -COR⁷, -CO₂R⁷, -OC(O)R⁷, -CONR¹⁰R¹¹, -N(R¹²)COR¹³, -C(R¹⁴)=N-OR¹⁵, -SO₂NR¹⁶R¹⁷; un groupe alkyle, alcényle, alcynyle, alcoxy, alcényloxy, cycloalkyle, cycloalkylalkyle, cycloalkyloxy, aryle, arylalkyle, aryloxy, aryloxyalkyle, un groupe hétérocyclyle ou hétérocyclylalkyle saturé ou insaturé, respectivement le cas échéant substitué,
R⁶ représente un groupe alkyle, cycloalkyle, cycloalkylalkyle, aryle ou arylalkyle, respectivement le cas échéant substitué,
R⁷ représente un groupe alkyle, cycloalkyle, aryle ou arylalkyle, respectivement le cas échéant substitué,
R⁸ et R⁹ représentent indépendamment l'un de l'autre l' hydrogène, -SO₂R⁷, -COR⁷, -CO₂R⁷, un groupe alkyle, alcényle, cycloalkyle, cycloalkylalkyle, aryle, arylalkyle ou un groupe hétérocyclyle ou hétérocyclylalkyle saturé ou insaturé, respectivement le cas échéant substitué,
R⁸ et R⁹ représentent en outre simultanément un groupe alcénylène ou alkylène, respectivement le cas échéant substitué, la chaîne alkylène pouvant être respectivement interrompue par -O-, -S- ou -NR¹⁸-,
R¹⁰ et R¹¹ représentent indépendamment l'un de l'autre l'hydrogène, -SO₂R⁷, un groupe alkyle, alcényle, cycloalkyle, cycloalkylalkyle, aryle, arylalkyle ou un groupe hétérocyclyle ou hétérocyclylalkyle saturé ou insaturé, respectivement le cas échéant substitué,
R¹⁰ et R¹¹ représentent en outre simultanément un groupe alkylène, respectivement le cas échéant substitué, la chaîne alkylène pouvant être interrompue par -O-, -Sou -NR¹⁸-,
R¹² et R¹³ représentent indépendamment l'un de l'autre l'hydrogène, un groupe alkyle, cycloalkyle, cycloalkylalkyle, aryle ou arylalkyle, respectivement le cas échéant substitué,
R¹² et R¹³ représentent en outre simultanément un groupe alkylène ou alcénylène, respectivement le cas échéant substitué,
R¹⁴ et R¹⁵ représentent indépendamment l'un de l'autre l'hydrogène, un groupe alkyle ou alcényle, respectivement le cas échéant substitué,
R¹⁶ et R¹⁷ représentent indépendamment l'un de l'autre l'hydrogène, un groupe alkyle ou cycloalkyle, respectivement le cas échéant substitué,
R¹⁶ et R¹⁷ représentent en outre simultanément un groupe alkylène, alcoxyalkylène ou alkylthioalkylène, respectivement le cas échéant substitué,
R¹⁸ représente l'hydrogène, -SO₂R⁷, -COR⁷ ou -CO₂R⁷; un groupe alkyle, alcényle, cycloalkyle, cycloalkylalkyle, aryle, arylalkyle ou un groupe hétérocyclyle ou hétérocyclylalkyle saturé ou insaturé, respectivement le cas échéant substitué,
w représente 0, 1 ou 2,
n représente 1, 2, 3 ou 4.

2. Δ¹-pyrrolines selon la formule (I) suivant la revendication 1, dans laquelle
R¹ représente un halogène, un groupe alkyle, halogénoalkyle, alcoxy, halogénoalcoxy ou -S(O)_{w}R⁴,
R² et R³ représentent indépendamment l'un de l'autre l'hydrogène, un halogène, un groupe alkyle, halogénoalkyle, alcoxy, halogénoalcoxy ou alcoxyalkyle,
R⁴ représente un groupe alkyle ou halogénoalkyle,
Het représente un groupe hétéroaryle à 5 à 14 membres, contenant 1 à 3 anneaux aromatiques, le cas échéant substitué de une à quatre fois, de manière identique ou différente, avec un ou plusieurs hétéroatomes de la série de l'azote, de l'oxygène et du soufre,
R⁵ représente le groupement -X-Y-Z-E, avec la condition que Y ne représente pas une liaison directe si X ne représente pas une liaison directe,
X représente une liaison directe, l'oxygène, -NR⁶-, un groupe carbonyle, carbonyloxy, oxycarbonyle, oxysulfonyle (OSO₂), alkylène, halogénoalkylène, alcénylène, halogénoalcénylène, alcynylène, alkylénoxy, oxyalkylène, oxyalkylénoxy, -S(O)_{w}-alkylène, cyclopropylène ou oxiranylène,
Y représente une liaison directe ou un groupe phénylène, naphtylène, tétrahydronaphtylène ou un groupe hétérocyclylène à 5 à 10 membres saturé ou insaturé avec un ou plusieurs hétéroatomes de la série de l'azote, de l'oxygène et du soufre, respectivement le cas échéant substitué de une à quatre fois, de manière identique ou différente, par des restes de la liste W¹,
Z représente une liaison directe ou -(CH₂)ₙ-,
E représente l'hydrogène, un halogène, un groupe hydroxy, cyano, formyle, nitro, trialkylsilyle, pentafluorothio, -S(O)_{w}R⁷, -OSO₂R⁷, -NR⁸R⁹, -COR⁷, -CO₂R⁷,-OC(O)R⁷, -CONR¹⁰R¹¹ -N(R¹²)COR¹³, -C(R¹⁴)=N-OR¹⁵, -SO₂NR¹⁶R¹⁷; un groupe alkyle, alcényle, alcynyle, alcoxy, alcényloxy, respectivement le cas échéant substitué par un halogène, un groupe cyano, alcoxy et/ou -NR⁸R⁹; ou un groupe, cycloalkyle, cycloalkylalkyle, cycloalkyloxy, aryle, arylalkyle, aryloxy, aryloxyalkyle, un groupe hétérocyclyle ou hétérocyclylalkyle saturé ou insaturé à 5 à 10 membres avec un ou plusieurs hétéroatomes de la série de l'azote, de l'oxygène et du soufre, respectivement le cas échéant substitué une fois ou plusieurs fois, de manière identique ou différente, par un halogène, un groupe cyano, nitro, alkyle, halogénoalkyle, alcényle, halogénoalcényle, alcoxy, halogénoalcoxy, alkylthio et/ou halogénoalkylthio,
W¹ représente un halogène, un groupe cyano, formyle, nitro, trialkylsilyle, alkyle, halogénoalkyle, alcoxy, halogénoalcoxy, alcényle, halogénoalcényle, alcényloxy, halogénoalcényloxy, alkylcarbonyle, alcoxycarbonyle, -S(O)_{w}R⁷, -C(R¹⁴)=N-OR¹⁵, -SO₂NR¹⁶R¹⁷, -(CH₂)ₚNR¹⁶R¹⁷, -(CH₂)ₚN(R¹⁶)COR¹⁷, -(CH₂)ₚN(R¹⁶)SO₂R¹⁷, -OSO₂R¹⁶, -OSO₂NR¹⁶R¹⁷,
R⁶ représente un groupe alkyle, halogénoalkyle ou un groupe cycloalkyle, cycloalkylalkyle, aryle ou arylalkyle, respectivement le cas échéant substitué une fois ou plusieurs fois, de manière identique ou différente, par un halogène, un groupe alkyle, halogénoalkyle, alcoxy, halogénoalcoxy, alkylthio et/ou halogénoalkylthio,
R⁷ représente un groupe alkyle, le cas échéant substitué une fois ou plusieurs fois, de manière identique ou différente, par un halogène et/ou -NR⁸R⁹; un groupe cycloalkyle, aryle ou arylalkyle, respectivement le cas échéant substitué une fois ou plusieurs fois, de manière identique ou différente, par un halogène, un groupe cyano, alkyle, halogénoalkyle, alcoxy, halogénoalcoxy, alkylthio, halogénoalkylthio,
R⁸ et R⁹ représentent indépendamment l'un de l'autre l'hydrogène, -SO₂R⁷, -COR⁷, -CO₂R⁷; un groupe alkyle ou alcényle, respectivement le cas échéant substitué une fois ou plusieurs fois, de manière identique ou différente, par un halogène, un groupe alkylcarbonyle, alkylcarbonyloxy, alkylamino, dialkylamino, alcoxy, halogénoalcoxy, alkylthio et/ou halogénoalkylthio; un groupe cycloalkyle, cycloalkylalkyle, aryle, arylalkyle ou un groupe hétérocyclyle ou hétérocyclylalkyle saturé ou insaturé à 5 à 10 membres avec un ou plusieurs hétéroatomes de la série de l'azote, de l'oxygène et du soufre, respectivement le cas échéant substitué une fois ou plusieurs fois, de manière identique ou différente, par un halogène, un groupe cyano, alkyle, halogénoalkyle, alcoxy, halogénoalcoxy, alkylthio et/ou halogénoalkylthio,
R⁸ et R⁹ représentent en outre simultanément un groupe alcénylène, le cas échéant substitué une fois ou plusieurs fois, de manière identique ou différente, par un halogène, un groupe cyano, alcoxy, halogénoalcoxy, alkylthio et/ou halogénoalkylthio; ou un groupe alkylène, le cas échéant substitué une fois ou plusieurs fois, de manière identique ou différente, par un halogène, un groupe cyano, alkyle, halogénoalkyle, alcoxy, halogénoalcoxy, alkylthio et/ou halogénoalkylthio, la chaîne alkylène pouvant être interrompue par -O-, -S- ou -NR¹⁸-,
R¹⁰ et R¹¹ représentent indépendamment l'un de l'autre l'hydrogène, -SO₂R⁷; un groupe alkyle ou alcényle, respectivement le cas échéant substitué une fois ou plusieurs fois, de manière identique ou différente, par un halogène, un groupe alkylamino, dialkylamino, alcoxy, et/ou alkylthio; un groupe cycloalkyle, cycloalkylalkyle, aryle, arylalkyle ou un groupe hétérocyclyle ou hétérocyclylalkyle saturé ou insaturé à 5 à 10 membres avec un ou plusieurs hétéroatomes de la série de l'azote, de l'oxygène et du soufre, respectivement le cas échéant substitué une fois ou plusieurs fois, de manière identique ou différente, par un halogène, un groupe cyano, alkyle, halogénoalkyle, alcoxy, halogénoalcoxy, alkylthio et/ou halogénoalkylthio,
R¹⁰ et R¹¹ représentent en outre simultanément un groupe alkylène, le cas échéant substitué une fois ou plusieurs fois, de manière identique ou différente, par un halogène, un groupe cyano, alkyle, halogénoalkyle, alcoxy, halogénoalcoxy, alkylthio et/ou halogénoalkylthio, la chaîne alkylène pouvant être interrompue par -O-, -S- ou -NR¹⁸,
R¹² et R¹³ représentent indépendamment l'un de l'autre l'hydrogène, un groupe alkyle, le cas échéant substitué une fois ou plusieurs fois, de manière identique ou différente, par un halogène, un groupe cyano, alcoxy et/ou alkylthio; un groupe cycloalkyle, cycloalkylalkyle, aryle ou arylalkyle, respectivement le cas échéant substitué une fois ou plusieurs fois, de manière identique ou différente, par un halogène, un groupe cyano, alkyle, halogénoalkyle, alcoxy, halogénoalcoxy, alkylthio et/ou halogénoalkylthio,
R¹² et R¹³ représentent en outre simultanément un groupe alkylène ou alcénylène, respectivement le cas échéant substitué une fois ou plusieurs fois, de manière identique ou différente, par un halogène, un groupe alkyle, halogénoalkyle, alcoxy, halogénoalcoxy, alkylthio et/ou halogénoalkylthio,
R¹⁴ et R¹⁵ représentent indépendamment l'un de l'autre l'hydrogène, un groupe alkyle, halogénoalkyle, alcényle ou halogénoalcényle,
R¹⁶ et R¹⁷ représentent indépendamment l'un de l'autre l'hydrogène, un groupe alkyle, halogénoalkyle, ou un groupe cycloalkyle, le cas échéant substitué une fois ou plusieurs fois, de manière identique ou différente, par un halogène ou un groupe alkyle,
R¹⁶ et R¹⁷ représentent en outre simultanément un groupe alkylène, alcoxyalkylène ou alkylthioalkylène, respectivement le cas échéant substitué une fois ou plusieurs fois, de manière identique ou différente, par un halogène ou un groupe alkyle,
R¹⁸ représente l'hydrogène, -SO₂R⁷, -COR⁷ ou -CO₂R⁷; un groupe alkyle ou alcényle, respectivement le cas échéant substitué une fois ou plusieurs fois, de manière identique ou différente, par un halogène, un groupe cyano, alkylamino, dialkylamino, alcoxy, halogénoalcoxy, alkylthio et/ou halogénoalkylthio; un groupe cycloalkyle, cycloalkylalkyle, aryle, arylalkyle, un groupe hétérocyclyle ou hétérocyclylalkyle saturé ou insaturé à 5 à 10 membres avec un ou plusieurs hétéroatomes de la série de l'azote, de l'oxygène et du soufre, respectivement le cas échéant substitué une fois ou plusieurs fois, de manière identique ou différente, par un halogène, un groupe cyano, alkyle, halogénoalkyle, alcoxy, halogénoalcoxy, alkylthio et/ou halogénoalkylthio,
w représente 0, 1 ou 2,
n représente 1, 2, 3 ou 4,
p représente 0, 1 ou 2.

3. Δ¹-pyrrolines selon la formule (I) suivant la revendication 1, dans laquelle
R¹ représente un halogène, un groupe alkyle en C₁-C₆, halogénoalkyle en C₁-C₆, alcoxy en C₁-C₆, halogénoalcoxy en C₁-C₆ ou -S(O)_{w}R⁴,
R² et R³ représentent indépendamment l'un de l'autre l'hydrogène, un halogène, un groupe alkyle en C₁-C₆, halogénoalkyle en C₁-C₆, alcoxy en C₁-C₆, halogénoalcoxy en C₁-C₆ ou (alcoxy en C₁-C₆)-(alkyle en C₁-C₆),
R⁴ représente un groupe alkyle en C₁-C₆ ou halogénoalkyle en C₁-C₆,
Het représente un groupe hétéroaryle à 5 à 14 membres, avec 1 à 4 hétéroatomes, contenant 1 à 3 anneaux aromatiques, le cas échéant substitué de une à quatre fois, de manière identique ou différente par R⁵, qui contient 0 à 4 atomes d'azote, 0 à 2 atomes d'oxygène non adjacents et/ou 0 à 2 atomes de soufre non adjacents (en particulier thiényle, benzothiényle, furyle, benzofuryle, indolyle, thiénothiényle, thiénofuryle, thiénobenzothiényle, thiénobenzofuryle, pyridinyle, pyrimidinyle, pyridazinyle, pyrazinyle, triazolyle ou tétrazolyle),
R⁵ représente le groupement -X-Y-Z-E, avec la condition que Y ne représente pas une liaison directe si X ne représente pas une liaison directe,
X représente une liaison directe, l'oxygène, -S(O)_{w}-, -NR⁶-, un groupe carbonyle, carbonyloxy, oxycarbonyle, oxysulfonyle (OSO₂), alkylène en C₁-C₆, halogénoalkylène en C₁-C₆, alcénylène en C₂-C₆, halogénoalcénylène en C₂-C₆, alcynylène en C₂-C₆, alkylénoxy en C₁-C₆, oxyalkylène en C₁-C₆, oxy(alkylénoxy en C₁-C₆), -S(O)_{w}-(alkylène en C₁-C₆), cyclopropylène ou oxiranylène,
Y représente une liaison directe ou un groupe 1,4-phénylène, 1,3-phénylène, 1,2-phénylène, 2,6-naphtylène, 2,7-naphtylène, 1,4-naphtylène, 2,6-(1,2,3,4-tétrahydro)naphtylène, 2,7-(1,2,3,4-tétrahydro)naphtylène, 1,4-(1,2,3,4-tétrahydro)naphtylène, 5,8-(1,2,3,4-tétrahydro)naphtylène, respectivement le cas échéant substitué de une à quatre fois, de manière identique ou différente, par des restes de la liste W¹; ou un groupe hétérocyclylène à 5 ou 6 membres, saturé ou insaturé, avec 1 à 3 hétéroatomes, respectivement le cas échéant substitué de une à quatre fois, de manière identique ou différente, par des restes de la liste W¹, qui contient 0 à 3 atomes d'azote, 0 à 2 atomes d'oxygène non adjacents et/ou 0 à 2 atomes de soufre non adjacents (en particulier furylène, thiénylène, pyrrolylène, oxazolylène, thiazolylène, pyridinylène, pyrimidinylène, pyridazinylène oder pyrazinylène),
Z représente une liaison directe ou -(CH₂)ₙ-,
E représente l'hydrogène, le fluor, le chlore, le brome, un groupe hydroxy, cyano, formyle, nitro, tri-(alkyle en C₁-C₆)silyle, pentafluorothio, -S(O)_{w}R⁷, -OSO₂R⁷, -NR⁸R⁹, -COR⁷, -CO₂R⁷, -OC(O)R⁷, -CONR¹⁰R¹¹, -N(R¹²)COR¹³, -C(R¹⁴)=N-OR¹⁵, -SO₂NR¹⁶R¹⁷; un groupe alkyle en C₁-C₂₀, alcényle en C₂-C₂₀, alcynyle en C₂-C₁₀, alcoxy en C₁-C₂₀, alcényloxy en C₂-C₂₀, respectivement le cas échéant substitué une fois ou plusieurs fois, de manière identique ou différente, par un halogène, un groupe cyano, alcoxy en C₁-C₁₀ et/ou -NR⁸R⁹; ou un groupe cycloalkyle en C₃-C₁₂, (cycloalkyle en C₃-C₇)- (alkyle en C₁-C₄), cycloalcoxy en C₃-C₁₂, aryle, aryle-(alkyle en C₁-C₄), aryloxy, aryloxy-(alkyle en C₁-C₄), un groupe hétérocyclyle ou hétérocyclyle-(alkyle en C₁-C₄) saturé ou insaturé à 5 à 10 membres avec 1 à 4 héteroatomes qui comprennent 0 à 4 atomes d'azote, 0 à 2 atomes d'oxygène non adjacents et/ou 0 à 2 atomes de soufre non adjacents (en particulier tétrazolyle, furyle, furfuryle, benzofuryle, tétrahydrofuryle, thiényle, thényle, benzothiényle, thiolanyle, pyrrolyle, indolyle, pyrrolinyle, pyrrolidino, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, pipéridino, morpholinyle, thiomorpholinyle, morpholine, thiomorpholino, triazinyle, triazolyle, quinolinyle ou isoquinolinyle), respectivement le cas échéant substitué de une à quatre fois, de manière identique ou différente, par un halogène, un groupe cyano, nitro, alkyle en C₁-C₆, halogénoalkyle en C₁-C₆, alcényle en C₂-C₆, halogénoalcényle en C₂-C₆, alcoxy en C₁-C₆, halogénoalcoxy en C₁-C₆, alkylthio en C₁-C₆, halogénoalkylthio en C₁-C₆,
W¹ représente le fluor, le chlore, le brome, un groupe cyano, formyle, nitro, alkyle en C₁-C₆, halogénoalkyle en C₁-C₆, alcoxy en C₁-C₆, halogénoalcoxy en C₁-C₆, alcényle en C₂-C₆, halogénoalcényle en C₂-C₆, alcényloxy en C₂-C₆, halogénoalcényloxy en C₂-C₆, alkylcarbonyle en C₁-C₆, alcoxycarbonyle en C₁-C₆, -S(O)_{w}R⁷, -C(R¹⁴)=N-OR¹⁵ -SO₂NR¹⁶R¹⁷,- (CH₂)ₚNR¹⁶R¹⁷, -(CH₂)ₚN(R¹⁶)COR¹⁷, -(CH₂)ₚN(R¹⁶)SO₂R¹⁷, -OSO₂R¹⁶, -OSO₂NR¹⁶R¹⁷,
R⁶ représente un groupe alkyle en C₁-C₆, halogénoalkyle en C₁-C₆ ou un groupe cycloalkyle en C₃-C₇, (cycloalkyle en C₃-C₆)-(alkyle en C₁-C₄), aryle ou aryle-(alkyle en C₁-C₄), respectivement le cas échéant substitué de une à quatre fois par un halogène, un groupe alkyle en C₁-C₆, halogénoalkyle en C₁-C₆, alcoxy en C₁-C₆, halogénoalcoxy en C₁-C₆, alkylthio en C₁-C₆ et/ou halogénoalkylthio en C₁-C₆,
R⁷ représente un groupe alkyle en C₁-C₂₀, le cas échéant substitué une fois ou plusieurs fois, de manière identique ou différente, par un halogène et/ou -NR⁸R⁹; un groupe cycloalkyle en C₃-C₆, aryle ou aryl-(alkyle en C₁-C₄), respectivement le cas échéant substitué de une à huit fois, de manière identique ou différente, par un halogène, un groupe cyano, alkyle en C₁-C₆, halogénoalkyle en C₁-C₆, alcoxy en C₁-C₆, halogénoalcoxy en C₁-C₆, alkylthio en C₁-C₆ et/ou halogénoalkylthio en C₁-C₆,
R⁸ et R⁹ représentent indépendamment l'un de l'autre l'hydrogène, -SO₂R⁷, -COR⁷, -CO₂R⁷; un groupe alkyle en C₁-C₂₀ ou alcényle en C₂-C₂₀, respectivement le cas échéant substitué une fois ou plusieurs fois, de manière identique ou différente, par un halogène, un groupe alkylcarbonyle en C₁-C₆, alkylcarbonyloxy en C₁-C₆, alkylamino en C₁-C₆, di(alkyle en C₁-C₆)amino, alcoxy en C₁-C₆, halogénoalcoxy en C₁-C₆, alkylthio en C₁-C₆, halogénoalkylthio en C₁-C₆; un groupe cycloalkyle en C₃-C₁₂, aryle, aryle-(alkyle en C₁-C₄) ou un groupe hétérocyclyle ou hétérocyclyle-(alkyle en C₁-C₄) saturé ou insaturé à 5 à 10 membres avec 1 à 4 héteroatomes qui comprennent 0 à 4 atomes d'azote, 0 à 2 atomes d'oxygène non adjacents et/ou 0 à 2 atomes de soufre non adjacents (en particulier tétrazolyle, furyle, furfuryle, benzofuryle, tétrahydrofuryle, thiényle, thényle, benzothiényle, thiolanyle, pyrrolyle, indolyle, pyrrolinyle, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, morpholinyle, thiomorpholinyle, triazinyle, triazolyle, quinolinyle ou isoquinolinyle), respectivement le cas échéant substitué de une à quatre fois, de manière identique ou différente, par un halogène, un groupe cyano, alkyle en C₁-C₆, halogénoalkyle en C₁-C₆, alcoxy en C₁-C₆, halogénoalcoxy en C₁-C₆, alkylthio en C₁-C₆, halogénoalkylthio en C₁-C₆,
R⁸ et R⁹ représentent en outre simultanément un groupe alcénylène, le cas échéant substitué une fois ou plusieurs fois, de manière identique ou différente, par un halogène, un groupe cyano, alcoxy en C₁-C₆, halogénoalcoxy en C₁-C₆, alkylthio en C₁-C₆, halogénoalkylthio en C₁-C₆; ou un groupe alkylène en C₃-C₁₂, le cas échéant substitué une fois ou plusieurs fois dans la partie alkylène, de manière identique ou différente, par un halogène, un groupe cyano, alkyle en C₁-C₆, halogénoalkyle en C₁-C₆, alcoxy en C₁-C₆, halogénoalcoxy en C₁-C₆, alkylthio en C₁-C₆, halogénoalkylthio en C₁-C₆, la chaîne alkylène pouvant être interrompue par -O-, -S- ou -NR¹⁸ -,
R¹⁰ et R¹¹ représentent indépendamment l'un de l'autre l'hydrogène, -SO₂R⁷; un groupe alkyle en C₁-C₆ ou alcényle en C₂-C₆, respectivement le cas échéant substitué de une à treize fois, de manière identique ou différente, par un halogène, un groupe alkylamino en C₁-C₆, di(alkyle en C₁-C₆)amino, alcoxy en C₁-C₆, halogénoalcoxy en C₁-C₆, alkylthio en C₁-C₆, halogénoalkylthio en C₁-C₆; un groupe cycloalkyle en C₃-C₇, (cycloalkyle en C₃-C₇)-(alkyle en C₁-C₄), aryle, aryle-(alkyle en C₁-C₄) ou un groupe hétérocyclyle ou hétérocyclyle-(alkyle en C₁-C₄) saturé ou insaturé à 5 à 10 membres avec 1 à 4 héteroatomes qui comprennent 0 à 4 atomes d'azote, 0 à 2 atomes d'oxygène non adjacents et/ou 0 à 2 atomes de soufre non adjacents (en particulier tétrazolyle, furyle, furfuryle, benzofuryle, tétrahydrofuryle, thiényle, thényle, benzothiényle, thiolanyle, pyrrolyle, indolyle, pyrrolinyle, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, morpholinyle, thiomorpholinyle, triazinyle, triazolyle, quinolinyle ou isoquinolinyle), respectivement le cas échéant substitué de une à quatre fois, de manière identique ou différente, par un halogène, un groupe cyano, alkyle en C₁-C₆, halogénoalkyle en C₁-C₆, alcoxy en C₁-C₆, halogénoalcoxy en C₁-C₆, alkylthio en C₁-C₆, halogénoalkylthio en C₁-C₆,
R¹⁰ et R¹¹ représentent en outre simultanément un groupe alkylène en C₁-C₆, -(CH₂)₂-O-(CH₂)₂-, -(CH₂)₂-S- (CH₂)₂- ou -(CH₂)₂-N(R¹⁸)-(CH₂)₂, respectivement le cas échéant substitué de une à quatre fois dans la partie alkylène, de manière identique ou différente, par un halogène, un groupe cyano, alkyle en C₁-C₆, halogénoalkyle en C₁-C₆, alcoxy en C₁-C₆, halogénoalcoxy en C₁-C₆, alkylthio en C₁-C₆, halogénoalkylthio en C₁-C₆,
R¹² et R¹³ représentent indépendamment l'un de l'autre l'hydrogène, un groupe alkyle en C₁-C₆, le cas échéant substitué de une à treize fois, de manière identique ou différente, par un halogène, un groupe cyano, alcoxy en C₁-C₆, alkylthio en C₁-C₆; un groupe cycloalkyle en C₃-C₇, aryle ou aryle-(alkyle en C₁-C₄), respectivement le cas échéant substitué de une à huit fois, de manière identique ou différente, par un halogène, un groupe cyano, alkyle en C₁-C₆, halogénoalkyle en C₁-C₆, alcoxy en C₁-C₆, halogénoalcoxy en C₁-C₆, alkylthio en C₁-C₆, halogénoalkylthio en C₁-C₆,
R¹² et R¹³ représentent en outre simultanément un groupe alkylène en C₃-C₁₀ ou alcénylène en C₃-C₁₀, respectivement le cas échéant substitué de une à huit fois, de manière identique ou différente, par un halogène, un groupe alkyle en C₁-C₆, halogénoalkyle en C₁-C₆, alcoxy en C₁-C₆, halogénoalcoxy en C₁-C₆, alkylthio en C₁-C₆, halogénoalkylthio en C₁-C₆,
R¹⁴ et R¹⁵ représentent indépendamment l'un de l'autre l'hydrogène, un groupe alkyle en C₁-C₆, halogénoalkyle en C₁-C₆, alcényle en C₂-C₆ ou halogénoalcényle en C₂-C₆,
R¹⁶ et R¹⁷ représentent indépendamment l'un de l'autre l'hydrogène, un groupe alkyle en C₁-C₆, halogénoalkyle en C₁-C₆ ou un groupe cycloalkyle en C₃-C₇, le cas échéant substitué de une à huit fois, de manière identique ou différente, par le fluor, le chlore, le brome et/ou un groupe alkyle en C₁-C₆,
R¹⁶ et R¹⁷ représentent en outre simultanément un groupe alkylène en C₃-C₆, (alcoxy en C₁-C₃)-(alkylène en C₁-C₃) ou (alkylthio en C₁-C₃)-(alkylène en C₁-C₃), respectivement le cas échéant substitué de une à neuf fois, de manière identique ou différente, par le fluor, le chlore, le brome et/ou un groupe alkyle en C₁-C₆,
R¹⁸ représente l'hydrogène, -SO₂R⁷ -COR⁷ ou -CO₂R⁷; un groupe alkyle en C₁-C₂₀ ou alcényle en C₂-C₂₀, respectivement le cas échéant substitué une fois ou plusieurs fois, de manière identique ou différente, par un halogène, un groupe cyano, alkylamino en C₁-C₆, di(alkyle en C₁-C₆)amino, alcoxy en C₁-C₆, halogénoalcoxy en C₁-C₆. alkylthio en C₁-C₆, halogénoalkylthio en C₁-C₆; un groupe cycloalkyle en C₃-C₁₂, (cycloalkyle en C₃-C₇)-(alkyle en C₁-C₄), aryle, aryle-(alkyle en C₁-C₄), un groupe hétérocyclyle ou hétérocyclyle-(alkyle en C₁-C₄) saturé ou insaturé à 5 à 10 membres avec 1 à 4 héteroatomes qui comprennent 0 à 4 atomes d'azote, 0 à 2 atomes d'oxygène non adjacents et/ou 0 à 2 atomes de soufre non adjacents (en particulier tétrazolyle, furyle, furfuryle, benzofuryle, tétrahydrofuryle, thiényle, thényle, benzothiényle, thiolanyle, pyrrolyle, indolyle, pyrrolinyle, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, morpholinyle, thiomorpholinyle, triazinyle, triazolyle, quinolinyle ou isoquinolinyle), respectivement le cas échéant substitué de une à quatre fois, de manière identique ou différente, par un halogène, un groupe cyano, alkyle en C₁-C₆, halogénoalkyle en C₁-C₆, alcoxy en C₁-C₆, halogénoalcoxy en C₁-C₆, alkylthio en C₁-C₆, halogénoalkylthio en C₁-C₆,
w représente 0, 1 ou 2,
n représente 1, 2 ou 3,
p représente 0, 1 ou 2.

4. Δ¹-pyrrolines selon la formule (I) suivant la revendication 1, dans laquelle
R¹ représente le fluor, le chlore, le brome, un groupe alkyle en C₁-C₄, halogénoalkyle en C₁-C₄ avec 1 à 9 atomes de fluor, de chlore et/ou de brome, alcoxy en C₁-C₄, halogénoalcoxy en C₁-C₄ avec 1 à 9 atomes de fluor, de chlore et/ou de brome, ou -S(O)_{w}R⁴,
R² et R³ représentent indépendamment l'un de l'autre l'hydrogène, le fluor, le chlore, le brome, un groupe alkyle en C₁-C₄, halogénoalkyle en C₁-C₄ avec 1 à 9 atomes de fluor, de chlore et/ou de brome, alcoxy en C₁-C₄, halogénoalcoxy en C₁-C₄ avec 1 à 9 atomes de fluor, de chlore et/ou de brome,
R⁴ représente un groupe alkyle en C₁-C₄ ou un groupe méthyle ou éthyle respectivement substitué par le fluor ou le chlore,
Het représente un groupe 2-thiényle, 3-thiényle, 2-benzo[b]thiényle, 2-furyle, 3-furyle, 2-benzo[b]furyle, 2-indolyle, 2-thiéno[3,2-b]thiényle, 2-thiéno[3,2-b]furyle, 5-thiéno[3,2-b]furyle, 2-thiéno[2,3-f][1]benzothiényle, 2-thiéno[2,3-f][1]benzofuryle, 6-thiéno[2,3-f][1]benzofuryle, 2-pyridinyle, 3-pyridinyle, 2-pyrimidinyle, 5-pyrimidinyle, 3-pyridazinyle, 4-pyridazinyle, 2-pyrazinyle, triazolyle ou tétrazolyle, respectivement le cas échéant substitué de une à trois fois, de manière identique ou différente, par R⁵,
R⁵ représente le groupement -X-Y-Z-E, avec la condition que Y ne représente pas une liaison directe si X ne représente pas une liaison directe,
X représente une liaison directe, l'oxygène, -S(O)_{w}-, -NR⁶-, un groupe carbonyle, carbonyloxy, oxycarbonyle, oxysulfonyle (OSO₂), alkylène en C₁-C₄, halogénoalkylène en C₁-C₄ avec 1 à 8 atomes de fluor, de chlore et/ou de brome, alcénylène en C₂-C₄, halogénoalcénylène en C₂-C₄ avec 1 à 6 atomes de fluor, de chlore et/ou de brome, alcynylène en C₂-C₄, alkylénoxy en C₁-C₄, oxy(alkylène en C₁-C₄), oxy(alkylénoxy en C₁-C₄) ou -S(O)_{w}-(alkylène en C₁-C₄),
Y représente une liaison directe ou un groupe 1,4-phénylène, 1,3-phénylène, 1,2-phénylène, 2,6-naphtylène, 2,7-naphtylène, 1,4-naphtylène, 2,6-(1,2,3,4-tétrahydro)naphtylène, 2,7-(1,2,3,4-tétrahydro)naphtylène, 1,4-(1,2,3,4-tétrahydro)naphtylène, 5,8-(1,2,3,4-tétrahydro)naphtylène, 2,4-furylène, 2,4-thiénylène, 2,4-pyrrolylène, 2,5-oxazolylène, 2,5-thiazolylène, 2,5-pyridinylène, 2,6-pyridinylène, 2,5-pyrimidinylène, 3,6-pyridazinylène ou 2,5-pyrazinylène, respectivement le cas échéant substitué de une à trois fois, de manière identique ou différente, par des restes de la liste W¹;
Z représente une liaison directe ou -(CH₂)ₙ-,
E représente l'hydrogène, le fluor, le chlore, le brome, un groupe hydroxy, cyano, formyle, nitro, trialkylsilyle, diméthyl-tert-butylsilyle, -S(O)_{w}R⁷, -OSO₂R⁷, -NR⁸R⁹, -COR⁷, -CO₂R⁷, -OC(O)R⁷, -CONR¹⁰R¹¹ -N(R¹²)COR¹³, -SO₂NR¹⁶R¹⁷; un groupe alkyle en C₁-C₁₆, alcényle en C₂-C₁₆, alcynyle en C₂-C₁₆, alcoxy en C₁-C₁₆, alcényloxy en C₂-C₁₆, respectivement le cas échéant substitué une fois ou plusieurs fois, de manière identique ou différente, par le fluor, le chlore, le brome, un groupe cyano, alcoxy en C₁-C₆ et/ou -NR⁸R⁹; ou un groupe cycloalkyle en C₃-C₁₀, (cycloalkyle en C₃-C₆)-(alkyle en C₁-C₄), cycloalkyloxy en C₃-C₁₀, phényle, phénoxy, benzyle, phényléthyle, benzyloxy, tétrazolyle, furyle, furfuryle, benzofuryle, tétrahydrofuryle, thiényle, thényle, benzothiényle, thiolanyle, pyrrolyle, indolyle, pyrrolinyle, pyrrolidino, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, piperidino, morpholinyle, thiomorpholinyle, morpholino, thiomorpholino, triazinyle, triazolyle, quinolinyle ou isoquinolinyle, respectivement le cas échéant substitué de une à trois fois, de manière identique ou différente, par le fluor, le chlore, le brome, un groupe cyano, alkyle en C₁-C₄, halogénoalkyle en C₁-C₄, alcényle en C₂-C₆, halogénoalcényle en C₂-C₆, alcoxy en C₁-C₄, halogénoalcoxy en C₁-C₄, alkylthio en C₁-C₄, halogénoalkylthio en C₁-C₄,
W¹ représente le fluor, le chlore, le brome, un groupe cyano, formyle, triméthylsilyle, diméthyl-tert-butylsilyle, alkyle en C₁-C₄, alcoxy en C₁-C₄, alcényle en C₂-C₄, alcényloxy en C₂-C₄; un groupe halogénoalkyle en C₁-C₄, halogénoalcoxy en C₁-C₄ avec respectivement 1 à 9 atomes de fluor, de chlore et/ou de brome, halogénoalcényle en C₂-C₄, halogénoalcényloxy en C₂-C₄ avec respectivement 1 à 8 atomes de fluor, de chlore et/ou de brome; un groupe alkylcarbonyle en C₁-C₄, alcoxycarbonyle en C₁-C₄, -S(O)_{w}R⁷, -SO₂NR¹⁶R¹⁷, -(CH₂)ₚNR¹⁶R¹⁷, -(CH₂)ₚN(R¹⁶)COR¹⁷, -(CH₂)ₚN(R¹⁶)SO₂R¹⁶, -OSO₂R¹⁶, -OSO₂NR¹⁶R¹⁷,
R⁶ représente un groupe méthyle, éthyle, n-propyle, i-propyle, n-butyle, i-butyle, s-butyle, t-butyle, trifluorométhyle, trifluoroéthyle, ou un groupe cyclopropyle, cyclopropylméthyle, cyclopentyle, cyclopentylméthyle, cyclohexyle, cyclohexylméthyle, phényle, benzyle ou phényléthyle, respectivement le cas échéant substitué de une à quatre fois, de manière identique ou différente, par le fluor, le chlore, le brome, un groupe alkyle en C₁-C₄, alcoxy en C₁-C₄, alkylthio en C₁-C₄, halogénoalkyle en C₁-C₄, halogénoalcoxy en C₁-C₄ et/ou halogénoalkylthio en C₁-C₄, avec respectivement 1 à 9 atomes de fluor, de chlore et/ou de brome,
R⁷ représente un groupe alkyle en C₁-C₁₀, le cas échéant substitué une fois ou plusieurs fois, de manière identique ou différente, par le fluor, le chlore, le brome et/ou -NR⁸R⁹; un groupe cyclopropyle, cyclopentyle, cyclohexyle, phényle ou benzyle, respectivement le cas échéant substitué de une à quatre fois, de manière identique ou différente, par le fluor, le chlore, le brome, un groupe cyano, alkyle en C₁-C₄, alcoxy en C₁-C₄, alkylthio en C₁-C₄, halogénoalkyle en C₁-C₄, halogénoalcoxy en C₁-C₄ et/ou halogénoalkylthio en C₁-C₄, avec respectivement 1 à 9 atomes de fluor, de chlore et/ou de brome,
R⁸ et R⁹ représentent indépendamment l'un de l'autre l'hydrogène, -SO₂R⁷, -COR⁷, -CO₂R⁷; un groupe alkyle en C₁-C₁₆ ou alcényle en C₂-C₁₆, respectivement le cas échéant substitué une fois ou plusieurs fois, de manière identique ou différente, par le fluor, le chlore, le brome, un groupe alkylcarbonyle en C₁-C₄, alkylcarbonyloxy en C₁-C₄, alkylamino en C₁-C₄, di(alkyle en C₁-C₄)amino, alcoxy en C₁-C₄, halogénoalcoxy en C₁-C₄, alkylthio en C₁-C₄, halogénoalkylthio en C₁-C₄; un groupe cycloalkyle en C₃-C₁₀, (cycloalkyle en C₃-C₆)-(alkyle en C₁-C₄), phényle, benzyle, phényléthyle, tétrazolyle, furyle, furfuryle, benzofuryle, tétrahydrofuryle, thiényle, thényle, benzothiényle, thiolanyle, pyrrolyle, indolyle, pyrrolinyle, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, morpholinyle, thiomorpholinyle, triazinyle, triazolyle, quinolinyle ou isoquinolinyle, respectivement le cas échéant substitué de une à trois fois, de manière identique ou différente, par le fluor, le chlore, le brome, un groupe cyano, alkyle en C₁-C₄, halogénoalkyle en C₁-C₄, alcoxy en C₁-C₄, halogénoalcoxy en C₁-C₄, alkylthio en C₁-C₄, halogénoalkylthio en C₁-C₄,
R⁸ et R⁹ représentent en outre simultanément un groupe alcénylène en C₂-C₁₀, le cas échéant substitué une fois ou plusieurs fois, de manière identique ou différente, par le fluor, le chlore, le brome, un groupe alcoxy en C₁-C₄, halogénoalcoxy en C₁-C₄, alkylthio en C₁-C₄, halogénoalkylthio en C₁-C₄; ou un groupe alkylène en C₃-C₁₀, le cas échéant substitué une fois ou plusieurs fois dans la partie alkylène, de manière identique ou différente, par le fluor, le chlore, le brome, un groupe cyano, alkyle en C₁-C₄, halogénoalkyle en C₁-C₄, alcoxy en C₁-C₄, halogénoalcoxy en C₁-C₄, alkylthio en C₁-C₄, halogénoalkylthio en C₁-C₄, la chaîne alkylène pouvant être interrompue par -O-, -S- ou -NR¹⁸-,
R¹⁰ et R¹¹ représentent indépendamment l'un de l'autre l'hydrogène, -SO₂R⁷; un groupe alkyle en C₁-C₆ ou alcényle en C₂-C₆, respectivement le cas échéant substitué de une à neuf fois, de manière identique ou différente, par le fluor, le chlore, le brome, un groupe alkylamino en C₁-C₄, di(alkyle en C₁-C₄)amino, alcoxy en C₁-C₄, halogénoalcoxy en C₁-C₄, alkylthio en C₁-C₄, halogénoalkylthio en C₁-C₄; un groupe cycloalkyle en C₃-C₆, (cycloalkyle en C₃-C₆)-(alkyle en C₁-C₄), phényle, benzyle, phényléthyle, tétrazolyle, furyle, furfuryle, benzofuryle, tétrahydrofuryle, thiényle, thényle, benzothiényle, thiolanyle, pyrrolyle, indolyle, pyrrolinyle, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, morpholinyle, thiomorpholinyle, triazinyle, triazolyle, quinolinyle ou isoquinolinyle, respectivement le cas échéant substitué de une à trois fois, de manière identique ou différente, par le fluor, le chlore, le brome, un groupe cyano, alkyle en C₁-C₄, halogénoalkyle en C₁-C₄, alcoxy en C₁-C₄, halogénoalcoxy en C₁-C₄, alkylthio en C₁-C₄, halogénoalkylthio en C₁-C₄,
R¹⁰ et R¹¹ représentent en outre simultanément un groupe alkylène en C₄-C₆, -(CH₂)₂-O-(CH₂)₂-, -(CH₂)₂-S-(CH₂)₂- ou -(CH₂)₂-N(R¹⁸)-(CH₂)₂, respectivement le cas échéant substitué de une à quatre fois dans la partie alkylène, de manière identique ou différente, par le fluor, le chlore, le brome, un groupe cyano, alkyle en C₁-C₄, halogénoalkyle en C₁-C₄, alcoxy en C₁-C₄, halogénoalcoxy en C₁-C₄, alkylthio en C₁-C₄, halogénoalkylthio en C₁-C₄,
R¹² et R¹³ représentent indépendamment l'un de l'autre l'hydrogène, un groupe alkyle en C₁-C₆, le cas échéant substitué de une à neuf fois, de manière identique ou différente, par le fluor, le chlore, le brome, un groupe alcoxy en C₁-C₄, alkylthio en C₁-C₄; un groupe cycloalkyle en C₃-C₆, (cycloalkyle en C₃-C₆)-(alkyle en C₁-C₄), phényle, benzyle ou phényléthyle, respectivement le cas échéant substitué de une à quatre fois, de manière identique ou différente, par le fluor, le chlore, le brome, un groupe alkyle en C₁-C₄, halogénoalkyle en C₁-C₄, alcoxy en C₁-C₄, halogénoalcoxy en C₁-C₄, alkylthio en C₁-C₄, halogénoalkylthio en C₁-C₄,
R¹² et R¹³ représentent en outre simultanément un groupe alkylène en C₃-C₈ ou alcénylène en C₃-C₈, respectivement le cas échéant substitué de une à quatre fois, de manière identique ou différente, par le fluor, le chlore, le brome, un groupe alkyle en C₁-C₄, halogénoalkyle en C₁-C₄, alcoxy en C₁-C₄, halogénoalcoxy en C₁-C₄, alkylthio en C₁-C₄, halogénoalkylthio en C₁-C₄,
R¹⁶ et R¹⁷ représentent indépendamment l'un de l'autre l'hydrogène, un groupe alkyle en C₁-C₆, halogénoalkyle en C₁-C₄ avec 1 à 9 atomes de fluor, de chlore et/ou de brome, ou un groupe cycloalkyle en C₃-C₆, le cas échéant substitué de une à quatre fois, de manière identique ou différente, par le fluor, le chlore, le brome et/ou un groupe alkyle en C₁-C₄,
R¹⁶ et R¹⁷ représentent en outre simultanément -(CH₂)₃-, -(CH₂)₄-, -(CH₂)₅-, -(CH₂)₂-O-(CH₂)₂- ou -(CH₂)₂-S-(CH₂)₂-,
R¹⁸ représente l'hydrogène, -SO₂R⁷ -COR⁷ ou -CO₂R⁷; un groupe alkyle en C₁-C₁₆ ou alcényle en C₂-C₁₆, respectivement le cas échéant substitué une fois ou plusieurs fois, de manière identique ou différente, par le fluor, le chlore, le brome, un groupe cyano, méthylamino, éthylamino, di(alkyle en C₁-C₆)amino, alcoxy en C₁-C₄, halogénoalcoxy en C₁-C₄, alkylthio en C₁-C₄, halogénoalkylthio en C₁-C₄; un groupe cycloalkyle en C₃-C₁₀, (cycloalkyle en C₃-C₆)-(alkyle en C₁-C₄), phényle, benzyle, phényléthyle, tétrazolyle, furyle, furfuryle, benzofuryle, tétrahydrofuryle, thiényle, thényle, benzothiényle, thiolanyle, pyrrolyle, indolyle, pyrrolinyle, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, morpholinyle, thiomorpholinyle, triazinyle, triazolyle, quinolinyle ou isoquinolinyle, respectivement le cas échéant substitué de une à trois fois, de manière identique ou différente, par le fluor, le chlore, le brome, un groupe cyano, alkyle en C₁-C₄, halogénoalkyle en C₁-C₄, alcoxy en C₁-C₄, halogénoalcoxy en C₁-C₄, alkylthio en C₁-C₄, halogénoalkylthio en C₁-C₄,
w représente 0, 1 ou 2,
n représente 1 ou 2,
p représente 0 ou 1.

5. Δ¹-pyrrolines selon la formule (I) suivant la revendication 1, dans laquelle
R¹ représente le fluor, le chlore, le brome, un groupe méthyle, trifluorométhyle, méthoxy, trifluorométhoxy, méthylthio ou trifluorométhylthio,
R² et R³ représentent indépendamment l'un de l'autre l'hydrogène, le fluor, le chlore, le brome, un groupe méthyle, trifluorométhyle, méthoxy ou trifluorométhoxy,
Het représente un groupe 2-thiényle, 3-thiényle, 2-benzo[b]thiényle, 2-furyle, 3-furyle, 2-benzo[b]furyle, 2-thiéno[3,2-b]thiényle, 2-thiéno[3,2-b]furyle, 5-thiéno[3,2-b]furyle, 2-thiéno[2,3-f][1]benzothiényle, 2-thiéno[2,3-f][1]benzofuryle, 6-thiéno[2,3-f][1]benzofuryle, 2-pyridinyle, 3-pyridinyle, 2-pyrimidinyle, 5-pyrimidinyle, 3-pyridazinyle, 4-pyridazinyle ou 2-pyrazinyle, respectivement le cas échéant substitué une ou deux fois, de manière identique ou différente, par R⁵,
R⁵ représente le groupement -X-Y-Z-E, avec la condition que Y ne représente pas une liaison directe si X ne représente pas une liaison directe,
X représente une liaison directe, l'oxygène, le soufre, -SO₂-, -NR⁶-, -CO-, -C(O)-O-, -O-C(O)-, -CH₂-, -(CH₂)₂-, -C=C- (E ou Z), -C≡C-, -CH₂O-, -(CH₂)₂O-, -OCH₂-, -O(CH₂)₂-, -O-CH₂-O-, -SCH₂-, -S(CH₂)₂-, -CH₂S- oder -(CH₂)₂S-,
Y représente une liaison directe ou un groupe 1,4-phénylène, 1,3-phénylène, 2,6-naphtylène, 2,7-naphtylène, 2,4-furylène, 2,4-thiénylène, 2,5-pyridinylène, 2,5-pyrimidinylène, 3,6-pyridazinylène ou 2,5-pyrazinylène, respectivement le cas échéant substitué une ou deux fois, de manière identique ou différente, par des restes de la liste W¹;
Z représente une liaison directe, méthylène ou éthylène,
E représente l'hydrogène, le fluor, le chlore, le brome, un groupe hydroxy, cyano, -S(O)_{w}R⁷,-OSO₂R⁷, -NR⁸R⁹, -COR⁷, -CO₂R⁷, -OC(O)R⁷, -CONR¹⁰R¹¹ -N(R¹²)COR¹³, -C(R¹⁴)=N-OR¹⁵, -SO₂NR¹⁶R¹⁷; un groupe alkyle en C₁-C₁₆, alcényle en C₂-C₁₆, alcoxy en C₁-C₁₆, alcényloxy en C₂-C₁₆, respectivement le cas échéant substitué une fois ou plusieurs fois, de manière identique ou différente, par le fluor, le chlore, le brome, un groupe cyano, alcoxy en C₁-C₆ et/ou -NR⁸R⁹; ou un groupe cyclopropyle, cyclopentyle, cyclohexyle, cyclopropyloxy, cyclopentyloxy, cyclohexyloxy, cyclopropylméthyle, cyclopentylméthyle, cyclohexylméthyle, phényle, phenoxy, benzyle, phényléthyle, benzyloxy, tétrazolyle, furyle, furfuryle, benzofuryle, tétrahydrofuryle, thiényle, thényle, benzothiényle, thiolanyle, pyrrolyle, indolyle, pyrrolinyle, pyrrolidino, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, pipéridino, morpholinyle, thiomorpholinyle, morpholino, thiomorpholino, triazinyle, triazolyle, quinolinyle ou isoquinolinyle, respectivement le cas échéant substitué de une à trois fois, de manière identique ou différente, par le fluor, le chlore, le brome, un groupe cyano, méthyle, éthyle, n-propyle, i-propyle, -CF₃, -CHF₂, -CClF₂, -CF₂CHFCl, -CF₂CH₂F, -CF₂CCl₃, -CH₂CF₃, -CF₂CHFCF_{3'} -CH₂CF₂H, -CH₂CF₂CF₃, -CF₂CF₂H, -CF₂CHFCF_{3'} vinyle, allyle, 1-propényle, butényle, -CF=CHF, -CF=CH₂, -CF=CCl₂, -CH=CF₂, -CF₂CF=CF₂, -CH=CFH, -CH₂CF=CF₂, -CF=CF₂, -CF₂CH=CF₂, méthoxy, éthoxy, n-propoxy, i-propoxy, n-butoxy, i-butoxy, s-butoxy, t-butoxy, trifluorométhoxy, difluorométhoxy, chlorodifluorométhoxy, trifluoroéthoxy, méthylthio, éthylthio, n-propylthio, i-propylthio, n-butylthio, i-butylthio, s-butylthio, t-butylthio, trifluorométhylthio, difluorométhylthio, chlorodifluorométhylthio, trifluoroéthylthio,
W¹ représente le fluor, le chlore, le brome, un groupe cyano, formyle, méthyle, éthyle, n-propyle, i-propyle, n-butyle, i-butyle, s-butyle, t-butyle, méthoxy, éthoxy, n-propoxy, i-propoxy, n-butoxy, i-butoxy, s-butoxy, t-butoxy, vinyle, allyle, trifluorométhyle, trifluoroéthyle, trifluorométhoxy, trifluoroéthoxy, -OCF₂CF₂H, -CH=CF₂, -CH=CCl₂, -OCF=CF₂, -CO-méthyle, -CO-éthyle, -CO₂-méthyle, -CO₂-éthyle, -CO₂(t-butyle), -S-méthyle, -SO-méthyle, -SO₂-méthyle, -SCF₃, -SOCF₃, -SO₂CF₃, -SCHF₂, -SOCHF₂, -SO₂CHF₂, -SO₂N-(méthyle)₂, -N-(méthyle)₂, -N-(éthyle)₂, -N(n-propyle)₂, -N(méthyle)CO-méthyle, -N(méthyle)CO-éthyle, -N(méthyle)CO-propyle, -N(méthyle)CO(t-butyle), 2-pyrrolidonyle, 2-pipéridonyle, -N(méthyle)SO₂-méthyle, -N(méthyle)SO₂-éthyle, -N(méthyle)SO₂CF₃, -N(éthyle)SO₂CF₃, -N(méthyle)SO₂(CF₂)₃CF₃ ou -OSO₂N-(méthyle)₂,
R⁶ représente un groupe méthyle, éthyle, n-propyle, i-propyle, n-butyle, i-butyle, s-butyle, t-butyle, trifluorométhyle, trifluoroéthyle, cyclopropyle, cyclopropylméthyle, cyclopentyle, cyclopentylméthyle, cyclohexyle, cyclohexylméthyle, phényle, benzyle ou phényléthyle,
R⁷ représente un groupe méthyle, éthyle, n-propyle, i-propyle, n-butyle, i-butyle, s-butyle, t-butyle, pentyle, hexyle, -CF₃, -CHF₂, -CCl₃, -CCl₂F, diméthylaminométhyle, diméthylaminoéthyle, diéthylaminométhyle, diéthylaminoéthyle, cyclopropyle, cyclopentyle, cyclohexyle, phényle, ou benzyle,
R⁸ et R⁹ représentent indépendamment l'un de l'autre l'hydrogène, -SO₂R⁷, -COR⁷, -CO₂R⁷; un groupe alkyle en C₁-C₁₆, alcényle en C₂-C₁₆, respectivement le cas échéant substitué une fois ou plusieurs fois, de manière identique ou différente, par le fluor, le chlore, le brome, un groupe alkylcarbonyle en C₁-C₄, alkylcarbonyloxy en C₁-C₄, alkylamino en C₁-C₄, di(alkyle en C₁-C₄)amino, alcoxy en C₁-C₄, halogénoalcoxy en C₁-C₄, alkylthio en C₁-C₄, halogénoalkylthio en C₁-C₄; un groupe cycloalkyle en C₃-C₈, cyclopropylméthyle, cyclopentylméthyle, cyclohexylméthyle, cyclopropyléthyle, cyclopentyléthyle, cyclohexyléthyle, phényle, benzyle, phényléthyle, tétrazolyle, furyle, furfuryle, benzofuryle, tétrahydrofuryle, thiényle, thényle, benzothiényle, thiolanyle, pyrrolyle, indolyle, pyrrolinyle, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, morpholinyle, thiomorpholinyle, triazinyle, triazolyle, quinolinyle ou isoquinolinyle, respectivement le cas échéant substitué de une à trois fois, de manière identique ou différente, par le fluor, le chlore, le brome, un groupe cyano, alkyle en C₁-C₄, halogénoalkyle en C₁-C₄, alcoxy en C₁-C₄, halogénoalcoxy en C₁-C₄, alkylthio en C₁-C₄, halogénoalkylthio en C₁-C₄,
R⁸ et R⁹ représentent en outre simultanément un groupe alcénylène en C₂-C₈, le cas échéant substitué une fois ou plusieurs fois, de manière identique ou différente, par le fluor, le chlore, le brome, un groupe alcoxy en C₁-C₄, halogénoalcoxy en C₁-C₄, alkylthio en C₁-C₄, halogénoalkylthio en C₁-C₄; ou un groupe alkylène en C₃-C₈, le cas échéant substitué une fois ou plusieurs fois dans la partie alkylène, de manière identique ou différente, par le fluor, le chlore, le brome, un groupe cyano, méthyle, éthyle, n-propyle, i-propyle, n-butyle, i-butyle, s-butyle, t-butyle, halogénoalkyle en C₁-C₄, méthoxy, éthoxy, n-propoxy, i-propoxy, n-butoxy, i-butoxy, s-butoxy, t-butoxy, halogénoalcoxy en C₁-C₄, méthylthio, éthylthio, n-propylthio, i-propylthio, n-butylthio, i-butylthio, s-butylthio, t-butylthio, halogénoalkylthio en C₁-C₄, la chaîne alkylène pouvant être interrompue par -O-, -S- ou -NR¹⁸-,
R¹⁰ et R¹¹ représentent indépendamment l'un de l'autre l'hydrogène, -SO₂CF₃, un groupe méthyle, éthyle, n-propyle, i-propyle, n-butyle, i-butyle, s-butyle, t-butyle, pentyle, hexyle, -CF₃, -CH₂CF₃, -(CF₂)₃CF₃, méthoxyméthyle, méthoxyéthyle, cyclopropyle, cyclopentyle, cyclohexyle, ou un groupe phényle ou benzyle, respectivement le cas échéant substitué de une à trois fois, de manière identique ou différente, par le fluor, le chlore, le brome, un groupe méthyle, trifluorométhyle, méthoxy, trifluorométhoxy,
R¹⁰ et R¹¹ représentent en outre simultanément -(CH₂)₄-, -(CH₂)₅-, -(CH₂)₆-, -CH₂-CH(CH₃)-CH₂-CH(CH₃)-CH₂-, -(CH₂)₂-O-(CH₂)₂-, -(CH₂)₂-S-(CH₂)₂- ou -(CH₂)₂-N(R¹⁸)-(CH₂)₂-,
R¹² et R¹³ représentent indépendamment l'un de l'autre l'hydrogène, un groupe méthyle, éthyle, n-propyle, i-propyle, n-butyle, i-butyle, s-butyle, t-butyle, n-hexyle, trifluorométhyle, trifluoroéthyle, cyclopropyle, cyclopentyle, cyclohexyle, ou un groupe phényle ou benzyle, respectivement le cas échéant substitué de une à quatre fois, de manière identique ou différente, par le fluor, le chlore, le brome, un groupe méthyle, trifluorométhyle, méthoxy, trifluorométhoxy,
R¹² et R¹³ représentent en outre simultanément -(CH₂)₃-, -(CH₂)₄-, -(CH₂)₅-, -(CH₂)₆-, respectivement le cas échéant substitué de une à quatre fois, de manière identique ou différente, par le fluor, le chlore, le brome, un groupe méthyle, éthyle, méthoxy, éthoxy, méthylthio, éthylthio, trifluorométhyle, trifluorométhoxy, trifluorométhylthio,
R¹⁶ et R¹⁷ représentent indépendamment l'un de l'autre l'hydrogène, un groupe méthyle, éthyle, n-propyle, i-propyle, n-butyle, i-butyle, s-butyle, t-butyle, n-hexyle, trifluorométhyle, trifluoroéthyle, cyclopropyle, cyclopentyle, ou cyclohexyle,
R¹⁶ et R¹⁷ représentent en outre simultanément -(CH₂)₃-, -(CH₂)₄-, -(CH₂)₅-, -(CH₂)₂-O-(CH₂)₂- ou (CH₂)₂-S-(CH₂)₂-,
R¹⁸ représente l'hydrogène, -SO₂R⁷, -COR⁷ ou -CO₂R⁷; un groupe alkyle en C₁-C₁₆, alcényle en C₂-C₆, le cas échéant substitué une fois ou plusieurs fois, de manière identique ou différente, par le fluor, le chlore, le brome, un groupe cyano, méthylamino, éthylamino, di(alkyle en C₁-C₆)amino, alcoxy en C₁-C₄, halogénoalcoxy en C₁-C₄, alkylthio en C₁-C₄, halogénoalkylthio en C₁-C₄; un groupe cycloalkyle en C₃-C₈, cyclopropylméthyle, cyclopentylméthyle, cyclohexylméthyle, cyclopropyléthyle, cyclopentyléthyle, cyclohexyléthyle, phényle, benzyle, phényléthyle, tétrazolyle, furyle, furfuryle, benzofuryle, tétrahydrofuryle, thiényle, thényle, benzothiényle, thiolanyle, pyrrolyle, indolyle, pyrrolinyle, pyrrolidinyle, oxazolyle, benzoxazolyle, isoxazolyle, imidazolyle, pyrazolyle, thiazolyle, benzothiazolyle, thiazolidinyle, pyridinyle, pyrimidinyle, pyridazyle, pyrazinyle, pipéridinyle, morpholinyle, thiomorpholinyle, triazinyle, triazolyle, quinolinyle ou isoquinolinyle, respectivement le cas échéant substitué de une à trois fois, de manière identique ou différente, par le fluor, le chlore, le brome, un groupe cyano, méthyle, éthyle, n-propyle, i-propyle, n-butyle, i-butyle, s-butyle, t-butyle, halogénoalkyle en C₁.C₄, méthoxy, éthoxy, n-propoxy, i-propoxy, n-butoxy, i-butoxy, s-butoxy, t-butoxy, halogénoalcoxy en C₁-C₄, méthylthio, éthylthio, n-propylthio, i-propylthio, n-butylthio, i-butylthio, s-butylthio, t-butylthio, halogénoalkylthio en C₁-C₄,

6. Δ¹-pyrrolines de la formule 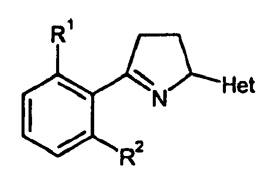 suivant la revendication 1, dans laquelle
R¹ représente le fluor ou le chlore,
R² représente l'hydrogène ou le fluor et
Het a les significations indiquées dans une ou plusieurs des revendications 1 à 5.

7. Composés des formules (I-1) à (I-16) 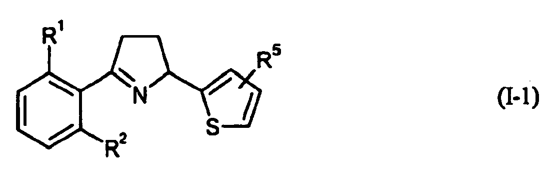 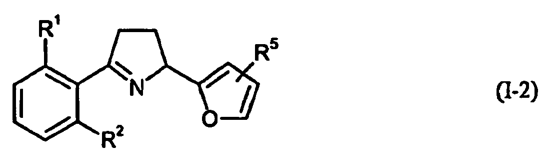 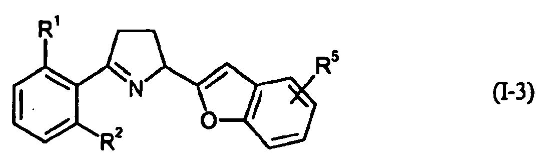 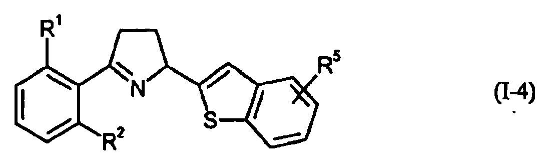 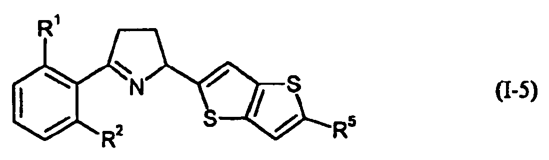 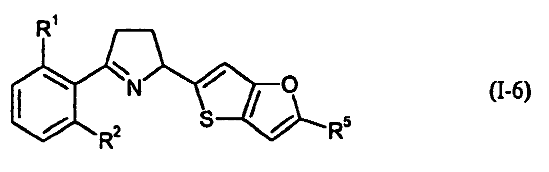 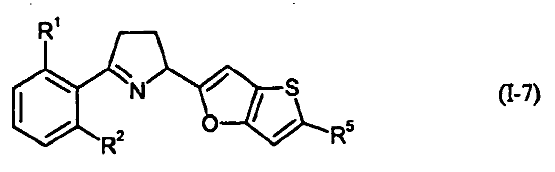 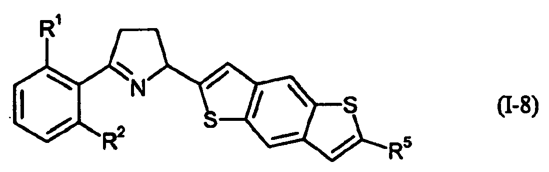 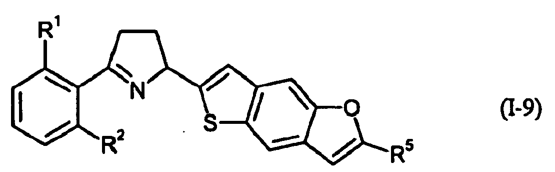 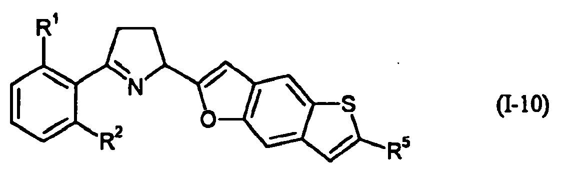 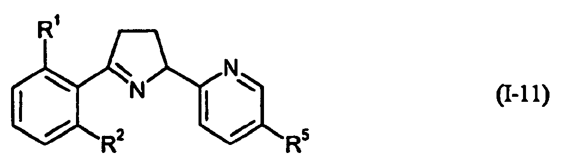 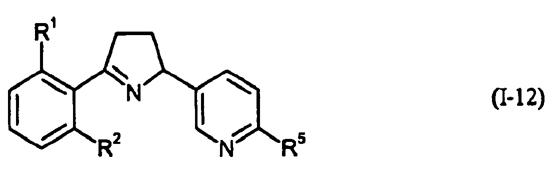 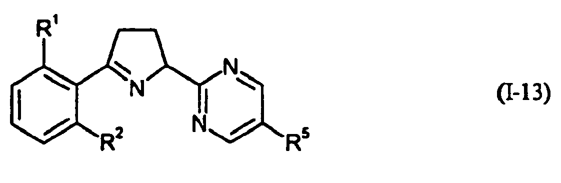 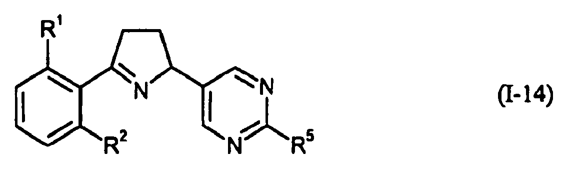 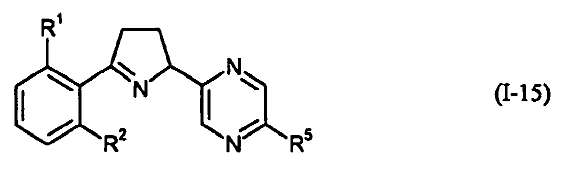 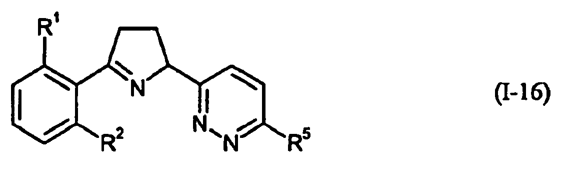 suivant la revendication 1, dans lesquelles
R¹ représente le fluor ou le chlore,
R² représente l'hydrogène ou le fluor et
R⁵ a les significations indiquées dans une ou plusieurs des revendications 1 à 5.

8. Composés des formules (I-d) avec configuration (R) 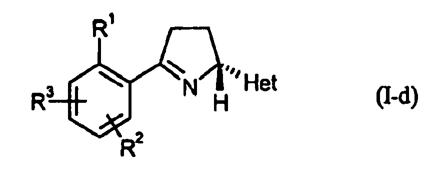 suivant la revendication 1, dans laquelle
R¹, R², R³ et Het ont les significations indiquées dans une ou plusieurs des revendications 1 à 5.

9. Δ¹-pyrrolines de la formule (I) suivant la revendication 1, dans laquelle Het représente un groupe 2-thiényle, 3-thinyle, 2-thiéno[3,2-b]thiényle, 2-pyridinyle ou 3-pyridinyle, respectivement le cas échéant substitué une ou deux fois, de manière identique ou différente, par R⁵.

10. Δ¹-pyrrolines de la formule (I) suivant la revendication 1, dans laquelle R¹ représente le fluor ou le chlore, R² l'hydrogène ou le fluor et R³ l'hydrogène.

11. Δ¹-pyrrolines de la formule (I) suivant la revendication 1, dans laquelle R² représente l'hydrogène ou le fluor, R² prenant la position 6 de l'anneau phényle auquel il est lié.

12. Δ¹-pyrrolines de la formule (I) suivant la revendication 1, dans laquelle X représente une liaison directe et Y un groupe phénylène, de préférence 1,4-phénylène.

13. Δ¹-pyrrolines de la formule (I) suivant la revendication 9, dans laquelle X représente une liaison directe et Y un groupe phénylène, de préférence 1,4-phénylène.

14. Procédé de fabrication de composés de la formule (I) selon la revendication 1, **caractérisé en ce que**
A) on traite des aminocétones de la formule (II) 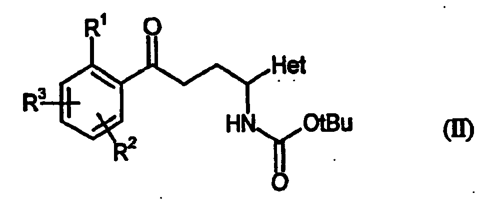 dans laquelle
R¹, R², R³ et Het ont les significations mentionnées dans la revendication 1,
avec un acide de Lewis Lewissäure ou un acide protonique, le cas échéant en présence d'un diluant
ou
B) on fait réagir des azides de la formule (III) 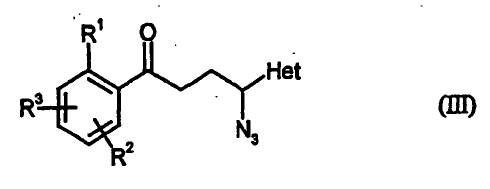 dans laquelle
R¹, R², R³ et Het ont les significations mentionnées dans la revendication 1,
avec une trialkylphosphine ou une triarylphosphine ou un trialkylphosphite ou un agent de réduction en présence d'un diluant et le cas échéant en présence d'un catalyseur,
ou
C) on fait réagir des amides de la formule (IV) 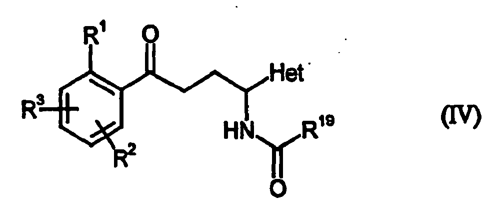 dans laquelle
R¹⁹ représente un groupe alkyle, halogénoalkyle, aryle ou aralkyle et
R¹, R², R³, Het ont les significations données dans la revendication 1,
avec un agent de désacylation azoté, le cas échéant en présence d'un diluant,
ou
qu'on obtient des Δ¹-pyrrolines de la formule (I-a) 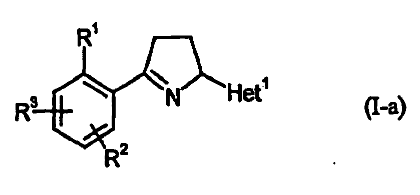 dans laquelle
Het¹ représente un groupe hétéroaryle substitué une fois par R⁵⁻¹,
R⁵⁻¹ représente le groupement -Y¹-E,
Y¹ représente un groupe phénylène ou hétérocyclylène, respectivement le cas échéant substitué et
R¹, R², R³ et E ont les significations mentionnées dans la revendication 1,
en ceci que
D) on fait réagir des Δ¹-pyrrolines de la formule (I-b) 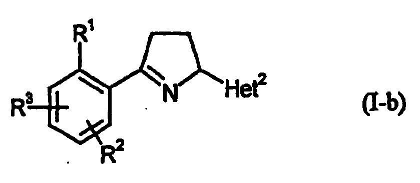 dans laquelle
Het² représente un groupe hétéroaryle substitué une fois par R⁵⁻²,
R⁵⁻² représente le chlore, le brome, l'iode, -OSO₂CF₃ ou -OSO₂(CF₂)₃CF₃,
R¹, R² et R³ ont les significations mentionnées dans la revendication 1,
avec des (hétéro)cycles de la formule (V)
A¹-Y¹-E (V)
dans laquelle
E a les significations données dans la revendication 1,
Y¹ a la signification indiquée ci-dessus,
A¹ représente le chlore, le brome, l'iode, -OSO₂CF₃ ou -OSO₂(CF₂)₃CF₃,
en présence d'un catalyseur, en présence d'un ester de l'acide diboronique et le cas échéant en présence d'un liant pour acide et le cas échéant en présence d'un diluant, dans une réaction tandem,
ou
E) on fait réagir des Δ¹-pyrrolines de la formule (VI) 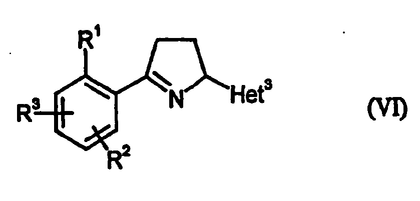 dans laquelle
Het³ représente un groupe hétéroaryle substitué une fois par A²,
A² représente un groupe -B(OH)₂, (4,4,5,5-tétraméthyl-1,3,2-dioxaborolan)-2-yle, (5,5-diméthyl-1,3,2-dioxaborinan)-2-yle, (4,4,6-triméthyl-1,3,2-dioxaborinan)-2-yle ou 1,3,2-benzodioxaborol-2-yle et
R¹, R² et R³ ont les significations données dans la revendication 1,
avec des hétérocycles de la formule (V)
A¹-Y¹-E (V)
dans laquelle
E a les significations données dans la revendication 1,
Y¹ et A¹ ont les significations indiquées ci-dessus,
le cas échéant en présence d'un catalyseur, le cas échéant en présence d'un liant pour acide et le cas échéant en présence d'un diluant,
ou
F) on fait réagir des Δ¹-pyrrolines de la formule (I-b) 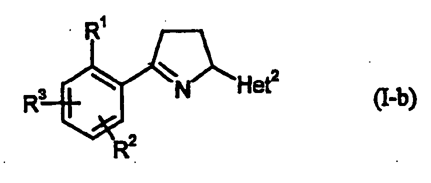 dans laquelle
R¹, R² et R³ ont les significations données dans la revendication 1,
Het² a la signification indiquée ci-dessus,
avec des dérivés de l'acide boronique de la formule (VII)
A²-Y¹-E (VII)
dans laquelle
E a les significations données dans la revendication 1,
Y¹ et A² ont les significations indiquées ci-dessus,
le cas échéant en présence d'un catalyseur, le cas échéant en présence d'un liant pour acide et le cas échéant en présence d'un diluant,
ou
G) on fait réagir des Δ¹-pyrrolines de la formule (I-c)
dans laquelle
Het⁴ représente un groupe hétéroaryle substitué une fois par R⁵⁻³,
R⁵⁻³ représente le brome ou l'iode et
R¹, R² et R³ ont les significations données dans la revendication 1,
avec des composés organométalliques de la formule (VIII)
M-Y¹-E (VIII)
dans laquelle
E a les significations données dans la revendication 1,
Y¹ a la signification indiquée ci-dessus,
M représente ZnCl, Sn(méthyle)₃ ou Sn(n-butyle)₃,
en présence d'un catalyseur, le cas échéant en présence d'un liant pour acide et le cas échéant en présence d'un diluant.

15. Δ¹-pyrrolines de la formule (I-d) 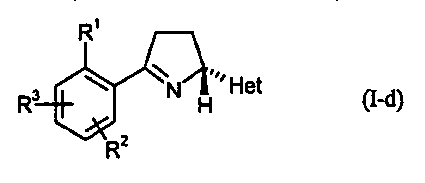 suivant la revendication 1, dans laquelle
R¹, R², R³ et Het ont les significations données dans la revendication 1.

16. Aminocétones de la formule (II) 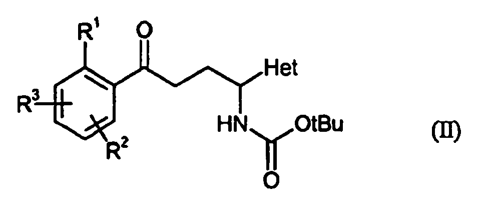 dans laquelle
R¹, R², R³ et Het ont les significations données dans la revendication 1.

17. Azides de la formule (III) 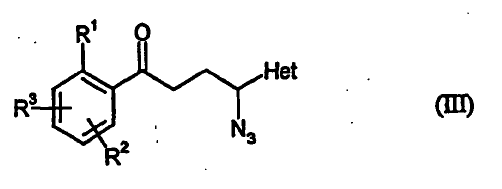 dans laquelle
R², R³ et Het ont les significations données dans la revendication 1.

18. Amides de la formule (IV) 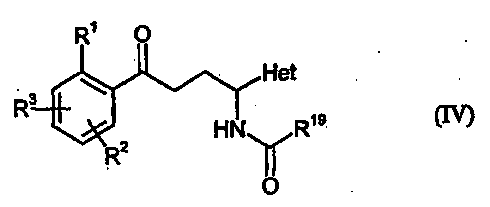 dans laquelle
R¹⁹ représente un groupe alkyle, halogénoalkyle, aryle ou arylakyle et
R¹, R², R³ et Het ont les significations données dans la revendication 1.

19. Lactames de la formule (IX) 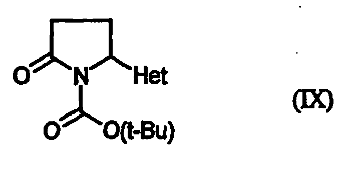 dans laquelle
Het a les significations données dans la revendication 1.

20. Halogénures de la formule (XV) 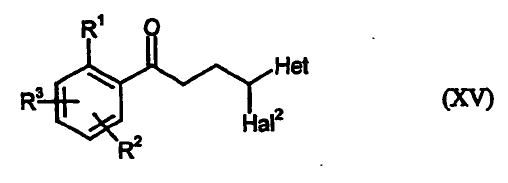 dans laquelle
R¹, R², R³ et Het ont les significations données dans la revendication 1 et
Hal² représente un halogène.

21. Pesticide, **caractérisé par** une teneur en au moins un composé suivant l'une quelconque des revendications 1 à 13 et 15 en plus de produits de coupage et/ou d'agents actifs en surface.

22. Utilisation de composés suivant l'une quelconque des revendications 1 à 13 et 15 pour la lutte contre les parasites, à l'exception du traitement thérapeutique du corps humain ou animal.

23. Procédé de lutte contre les parasites, **caractérisé en ce qu'**on laisse agir des composés suivant l'une quelconque des revendications 1 à 13 et 15 sur les parasites et/ou leur biotope, à l'exception du traitement thérapeutique du corps humain ou animal.

24. Utilisation de composés suivant l'une quelconque des revendications 1 à 13 et 15 pour la préparation d'un médicament à usage vétérinaire à l'encontre de parasites animaux, en particulier les ectoparasites.

25. Procédé de fabrication de pesticides, **caractérisé en ce qu'**on mélange des composés suivant l'une quelconque des revendications 1 à 13 et 15 avec des produits de coupage et/ou des agents actifs en surface.
